# EUROPEAN PATENT APPLICATION

(11) **EP 2 392 649 A2**
(43) Date of publication of application: **07.12.2011**
(21) Application number: 11163622.1
(22) Date of filing: 04.08.2009
(51) Int. Cl.: C12N 9/00, C12N 9/10

(54) **Adipoyl-7-ADCA producing strains**

(30) Priority: 05.08.2008 EP 08161838
(62) Divisional of application: 09781469.3
(71) Applicant: DSM IP Assets B.V., 6411 TE Heerlen (NL)
(72) Inventor: Berg, Van Den, Marco Alexander, 2685 EH, Poeldijk (NL); Bovenberg, Roelof, Ary, Lans, 3062 DA, Rotterdam (NL)
(74) Representative: De Vroom, Erik

(57) **Abstract**

The present invention relates to a mutant microbial strain derived from a parent microbial strain capable of producing an N-adipoylated β-lactam compound when cultured in a medium comprising adipic acid characterized in that the mutant microbial strain has an improved incorporation of the adipic acid from the culture medium into the N-adipoylated β-lactam compound.

## Description

### Field of the invention

The present invention relates to N-adipoylated β-lactam compound producing strains, to a method for their construction as well to the identification and over expression of genes and enzymes involved in the incorporation of adipic acid from the fermentation medium into the to N-adipoylated β-lactam compound.

### Background of the invention

Semi-synthetic β-lactam antibiotics (SSA's) are produced on an industrial scale starting from β-lactam intermediates such as 6-aminopenicillanic acid (6-APA), 7-amino-desacetoxy-cephalosporanic acid (7-ADCA), 7-aminocephalosporanic acid (7-ACA) and 7-amino-3-chloro-3-cephem-4-carboxylate (7-ACCA), 7-amino-3-[(Z/E)-1-propen-1-yl]-3-cephem-4-carboxylate (7-PACA), 7-aminodeacetylcephalosporanic acid (7-ADAC), 7-amino-3-carbamoyloxymethyl-3-cephem-4-carboxylic acid (7-ACCCA) and others.

The first generation 7-ADCA product was derived from PenG whereby both the expansion of the 5-membered penem ring to the 6-membered cephem ring and the subsequent cleavage of the phenylacetic acid side chain of the phenylacetyl-7-ADCA were carried out using chemical reactions. The next generation 7-ADCA product was still obtained from PenG but after the chemical ring expansion, the phenylacetic acid side chain of the phenylacetyl-7-ADCA was cleaved off enzymatically using a suitable (penicillin) acylase. Other processes have been developed wherein also the ring expansion of PenG to phenylacetyl-7-ADCA is carried out in vitro using a suitable expandase enzyme, but these processes are of little industrial importance.

The most recent and most elegant production process for 7-ADCA comprises the culturing of a *Penicillium chrysogenum,* transformed with and expressing a gene encoding a suitable expandase. This engineered *Penicillium chrysogenum* strain, when grown in the presence of adipic acid as the side chain precursor in the fermentation vessel, produces and excretes adipoyl-7-ADCA - see WO93/05158. In this production process, the adipoyl-7-ADCA is recovered from the fermentation broth, subjected to a suitable acylase to cleave off the adipic acid side chain after which the 7-ADCA thus obtained is further purified, crystallized and dried. Other side chains precursors have been disclosed in WO95/04148 (2-(carboxyethylthio)acetic acid and 3-(carboxymethylthio)-propionic acid), WO95/04149 (2-(carboxyethylthio)propionic acid), WO96/38580 (phenyl acetic acid) and WO98/048034 and WO98/048035 (various dicarboxylic acids). The expandase takes care of the expansion of the 5-membered ring of the various N-acylated penicillanic acids, thereby yielding the corresponding N-acylated desacetoxycephalosporanic acids.

However, in addition to being used as side chain precursor, adipate may be degraded and used as a carbon source in the primary metabolism. This was demonstrated by Robin et al. (Appl. Microbiol. Biotechnol (2001) 57, 357-362)) in batch cultures with sucrose as the primary carbon source. After depletion of the glucose and fructose (derived from the sucrose), the formation of adipoyl-6-APA and adipoyl-7-ADCA commenced and in this stage only up to 2% of the adipic acid was incorporated in the β-lactam compounds while the remainder was used as a carbon source. It was suggested by the authors that, due to the similarity between adipic acid and fatty acids, the adipic acid degradation was likely to occur by β-oxidation. In a later study, Thykaer et al. (Metabolic Engineering (2002), 4, 151-158), on the basis of a metabolic network analysis of an adipoyl-7-ADCA producing strain of *Penicillium chrysogenum,* arrived at the conclusion that the adipate degradation takes place in the microbodies (glyoxysomes) by β-oxidation enzymes and not in the cytosol or mitochondria.

The nature of the β-oxidation pathway in (filamentous) fungi and yeast in general, and in *Penicillium chrysogenum* in particular, is still obscure in terms of the intracellular localization, the enzymes involved, as well as the role of the β-oxidation pathway in the total metabolism of the micro-organism. In order to reduce the adipic acid degradation, it has been suggested by Thykaer *et al.* (2002) to delete the enzyme responsible for the adipate degradation, however, without specifying any of such enzymes to be a suitable candidate.

Since adipate is costly compared to glucose or glycerol, adipate degradation is undesirable. Instead, from an industrial production process point of view, an incorporation yield of adipate into N-adipoylated β-lactam compounds close to 100% is very desirable.

Surprisingly we have found that over expression of a gene encoding an adipoyl-CoA forming enzyme in a microbial strain capable of producing an N-adipoylated β-lactam compound result in a mutant microbial strain having an improved incorporation of the adipic acid from the culture medium into the N-adipoylated β-lactam compound.

### Detailed description of the invention

In a first aspect, the invention provides a method for the identification of one or more genes of a microbial strain capable of producing an N-adipoylated β-lactam compound and which encode one or more enzymes which are involved in the incorporation of the adipic acid from the culture medium into the N-adipoylated β-lactam compound by the microbial strain when cultured in a medium comprising adipic acid. The incorporation of the adipic acid from the culture medium into the N-adipoylated β-lactam compound may comprise the following steps:
1. Transport of the adipic acid from the culture medium into the interior of the microbial cell.
2. Conversion of the intracellular adipic acid into adipoyl-CoA by an enzyme such as a CoA-ligase (EC 6.2.1.xx) or a CoA-transferase (EC 2.8.3.xx)
3. Transfer of the adipatoyl-moiety from adipoyl-CoA towards isopenicillin N by the enzyme acyl coenzyme A: isopenicillin N acyltransferase (EC 2.3.1.164) resulting in adipoyl-6-APA.
4. Optionally, the adipoyl-6-APA may be converted into adipoyl-7-ADCA by means of the enzyme expandase (EC 1.14.20.1).

The method for the identification of one ore more genes of the microbial strain encoding one or more enzymes which are involved in the incorporation of the adipic acid from the culture medium into the N-adipoylated β-lactam compound may comprise the following steps:
a. Selection of a nucleotide sequence of one or more known genes and/or the amino acid sequence of one or more known enzymes, optionally encoded by said genes, which are involved in activation of acids into the respective acyl-CoA compounds.
b. Using the selected sequence from step (a) as a probe in a BLAST search for the identification of homologous sequences among available nucleotide or amino acid sequences of the microbial strain capable of producing an N-adipoylated β-lactam compound.

In a preferred embodiment, genes encoding one or more enzymes which are involved in the incorporation of the adipic acid from the culture medium into the N-adipoylated β-lactam compound may be selected from the group consisting of genes encoding enzymes that are capable of catalyzing the conversion of the intracellular adipic acid into adipoyl-CoA such as CoA-ligase and CoA-transferase.

The CoA-ligase (EC 6.2.1.xx) preferably has the capability of synthesizing CoA-derivates of dicarboxylic acids such as oxalic acid, malonic acid, succinic acid, glutaric acid, carboxy-methyl-thio-propionic acid, thio-di-propionic acid, trans-β-hydromuconic acid and/or adipic acid. A highly preferred CoA-ligase (EC 6.2.1.xx) has the capability of synthesizing adipoyl-CoA.

The CoA-transferase (EC 2.8.3.xx) preferably has the capability of synthesizing CoA-derivates of dicarboxylic acids such as oxalic acid, malonic acid, succinic acid, glutaric acid and/or adipic acid. A highly preferred CoA-transferase (EC 2.8.3.xx) has the capability of synthesizing adipoyl-CoA.

In a preferred embodiment, the selected sequence (probe) is a known nucleotide sequence of the gene or a known amino acid sequence of the enzyme optionally encoded by said gene and may be selected from (but is not limited to) the group consisting of the following genes:

| | |
|---|---|
| Probe A. | Acetyl-coenzyme A synthetase (EC 6.2.1.1) from *Saccharomyces cerevisiae* (Entrez Accession No. AAB35143). |
| Probe B. | Phenylacetate-CoA ligase (EC 6.2.1.30) from *Penicillium chrysogenum* (Entrez Accession No. CAA04820) |
| Probe C. | Very long-chain fatty acyl-CoA synthase (EC 6.2.1.3) Saccharomyces cerevisiae (Entrez Accession No. CAA84983) |
| Probe D. | Acyl-CoA synthetase from Aspergillus oryzae (Entrez Accession No. Q2UHE5) |
| Probe E. | Succinyl-CoA:3-ketoacid-coenzyme A transferase subunit A (EC 2.8.3.5) from *Homo sapiens* (Entrez Accession No. BAB40810) |
| Probe F. | Formyl-coenzyme-A transferase (EC 2.8.3.2) from *Escherichia coli* (Entrez Accession No. P69902) |

In a preferred embodiment, the method utilizes as a probe the amino acid sequence of the acetyl-coenzyme A synthetase (EC 6.2.1.1) from *Saccharomyces cerevisiae* (Entrez Accession No. AAB35143) (Probe A) and/or the amino acid sequence of the phenylacetate-CoA ligase (EC 6.2.1.30) from *Penicillium chrysogenum* (Entrez Accession No. CAA04820) (Probe B).

For the purpose of the present invention, the BLAST algorithm is used to identify homologous sequences (Altschul, et al., 1990, J. Mol. Biol. 215: 403-410). Software for performing BLAST analyses is publicly available through the National Center for Biotechnology Information (http://www.ncbi.nlm.nih.gov/). The BLAST algorithm parameters W, T, and X determine the sensitivity and speed of the alignment. The BLAST program uses as default a word length (W) of 11, the BLOSUM62 scoring matrix (see Henikoff & Henikoff, Proc. Natl. Acad. Sci. USA 89:10915 (1989)) alignments (B) of 50, expectation (E) of 10, M=5, N=-4, and a comparison of both strands.

In addition to the method of the invention described above, the genes encoding one or more enzymes which are involved in the incorporation of the adipic acid from the culture medium into the N-adipoylated β-lactam compound, may further be selected on the basis of their ratio of the transcript level of the gene when the parent strain is grown in a medium containing adipic acid with the transcript level of the gene when the parent strain is grown in a control medium without adipic acid (defined herein as "adipate/control" ratio). Preferably, such a gene has an "adipate/control" ratio of more than 1, preferably ≥ 2, preferably ≥ 3, preferably ≥ 4, preferably ≥ 5, preferably ≥ 10, preferably ≥ 15, preferably ≥ 20, preferably ≥ 30, preferably ≥ 40, preferably more ≥ 50, preferably ≥ 60, preferably ≥ 70, preferably ≥ 80, most preferably ≥ 90. As a second control, the ratio of the transcript level of the gene when the parent strain is grown in a medium containing phenyl acetic acid with the transcript level of the gene when the parent strain is grown in a control medium without phenyl acetic acid (designated as "PAA/control") may be determined.

A third ratio may be calculated by dividing the "adipate/control" ratio by the "PAA/control" ratio thus yielding the "adipate/PAA" ratio. Preferably, the gene of the invention has an "adipate/PAA" ratio of ≥ 1, preferably ≥ 2, preferably ≥ 3, preferably ≥ 4, preferably ≥ 5, preferably ≥ 10, preferably ≥ 15, preferably ≥ 20, preferably ≥ 30, preferably ≥ 40, preferably more ≥ 50, preferably ≥ 60, preferably ≥ 70, preferably ≥ 80, most preferably ≥ 90. Preferred genes combine a high "adipate/control" ratio with a low "PAA/control" ratio. For example, a gene with an "adipate/control" ratio of around 50 and a PAA/control ratio of around 10 (i.e. a adipate/PAA of 5) is preferred over a gene with an adipate/control ratio of around 50 and a PAA/control ratio of around 50 (i.e. a adipate/PAA of 1).

In a second aspect, the invention provides a gene encoding a polypeptide which is involved in the incorporation of the adipic acid from the culture medium into the N-adipoylated β-lactam compound. Preferably, genes encoding one or more enzymes which are involved in the incorporation of the adipic acid from the culture medium into the N-adipoylated β-lactam compound may be selected from the group consisting of genes encoding enzymes that are capable of catalyzing the conversion of the intracellular adipic acid into adipoyl-CoA such as CoA-ligase and CoA-transferase as described hereinbefore.

In one embodiment, the genes encoding a polypeptide which is an enzyme having CoA-ligase (EC 6.2.1.xx) activity,
- may have a genomic nucleotide sequence selected from the group consisting of Pc13g14420 (SEQ ID No:1); Pc21g22010 (SEQ ID No:2); Pc22g20270 (SEQ ID No:3); Pc22g00960 (SEQ ID No:4); Pc12g05520 (SEQ ID No:5); Pc13g12270 (SEQ ID No:6); Pc18g05710 (SEQ ID No:7); Pc20g13500 (SEQ ID No:8); Pc13g01890 (SEQ ID No:9); Pc20g10840 (SEQ ID No:10); Pc13g05130 (SEQ ID No:11); Pc13g10810 (SEQ ID No:12); Pc22g06680 (SEQ ID No:13); Pc22g14900 (SEQ ID No:14); Pc22g16410 (SEQ ID No:15); Pc21g13540 (SEQ ID No:16); Pc21g20650 (SEQ ID No:17); Pc21g23730 (SEQ ID No:18); Pc21g21960 (SEQ ID No:19); Pc22g24780 (SEQ ID No:20); Pc06g01160 (SEQ ID No:21); Pc12g09980 (SEQ ID No:22); Pc15g00420 (SEQ ID No:23); Pc21g07810 (SEQ ID No:24) and Pc21g09470 (SEQ ID No 25); or
- may have a corresponding cDNA nucleotide selected from the group consisting of SEQ ID No: 32;SEQ ID No: 33;SEQ ID No: 34;SEQ ID No: 35;SEQ ID No: 36;SEQ ID No: 37;SEQ ID No: 38;SEQ ID No: 39;SEQ ID No: 40; SEQ ID No: 41; SEQ ID No: 42; SEQ ID No: 43; SEQ ID No: 44; SEQ ID No: 45; SEQ ID No: 46; SEQ ID No: 47; SEQ ID No: 48; SEQ ID No: 49; SEQ ID No: 50; SEQ ID No: 51; SEQ ID No: 52; SEQ ID No: 53; SEQ ID No: 54; SEQ ID No: 55; SEQ ID No: 56;
- may encode a corresponding amino acid sequence selected from the group consisting of SEQ ID No: 63; SEQ ID No: 64; SEQ ID No: 65; SEQ ID No: 66; SEQ ID No: 67; SEQ ID No: 68; SEQ ID No: 79; SEQ ID No: 70; SEQ ID No: 71 SEQ ID No: 72; SEQ ID No: 73; SEQ ID No: 74; SEQ ID No: 75; SEQ ID No: 76; SEQ ID No: 77; SEQ ID No: 78; SEQ ID No: 79; SEQ ID No: 80; SEQ ID No: 81; SEQ ID No: 82; SEQ ID No: 83; SEQ ID No: 84; SEQ ID No: 85; SEQ ID No: 86; SEQ ID No: 87;

In a second embodiment, the genes encoding a polypeptide which is an enzyme having CoA-transferase (EC 2.8.3.xx) activity
- may have a genomic nucleotide sequence selected from the group consisting of Pc20g15639 (SEQ ID No: 26); Pc13g02780 (SEQ ID No: 27); Pc22g13680 (SEQ ID No: 28); Pc22g03940 (SEQ ID No: 29); Pc14g00590 (SEQ ID No: 30); Pc16g00210 (SEQ ID No:31).
- may have a corresponding cDNA nucleotide selected from the group consisting of SEQ ID No: 57; SEQ ID No: 58; SEQ ID No: 59; SEQ ID No: 60; SEQ ID No: 61; SEQ ID No: 62;
- may encode a corresponding amino acid sequence selected from the group consisting of SEQ ID No: 88; SEQ ID No: 89; SEQ ID No: 90; SEQ ID No: 91; SEQ ID No: 92; SEQ ID No: 93
Tables 1 and 2 show how the SEQ ID No's of the genomic nucleotide sequences, the cDNA sequences and the amino acid sequences are related to one another.

The nucleotide sequences of the genes of the present invention are not limited to the sequences listed above but also comprise sequences that are "substantially homologous" to said sequences with the proviso that said genes encode an enzyme having CoA-ligase (EC 6.2.1.xx) activity or CoA-transferase (EC 2.8.3.xx) respectively.

The amino acid sequences are not limited to the sequences listed above but also comprise sequences that are "substantially homologous" to said sequences with the proviso that the polypeptide having such an amino acid sequence is having CoA-ligase (EC 6.2.1.xx) activity or CoA-transferase (EC 2.8.3.xx) respectively.

It is defined herein that a sequence (sequence 1) is "substantially homologous" to another sequence (sequence 2) when sequence 1 possesses a degree of identity to sequence 2 of at least 75%, preferably at least 80%, preferably at least 85%, preferably at least 90%, preferably at least 95%, still more preferably at least 96%, still more preferably at least 97%, still more preferably at least 98% and most preferably at least 99%. This definition of "substantially homologous" applies to nucleotide sequences as well as to amino acid sequences.

The invention provides the following genes encoding CoA-ligase (EC 6.2.1.xx):
1. Pc13g14420 with a genomic nucleotide sequence as depicted in SEQ ID No. 1 and the corresponding cDNA sequence as depicted in SEQ ID No. 32 and the corresponding amino acid sequence as depicted in SEQ ID No. 63 as well as sequences that are more than 55% homologues to these sequences, more preferably more than 60%, more preferably more than 70%, more preferably more than 80%, more preferably more than 90%, more preferably more than 95% homologues to these sequences.
2. Pc21g22010 with a genomic nucleotide sequence as depicted in SEQ ID No. 2 and the corresponding cDNA sequence as depicted in SEQ ID No. 33 and the corresponding amino acid sequence as depicted in SEQ ID No. 64 as well as sequences that are more than 45% homologues to these sequences, more preferably more than 50%, more preferably more than 60%, more preferably more than 70%, more preferably more than 80%, more preferably more than 90%, more preferably more than 95% homologues to these sequences.
3. Pc22g20270 with a genomic nucleotide sequence as depicted in SEQ ID No. 3 and the corresponding cDNA sequence as depicted in SEQ ID No. 34 and the corresponding amino acid sequence as depicted in SEQ ID No. 65
4. Pc22g00960 with a genomic nucleotide sequence as depicted in SEQ ID No. 4 and the corresponding cDNA sequence as depicted in SEQ ID No. 35 and the corresponding amino acid sequence as depicted in SEQ ID No. 66 as well as sequences that are more than 70% homologues to these sequences, more preferably more than 80%, more preferably more than 90%, more preferably more than 95% homologues to these sequences.
5. Pc12g05520 with a genomic nucleotide sequence as depicted in SEQ ID No. 5 and the corresponding cDNA sequence as depicted in SEQ ID No. 36 and the corresponding amino acid sequence as depicted in SEQ ID No. 67 as well as sequences that are more than 85% homologues to these sequences, more preferably more than 90%, more preferably more than 95% homologues to these sequences.
6. Pc13g12270 with a genomic nucleotide sequence as depicted in SEQ ID No. 6 and the corresponding cDNA sequence as depicted in SEQ ID No. 37 and the corresponding amino acid sequence as depicted in SEQ ID No. 68 as well as sequences that are more than 80% homologues to these sequences, more preferably more than 90%, more preferably more than 95% homologues to these sequences.
7. Pc18g05710 with a genomic nucleotide sequence as depicted in SEQ ID No. 7 and the corresponding cDNA sequence as depicted in SEQ ID No. 38 and the corresponding amino acid sequence as depicted in SEQ ID No. 69 as well as sequences that are more than 80% homologues to these sequences, more preferably more than 90%, more preferably more than 95% homologues to these sequences.
8. Pc20g13500 with a genomic nucleotide sequence as depicted in SEQ ID No. 8 and the corresponding cDNA sequence as depicted in SEQ ID No. 39 and the corresponding amino acid sequence as depicted in SEQ ID No. 70 as well as sequences that are more than 80% homologues to these sequences, more preferably more than 90%, more preferably more than 95% homologues to these sequences.
9. Pc13g01890 with a genomic nucleotide sequence as depicted in SEQ ID No. 9 and the corresponding cDNA sequence as depicted in SEQ ID No. 40 and the corresponding amino acid sequence as depicted in SEQ ID No. 71 as well as sequences that are more than 75% homologues to these sequences, more preferably more than 80%, more preferably more than 95% homologues to these sequences.
10. Pc20g10840 with a genomic nucleotide sequence as depicted in SEQ iD No. 10 and the corresponding cDNA sequence as depicted in SEQ ID No. 41 and the corresponding amino acid sequence as depicted in SEQ ID No. 72 as well as sequences that are more than 55% homologues to these sequences, more preferably more than 60%, more preferably more than 70%, more preferably more than 80%, more preferably more than 90%, more preferably more than 95% homologues to these sequences.
11. Pc13g05130 with a genomic nucleotide sequence as depicted in SEQ ID No. 11 and the corresponding cDNA sequence as depicted in SEQ ID No. 42 and the corresponding amino acid sequence as depicted in SEQ ID No. 73 as well as sequences that are more than 80% homologues to these sequences, more preferably more than 90%, more preferably more than 95% homologues to these sequences.
12. Pc13g10810 with a genomic nucleotide sequence as depicted in SEQ ID No. 12 and the corresponding cDNA sequence as depicted in SEQ ID No. 43 and the corresponding amino acid sequence as depicted in SEQ ID No. 74 as well as sequences that are more than 80% homologues to these sequences, more preferably more than 90%, more preferably more than 95% homologues to these sequences.
13. Pc22g06680 with a genomic nucleotide sequence as depicted in SEQ ID No. 13 and the corresponding cDNA sequence as depicted in SEQ ID No. 44 and the corresponding amino acid sequence as depicted in SEQ ID No. 75.
14. Pc22g14900 with a genomic nucleotide sequence as depicted in SEQ ID No. 14 and the corresponding cDNA sequence as depicted in SEQ ID No. 45 and the corresponding amino acid sequence as depicted in SEQ ID No. 76.
15. Pc22g16410 with a genomic nucleotide sequence as depicted in SEQ ID No. 15 and the corresponding cDNA sequence as depicted in SEQ ID No. 46 and the corresponding amino acid sequence as depicted in SEQ ID No. 77 as well as sequences that are more than 80% homologues to these sequences, more preferably more than 90%, more preferably more than 95% homologues to these sequences.
16. Pc21g13540 with a genomic nucleotide sequence as depicted in SEQ ID No. 16 and the corresponding cDNA sequence as depicted in SEQ ID No. 47 and the corresponding amino acid sequence as depicted in SEQ ID No. 78 as well as sequences that are more than 80% homologues to these sequences, more preferably more than 90%, more preferably more than 95% homologues to these sequences.
17. Pc21g20650 with a genomic nucleotide sequence as depicted in SEQ ID No. 17 and the corresponding cDNA sequence as depicted in SEQ ID No. 48 and the corresponding amino acid sequence as depicted in SEQ ID No. 79 as well as sequences that are more than 80% homologues to these sequences, more preferably more than 90%, more preferably more than 95% homologues to these sequences.
18. Pc21g23730 with a genomic nucleotide sequence as depicted in SEQ ID No. 18 and the corresponding cDNA sequence as depicted in SEQ ID No. 49 and the corresponding amino acid sequence as depicted in SEQ ID No. 80 as well as sequences that are more than 85% homologues to these sequences, more preferably more than 90%, more preferably more than 95% homologues to these sequences.
19. Pc21g21960 with a genomic nucleotide sequence as depicted in SEQ ID No. 19 and the corresponding cDNA sequence as depicted in SEQ ID No. 50 and the corresponding amino acid sequence as depicted in SEQ ID No. 81 as well as sequences that are more than 80% homologues to these sequences, more preferably more than 90%, more preferably more than 95% homologues to these sequences.
20. Pc22g24780 with a genomic nucleotide sequence as depicted in SEQ ID No. 20 and the corresponding cDNA sequence as depicted in SEQ ID No. 51 and the corresponding amino acid sequence as depicted in SEQ ID No. 82 as well as sequences that are more than 70% homologues to these sequences, more preferably more than 80%, more preferably more than 90%, more preferably more than 95% homologues to these sequences.
21. Pc06g01160 with a genomic nucleotide sequence as depicted in SEQ ID No. 21 and the corresponding cDNA sequence as depicted in SEQ ID No. 52 and the corresponding amino acid sequence as depicted in SEQ ID No. 83 as well as sequences that are more than 65% homologues to these sequences, more preferably more than 70%, more preferably more than 80%, more preferably more than 90%, more preferably more than 95% homologues to these sequences.
22. Pc12g09980 with a genomic nucleotide sequence as depicted in SEQ iD No. 22 and the corresponding cDNA sequence as depicted in SEQ ID No. 53 and the corresponding amino acid sequence as depicted in SEQ ID No. 84 as well as sequences that are more than 45% homologues to these sequences, more preferably more than 50%, more preferably more than 60%, more preferably more than 70%, more preferably more than 80%, more preferably more than 90%, more preferably more than 95% homologues to these sequences.
23. Pc15g00420 with a genomic nucleotide sequence as depicted in SEQ ID No. 23 and the corresponding cDNA sequence as depicted in SEQ ID No. 54 and the corresponding amino acid sequence as depicted in SEQ ID No. 85 as well as sequences that are more than 80% homologues to these sequences, more preferably more than 90%, more preferably more than 95% homologues to these sequences.
24. Pc21g07810 with a genomic nucleotide sequence as depicted in SEQ ID No. 24 and the corresponding cDNA sequence as depicted in SEQ ID No. 55 and the corresponding amino acid sequence as depicted in SEQ ID No. 86 as well as sequences that are more than 70% homologues to these sequences, more preferably more than 80%, more preferably more than 90%, more preferably more than 95% homologues to these sequences.
25. Pc21g09470 with a genomic nucleotide sequence as depicted in SEQ ID No. 25 and the corresponding cDNA sequence as depicted in SEQ ID No. 56 and the corresponding amino acid sequence as depicted in SEQ ID No. 87 as well as sequences that are more than 70% homologues to these sequences, more preferably more than 80%, more preferably more than 90%, more preferably more than 95% homologues to these sequences.
The invention provides the following genes encoding CoA-transferase (EC 2.8.3.xx):
26. Pc20g15630 with a genomic nucleotide sequence as depicted in SEQ ID No. 26 and the corresponding cDNA sequence as depicted in SEQ ID No. 57 and the corresponding amino acid sequence as depicted in SEQ ID No. 88 as well as sequences that are more than 60% homologues to these sequences, more preferably more than 70%, more preferably more than 80%, more preferably more than 90%, more preferably more than 95% homologues to these sequences.
27. Pc13g02780 with a genomic nucleotide sequence as depicted in SEQ ID No. 27 and the corresponding cDNA sequence as depicted in SEQ ID No. 58 and the corresponding amino acid sequence as depicted in SEQ ID No. 89 as well as sequences that are more than 85% homologues to these sequences, more preferably more than 90%, more preferably more than 95% homologues to these sequences.
28. Pc22g13680 with a genomic nucleotide sequence as depicted in SEQ ID No. 28 and the corresponding cDNA sequence as depicted in SEQ ID No. 59 and the corresponding amino acid sequence as depicted in SEQ ID No. 90 as well as sequences that are more than 80% homologues to these sequences, more preferably more than 90%, more preferably more than 95% homologues to these sequences.
29. Pc22g03940 with a genomic nucleotide sequence as depicted in SEQ ID No. 29 and the corresponding cDNA sequence as depicted in SEQ ID No. 60 and the corresponding amino acid sequence as depicted in SEQ ID No. 91 as well as sequences that are more than 95% homologues to these sequences.
30. Pc14g00590 with a genomic nucleotide sequence as depicted in SEQ ID No. 30 and the corresponding cDNA sequence as depicted in SEQ ID No. 61 and the corresponding amino acid sequence as depicted in SEQ ID No. 92 as well as sequences that are more than 80% homologues to these sequences, more preferably more than 90%, more preferably more than 95% homologues to these sequences.
31. Pc16g00210 with a genomic nucleotide sequence as depicted in SEQ ID No. 31 and the corresponding cDNA sequence as depicted in SEQ ID No. 62 and the corresponding amino acid sequence as depicted in SEQ ID No. 93 as well as sequences that are more than 80% homologues to these sequences, more preferably more than 90%, more preferably more than 95% homologues to these sequences.

For the purpose of the present invention, the homology between two amino acid sequences refers to the percentage of amino acids that are identical between the two sequences. The homology is determined using the BLAST algorithm, which is described in Altschul et al. (J. Mol. Biol. 215: 403-410 (1990)). Software for performing BLAST analyses is publicly available through the National Center for Biotechnology Information (http://www.ncbi.nlm.nih.gov). The BLAST algorithm parameters W, T, and X determine the sensitivity and speed of the alignment. The BLAST program uses as defaults a word length (W) of 11, the BLOSUM62 scoring matrix (see Henikoff & Henikoff, Proc. Natl. Acad. Sci. USA 89: 10915 (1989)) alignments (B) of 50, expectation (E) of 10, M=5, N=-4, and a comparison of both strands.

A substantially homologous polypeptide may encompass polymorphisms that may exist in cells from different populations or within a population due to natural allelic or intra-strain variation. A substantially homologous polypeptide may further be derived from a fungus other than the fungus where the specified amino acid and/or DNA sequence originates from, or may be encoded by an artificially designed and synthesized DNA sequence.

For the purpose of the present invention, the homology between two nucleotide sequences refers to the percentage of bases that are identical between the two sequences. DNA sequences related to the specified DNA sequences and obtained by degeneration of the genetic code are also part of the invention. Homologues may also encompass biologically active fragments of the full-length sequence.

Substantially homologous polypeptides may contain only conservative substitutions of one or more amino acids of the specified amino acid sequences or substitutions, insertions or deletions of non-essential amino acids. Accordingly, a non-essential amino acid is a residue that can be altered in one of these sequences without substantially altering the biological function. For example, guidance concerning how to make phenotypically silent amino acid substitutions is provided in Bowie, J.U. et al., (Science 247:1306-1310 (1990)) wherein the authors indicate that there are two main approaches for studying the tolerance of an amino acid sequence to change. The first method relies on the process of evolution, in which mutations are either accepted or rejected by natural selection. The second approach uses genetic engineering to introduce amino acid changes at specific positions of a cloned gene and selects or screens to identify sequences that maintain functionality. As the authors state, these studies have revealed that proteins are surprisingly tolerant of amino acid substitutions. The authors further indicate which changes are likely to be permissive at a certain position of the protein. For example, most buried amino acid residues require non-polar side chains, whereas few features of surface side chains are generally conserved. Other such phenotypically silent substitutions are described in Bowie et al, and the references cited therein.

The term "conservative substitution" is intended to mean that a substitution in which the amino acid residue is replaced with an amino acid residue having a similar side chain. These families are known in the art and include amino acids with basic side chains (e.g. lysine, arginine and histidine), acidic side chains (e.g. aspartic acid, glutamic acid), uncharged polar side chains (e.g., glycine, asparagines, glutamine, serine, threonine, tyrosine, cystein), non-polar side chains (e.g., alanine, valine, leucine, isoleucine, proline, phenylalanine, methionine, tryptophane), branched side chains (e.g., threonine, valine, isoleucine) and aromatic side chains (e.g., tyrosine, phenylalanine tryptophane, histidine).

Variants of the amino acid sequences of the present inventions leading to an improved catalytic function (i.e. conversion of adipate into adipoyl-CoA) may be obtained by modifying the corresponding genes of the present invention. Among such modifications are:
1. Error prone PCR to introduce random mutations, followed by a screening of obtained variants (essentially as described in example 4) and isolating of variants with improved kinetic properties
2. Family shuffling of related variants of the genes encoding the adipoyi-CoA forming enzyme(s), followed by a screening of obtained variants (essentially as described in example 4) and isolating of variants with improved kinetic properties

Variants of the genes of the present invention leading to an increased level of mRNA and/or protein, resulting in more enzyme activity (i.e. conversion of adipate into adipoyl-CoA) may be obtained by modifying the polynucleotide sequences of said genes. Among such modifications are:
1. Improving the codon usage in such a way that the codons are (optimally) adapted to the parent microbial host.
2. Improving the codon pair usage in such a way that the codons are (optimally)adapted to the parent microbial host
3. Addition of stabilizing sequences to the genomic information encoding the adipoyl-CoA forming enzyme(s) resulting in mRNA molecules with an increased half life

Preferred methods to isolate variants with improved catalytic properties or increased levels of mRNA or protein are described in WO03010183 and WO0301311. Preferred methods to optimize the codon usage in parent microbial strains are described in PCT/EP2007/05594. Preferred methods to add stabilizing elements to the genes encoding the adipoyl-CoA forming enzyme(s) are described in WO2005059149.

In a third aspect, the invention provides a mutant microbial strain derived from a parent microbial strain capable of producing an N-adipoylated β-lactam compound when cultured in a medium comprising adipic acid characterized in that the mutant microbial strain has an improved incorporation yield of the adipic acid from the culture medium into the N-adipoylated β-lactam compound compared to the parent microbial strain.

The incorporation yield is defined herein as the molar percentage of adipic acid incorporated in the N-adipoylated β-lactam compound relative to the total molar amount of adipic acid consumed. The total molar amount of adipic acid consumed is equal to the total molar amount of adipic acid added to the fermentation process minus the molar amount of adipic acid remaining after the fermentation process. The improved incorporation yield may be expressed as the relative improvement if the mutant microbial strain compared to the parent microbial strain. For instance, when the parent microbial strain has an incorporation yield of 5% as defined above and the mutant microbial strain of 6%, than the improved incorporation yield of the mutant is 20% (6/5*100 - 100).

Preferably, the mutant strain of the invention has an improved incorporation yield of at least 5%, more preferably at least 7.5%, more preferably at least 10%, more preferably at least 15%, more preferably at least 20%, more preferably at least 30%, more preferably at least 40%, more preferably at least 50%, more preferably at least 60%, more preferably at least 70%, more preferably at least 80%, more preferably at least 90%, more preferably at least 95%, more preferably at least 97%, more preferably at least 98%, more preferably at least 99%, more preferably at least 100%,

The maximal improved incorporation yield that can be obtained is dependent on the actual incorporation yield of the parent microbial strain. When the parent microbial strain has an incorporation yield of 5% and 100% is the theoretical maximal incorporation yield, then the mutant microbial strain may have a maximal improved incorporation yield of 1900% (100/5*100 - 100). Likewise, when the parent microbial strain has already an incorporation yield of 50% and 100% is the theoretical maximal incorporation yield, then the mutant microbial strain may have a maximal improved incorporation yield of 100% (100/50*100 - 100).

In a preferred embodiment, one or more genes of the present invention as summarized hereinbefore and which have been identified by the method of the invention as described hereinbefore and which encode one or more enzymes which are involved in the incorporation of the adipic acid from the culture medium into the N-adipoylated β-lactam compound, preferably CoA-ligase and/or CoA-transferase, is over expressed in the mutant microbial strain of the invention compared to the parent microbial strain in which said gene is not over expressed.

Over expression of a gene is defined herein as the expression of the gene which results in an activity of the enzyme encoded by said gene in the mutant microbial strain being at least 1.5-fold the activity of the enzyme in the parent microbial; preferably the activity of said enzyme is at least 2-fold, more preferably at least 3-fold, more preferably at least 4-fold, more preferably at least 5-fold, even more preferably at least 10-fold and most preferably at least 20-fold the activity of the enzyme in the parent microbial.

The N-adipoylated β-lactam compound produced by the microbial strain may be any N-adipoylated β-lactam wherein the β-lactam moiety is a penem or cephem. Preferred N-adipoylated β-lactam compounds are adipoyl-derivates of the intermediates lister before: 6-aminopenicillanic acid (6-APA), 7-amino-desacetoxy-cephalosporanic acid (7-ADCA), 7-aminocephalosporanic acid (7-ACA) and 7-amino-3-chloro-3-cephem-4-carboxylate (7-ACCA), 7-amino-3-[(Z/E)-1-propen-1-yl]-3-cephem-4-carboxylate (7-PACA), 7-aminodeacetylcephalosporanic acid (7-ADAC), 7-amino-3-carbamoyloxymethyl-3-cephem-4-carboxylic acid (7-ACCCA) and others. Most preferred are N-adipoylated cephalosporins, most preferred is adipoyl-7-ADCA.

The microbial strain capable of producing an N-adipoylated β-lactam compound may be selected from the group consisting of a fungus, bacterium or yeast. Preferably the microbial strain of the present invention is a fungus, more preferably a filamentous fungus. A preferred filamentous fungus may be selected from the group consisting of *Aspergillus, Acremonium, Trichoderma* and *Penicillium.* More preferably the mutant microbial strain of the present invention belongs to the species *Penicillium,* most preferably *Penicillium chrysogenum.* A preferred bacterium may be selected from the groups consisting of *Streptomyces, Nocardia,* or *Flavobacterium.*

In a preferred embodiment, the mutant microbial strain of the present invention capable of producing an N-adipoylated β-lactam compound belongs to the species *Penicillium,* most preferably is *Penicillium chrysogenum,* which has been transformed with a gene encoding an expandase, preferably the *Streptomyces clavuligerus cefE* gene, which enables the strain to produce adipoyl-7-ADCA when cultured in the presence of the precursor adipic acid.

In another embodiment, the mutant microbial strain of the present invention capable of producing an N-adipoylated β-lactam compound belongs to the species *Penicillium,* most preferably is *Penicillium chrysogenum,* and in addition to an expandase gene, preferably the *Streptomyces clavuligerus cefE* gene, has been transformed with a hydroxylase gene, preferably the *Streptomyces clavuligerus cefF* gene, whose expression product converts the 3-methyl side chain of adipoyl-7-ADCA to 3-hydroxymethyl, to give adipoyl-7-aminodeacetylcephalosporanic acid (adipoyl-7-ADAC).

In another embodiment, the mutant microbial strain of the present invention capable of producing an N-adipoylated β-lactam compound belongs to the species *Penicillium,* most preferably is *Penicillium chrysogenum,* has been transformed with a expandase/hydroxylase gene, preferably the *Acremonium chrysogenum cefEF* gene, whose expression product converts the 3-methyl side chain of adipoyl-7-ADCA to 3-hydroxymethyl, to give adipoyl-7-aminodeacetylcephalosporanic acid (adipoyl-7-ADAC).

In another embodiment the mutant microbial strain of the present invention capable of producing an N-adipoylated β-lactam compound belongs to the species *Penicillium,* most preferably is *Penicillium chrysogenum,* and in addition to the genes encoding expandase, preferably the *Streptomyces clavuligerus cefE* gene, and hydroxylase, preferably the *Streptomyces clavuligerus cefF* gene, is further transformed with an acetyltransferase gene, preferably the *Streptomyces clavuligerus cefG* gene, whose expression product (i.e. the acyltransferase) converts the 3-hydroxymethyl side chain to the 3-acetyloxymethyl side chain to give adipoyl-7-ACA.

In a further embodiment the mutant microbial strain of the present invention capable of producing an N-adipoylated β-lactam compound belongs to the species *Penicillium,* most preferably is *Penicillium chrysogenum* and has been transformed with genes encoding an expandase, preferably the *Streptomyces clavuligerus cefE* gene, a hydroxylase, preferably the *Streptomyces clavuligerus cefF* gene, and an O-carbamoyl transferase enzyme, preferably the *Streptomyces clavuligerus cmcH* gene, resulting in adipoyl-7-amino-3-carbamoyloxymethyl-3-cephem-4-carboxylic acid.

In another embodiment the mutant microbial strain of the present invention belongs to the species *Penicillium,* most preferably *Penicillium chrysogenum,* optionally transformed with one or more of the above mentioned genes encoding an expandase, a hydroxylase, an expandase/hydroxylase, an acetyltransferase and/or an O-carbamoyl transferase, and in addition has deletions in one or more genes encoding enzymes involved in the consumption of adipic acid. Preferred examples of such modifications are (but not limited to) β-oxidation encoding enzymes. The respective enzymes involved may be a CoA ligase (EC 6.2.1.xx), an acyl-CoA dehydrogenase (EC 1.3.99.xx), an acyl-CoA oxidase (EC 1.3.3.xx), an enoyl-CoA hydratase (EC 4.2.1.17), a 3-Hydroxyacyl-CoA dehydrogenase (EC 1.1.1.35) or an acetyl-CoA C-acyltransferase (EC 2.3.1.16), respectively.

In yet another embodiment, the mutant microbial strain of the present invention belongs to the species *Penicillium,* most preferably *Penicillium chrysogenum,* optionally transformed with one or more of the above mentioned genes encoding an expandase, a hydroxylase, an expandase/hydroxylase, an acetyltransferase, an O-carbamoyl transferase and/or contains deletions in genes encoding enzymes involved in the consumption of adipic acid and in addition is also transformed with genes encoding enzymes capable of increasing the secretion of N-adipoylated β-lactam in to the medium. Preferred examples of such genes are (but not limited to): the Acremonium chrysogenum *cefT* gene or homologs thereof and the Acremonium chrysogenum *cefM* gene or homologs thereof.

A highly preferred embodiment of the mutant microbial strain of the present invention has the following characteristics:
1) It belongs to the species *Penicillium,* most preferably *Penicillium chrysogenum,* and,
2) optionally has been transformed with genes encoding:
   a. an expandase, preferably the *Streptomyces clavuligerus cefE* gene,
   b. a hydroxylase, preferably the *Streptomyces clavuligerus cefF* gene,
   c. an expandase/hydroxylase, preferably the *Acremonium chrysogenum cefEF* gene,
   d. an acetyltransferase gene, , preferably the *Streptomyces clavuligerus cefG* gene, and/or
   e. an O-carbamoyl transferase, preferably the *Streptomyces clavuligerus cmcH* gene.
   f. an N-adipoyl β-lactam transporter, preferably the *Acremonium chrysogenum* cefT gene,
   including all the optimized variants (via methods as outlined above) that can be obtained from these examples.
3. Optionally, genes encoding adipate degrading enzymes or adipate consuming enzymes are also modified in such a way that the degradation or consumption is lowered as compared to the parent microbial strain.

In a fourth aspect, the invention provides a method for the construction of the mutant microbial strains of the invention. Over expression of the gene of the invention in the mutant microbial strain of the invention is obtained with the use of nucleic acid constructs, e.g. expression constructs, which contain one or more of the selected genes, each operably linked to one or more control sequences, which direct the expression of the encoded polypeptide in a suitable expression host. The nucleic acid constructs may be on one DNA fragment, or, preferably, on separate fragments. Expression will be understood to include any step involved in the production of the polypeptide and may include transcription, post-transcriptional modification, translation, post-translational modification, and secretion. The term nucleic acid construct is synonymous with the term expression vector or cassette when the nucleic acid construct contains all the control sequences required for expression of a coding sequence in a particular host organism. The term "control sequences" is defined herein to include all components, which are necessary or advantageous for the expression of a polypeptide. Each control sequence may be native or foreign to the nucleic acid sequence encoding the polypeptide. Such control sequences may include, but are not limited to, a promoter, a leader, optimal translation initiation sequences (as described in Kozak, 1991, J. Biol. Chem. 266:19867-19870), a secretion signal sequence, a pro-peptide sequence, a polyadenylation sequence, a transcription terminator. At a minimum, the control sequences include a promoter, and transcriptional and translational stop signals. The term "operably linked" is defined herein as a configuration in which a control sequence is appropriately placed at a position relative to the coding sequence of the DNA sequence such that the control sequence directs the production of a polypeptide.

The control sequence may include an appropriate promoter sequence containing transcriptional control sequences. The promoter may be any nucleic acid sequence, which shows transcription regulatory activity in the cell including mutant, truncated, and hybrid promoters, and may be obtained from genes encoding extra cellular or intracellular polypeptides. The promoter may be either homologous or heterologous to the cell or to the polypeptide.

Preferred promoters for filamentous fungal cells are known in the art and can be, for example, the glucose-6-phosphate dehyrogenase gpdA promoters, protease promoters such as *pep*A, *pep*B, *pep*C, the glucoamylase *gla*A promoters, amylase *amy*A, *amy*B promoters, the catalase *cat*R or catA promoters, glucose oxidase *gox*C promoter, beta-galactosidase *lac*A promoter, alpha-glucosidase *agl*A promoter, translation elongation factor *tef*A promoter, xylanase promoters such as *xln*A, *xln*B, *xln*C, *xln*D, cellulase promoters such as *egl*A, *egl*B, *cbh*A, promoters of transcriptional regulators such as *are*A, *cre*A, *xln*R, *pac*C, *prt*T, etc or any other, and can be found among others at the NCBI website (http://www.ncbi.nlm.nih.gov/entrez/).

In a preferred embodiment, the promoter may be derived from a gene, which is highly expressed (defined herein as the mRNA concentration with at least 0.5% (w/w) of the total cellular mRNA). In another preferred embodiment, the promoter may be derived from a gene, which is medium expressed (defined herein as the mRNA concentration with at least 0.01% until 0.5% (w/w) of the total cellular mRNA). In another preferred embodiment, the promoter may be derived from a gene, which is low expressed (defined herein as the mRNA concentration lower than 0.01% (w/w) of the total cellular mRNA).

In an even more preferred embodiment, Micro Array data is used to select genes, and thus promoters of those genes, that have a certain transcriptional level and regulation. In this way one can adapt the gene expression cassettes optimally to the conditions it should function in. Amongst the suitable promoters to be selected via this method are promoters of genes that have a very high "adipate/control" ratio as explained above. Also, strong and/or constitutive promoters as isolated and/or disclosed by WO2007071399 can be considered to be suitable promoters.

Alternatively, one could clone random DNA fragments in front of the polynucleotides of this invention. Using an acetamide plate assay as disclosed by WO2007118836_one can easily screen for active promoters, as these should facilitate growth on acetamide as the sole nitrogen source. These DNA fragments can be derived from many sources, i.e. different species, PCR amplified, synthetically and the like.

The control sequence may also include a suitable transcription terminator sequence, a sequence recognized by a filamentous fungal cell to terminate transcription. The terminator sequence is operably linked to the 3' terminus of the nucleic acid sequence encoding the polypeptide. Any terminator, which is functional in the cell, may be used in the present invention. Preferred terminators for filamentous fungal cells are obtained from the genes encoding *Aspergillus oryzae* TAKA amylase, *Aspergillus niger* glucoamylase, *Aspergillus nidulans* anthranilate synthase, *Aspergillus niger* alpha-glucosidase, trpC gene and *Fusarium oxysporum* trypsin-like protease.

The control sequence may also include a suitable leader sequence, a non-translated region of an mRNA, which is important for translation by the filamentous fungal cell. The leader sequence is operably linked to the 5' terminus of the nucleic acid sequence encoding the polypeptide. Any leader sequence, which is functional in the cell, may be used in the present invention. Preferred leaders for filamentous fungal cells are obtained from the genes encoding *Aspergillus oryzae* TAKA amylase and *Aspergillus nidulans* triose phosphate isomerase and *Aspergillus niger* glaA. Other preferred initiator sequences are isolated and/or disclosed by WO2006077258.

The control sequence may also include a polyadenylation sequence, a sequence which is operably linked to the 3' terminus of the nucleic acid sequence and which, when transcribed, is recognized by the filamentous fungal cell as a signal to add polyadenosine residues to transcribed mRNA. Any polyadenylation sequence, which is functional in the cell, may be used in the present invention. Preferred polyadenylation sequences for filamentous fungal cells are obtained from the genes encoding *Aspergillus oryzae* TAKA amylase, *Aspergillus niger* glucoamylase, *Aspergillus nidulans* anthranilate synthase, *Fusarium oxysporum* trypsin-like protease and *Aspergillus niger* alpha-glucosidase.

The nucleic acid construct may be an expression vector. The expression vector may be any vector (e.g. a plasmid or virus), which can be conveniently subjected to recombinant DNA procedures and can bring about the expression of the nucleic acid sequence encoding the polypeptide. The choice of the vector will typically depend on the compatibility of the vector with the cell into which the vector is to be introduced. The vectors may be linear or closed circular plasmids.

The vector may be an autonomously replicating vector, i.e. a vector, which exists as an extrachromosomal entity, the replication of which is independent of chromosomal replication, e.g. a plasmid, an extrachromosomal element, a minichromosome, or an artificial chromosome. An autonomously maintained cloning vector for a filamentous fungus may comprise the AMA1-sequence (see e.g. Aleksenko and Clutterbuck (1997), Fungal Genet. Biol. 21: 373-397).

Alternatively, the vector may be one which, when introduced into the cell, is integrated into the genome and replicated together with the chromosome (s) into which it has been integrated. The integrative cloning vector may integrate at random or at a predetermined target locus in the chromosomes of the host cell. In a preferred embodiment of the invention, the integrative cloning vector comprises a DNA fragment, which is homologous to a DNA sequence in a predetermined target locus in the genome of host cell for targeting the integration of the cloning vector to this predetermined locus. In order to promote targeted integration, the cloning vector is preferably linearized prior to transformation of the host cell. Linearization is preferably performed such that at least one but preferably either end of the cloning vector is flanked by sequences homologous to the target locus. The length of the homologous sequences flanking the target locus is preferably at least at least 0.1 kb, even preferably at least 0.2kb, more preferably at least 0.5 kb, even more preferably at least 1 kb, most preferably at least 2 kb. Preferably, the parent microbial strains are modified for improved frequency of targeted DNA integration as described by WO05095624 and/or WO2007115886.

The vector system may be a single vector or plasmid or two or more vectors or plasmids, which together contain the total DNA to be introduced into the genome of the host cell.

The DNA constructs may be used on an episomal vector. However in the present invention the constructs are preferably integrated in the genome of the host strain.

In a fifth aspect the invention provides the use of the genes of the invention for the construction of the mutant microbial strain of the invention capable of producing an N-adipoylated β-lactam compound when cultured in a medium comprising adipic acid whereby the mutant microbial strain has an improved incorporation yield of the adipic acid from the culture medium into the N-adipoylated β-lactam compound compared to the parent microbial strain.

In one embodiment, the mutant microbial strain may contain more than one of the genes of the present invention, all being overexpressed and the combination leading to an improved incorporation yield of the adipic acid from the culture medium into the N-adipoylated β-lactam compound compared to the parent microbial strain.

In another embodiment, the mutant microbial strain of the present invention contains one or more of the genes of the present invention encoding enzymes that are capable of catalyzing the conversion of the intracellular adipic acid into adipoyl-CoA such as CoA-ligase and CoA-transferase as described hereinbefore, as well as functionally inactivated genes encoding enzymes involved in one or more degradation pathways of adipic acid. The combination of functionally inactivated genes involved in the degradation of adipic acid and the overexpression of genes involved in the incorporation of adipic acid into the N-adipoylated β-lactam compound in one single microbial strain, leads to an even further improved incorporation yield of the adipic acid from the culture medium into the N-adipoylated β-lactam compound compared to the parent microbial strain. "Functionally inactive" is defined herein as the inactivation of a gene which results in a residual activity of the encoded enzyme of preferably less than 50%, more preferably less than 40%, more preferably less than 30%, more preferably less than 20%, more preferably less than 10%, more preferably less than 5%, more preferably less than 2%. "Inactivation of a gene" is defined herein as the modification of a gene in order to obtain a functionally inactive gene as defined hereinbefore. Methods for the modification of a gene in order to obtain a functionally inactive gene are known in the art and may include: inactivation of the gene by base pair mutation resulting in a(n early) stop or frame shift; mutation of one or more codons which encode one or more a critical amino acids (such as the catalytic triad for hydrolases); mutations in the gene resulting in mutations in the amino acid sequence of the enzyme which lead to a decreased half-life of the enzyme; modifying the mRNA molecule in such away that the mRNA half-life is decreased; insertion of a second sequence (i.e. a selection marker gene) disturbing the open reading frame; a partial or complete removal of the gene; removal/mutation of the promoter of the gene; using anti-sense DNA or comparable RNA inhibition methods to lower the effective amount of mRNA in the cell. Most preferably the gene has been made functionally inactive by deletion resulting in a total absence of the encoded polypeptide and hence enzyme activity.

In a sixth aspect, the invention provides a process for the production of an N-adipoylated β-lactam compound by culturing the mutant microbial strain of the invention whereby the process for the production of the N-adipoylated β-lactam compound has an improved incorporation yield of the adipic acid from the culture medium into the N-adipoylated β-lactam compound compared to the process for the production of an N-adipoylated β-lactam using the parent microbial strain.

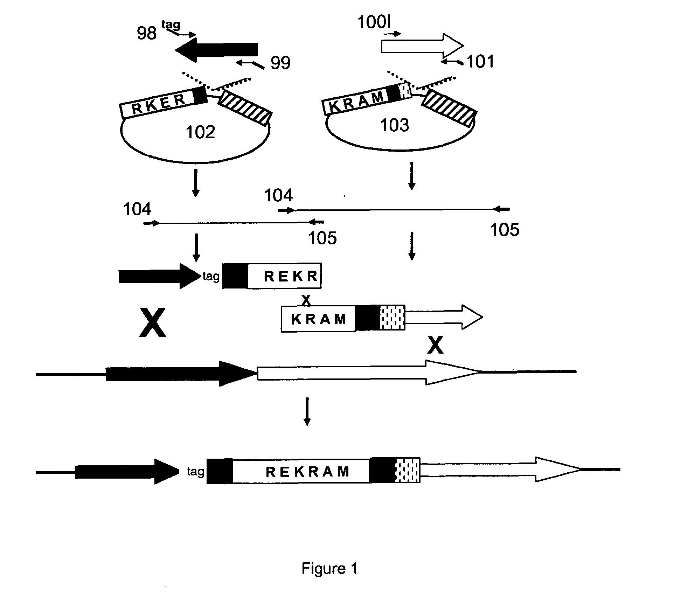

Figure 1 is a representation of the steps involved in deleting the *Penicillium chrysogenum* gene Pc22g20270. Legend:
- solid arrow, Pc22g20270 promoter;
- open arrow, Pc22g20270 ORF;
- hatched box, trpC terminator;
- dashed box, ccdA gene;
- solid box, lox site;
- crosses, recombination event;
- downwards arrows, subsequent steps in the procedure; REKR and KRAM, overlapping non-functional amdS selection marker fragments; REKRAM, functional amdS selection marker gene.
Numbers indicate the SEQ ID NO.'s of the oligonucleotides "tag" indicates the presence of a specific nucleotide sequence which can be used for mutant identification.

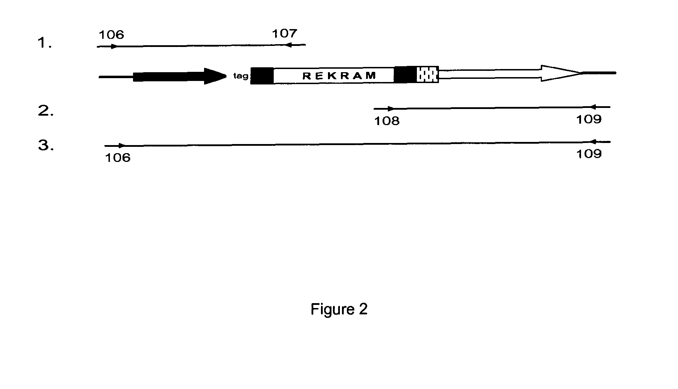

Figure 2 is a representation of the steps involved in confirming the actual deletion of the *Penicillium chrysogenum* gene Pc22g20270. Legend: solid arrow, Pc22g20270 promoter; open arrow, Pc22g20270 ORF; hatched box, *trpC* terminator; solid box, lox site; REKRAM, functional *amdS* selection marker gene. Numbers indicate the SEQ ID NO.'s of the oligonucleotides for the three PCR reactions indicated (see also table 4).

### General materials and methods

BLAST algorithms are used to identify homologous sequences (Altschul, et al., 1990, J. Mol. Biol. 215: 403-410). Software for performing BLAST analyses is publicly available through the National Center for Biotechnology Information (http://www.ncbi.nlm.nih.gov/). The BLAST algorithm parameters W, T, and X determine the sensitivity and speed of the alignment. The BLAST program uses as defaults a word length (W) of 11, the BLOSUM62 scoring matrix (see Henikoff & Henikoff, Proc. Natl. Acad. Sci. USA 89: 10915 (1989)) alignments (B) of 50, expectation (E) of 10, M=5, N=-4, and a comparison of both strands.

Standard procedures were carried out as described elsewhere (Sambrook, J. et al. (1989), Molecular cloning: a laboratory manual, 2nd Ed., Cold Spring Harbor Laboratory Press, Cold Spring Harbor, New York). DNA was amplified using the proofreading polymerases Turbo-Pfu-Polymerase (Stratagene, The Netherlands) or Phusion (Finnzymes), following the manufacturers protocol, while the verification of constructed strains and plasmids was achieved by using Taq polymerase. Restriction enzymes were from Invitrogen or New England Biolabs. For routine cloning, *Escherichia coli* strains Top10 and DH10B (Invitrogen) were employed. Verification of the constructed plasmids was carried out by restriction analysis and subsequent sequencing.

### EXAMPLES

### Example 1

### Identification of Penicillium chrysogenum genes encoding putative adipoyl-CoA-synthesizing enzymes

### 1. CoA ligase (EC 6.2.1.xx)

Using probe A, probe B, probe C and probe D (see text), the genome of *Penicillium chrysogenum* strain Wisconsin54-1255 was searched and subsequent annotation revealed 25 genes encoding a putative CoA-ligase activity (EC 6.2.1.xx); see table 1.

### 2. CoA-transferase (EC 2.8.3.xx)

Using probe E and probe F (see text), the genome of *Penicillium chrysogenum* strain Wiscons54-1255, was searched and revealed 6 genes encoding a putative CoA-transferase (EC 2.8.3.xx); see table 2.

### Example 2

### MicroArray analyses of Penicillium chrysogenum genes encoding putative CoA ligases (EC 6.2.1.xx) and/or CoA transferases (EC 2.8.3.xx)

To identify which of the 31 identified putative adipoyl-CoA synthesizing enzymes, in particular CoA ligases (EC 6.2.1) and/or CoA transferases (EC 2.8.3.xx), are suitable candidates for over expression in order to increase the synthesis of the N-adipoylated β-lactam compound, a MicroArray study was performed. The *P. chrysogenum* genome sequence was used to prepare a proprietary DNA microarray, using the Affymetrix Custom GeneChip program (Affymetrix, Inc., Santa Clara, CA): GeneChip, DSM_PENa520255F.

*P. chrysogenum* strains with and without the *Streptomyces clavuligerus cefE* gene encoding expandase, were inoculated in 100 ml shake flasks with 25 ml of β-lactam production medium (as described in US20020039758), with either 10 g/I adipate, 3 g/l phenylacetic acid (PAA) or no precursor (control). The cultures were incubated at 25 °C with 280 rotations per minute. After 90 hours the total broth was sampled and rapidly cooled. The cells were washed and the cell pellet was frozen in liquid nitrogen. The cells were subsequently disrupted by grinding the frozen pellet with a pestle and mortar, and RNA was isolated using Trizol. The quality and amount of total RNA was routinely checked on a Bioanalyzer (Agilent). Twenty microgram of total RNA was used for a standard cDNA synthesis and labelling reaction (according to Affymetrix instructions). Hybridisation of the GeneChips^{®} was performed according to the suppliers instructions (Affymetrix, Santa Clara, USA) Hybridized arrays were scanned and analyzed using the Affymetrix GeneChip^{®}Operating Software (GCOS, Affymetrix, Santa Clara, USA). All experiments were done in triplicate and the average of the three measurements was taken as the relative level of transcript.

Genes specifically induced by adipate were identified by determining the ratios between the transcript level on adipate-containing medium and the transcript level on the PAA-containing or control medium. The PAA-containing medium was used to identify a-specific responses by looking at the ratios between the transcript level on control medium and the transcript level on the PAA-containing medium. In Tables 1 and 2 the results are shown.

**Table 1 Transcript levels of Penicillium chrysogenum genes encoding an enzyme with a putative CoA ligase activity (EC 6.2.1.xx).**

| GeneID | SEQ ID No | | | Homology to (Probe) | Ratio | | |
|---|---|---|---|---|---|---|---|
| | gDNA | CDNA | Amino acid | % | adipate/PAA | PAA/control | adipate/control |
| Pc13q14420 | 1 | 32 | 63 | 52 (D) | 5.3 | 2.8 | 15.1 |
| Pc21g22010 | 2 | 33 | 64 | 52 (B) | 4.3 | 0.2 | 0.8 |
| Pc22g20270 | 3 | 34 | 65 | 55 (B) | 3.3 | 1.9 | 6.4 |
| Pc22g00960 | 4 | 35 | 66 | 41 (A) | 3.2 | 1.8 | 5.9 |
| Pc12g05520 | 5 | 36 | 67 | 43 (B) | 2.8 | 1.4 | 4.0 |
| Pc13g12270 | 6 | 37 | 68 | 53 (B) | 2.1 | 3.9 | 8.3 |
| Pc18g05710 | 7 | 38 | 69 | 53 (C) | 1.7 | 1.6 | 2.6 |
| Pc20g13500 | 8 | 39 | 70 | 54 (C) | 1.7 | 6.7 | 11.6 |
| Pc13g01890 | 9 | 40 | 71 | 41 (B) | 1.6 | 2.1 | 3.4 |
| Pc20g10840 | 10 | 41 | 72 | 40 (C) | 1.6 | 0.4 | 0.6 |
| Pc13g05130 | 11 | 42 | 73 | 51 (A) | 1.5 | 1.7 | 2.6 |
| Pc13g10810 | 12 | 43 | 74 | 42 (A) | 1.2 | 2.1 | 2.5 |
| Pc22g06680 | 13 | 44 | 75 | 75 (A) | 1.2 | 0.8 | 0.9 |
| Pc22g14900 | 14 | 45 | 76 | 100 (B) | 1.1 | 2.0 | 2.1 |
| Pc22g16410 | 15 | 46 | 77 | 43 (B) | 1.1 | 4.7 | 5.3 |
| Pc21g13540 | 16 | 47 | 78 | 59 (D) | 1.0 | 1.4 | 1.4 |
| Pc21g20650 | 17 | 48 | 79 | 51 (B) | 1.0 | 2.1 | 3 |
| Pc21g23730 | 18 | 49 | 80 | 41 (B) | 0.8 | 2.6 | 2.1 |
| Pc21g21960 | 19 | 50 | 81 | 86 (D) | 0.7 | 1.5 | 1.1 |
| Pc22g24780 | 20 | 51 | 82 | 52 (B) | 0.3 | 22.1 | 6.4 |
| Pc06g01160 | 21 | 52 | 83 | 50 (B) | -* | - | - |
| Pc12g09980 | 22 | 53 | 84 | 42 (B) | - | - | - |
| Pc15g00420 | 23 | 54 | 85 | 52 (C) | - | - | - |
| Pc21g07810 | 24 | 55 | 86 | 46 (B) | - | - | - |
| Pc21q09470 | 25 | 56 | 87 | 41 (B) | - | - | - |

| | | | | | | | |
|---|---|---|---|---|---|---|---|
| * - = no detectable transcripts under the conditions studied | | | | | | | |

**Table 2 Transcript levels of Penicillium chrysogenum genes encoding an enzyme with a putative CoA transferase activity (EC 2.8.3.xx).**

| GeneID | SEQ ID No | | | Homology to (Probe) | Ratio | | |
|---|---|---|---|---|---|---|---|
| | gDNA | CDNA | Amino acid | % | adipate/PAA | PAA/control | adipate/control |
| Pc20g15630 | 26 | 57 | 88 | 50 (F) | 24.9 | 2.8 | 70.5 |
| Pc13g02780 | 27 | 58 | 89 | 47 (F) | 12.0 | 1.1 | 12.6 |
| Pc22g13680 | 28 | 59 | 90 | 44 (F) | 5.8 | 4.3 | 5.8 |
| Pc22g03940 | 29 | 60 | 91 | 68 (E) | 1.2. | 4.4 | 5.5 |
| Pc14g00590 | 30 | 61 | 92 | 63 (E) | 0.9 | 2.7 | 2.4 |
| Pc16g00210 | 31 | 62 | 93 | 45 (F) | 0.6 | 2.0 | 1.1 |

### Example 3

### Overexpression of a Penicillium chrysogenum gene encoding a putative CoA-ligase leads to increased adipoyl-CoA ligase activity

### Construction of expression vector

The *Penicillium chrysogenum* Pc22g14900 gene encoding a putative CoA-ligase with an amino acid sequence identical to probe B (Probe B = Phenylacetate-CoA ligase (EC 6.2.1.30) from Penicillium chrysogenum (Entrez Accession No. CAA04820)) was PCR amplified using the oligonucleotides of SEQ ID NO 94 and 95, introducing *Nde*I and *Nsi*I sites upstream and downstream of the gene for cloning. Digestion of the amplified fragment with *Ndel* and *Nsil* enabled to clone Pc22g14900 into the plasmid pISEWAn (as described in WO04106347) pre-digested with the same restriction enzymes resulting in the plasmid piPCLA. Therefore, the expression of Pc22g1400 was controlled by the *pcbC* (encoding IPN synthase from *P. chrysogenum*) promoter and the *penDE* (encoding acyltransferase of *P. chrysogenum*) terminator.

### Transformation to P. chrysogenum

The Pc22g14900 expression cassette was obtained from the pIPCLA plasmid after digestion with *Not*I and the isolated fragment was transfected by co-transformation with the *amdS* selection marker in parent microbial hosts (i.e. *P. chrysogenum* strains expressing the expandase gene, *cefE*). The integration of the *amdS* marker enables the *P. chrysogenum* transformants to grow on selection medium containing acetamide as the sole nitrogen source. The *amdS* fragment was isolated after digestion with *Hind*III from plasmid pHELY-A1 (as described in WO04106347).

Techniques involved in the transfer of DNA to protoplasts of *P. chrysogenum* are well known in the art and are described in many references, including Finkelstein and Ball (eds.), Biotechnology of filamentous fungi, technology and products, Butterworth-Heinemann (1992); Bennett and Lasure (eds.) More Gene Manipulations in fungi, Academic Press (1991); Turner, in: Pühler (ed), Biotechnology, second completely revised edition, VHC (1992). The Ca-PEG mediated protoplast transformation and the acetamide selection procedure used is described in EP635574.

Hundreds of acetamide-positive clones were transferred to a second acetamide-containing plate and subsequently transferred to induce sporulation (for example on YEPD media per liter: yeast extract, 10 g; peptone, 10 g; glucose, 20 g). Subsequently, several clones were colony purified and the presence of the Pc22g14900 expression cassette was determined via PCR. To this end a piece of sporulated colony was resuspended in 50 µl of water and boiled for 10 minutes at 98 degrees Celsius. The cell debris was spun down and one µl of the supernatant was used in a specific PCR reaction. Using a primer set with the forward primer (SEQ ID NO 96) annealing at the *pcbC* promoter of the Pc22g14900 cassette and the reverse primer annealing (SEQ ID NO 97) downstream of the ATG of the Pc22g14900 gene. Only co-transformants with the Pc22g14900 expression cassette intact integrated should give a 262 bp fragment.

### Determining the adipoyl-CoA ligase activity of P. chrysogenum transformants

One acetamide-positive transformant which gave the 262 bp fragment in the specific Pc22g14900 expression cassette PCR was selected for further testing. This mutant and the parent strain were cultivated as described in example 2 and after 6 days the cultures were sampled. The supernatant was separated from the cells via filtration. The cells were washed twice with ice-cold physiological salt (0.9% NaCl) and the pellet was frozen in liquid nitrogen. The frozen pellet was subsequently freeze-dried.

The adipoyl-7-ADCA concentration in the filtrate was determined via HPLC (as described in US 6,410,259) - see Table 3. The CoA-ligase activity and the formation of acyl-CoA compounds were determined as described in WO97/38107. The frozen cells were grinded and 50 mg resuspended in 2 ml extraction buffer (per 10.4 ml: 35% ammonium sulphate, 10 ml; 1 tablet of Complete^{™} Protease Inhibitor (Roche) in 2 ml milliQ water, 0.4 ml; β-mercaptoethanol, 3.9 µl; ATP, 27.1 mg; pH 8.56). The mixture was stirred for 15 minutes at 4 °C and afterwards the cell debris was spun down for 5 minutes at 14000 rpm. The supernatant (=cell-free extract) was used directly. The protein concentration was determined with Bradford reagent.

Three substrate mixtures were prepared to determine the CoA-enzyme activity:
1. adipate mixture: 55.1 mg/ml ATP, 250 µl; 15.35 mg/ml CoA, 250 µl; 1.0 M adipate, 240 µl; 50.83 mg/ml MgCl₂, 50 µl; 50 mM Tris-HCl, 360 µl; pH 7.04
2. hexanoate mixture: 55.1 mg/ml ATP, 250 µl; 15.35 mg/ml CoA, 250 µl; 0.4 M hexanoate, 75 µl; 50.83 mg/ml MgCl2, 50 µl; 50 mM Tris-HCl, 525 µl; pH 7.06
3. phenylacetic acid (PA) mixture: 55.1 mg/ml ATP, 250 µl; 15.35 mg/ml CoA, 250 µl; 0.4 M PA, 75 µl; 50.83 mg/ml MgCl₂, 50 µl; 50 mM Tris-HCl, 525 µl; pH 7.00
230 µl of these mixtures were incubated with 10 µl of cell-free extract (which was 1:10 diluted in case of the adipate and hexanoate mixtures). The mixtures were incubated for 0, 10, 20 and 30 minutes, at which time points 50 µl samples were taken and processed as described in WO9738107. The CoA-ligase activity with the different substrates was calculated from the obtained results (see table 3).

**Table 3. Adipoyl-7-ADCA productivity and CoA-ligase activity of the P. chrysogenum strain overexpressing the CoA-ligase PC22g14900.**

| Strain | Adipoyl-7ADCA (control=100) | Adipoyl-CoA ligase (nmol/min.mg) | Hexanoyl-CoA ligase (nmol/min.mg) | PA-CoA ligase (nmol/min.mg) |
|---|---|---|---|---|
| Parent | 100% | 51 | 100 | 100 |
| Transformant A | 112% | 159 | 222 | 755 |

As can be seen in Table 3, the 3-fold overexpression of the adipoyl-CoA ligase activity leads to an increased production of the N-adipoylated β-lactam compound adipoyl-7-ADCA.

### Example 4

### Deletion of Penicillium chrysogenum gene Pc22g20270 leads to a decreased incorporation yield of the adipic acid from the culture medium into the N-adipoylated β-lactam compound compared to the non-mutant parent strain.

### Construction of deletion mutants

The gene Pc22g20270 was identified as a gene with an "adipate/control" ratio of 6.4 and an "adipate/PAA" ratio of 3.3 (see Table 1). In order to prevent the transcription of this gene a selection marker gene was inserted between the promoter and the open reading frame (ORF). To this end the promoter and the ORF were PCR amplified using the oligonucleotides SEQ ID NO. 98 plus 99 and SEQ ID NO. 100 plus 101, respectively. Phusion Hot-Start Polymerase (Finnzymes) was used to amplify the fragments. The ampliefied fragments are approximately 1500 and 1800 basepairs (bp) in length respectievley and contain a 14 bp tail suitable for the so-called STABY cloning method (Eurogentec).

From the standard STABY vector, pSTC1.3, two derivatives were obtained. One, pSTamdSL, was used for cloning the PCR amplified Pc22g20270 promoter. The other, pSTamdSR, was used for cloning the PCR amplified Pc22g20270 terminator. pSTamdSL (SEQ ID NO. 102) was constructed by insertion of an inactive part of the *amdS* selectionmarker gene (see for example the *PgpdA-amdS* cassette of pHELY-A1 in WO04106347) by PCR amplification of the last 2/3 of the gene (*amdS*) and cloning it in the *Hind*III*-Bam*HI sites of pSTC1.3. pSTamdSR (SEQ ID NO. 103) was constructed by insertion of another inactive part of the *amdS* selectionmarker gene (see for example the *PgpdA-amdS* cassette of pHELY-A1 in WO 04106347) by PCR amplification of the *PgpdA* promoter and the first 2/3 of the gene wherein the EcoRV sites where removed and cloning it in the HindIII-PmeI sites of pSTC1.3. Also, a strong terminator was inserted in front of the P*gpdA-amdS;* the trpC terminator was PCR amplified and introduced via the *Sbf*I*-Not*I sites of the P*gpdA-amdS* fragment. Both vectors do contain an overlapping but non-functional fragment of the fungal selection marker gene *amdS,* encoding acetamidase and allowing recipient cells that recombine the two fragments into a functional selectionmarker to grow on agar media with acetamide as the sole nitrogen source (EP 635,574; WO97/06261; Tilburn et al., 1983, Gene 26: 205-221). The PCR fragments were ligated into the vectors overnight using T4 ligase (Invitrogen) at 16 °C, according to the STABY-protocol (Eurogentec) and transformed to chemically competent CYS21 cells (Eurogentec). Ampicillin resistant clones were isolated and used to PCR amplify the cloned fragments fused to the non-functional *amdS* fragments (see Fig. 1). This was done using the oligonucleotides SEQ ID NO. 104 and 105. The thus obtained PCR fragments were combined (see Fig. 1) and used to transform a *P*. *chrysogenum* strain with the *hdfA* gene deleted (WO05095624). In this strain the non-homologous end-joining pathway is disturbed and therefore the random integration of DNA is drastically reduced. And as the combined PCR fragments themselves should recombine also to form a functional *amdS* selection marker gene (i.e. the so-called bipartite or split-marker method), correct targeted integrants should undergo a triple homologous recombination event (see Fig. 1).
Transformants were obtained on acetamide containing agar (EP 635,574; WO97/06261) and these were subsequently transferred to a second acetamide selection plate to induce sporulation.

### Verification of deletion mutants

The mutants obtained were used for further characterisation: verification of the actual gene deletion by PCR and verification of the N-adipoylated β-lactam titer and yield on adipic acid after cultivation in 25 ml batch cultures (in 100ml shake flasks).

To isolate chromosomal DNA of the correct quality (i.e. enabling PCR amplification up to 9 kb) spores of mutant Pc22g20270 were used to inoculate 3 ml of medium in a 24-well MTP plate and grown for 2-3 days at 550 rpm, 25°C and 80% humidity. Cells are washed and protoplasted using standard buffers (see Swinkels, B.W., Selten, G.C.M., Bakhuis, J.G., Bovenberg, R.A.L., Vollebregt, A.W. 1997. The use of homologous *amdS* genes as selectable markers. WO9706261). Protoplastation was done for 2 hours at 37°C, using Glucanex at 10 mg/ml in the 24-well plates. Cells (protoplasts and remaining mycelium) are washed again and DNA was isolated using the Puragen DNA isolation kit (Gentra) according to the suppliers' instructions. The DNA was air-dried and dissolved in 100 ul water. PCR reactions were performed in a final 50 µl, with the following composition:

| | |
|---|---|
| 5 µl | DNA template (5x diluted from sample preparation) |
| 21.5 µl | water |
| 10 µl | GC buffer (Finnzymes) |
| 1 µl | dNTP (stocksolution of 10mM) |
| 2 µl | DMSO |
| 5 µl | Forward oligonucleotide (stocksolution of 2µM) |
| 5 µl | Reverse oligonucleotide (stocksolution of 2µM) |
| 0.5 µl | Phusion Polymerase (Finnzymes) |

To verify the correct deletion 3 PCR reactions are performed (see Figure 2). The first PCR reaction is to confirm the correct integration at left flanking, using for the locus of gene Pc22g20270 the specific forward oligonucleotide of SEQ ID NO. 106 and the reverse oligonucleotide of SEQ ID NO. 107; the former being specific for this gene locus and choosen just upfront of the fragment used for gene targeting and the latter annealing in the *amdS* selection marker, which can be used to verify all individual gene mutations. The second PCR reaction is to confirm the correct integration at right flanking, using for the locus of gene Pc22g20270 the forward oligonucleotide of SEQ ID NO. 108 and the specific reverse oligonucleotide of SEQ ID NO. 109; the former being specific for this gene locus and chosen just downstream of the fragment used for gene targeting and the latter annealing in the *amd*S selectionmarker, which can be used to verify all individual gene mutations. The third PCR reaction is to confirm the absence of the WT fragment and the correct integration at the locus of gene; for this one can combine the two locus specific oligonucleotides of the first two PCR reactions, i.e. in the case of locus of gene Pc22g20270 the forward oligonucleotide of SEQ ID NO. 106 and the reverse oligonucleotide of SEQ ID NO. 109; if the gene targeting is correct this yields a much larger band than in the case of the WT (see table 4).

The PCR amplification is performed in a Tetrad machine of Biorad using the following program:
Step 1:30 sec at 98°C
Step 2:10 sec at 98°C
Step 3:30 sec at 55°C
Step 4:1.5-4.5 min at 72°C
   (the actual extention time is set by using 0.5 min/kb to be amplified)
Step 5: Repeat steps 2-4 for 35 cycles
Step 6:10min at 72°C

**Table 4. PCR fragments in mutant at gene locus Pc22g20270 (see also Figure 2).**

| PCR | Target | Fwd oligo | Rev oligo | Expected if correct | Expected if WT |
|---|---|---|---|---|---|
| 1 | Left flank | SEQ ID NO 106 | SEQ ID NO 107 | 1.9 kb | No band |
| 2 | Right flank | SEQ ID NO 108 | SEQ ID NO 109 | 3.4 kb | No band |
| 3 | Gene Locus | SEQ ID NO 106 | SEQ ID NO 109 | 8.3 kb | 4.5 kb |

The same procedure can be applied to all the mutants obtained according to the present invention; in all cases the oilgonucleotides of SEQ ID NO. 107 and 108 stay the same, but the oligonucleotides of SEQ ID NO. 106 and SEQ ID NO. 109 are gene specific.

### Productivity of deletion mutants

The spores of mutants for locus Pc22g20270 were inoculated in 25 ml medium as described in example 2, in 100 ml shake flasks and incubated for 168 hours at 25 °C and 280 rpm. As a control strain, *P. chrysogenum* strain DS17690 (S917), deposited at the Centraalbureau voor Schimmelcultures, Utrecht, The Netherlands on April 15, 2008 with deposition number CBS 122850, was inoculated and grown in the same way. Subsequently, the cells were removed by centrifugation and 1 ml of the supernatant was used for NMR analysis. Quantitative ¹H NMR experiments were performed at 600 MHz on a Bruker Avance 600 spectrometer. To a known quantity of filtrate, a known quantity of internal standard (for example maleic acid), dissolved in phosphate buffer was added prior to lyophilisation. The residue was dissolved in D₂O and measured at 300°K. The delay between scans (30 s) was more than 5 times T₁ of all compounds, so the ratio between the integrals of the compounds of interest and the integral of the internal standard is an exact measure for the quantity of the penicillins, intermediates (6-APA and IsopenicillinN), degradation products (8-HPA), remaining sugar and remaining side-chain (adipate). Compared to the DS17690 strain, the functional inactivation of gene Pc22g20270 decreased the adipoyl-6APA titer in shake flask cultivation significantly. With the titer of DS17690 set at 100, the Pc22g20270 microbial mutant strain produced 89% adipoyl-6APA, while using phenyl acetic acid as a side chain the mutant microbial strain of Pc22g20270 had the same penicillinG productivity as the DS17690 strain.

These results suggest that the putative acyl-CoA ligase encoded by gene Pc22g20270 has a functional role in activating adipic acid as the side chain precursor for the production of N-adipoylated β-lactams. The relative yield of N-adipoylated β-lactams on the side chain precursor adipic acid (the incorporation yield as defined in the text) was 84% of the incorporation yield of DS17690.

Thus, we may conclude that by deleting gene Pc22g20270 the adipic acid incorporation yield is decreased as compared to the parent strain and therefore gene Pc22g20270 should be over expressed to obtain mutant microbial strains with an improved incorporation yield of the adipic acid from the culture medium into the N-adipoylated β-lactam compound compared to the non-mutant parent strain.

## Claims

1. A mutant microbial strain capable of producing an N-adipoylated β-lactam compound when cultured in a culture medium comprising adipic acid **characterized in that** one ore more genes encoding an adipoyl-CoA forming enzyme is over expressed in the mutant strain resulting in an improved incorporation yield of the adipic acid from the culture medium into the N-adipoylated β-lactam compound compared to the non-mutant parent strain wherein said gene is not over expressed.

2. The mutant microbial strain of claim 1 **characterized in that** the improved adipate incorporation yield is at least 5%.

3. The mutant microbial strain of any of claims 1 and 2 wherein the adipoyl-CoA forming enzyme is acid CoA ligase (EC 6.2.1.xx).

4. The mutant microbial strain of any of claims 1 and 2 wherein the adipoyl-CoA forming enzyme is acid-CoA transferase (EC 2.8.3.xx).

5. The mutant microbial strain of 3 wherein the gene encoding acid CoA ligase (EC 6.2.1.xx) is selected from the group consisting of Pc13g14420 (SEQ ID No:1); Pc21g22010 (SEQ ID No:2); Pc22g20270 (SEQ ID No:3); Pc22g00960 (SEQ ID No:4); Pc12g05520 (SEQ ID No:5); Pc13g12270 (SEQ ID No:6); Pc18g05710 (SEQ ID No:7); Pc20g13500 (SEQ ID No:8); Pc13g01890 (SEQ ID No:9); Pc20g10840 (SEQ ID No:10); Pc13g05130 (SEQ ID No:11); Pc13g10810 (SEQ ID No:12); Pc22g06680 (SEQ ID No:13); Pc22g14900 (SEQ ID No:14); Pc22g16410 (SEQ ID No:15); Pc21g13540 (SEQ ID No:16); Pc21g20650 (SEQ ID No:17); Pc21g23730 (SEQ ID No:18); Pc21g21960 (SEQ ID No:19); Pc22g24780 (SEQ ID No:20); Pc06g01160 (SEQ ID No:21); Pc12g09980 (SEQ ID No:22); Pc15g00420 (SEQ ID No:23); Pc21g07810 (SEQ ID No:24) and Pc21g09470 (SEQ ID No 25) and sequences that are substantially homologous to said sequences.

6. The mutant microbial strain of claim 4 wherein the gene encoding acid-CoA transferase (EC 2.8.3.xx) is selected from the group consisting of Pc20g15639 (SEQ ID No: 26); Pc13g02780 (SEQ ID No: 27); Pc22g13680 (SEQ ID No: 28); Pc22g03940 (SEQ ID No: 29); Pc14g00590 (SEQ ID No: 30); Pc16g00210 (SEQ ID No: 31) and sequences that are substantially homologous to said sequences.

7. The strain of any of the preceding claims wherein the N-adipoylated β-lactam compound is selected from the group consisting of adipoyl-6-APA, adipoyl-7-ADCA, adipoyl-7-ACA, adipoyl-7-ACCA, adipoyl-7-PACA, adipoyl-7-ADAC, adipoyl-7-ACCCA.

8. The strain of any of the preceding claims wherein the strain is a fungus, bacterium or yeast.

9. The strain of any of the preceding claims wherein the fungus belongs to the genus *Penicillium.*

10. The strain of any of the preceding claims wherein the fungus is *Penicillium chrysogenum,* preferably transformed with and expressing a gene encoding an expandase or an expandase/hydroxylase.

11. A method for the construction of the mutant strain as defined in claims 1-10 comprising over expression one ore more genes encoding an adipoyl-CoA forming enzyme.

12. The method according to claim 11, wherein the adipoyl-CoA forming enzyme is acid CoA ligase (EC 6.2.1.xx).

13. The method according to claim 11, wherein the adipoyl-CoA forming enzyme is acid-CoA transferase (EC 2.8.3).

14. A gene encoding acid CoA ligase (EC 6.2.1.xx) and which is selected from the group consisting of Pc13g14420 (SEQ ID No:1); Pc21g22010 (SEQ ID No:2); Pc22g20270 (SEQ ID No:3); Pc22g00960 (SEQ ID No:4); Pc12g05520 (SEQ ID No:5); Pc13g12270 (SEQ ID No:6); Pc18g05710 (SEQ ID No:7); Pc20g13500 (SEQ ID No:8); Pc13g01890 (SEQ ID No:9); Pc20g10840 (SEQ ID No:10); Pc13g05130 (SEQ ID No:11); Pc13g10810 (SEQ ID No:12); Pc22g06680 (SEQ ID No:13); Pc22g14900 (SEQ ID No:14); Pc22g16410 (SEQ ID No:15); Pc21g13540 (SEQ ID No:16); Pc21g20650 (SEQ ID No:17); Pc21g23730 (SEQ ID No:18); Pc21g21960 (SEQ ID No:19); Pc22g24780 (SEQ ID No:20); Pc06g01160 (SEQ ID No:21); Pc12g09980 (SEQ ID No:22); Pc15g00420 (SEQ ID No:23); Pc21g07810 (SEQ ID No:24) and Pc21g09470 (SEQ ID No 25) or an acid-CoA transferase (EC 2.8.3.xx) and which is selected from the group consisting of Pc20g15639 (SEQ ID No: 26); Pc13g02780 (SEQ ID No: 27); Pc22g13680 (SEQ ID No: 28); Pc22g03940 (SEQ ID No: 29); Pc14g00590 (SEQ ID No: 30); Pc16g00210 (SEQ ID No: 31) and sequences that are substantially homologous to said sequences.

15. A gene encoding acid-CoA transferase (EC 2.8.3.xx) and which is selected from the group consisting of Pc20g15639 (SEQ ID No: 26); Pc13g02780 (SEQ ID No: 27); Pc22g13680 (SEQ ID No: 28); Pc22g03940 (SEQ ID No: 29); Pc14g00590 (SEQ ID No: 30); Pc16g00210 (SEQ ID No: 31) and sequences that are substantially homologous to said sequences.

16. A polypeptide encoded by the gene as defined in claim 14 or claim 15

17. A process for the production of an N-adipoylated β-lactam compound comprising culturing the strain as defined in any of claims 1-10 in a fermentation medium comprising adipic acid.


SEQUENCE LISTING

<110> DSM IP Assets B.V.

<120> ADIPOYL-7-ADCA PRODUCING STRAINS

<130> 26946-WO-PCT

<160> 109

<170> PatentIn version 3.3

<210> 1
<211> 1798
<212> DNA
<213> Penicillium chrysogenum

<400> 1
atgggagacc gcactctatt catacctcca catgtgggag ataacgtgtt gccaaatctt 60

cccttttttc acagactatt gcgttatgcg caacgtaagc cttcgcccat tgtggttcgg 120

gacttggttg ccgacgcgga aaagacgtac caccaccttg tttcggacgt gttggcattt 180

cgcaaggtct tggaaagatc catcagccat gaagctcgac gcgatttgaa tgcggacaag 240

gaggtatata ttggattgct tgcaccggga ggatatgaat acaccgtcgg cttcatcgcc 300

atcctggcaa tcggtgcagc agtcgtacct atgggtgagt ttcaacattc tggaagaaag 360

tccttggcta aaagccagca gctgctgcat taccagcaga agaagcctcg tacttcctat 420

tgaaggcgcg atgtgtaggt ctggtagcca gcacagccag cgaaaagacc gcccagtccg 480

tggttcgcta tatgggagaa agcaaaggca tgcacatccc ctgcatatct ccaatcgcat 540

ctcatttccg ccacacgctt ctcccgtcag atgaaatgac catctcctcc ggtccagttc 600

cggatatgaa cgccgcagca ctggtaatat tcacttcagg cacgacagga cccccaaagg 660

gcgctgtgca gcgtcgcagt tatatttccg gaaatggaga ggccgatgca gcctattacc 720

gaatcacaga caaggacaca gtcctccatg tccttccagt gcatcatgcc tccggtgtgg 780

gattgacttt cctcccgttc cttgcagccg gcgcatgtat tgagttccgc tgtggtagtt 840

tcgatacagc gtggacgtgg gagcgctggc gacggggagg tcttaccttt ttctctggag 900

ttcccaccat atacatgcgt atgatgcggt actacgaaga gaacatttcg caccaagctc 960

ctgagatacg tgaccagtat gtcgccggtg cgcgtcagat tagagcgatg ctttgtggta 1020

cttcggctct tccagggcca gtacaagagt tctggcataa cattaggagc aaaccaattc 1080

tcacccgata tggtgcgacg gaatttggtg cagtcatcaa gactgaactt gattccgatg 1140

gaacgccgca aaacagtgtt ggttgtgttg ctgaggcggt ctcactcaag cttaccgagg 1200

aaggtcagat cttagtgaag tgtccttata tgttttccaa gtaagccaaa tttcctttcg 1260

agaagtggtg gtccattctc acacgccttt taacaggtat ctcttcgacg agaaagcgac 1320

agcggatgca catgacgccg aggggtactt caagacgggg gatattgctc gccgtgaagg 1380

aaaatattat ttcatcctag gccgtgcctc gatcgacatc atcaagtcag gtggctacaa 1440

gatctccgca ttggatatcg agcgcgagat tctgggtctg gactatgttt ctgaggtcat 1500

ggttgtgggc gtcgaagatg aggagttcgg tcaacgagtt gctgctactg tcagcctcaa 1560

acaagatcaa aagacaacac gcaaaagcct cacaatcgcg gagctgcggg aagatctgcg 1620

gagtaagatg gctgggtata agatgccaac tgtccttcgt gtggtccaag gtgaattgcc 1680

caagtcaggc acggggaagg tgcagaagaa gatattggga ccgcagtttt ttccgcctaa 1740

ctatcgacag ctgcctgagg tgcaagtctg gagcagggag aataaggcga ggctatag 1798

<210> 2
<211> 1890
<212> DNA
<213> Penicllium chrysogenum

<400> 2
atgaggaaga ccttgcgtca tggcttggga ttacctactt acatatacat acctaggtag 60

gtagctactt gaactccccg tactcatcac agccatcaca tagatatccc tccaattgat 120

acttgggatt gtctcttcga aagagaagac agacccttcc tagatgacca cagtacgtac 180

attccttcaa catcaaagcc ggatactaac aaccatagtc atcttcaagt cgccgccggg 240

cgaagtccga accttcaacg acatccggtc ccaagcccta agcttcggcg ccaatctcca 300

agtccaatgg gaatggcaaa aaggcgatat cctcttactc tttgcgccga atgacatcga 360

tgtccccacg ttgttttggg gatgccattg ggctggggga gtagtctcac cagccaaccc 420

ggcattcact gctgatgagt tgaaatacca attgagtgac acaggggcaa aggcaatggt 480

tgtccacacc tcgctcttag acactgcact caccgctgca aagcgcattg actttcctat 540

tgcgcacatg ttggtttttg gccctccctc ctcagaagca gagctgcagc atgtcgagtc 600

catgttggga ggtggagctc ctggagcaag acgtccgagg atagacccag tcgtcgatac 660

agccttcctg gtatactcgt ccgggactac cggacggccc aaggggacaa gaataactca 720

taccaatctt gtgacgaacc tcgtcttgca gggacgagtt gaaggtccgc acatgaactg 780

gagacaggat cggttcctgt cattcttacc aacataccac atatatggta tgtcaatatt 840

tgacggagaa tcctcatttg caggctaacc agtctactca aaggacttac atgtctagta 900

catttccctc ttttcctggg aataaagaca atagtaatgg agaagttctc cgtcaaaggt 960

ttcttacaca atgtgaaagc cgaatcgatc acccacatct acgccgtccc acctgttgtc 1020

ctgtacctcg ccaaagatcc cgcaatgacg cgcgaacaac tatcatcact gagaatggta 1080

acctcagctg ctgcaccact agcatcagat ctgatccacg ctgtgtacga tcgtctgaag 1140

gtccctgtcc gccaggcata cggactgacc gagtcgacgg ctgttacgca tctccaggta 1200

cctctatccg atgtatccaa tagttgccag ctcgactaac ttattcaaca gagatgggac 1260

gaatgggata aagcaatggg atcaagcggc cctctatatc ccgcagtcga aacgaagttc 1320

atcgacgacc aagggaaccc cgtaacaaaa ggcgacggcg aactatgtct ccgaggaccg 1380

accctcttcc caggctacca taacaacgct gaagccacgg cacggtctat caccagcgat 1440

ggatggttca aaaccggtga tattggattc caagacgagg agggaaatct atttatcaca 1500

gatcgattaa aggaccttat taaattcaag ggtttccaaa ttcccccggc ggagatagag 1560

agtgccctgc atgaacatcc gcttgtgcat gacgccgctg tgattggact tgctgttgag 1620

aagattgcga ccgaggtacc ggttgcttat gtggttcttg agaagacgga taaaccggcc 1680

gagcaggttg cagaggaatt ggtggcttat gttagtggga aactggctcc tcataagagg 1740

cttcgtggtg ggattgtcct gatcgacgag attcccaagg gtcccgctgg gaagattctg 1800

aagcgggtgt tgaagactag ggcagaaggt gtcgatcaag ggaaggctat tggtgcttca 1860

atctatgacg atcgtccttc caagctgtga 1890

<210> 3
<211> 1835
<212> DNA
<213> Penicillium chrysogenum

<400> 3
atgcccgtat cctctaacta ccccctggtc gacatcccag aagtggatct ttggaccttt 60

ctatttgagc gcaaggacag agcttaccca gatgacaaga gtgagtgata tccaatgaga 120

aacccctagt gctacgctat taaatacggc attgtatttc tcttagtttt ctcaacactg 180

acaccccacc agttatctac caagatgccg atacccaacg tcattacaca tacaaatcgc 240

tacgtgatgc ctcgctagat ttcggcaagg gcctcaaagc cctgtatgag tggcgcaagg 300

gcgatgtact ggccctcttc acgccaaaca gcatcgatac acccgtggtg atgtggggca 360

cactttgggc cggcggaacc atctcgcctg caaacccggg ttacacggtt gacgagctgg 420

ccttccagct caagaactcc catgccaagg gccttgtaac gcaagcctcg gtgctgcccg 480

ttgcgcggga agcggcgaag aaagttggca tgcctgaaga ccgcattatc ctgattgggg 540

accagcgtga tcccgatgcg cgcgtcaagc atttcacctc tgtgcgcaat atctctggtg 600

ctacccgcta ccgcaagcag aagatcactc ctgcgaagga tgtggctttt ttggtttatt 660

cttctgggac cacgggtgtg cctaagggtg tcatgatatc gcatcggaac atcgttgcca 720

atatcaggca gcagttcatt gctgagggtg aaatgttaag ctggaatggt ggtcctgatg 780

gaaagggtga tcgggtcctg gcatttttgc cgttctatca tatctatggt atggatttca 840

attgcgcagg tgctcgaaca atattctaac tagagccttt aggattgact tgcttaatta 900

cccaggcttt atacaagggt tatcacttga ttgtcatgtc caaattcgac attgaaaagt 960

ggtgcgccca cgtgcagaac tatcgctgct cgtttagtta tatcgtccct cccgtggtgc 1020

tattgttagg caagcacccg gtggtcgaca aatatgacct ttcaagtctg cgcatgatga 1080

actcaggcgc tgcaccactc actcaagagc tggtagaagc agtctactcc cgcatcaagg 1140

ttggcattaa gcagggctac ggactcagcg agaccagccc gactacacac tcccaacggt 1200

gggaggactg gcgcgaagcc atgggttccg tcggccgctt gatgcctaat atgcaagcca 1260

agtacatgac tatgcctgag gatgggtccg agcctaagga ggttggcgag ggcgaggttg 1320

gcgaattgta cctgaaagga cctaacgttt tcctgggcta tcacgagaac ccagaggcta 1380

ccaagggctg cctgtctgag gatgggtggt tccagactgg tgacgttgga taccaggacg 1440

ccaagggtaa tttctacatt accgatcgtg tcaaggaact tatcaagtat aagggcttcc 1500

aggtgcctcc tgctgagttg gaagggtatc tggttgacaa cgatgctatt gatgatgttg 1560

ctgtcattgg tattgagagt gagacacacg gttccgaggt tcctatggct tgtgtggttc 1620

gcagcgcgaa gagcaagtct tccggaacaa gcgagaagga tgaagcagca aggattatca 1680

aatggctgga tagcaaggtc gcaagccaca agcgtctgcg cggtggcgtc cactttgtgg 1740

atgagatccc caagaatccc agtgggaaga tcctacgtcg gattctgaag cagaagttca 1800

agggggcggc cgaggcacca aaagccaagc tgtaa 1835

<210> 4
<211> 2529
<212> DNA
<213> Penicillium chrysogenum

<400> 4
atgacatctc ttccgcgtaa actatggcag catccagccc ccgagtcgac ccagatgggt 60

cgcttccagc gagacctaga gaaatccacc ggacacaagt tcgacgtgag tgagattggt 120

ctgtggaaag atactatcta tctaacatct cccagtcctt ccatgatatg tacctgtatt 180

cagtcaagaa ccgctcagcg ttctgggaat tctgctggaa atacttccaa ctgatccacg 240

agggctccta cacaaaggta gttgatgaaa ccgctcgaat ggatagcgtg cccgaatggt 300

tccccggtgt acgtctgaac ttcgccgaga acctgctttt ctcacgcatt gctggcgaca 360

aaacagacaa ggaagacgac aagattgctg ttagcgaagt tcgagaaggc gctgcgcatg 420

aggttatcca tctcacttgg ggtgagctcc gacgacggac tggagcgttg gtccaggcta 480

tgaaggccca tggggtcgtt caggtgatcc gtattgctct ctgcgcggcg aatagcatcg 540

atacactatt tgtgttcttg gcgtcaacgg ctctgggtgc tatcttctca tctagttcca 600

cggatatggg tactaaaggc gttttggata gactgcttca gatcaagcct cgctggctgt 660

ttatggacga cctggcggtt tataatggta agaccatcga cttgcgatcg aagattgggg 720

aaatcgtgaa aggcatggag tctgtccctg aatttgaggg agtcgtctca ctacctcggt 780

tccattcgcg gccggcggat atcaactcca tctctaggac caagactctg gcggagttcc 840

tcgaaaaggc gggcggaaat gagaaacttg aatttgaaag agttggattt cgcgatccct 900

tcttggtggt ttactcgtct ggcacgacag gacagccgaa gtgtatcgtc cattctgtgg 960

gcggcgtgct tctaaactcg agcaaggagg gaagattgca tagtgatctc gggcctgact 1020

gtgtgacctt acaatatacg acaaccggat ggattatgta tatgagtgct gtgcagactt 1080

tactctttgg ggcacgggtg gtgctgtatg atggaagccc atttatccca ggtatcactg 1140

ctttggttga ccttgcagca caggagaagt aagtcgtcct tctgttgaca tgacattcac 1200

tccacgatgt acatttgaag tccccggcta atcaatggca gggtgaccca ccttggaata 1260

tcaccccggt ggctccatga gttacaacag gctaagatca aaccccgcga gaaagttgat 1320

ctcagctcgt tacgggttgt cacgagcact ggcatggtcc ttcgggatgc attgttcgag 1380

tggttctatg acgaagggtt cccaccccat actcgtctga ataatatttc tggtggcact 1440

gatattgcag gatcctttgg aacaggcaac ccccttgtcc cactatatgt tggtgggtgc 1500

gccggatgca gcctgggcat tcccgtggag gtttacgact ctacgattga gggcggcgac 1560

gggatcaagg gagtacccgt ggaggacggt gttccaggcg aattggtcgc cacatctgct 1620

tttcccaaca tgcccacact attctggggg gatgagagcg gcaagaagta ccatgatgcg 1680

tattttgaga gattcgacag tgaggccact cccccctgtg cctatgacta tggtcgctaa 1740

ttctcttcag acgtgtggac acacggcgac ttcgtttcca tccgtccaat cacgaaacaa 1800

attgtgtttc acggacgcgc tgatggggtg ctcaacccgt cgggagtgcg atttggatcg 1860

gccgagatct atcgggtact tgagggccaa ttttcgaaag agattagcga ttccatatgc 1920

gtaggccagc gacgaccaac agatacggac gagcgggtga tactcttttt gctgatgagg 1980

ccagggatgg cgttcacacc ggacctggtt gcacgagtga agagcgccat ccgttcagag 2040

ctgagccctc gccatgtacc catgttcacg tttgagaccc atgaaatccc ggtatgtgct 2100

tttgtagtga aaaacaagat ctgtaagagc ggaactgacc cacgtatatc gtcatagacc 2160

acggtcaacc tgaaaaaggt ggagctccct gtgaagcaga ttgtgtctgg aaaaatcatt 2220

aagccgtcgg gtacactgct caacccgaag agcttggact tctactatca attcgccaaa 2280

gtggaaactc tgcgcgagag taagctctga acggactctt ggacatcttc ccatcccaag 2340

tgactccata agagaggggc agaccgggtc catcgggatc gtcacttcgc tctgccaagc 2400

tttgaccaag ctttagcaac accgggatct cttttggggg agttttgtat ataagaatac 2460

atggtagaag ataggaagta gattgaaata gaaatattga acaacgcata tgaaaggaat 2520

agtaagtag 2529

<210> 5
<211> 2302
<212> DNA
<213> Penicillium chrysogenum

<400> 5
atgttcttct catcacctcc ccatttggct aaggccgagg agcttaagca agcgcctcca 60

aagggcgtag cttactctgt cgccattcct ggctctgaac agcccggccg aagtcgggta 120

taccgagcat ggaatgcgca gaaggaactt ttgacgacac tggactccca ggtacagagg 180

ctcagccaat gccagaatat cttaatactg accaattcta ggtcaccacg gcccatgata 240

tgttcgaatc gaccgccaac cgccagccca agaaccactg cctcggttgg cgtccgtata 300

actccacgac caagtccttc gatccatacc agtggctcac ttacgagact gtgcagaaga 360

gacgcgctgc gttcggtgct ggtctagtag agctgcacca caagcacgac tgccatcgct 420

ccggccagta tggtgttggt ctctggtccc agaaccgacc ggaatggcag attaccggtg 480

agttgtgctg tgagggcgaa gaattcaaga gataaaacgc taaatcgaaa ctatgtacag 540

atttggcttg catttcccaa tctctttact ccgtctcgat ctacgacgtc ctgtccccag 600

acgctaccga atatattatc aaccacgctg agctcagctg tgttgtcacc tctctacccc 660

atatcccaac gctcctcaag ctcaagcctt ctctcccgaa cctgaagatc atcatcagtt 720

tggacccatt agatggcggt gagcaggatg gtcactccaa gcgtgccctt atcgagtcca 780

tggccgcggg tcagggcctg gcgatctaca ctatggatca ggttgaggag ctgggcttgg 840

cctcaaagcg cgggtacaat gccccttctg cttcggacat tgtcactatc aactatacct 900

ccggaacgac cggcccccct aagggtgttg ttctgacgca tggcaatgcc gtggcggcca 960

cgtcctgcgg tttgatcact gtcggccaat cccgaggtga tacgatggtc tcttatctgc 1020

cgctggccca tatttatgcc cgtctggccg agcacactgc tttctggggt ggtgcccgga 1080

ttggttattt ccacgggaac attgtggagt tggtcgatga tttgaagttg ttgaaaccca 1140

ctggtttcat gtctgttcct cgtctttaca gtcggtttgg tagcgcaatc cgttctgcga 1200

cagttgagca gcctggtttc aagggcgctc tttcacgaca catcattgcg gccaagactg 1260

cgaacatgaa gaaccccgat ccctccaagg cgacagtccg gcacgctgtg tacgatcgcc 1320

tgtgggctaa gaaggtggct gccgctctcg gtctggagcg agccaggtac atggtgtctg 1380

gctccgcgcc cttggacccg actctacatg acttcctgcg tgttgccacc ggtaccgata 1440

tccttcaggg ttacggcttg actgagtcct acgcctcagc caccgcccag ccgacctacg 1500

atctgacctc cggcaactgc ggtagccttg ctccctgtgt cgaggcatgc ctggcctctc 1560

tgccagacat ggagtactcc gtggatgaca agccgttccc ccgtggtgag ctcttgctgc 1620

gcggcaacaa catgttccgc gagtactaca agaacgagga agagaccagc aaggccgtca 1680

ctgaggatgg ctggttccgc accggcgatg tctgcacaat cgacgaaaag ggccgcttca 1740

tcatcattga ccgccgcaag aacgtactca agctcgccca gggcgagtac atctcccccg 1800

agcgtctgga gggtgttgtc cttgccgagc ttggctacat cgcccaggct tatgtccacg 1860

gcgacagcat gcagacattc ctagttggca ttttcggtgt cgcacctgac ctcttcgctc 1920

cgtttgccag caaggttctg ggcaagacca ttgccccgac cgacttggaa gcgattaagg 1980

aatctctgcg cgatgacaag atccgccgtg ctgtgctccg ggacctcgaa cgcgtggcca 2040

agaagcacaa gtttgctggc tacgagagaa tccgcaacgt ctctctcaag gtggagccat 2100

tcaccgttga gaacaacttg ctgaccccga cgtaagtttt tatttccacc tttccgtaat 2160

ccgagcattg ctaacattat cccctccctt ctatagtctc aagctcaagc gtccccctgc 2220

tgtcaagctt taccgttccc ttctggatca gctctacgaa caggccaccg aggagcagtc 2280

tgcccccaag gctaagctat aa 2302

<210> 6
<211> 1915

<212>  DNA
<213>  Penicillium chrysogenum

<400>  6
   atgccgtata aatcccgctg gacgattgac atcccagaca cccatttagc ctctcttctt      60
   ctcaaatccc caacggcacc tctctccaaa acacacaagt gctacctgga cgccgcccgc     120
   ccagaaaccc actacctcac cacccatgat ctccgcctct ggagccagcg gctagcagcc     180
   ggcctacgca aatccggact gcagcgcgga gaccgcgtcc tcctcttctc aggcaacgac     240
   ctcttcttcc ccgtagtctt cctaggcgtg ataatggcgg gggggatctt caccggcgcg     300
   aatccaacct tcgtagcccg cgaactagcc taccaactcc aagacagcgg cgccacatac     360
   ctcctctgcg caagcaacag tctagaaacg ggactggaag ccgccaaaca agccaaacta     420
   ccgcagagcc acatcttcgc ctacgacacc tccatctacg acggcgtaac aaacccccaa     480
   aagggctgcg catactggag cgacctcctc gcctcagagg aagaaggtgc agccttcacc     540
   tgggacgagc tgagtacacc agccctctcg tccacaacac tcgcactcaa ctactccagc     600
   ggcacaacgg gccggcccaa gggcgtcgag atctcgcacc ggaactacgt cgccaatatg     660
   ctccagtatt gtcatacggc aagtctacat ccggactaca aggctcgtct tgagcgctcc     720
   cgctggctat gtttcttgcc tatgtatcac gcgatggcgc agaatatctt tattgcggcg     780
   gcgctgtacc gcgccacgcc ggtgtatatc atgtctaagt ttgactttgt caagatgctg     840
   gagtatacgc agcgattccg gattacggat tttatactcg tgccgccggt tgttgttgcg     900
   ttggcgaaac atccggctgt tgggcagtat gatttgagta gtgtggagtt ggttgggagt     960
   ggtgcagcgc cgttggggag ggaggtttgt gaggaggttg agaagttgtg gcctcccggg    1020
   aagattaata ttaaacaggg atgggggatg actgagtgag ttcttatttt tttttttttt    1080
   tttttctctt taaattgttt tgctgttttc tgttgggcta ttttgatgga aagctcatct    1140
   tccatatagg gcgacttgct cggtcacggg atggaatcca gccgaaatta gtacatctgc    1200
   gtctgtcggt gagctcaatg ccaattgcga ggcgaagatt atgttcgatg gggttgaggt    1260
   gaaggaacgc aactctcgtg gcgagttgtg ggttcgtgct cccaatgtga tgaaaggcta    1320
   ctggcgcaat gagaaggcca ctaaagagac caagacggag gatgggtggc tcctgacggg    1380
   ggatattgcg tttgtggatg acgatggcaa attccatgtg gtggatcgaa tgaaggtatg    1440
   tttcaaagaa gaagaagaat gcaggaaact aacttggact ctgtacagga attaatcaaa    1500
   gtcaaaggca accaggtggc gcccgccgag ttagaggctc ttttacttga acatccggcg    1560
   atatcagatg ttgcggtgat cggggttgtc atgtaagttt gttttttttt ttatggatca    1620
   tttatggatc tacgatctat tgtttttctc tctttttttc tctttttccc tctcttctaa 1680
   cccgattagc aacaacgatg agcgccctcg cgcatatgtg gtgcttcgac ccggccaatc 1740
   cgccaccgcc aacgagatcg cgcattactt ggacaacaag gtctccgcgt tcaagcggat 1800
   taccggcgga gttgtattcc tcgaggccat accgaagaat ccttcgggca agattcttcg 1860
   catgaaattg cgtgaacagg ccaaagagga gctacgtgtc accgctaaac tctag 1915

<210> 7
<211> 2030
<212> DNA
<213> Penicillium chrysogenum

<400> 7
atggctaaca agcggtctct ttgcgcagaa attcccttga gtcttgcact ccctgccgcg 60

gcaactacac ttgcatatct caacgcaaga tggtcgctct cctacgattt gaatctcctc 120

aagggcttgc tgaagatgtc tgcgaagtcc cgcatcgccg aacgtggcga ccgcttgaac 180

ctcttctaca cacttgaagc ctatgcactc gatcctaaaa ctgcagacaa cgatttcatt 240

gtgtacaatg gacgaacgac gacattccac gaaacctaca tcacggctct tcgctacggg 300

gcgtggttca agacagtcca cggcattaag cgcaaggaga tcgtcgcgat cgatttcatg 360

aactcatcta ccttcatctt catgctgttg ggactctgga gcattggagc agtccctgca 420

tttatcaact acaacttgtc tggcaagccg ctcacccact ctattcggac gtcctccgca 480

aggctcgtgg tggttgacga agaagtccgc cactgcttcc cggaggagca ggagaagatt 540

ttgacatccc cgaacttccg tgatggcaag ggaccggttg aaattgtgtt ccatacccca 600

gaggtagaag cgcagatctt ggggatggag cctatgcgag aggatgacaa ggctcgcagt 660

ggcttgatcc ctcgcgatat ggccattttg atttacacaa gtggcaccac cgggcttcct 720

aaacccgcta ttgtcagctg gaagaagtgc tggtctggta gtttgttcgt taaagattgg 780

ttgaacatta ctccctctga cagattcttc acggtaaggt ctaccaaatt tacaaatgtt 840

gctgtccaag ctaacctctt aactagtgca tgcctcttta ccatagttcc gctgcgatac 900

ttgggtttat aacttgcttg atgggcgggt ccaccctgat cattggccgc cggttctctg 960

cccgcaactt catgaaggag gctcgggaga acggtgccac agttatccaa tacgtgggcg 1020

aaacgctgcg ttatctattg ggcgtagcac ctgaaattga cccggtcacc ggagacgacc 1080

ttgacaagaa gcacaatatc cgcttggcgt ttggaaatgg attgcgtcct gatatctgga 1140

accgcttcaa ggagcgcttc aacattccca caatcgccga gttctacgcc gccaccgaag 1200

gcactgcggg ctcatggaac atttcgtcaa acgacttttc cgcgggtgcc atcggccgaa 1260

atggtgcttt aggaaacatt attcttggtc gtggctctgc cattgtggat gttgaccacg 1320

agacccagga accatggcgt gatcccaaga ctggtctctg caagaaggtg cctcgcggtg 1380

accccggtga gttgctgttc gccatcgatg cagcagatcc cactgccaat ttccagggct 1440

acttcggaaa caagaaggcg acagaaggca agattatccg tgatgtaatc aagaagggcg 1500

atgcctattt ccgaaccggt gacatggttc gatgggacaa agacggccgc tggttcttct 1560

cggatcgact gggtgatacc ttccgatgga agagcgagaa cgtatcaacc agcgaggttt 1620

ctgaggttct aggcgcgcat cctgaagtgc atgaagccaa tgtttacggc gtgtctcttc 1680

ctaatcatga tggtcgtgcc ggttgtgcag ccatcctctt caaccagcag ataacaagcg 1740

gtggcctctc tgattccgca ctggagccct cgcgcgaggt gcttgatact ctggctgcac 1800

acactctcca gaacctgccc cggtttgcgg ctccgctgtt cttgcgggtt acgcctgtga 1860

cgcagtcaac gggtaacaac aagcaacaga agcatgttct ccgtactgaa ggtgtggatc 1920

cggctcttgt gtcgaagaag gacagactgt actggcttca gggcaataca tatgtcccct 1980

ttgggcagag ggactgggaa cggttgactg gtggtcaagt tcgactgtga 2030

<210> 8
<211> 2072
<212> DNA
<213> Penicillium chrysogenum

<400> 8
atggatatag gtacgtggcc aaacagttat tccaattgac ttcactcaca accttggaaa 60

ttacaggaac cgccgccgct gccctcgtcg gaggagctac aatcgcagga tatttgaatg 120

ccaagtttca tatccagaag gatatatcag gactgttcac gctgaagaac gctgaacgac 180

aatatgccaa tgccggtatg tttatatact attcctataa gtgcaaatca tatactgatc 240

agctcttagc tagtcaaaac caaggaaatc catggttcgt gctagtgcag accgtgaagc 300

gataccccga catgatctgt ctctggactc gcgagagatc atacacatac cgagaaatcc 360

aagaccaagc ctgccaatat gctcatttct tcctatcaca tggcgtgaag aaaggcgacc 420

tcgttgccct ttacctccag aacagcaacg agtacctcgt tgcatgggtc gccctctgga 480

gcattggctg tgcccccgcc gccataaatt acaacctcac cggtgacgca ttactgcact 540

gcctgaaaat cagcgacgcg accattctcc tcgtcgacga aaatgccgac tgtcgcgccc 600

gcgtcgaaga aagccatgac gctatcacag gcaatctggg aatgaagcca atgacactag 660

atagcgcctt aaaggcgcat atcagaacat tcccaacaac cctcccaccc aaggaactga 720

gcaagcatgt ggccggggac ttccccgcaa ttcttctata cacctctggc accacgggta 780

tgcccaaggg ctgtgcgttc acaatgtcca gactatatac cactctattc gtccgccgtg 840

ccatgatggg agatacgcca ggcccaggag gtgatcgctg gtacagctgt atgcctttat 900

atcacggaac ggcggctatc accataatct cgtgtctggt aatgggtgta agcgtcgcca 960

ttgcgcccaa gttcagcgtc agtagattct ggacagatgt ccgcgactcg gagtcgacca 1020

tcttcgtgta cgttggagag actgcacggt atttgctcgc accgcctccg tcgcctctag 1080

accgaaacca caaggtacgc tgtatgtacg gcaacggtct ccgtccggat atctgggaac 1140

ggttccgtga acggtttggt gttgcggaag tcggggagtt cttcaatagc acagaaggtg 1200

tctttggtct cttcaactac aaccgcggcc ctttcacggc tggaagtgtt ggtcatcacg 1260

gtttgatcat gcgtggtata ctgcataatg tcttcgtccc tgttgccatt gatccagaaa 1320

ctggagatat tctgcgggat cccaagtctg gctttgcggt tcgctcgcct tatgagcagg 1380

gaggtgaaat tatcgtcaac gttcccagtg aagaagcttt ccagggttac tggcacaatg 1440

atgaggcgac caataagaag tttttacgtg atgtcttcaa gaagggggat ctctattatc 1500

gatctggaga tgctctgcgc cggcagagcg atggccggtg gtacttcctc gaccgactcg 1560

gggatacctt ccgatggaag agtgagaatg tgtaagttgc tgatatgcgc cggaaattct 1620

gggagcagat ttgttaatga tgtcaacgca acagagcaac cgccgaggtt tcagaggtac 1680

taggccagtt ccctggcatc accgaagcca acgtttacgg cgtccgtctc cccaatcacg 1740

agggacgcgc cggctgtgca gcgattcaaa ttagcccaga tgcgcgtcag acgtttgact 1800

acacggagct tgcgagattc gtacgctcca agctgccgaa atatgctgtt ccattgttcc 1860

ttcgcatcgt cgagaatccc actcacatcc acaaccataa gcagaacaag gtgccacttc 1920

gtgatgaggg ggttgatact gccttgattg ggactaaagc ccctgagggt aaagacgacc 1980

acttcctgtg gattgcgcct ggtgaggaga gttattcacc gtatgggcag aaagagtggg 2040

agcaattgtc gagtgggagt gttcggctgt ga 2072

<210> 9
<211> 1955
<212> DNA
<213> Penicillium chrysogenum

<400> 9
atgtctggtt caaagtctcg gttgtctggg ctccttggcc atttcacagg ctcaacaccg 60

ccggttgaac accgtgttaa tactcatacg ctatccccaa ctttctttct tccgcgtgct 120

gctgccgttg agcccaatgt aagctctctg caagtggttc ttaggtagtg cttcaatggt 180

ctaacgaaaa attacacagg ctgaggcgat ctatcatgta accgccaaca acaaaatcct 240

tcgtaggagc tatggcgaga ctgcagaccg agcgaggggt atggcttact acctcaaaaa 300

acatgggctc agcagagtgg ggatactatg cccgaatacc ccagccttcc ttgagtcaat 360

tttcggaatc gccgcagctg gcgctgttaa tatcggtatc ctatcgtctt ttgaagagcg 420

atatccattg agctaacata tttgcagctg tcaattatcg tctgaaacaa gaagacattg 480

cttatatatt tgaccatggt gatgcggagg tgatcatcgt cgacgaggaa tatgttccac 540

ttctggagca ctatcgatcc caacaccccc gcattcctat tatagtagac accgacactg 600

atgcgacgga gggtgagcta acgggcccct ttgacgaggc tgttctggaa ggcctcagac 660

acgacatcga tacaggttct cgaggctggg aaggactcga aagccaagct gccgatgaag 720

aatccacaat tgcattggca tatactagcg ggactacagc ccgtcctaag ggtgttgagt 780

tcagtcatcg ggctgctatc tggcaacatt gggaaacgtc attgaaactg gtctgaactc 840

accaccgcgg acgtgcccgt tatctatgga cgctacccat gttccacgct atgggttcgt 900

gcatcttctt cgcttgagtg atgtgcaatc aacctgcata actaacatat gtatttatat 960

aggctggaca tttccatggg cagtgacagc cgttcgcgga actcattatt gcctacgcaa 1020

gatagactac cccgaaatct ggaggctgct gaaagaggag catatcaccc acttcaatgc 1080

tgcaccgact gtgaacacct tgctctgtaa cgcgaaagaa gcagagaggc tgcccgagcc 1140

tgttcgcgtc acggtggcag caagcccgcc cacaccactt ctcttcgaac aaatgaccga 1200

cctaaacttg caccctgtac atgtttatgg aatgacggag acatacggcc caatcaccaa 1260

gggctaccat ttgccagaat gggacgagct ccctcttaag gacaaatacc agaggatggc 1320

tcgccaaggc cacggcttta ttacaagtct cccagctcgg gtcattaaga ccgaagtgcc 1380

tgcagggact atcacagatg ttcgcaagga tggccaggag attggcgaga ttgtttttgt 1440

tggaaatatc tgtgcccggg gatactacaa ggaccccgag gcgactcgga aattatttgc 1500

aggaggtgtg ttgcactctg gggacttggc tgtctggcat cccgacggtg caattcagat 1560

tcttgatcgt gcaaaggata tcatcatcag tggtatgtgt ggcgagcggc tcaactgtca 1620

agggaccaaa ttgctaacaa tgattaggcg gcgaaaacat ctcctccgtt gcactggagt 1680

ctatgctagt tatgcatcct gatattttgg aggctggtgt cgttgctgtt cctgactccc 1740

actggggcga acgacctaaa gcctttgtta cagtgaagga aggcaagagc ctacaaggct 1800

cagacttgat agactgggct cgaaatacta gcggaatcag caagttcatg attccacgag 1860

aagtggaagt ggttactgaa ctacctaaga ccagcactgg caaaatccgg aagaacatcc 1920

tacgtgactg ggtgaagggt ccgagagaag cttag 1955

<210> 10
<211> 1898
<212> DNA
<213> Penicillium chrysogenum

<400> 10
atgagcctgc aagactcaca tcacagtttc cagccaaagc cttcctataa acagggtctt 60

ctggagccgg cacttcggaa ctggacaatt ggggatctac tccagcgtca agccgagcaa 120

tttccaggca acattgcgat ttcctgcccc ggcacgtcaa actccatcac ctatcgccaa 180

ttgaacgacc gtaccaagct tcttggaaaa gctctgatag caagtggaat ctccgttgga 240

gatcgcgttg gcatctttgc aggaaatgtt ttggaatatg tggaggttgc gctggcaact 300

gcgcgcattg gagcaatcat cgttctgttg aacacctttt acacaacgga ggaaatcaaa 360

agagcccttc ggtttactgg tcagtacata cttggtacaa ttgggaactc aatttttaat 420

ctcaatatat agggtgttca ttgctattca tcactgagag ccttggaaaa cgcagtctgt 480

tgccatgcat cgaccaactc aacgagataa ttgaaaacca gaaaagcgag ttcccagatc 540

tccgaagcat ggttcttctc tcaggacagt gttcagaatc gtccaatctg cagtcctatg 600

cggacttttt caatatccca cccaccggtg cgaaggccgc ttctgcttgc aaggcagcag 660

aaacacaagt tactccagag acgatctgta acttccaatt cactagtggc accactggga 720

tgccgaaggc ggtgatgctc actcacttgt aagccttcct aggcacgacg tgttcgcata 780

caaactgact cgagggaata gtaacgtcgt caacaatggt tttctaatag gtgaccgagt 840

gtgcctcagt ccaaatgata ccatatgttg tccgtggcct ttgttccact cgtctggatt 900

tgttgttggc ctgataacat cactgtgtca tggtgcaact ctcgttctac cgtcaccagt 960

attcgatccc gctgccacgg cacgagcact gatctccgaa aggtgtactg ggcttcaagg 1020

cgtcccaacc atgttcgctg ctgttctcga gtggtaccgt caacgtggta ctcgtccgcc 1080

acctcttcga acaggaatta tcggtggatc accagtttcc ccggctcttc tcagagagct 1140

ccagcatgaa tttgctctgg aagacctggg tattgcatat ggtgggttcc caatcgctct 1200

tcaactttgc ttcgcacagt actaatctga ttctgctagg tatgactgaa acctcgccac 1260

tgagcttcct gtcaaaaggc tttgagccag aagggaccca ctcatggatg gaaatcttac 1320

cacacacgac ggcaaagata gtggatgcgc aaggaaccat tgtccccatt ggaagtcctg 1380

gagaactgtg cgtctcagga tatctcttgc agcaaggata ctatcaaaac cccgggaaga 1440

cgagtgaggc catgcgcgtg catgaggatg gagttctctg gatacattca ggcgacgaag 1500

caatcatgga tgagcaagga cgttgtcgga tcagcggcag gatcaaggat accatcattc 1560

gcggcggaga gaacatatat ccagcagaga tcgaggatcg cctgaatgag cacccggcaa 1620

tctcgatgtc ggcggtagtg ggtatccaag atgcaaagta cggagaggca gtggcggcat 1680

tcttgcaact caaacatggc gaaaacccct gtgcacaggc acacatctcg gaatgggttc 1740

agcaaaccct ggggaaacat aaggtaccaa cactcgtatt tcacctcggt gttgatggcg 1800

ttcctggcga tttccctaag acagcaagcg ggaagatcaa gaaggttgac ctggtggcca 1860

ttggaactca gcttgtccgc ggcgcgggta aactgtag 1898

<210> 11
<211> 2309
<212> DNA
<213> Penicillium chrysogenum

<400> 11
   atgtctccgc acccccaaac aacaacccac tcacactccc ttggcgaccc agaaacattc 60
   tggtcccatc acgcggccag actccactgg caccacaaac cttcacgtgc cctaacacgg 120
   aagacaaagt tcctagcgag cggaaccaag catgagcact ggtcgtggtt cgctgacggc 180
   gaaatctcca caacctacaa ttgtgtcgac cggcatgtgc tcgcaggcca tggcgacaat 240
   gttgcgatcg tatgggaatc gccagttacc ggtgtaacag agaagtatac ttatgcccgt 300
   ctactcgatg aggtcgaggt gcttgccggt gtgctgaggg aggaaggggt acagaaaggc 360
   gatgttgtta ttatctacag tatgcaaacc aacaacttcg tcatcgtaca agtggggaac 420
   cattaaagac agccaaaatt gaccacgtat cgcatctagt gccaatgatc cccgcagccc 480
   taatagctgc gttggcaata acccgcctag gcgcaatcca cgccgccgtc ttcggtggct 540
   tcgccccgca agctctagca cagcgcattg aggccgcgcg tccgagagct ataatgacag 600
   cgtcatgtgg aatagagggg tccaaggggc ccattcccta ccgaccgctc gtcgagggcg 660
   ccctcaaggc aagcagcttc aagccgagca aggtcattgt ctggcagcgt gaccaactcc 720
   gctggaatca gcccgataag cgcggtggcc agcgtaattg gcagagactc gttaagagtg 780
   cacggttccg tggggttaag gctggacctg tccctgttaa gagtacggac gcgctttata 840
   ttatttatac aagtggtgag atattctaag aacggaatcc ggttctcggt ctggtgttat 900
   ttatactcac atgtttccac aggaacaact ggcctcccca aaggcgtcct ccgcgaagca 960
   ggcgggcatg ccgtgggcct ggaactctcc ataaagtccc tcttcgggat aacaggtccc 1020
   ggggacacaa tgttctgcgc atcagacatc ggctgggtcg tcggacactc gtacatcctc 1080
   tatgcaccgc tgctagtcgg cgccacaacc gtcctgttcg aaggtaaacc cgtcggcacg 1140
   ccggatgcag gaacgttctg gcgtatcatc gagaaacaca atgtgaatgc gctcttcacg 1200
   gcaccaactg ctatgcgagc catccggaaa gacgacccgg acaaccagct tatcggcgag 1260
   gtcgggaagc ggggaggatt gaaatcgctc cgtgcgcttt tcctcgccgg tgagcgtagc 1320
   gagccgggta ttgttagctc ataccaaggc ctgcttggta agtatgccgc ggccggtgct 1380
   cttgttattg ataactggtg gtcctccgaa tccggatccc cgattacggg tgttgccttg 1440
   gatgggaggg ctgcccttgc tagtccggca tccggaccag atcatcctcc cctcacgccc 1500
   cgtcccggct cagcaggctt cccgatgcct gggtttgatg tccgcattgt tgatgacgag 1560
   ggtaacgaag tcccgcgcgg aacaatgggc aatattgtta tggctccgcc gctcgccccg 1620
   tcagcgttca caaccctttt cgaggacgaa gaacggtttt accgtgggta tcttaagcgc 1680
   tttaatggcc gctggattga taccggtgat gcggggatga ttgataagga tggctacgtg 1740
   catattatgt cgcggagtga cgatatcatc aacgttgccg cgcataggtt cagcaccggt 1800
   aaggaattgg attttcctcg ctccccaggg ccacgctctg aggatacatg atctgaccca 1860
   tttctatagg atccattgaa caagcaatcc tctcgcatcc gtcgattagc gaagcaagcg 1920
   tcgtcggcat ccccgacccg ctaaaaggcc acctcccctt cgcatttatc cagcccaaag 1980
   ctggcgcagt ccctggagaa aatgggcatc ttccggcaac gccgagcccg cagttatttg 2040
   ccgaggttaa cgcgctggtc cgcgagcaga tcggggcgat tgcgtcgctg ggtgggatta 2100
   tacagggtcg ggggatgatt cccaagacgc ggagtgggaa gacgcttagg cgtgttctta 2160
   gggagcttgt tgaaattgga gtgaaggggg agtatgcggc ggaggttagt gtgccgccta 2220
   ctgttgagga tgcggaggtc gtttctgttg cgaggaagag ggttagagag tattttgagg 2280
   caaagggggt tgggaagtcg aagctttag 2309

<210> 12
<211> 2160
<212> DNA
<213> Penicillium chrysogenum

<400> 12

atgcctcata tcgacgtaaa cgttgagcca gaggccaagg agctctggcg gccctcctct 60

cctgagacaa ctcagatcta cgatttcatg actaaagtga acaagaagta tggcttgtca 120

ctgaacaact acgatacact ctggaaatgg agtgtctctg aaccagccca gttctgggag 180

gaaatctggc actataccaa gatcaacgcc cacaccccat accaacacgt gagtaattcc 240

catttcgagc ctggatccac caccatcttg gacatgagat atccgatcct aatccgatct 300

acataggtcc ttgattcaaa agatgtgcta tttcctagac cttcattctt cgaagggagc 360

actttgaact tcgccgagaa tctgttgtat cccgccacct cgccggatga agattccgta 420

gcagtgattg gcgccactga agtagcccgc gaattcgtct cgtggaagga gctacgagaa 480

cgagttcgcc tgtgcgcaaa cgctctgaaa gaagcaggtc tgaagaccgg cgatcgggtc 540

gctggcttcg tgggcaatca tgctaatacc gtcgtggcaa tgttggccgc tgcttcaatc 600

ggggcctttt ggactggcgt gtcacctgac actggtgtgc atgctgtttt ggagcgtttg 660

aagcagatcc aacctaagat cctctttgcg gacaatggat ccttctacaa tggcaaggtg 720

catagctcgc atgccaaagt acgggagatt gtctctgaat tgccggacct tgagcttttg 780

gtgcttctta ttaccacccc ggaacttgaa gttaacctgg atgaccttcg acctgcaaat 840

gggaaagcaa aagtttatgg tgatttcgtg tctgaggtca aggactctca ggcgccatta 900

gagtttgcaa gcttgagacc agatcatccc gtctatatcc tgtattcgtc tggtacaacg 960

ggggctccca aacctatcgt tcatgggtct ctgggcacat tgttgcagca caagaaagaa 1020

catcttcttc actgtgacat tcgaccaggt gatcgcttgt tctacttcac aacgacaacc 1080

tggatgatgt ggcactggct ggtgtcgggc ttggccagcg gagcaacaat cgtcctgtac 1140

gatggatcac ccttccgacc ctttgatgct gaaggcggga agggagaaat ggccatgccg 1200

cggctgatcg aagagcttca aattacccat ttcggtacat cggccaaata tctctctctc 1260

ctcgaacaag ccgctttgaa cccgcgcaag catgcccata gaccagtgtc gttgaagacc 1320

ctaagggcca tcttcagtac tggatccccg ttggcaccat caacattcga atacgtctat 1380

tcctctttcc accacgacat catgcttggt tccatcaccg gtggcacaga tatcctctct 1440

ctgttcgctt ccggctgccc cattctccca gtttacaaag gagaaattca atgccgttct 1500

ttggggatgg acatctcagt gtttgactat gctggcaaag acattagtgc aactggtgaa 1560

cccggtgatc ttgtctgcgt cacacccttc ccggcccaac cagtcatgtt ctggcccccg 1620

ggaccaacag gcttggagaa gtatcggaaa agttacttcg atgtgtttgg gtcctcggtc 1680

tggcaccatg gggactacgt gcgtttgaac ccacaaaccg gaggtgtggt catgctaggc 1740

cgcagcgacg gcgtactgaa gccatccgga gtgcgatttg gaagtgccga gatatacaat 1800

attctactta agcatttcgc cgaagatgtg gaagactccc tttgcattgg tcggaggcga 1860

gatggcgttg acaccgatga gacggtcgtt ctgtttgtcc gactttcttc tgccaacgag 1920

tccggtatgc cggcggatct cgccgcacgc atccaggcca ctattcggaa ggagcttagc 1980

ccccgccatg tgcctggcat tatcgatgca tgtcccgaga ttccggtgac ttcaaatggc 2040

aagaaggtcg aaaatgctgt gaagcagatt ttgtgtgggc ttaacatcaa gattggtgcg 2100

agtgttgcga atgcttcatg tcttgattgg tatagaaatt gggcctcgga aaccccttag 2160

<210> 13
<211> 2340
<212> DNA
<213> Penicillium chrysogenum

<400> 13
atgtcggacg gcccaattca gcctcccaag cccgcagtgg taagaatcac cgacctccag 60

accgagatga ccagacccgt gtcgcactgg tgaccgaagt atcatgggct aactggtgat 120

ataggtgcat gaggcacacg aggtcgacac tttccacgtc cccaaggcgt tccacgataa 180

gcacccctcc ggcactcaca tcaaggacat tgaggagtac aagaagcttt acgaagaatc 240

aatcaagagc cccgacacct tctgggcacg catggcccgc gagctcctca catttgacaa 300

ggactttgaa accacacatc acggctcgtt tgagaacggc gacaatgcct ggttcgtcga 360

gggtcggttg aacgcatcgt tcaactgtgt cgatcgccat gccctcaaga acccagataa 420

ggtcgccatt atttatgagg ccgacgagcc caacgagggc cgtaagatca cctacggaga 480

gctgatgcgc gaggtgtccc gggttgcctg gactctgaag gagcgtggcg tcaagaaggg 540

cgacacggtc ggtatctacc tgcccatgat tcccgaggcc gtaatcgctt tcctggcttg 600

ctcgcgtatt ggtgccgtgc actccgttgt cttcgctggt ttctcttccg actccctccg 660

ggaccgtgtc ctggacgcct cctccaaggt catcattacc tccgacgagg gcaagcgcgg 720

tggcaagatc attggcacta agaagattgt ggacgaggcc atgaagcagt gccccgatgt 780

gcacaccgtg ctggtgtaca agcgcaccgg tgccgaggtg ccctggaccg ctggccgtga 840

catttggtgg cacgaggagg tcgagaagta ccccaactac ctcgcccctg agtcggtcag 900

ctccgaggat cctctcttcc tgttgtacac ctccggttcc accggtaagc ccaagggtgt 960

tatgcacacc actgccggtt acctgctcgg tgcggccatg actggaaagt acgtgtttga 1020

tatccacgac gatgatcgct acttctgcgg tggtgatgtc ggttggatta caggtcacac 1080

ctatgtcgtg tacgcccctc tattgcttgg ctgcgccacc gtcgtgttcg agagtacccc 1140

cgcctaccct aacttctcgc gctactggga tgtcattgac aagcacgacg tcacacaatt 1200

ctacgttgca cccaccgctc tgcgtctgct gaagcgcgct ggagatgagc acattcacca 1260

caagatgcac agtctgcgta ttcttggctc cgtcggagag cccattgccg cggaagtctg 1320

gaagtggtac ttcgagtgtg ttggcaagga ggaagctcac atctgcgacg ttcgttcccc 1380

cttacccttg gaccttttgg aataacttct aatttttgga tctgtagaca tactggcaaa 1440

ccgagaccgg ctcacatgtc atcacccctc tcggcggtat cacccccacc aagcccggca 1500

gtgcctccct acccttcttc ggtatcgagc ctgccattat cgaccccgtc tccggagagg 1560

agattgtcgg caatgatgtc gagggtgttt tggccttcaa gcagccgtgg cccagcatgg 1620

cccgcaccgt gtggggtgcc cacaagcgtt acatggacac ttacttgaac gtgtacaagg 1680

gttactacgt aagacgcttc gcagcctgcc ttgcagggtt gatactaact catatatagt 1740

tcaccggaga tggtgctggc cgtgaccacg acggctatta ctggatccgc ggtcgtgttg 1800

acgatgtcgt caacgtttct ggacaccgtc tgtccaccgc tgagatcgag gccgctcttc 1860

tcgagcaccg taagtccaac cacagtatct gccaaaaatt gcaactgagc ccaaactaac 1920

tatgaacagc ttccgttgcc gaggctgctg tcgttggtat tgccgacgag ctgaccggtc 1980

aggctgtcaa tgcctttgtc tctctcaagg agggcaagcc cacagaacag atcagcaagg 2040

accttgcaat gcaagttcgc aagtccattg gtcccttcgc cgcccccaag gctgtcttcg 2100

tcgtggatga cctccccaag acccgcagtg gcaagatcat gcgccgaatc ctccggaaga 2160

ttctcagtgg cgaggaggac agcctcggtg atacatcaac ggtaagcatc atctctcagc 2220

aagatagtac ccgcaatcgt atcgtccgaa caatagctaa cgaaatattc ttcacagctc 2280

tccgacccca gtgtcgtgga caagatcata gaaaccgtcc acagtgctcg ccagaagtaa 2340

<210> 14
<211> 2025
<212> DNA
<213> Penicillium chrysogenum

<400> 14
atggtttttt tacctccaaa ggagtccggt caattggacc caattcccga caatattcca 60

atcagcgagt ttatgctcaa tgagagatat ggacgagtgc gacacgccag ctcccgggac 120

ccatacacct gtggtattac cgggaagtca tactcgtcga aagaggtagc caatcgcgtc 180

gactcgctgg ctcgtagtct atcaaaggaa tttggttggg cgccgaatga agggtcagaa 240

tgggataaga cattggccgt gtttgccctc aacactgtag gtggagtata ctgtatcaag 300

aagaatggca gggcgcttat tctttcgttt tcccgattag atcgattcct tacccctatt 360

ctgggccgtt cacagactgg gcggtgttct cactcccgcc aacgcatcat actccgccgc 420

cgagctgacg catcagctgc ttgattccaa ggccaaggcc cttgtgactt gtgttcctct 480

cctctccatc tcactggaag ctgcagccaa agctggtctc ccgaagaaca gaatctactt 540

actcgatgta cctgagcagc ttcttggcgg agtcaagcct ccagcaggat acaagtccgt 600

ttccgaactg acccaggctg ggaagtctct cccgccagtg gatgaattgc gatggagcgc 660

gggtgaaggt gcccggcgaa cagcatttgt gtgctactca agtggaacgt ctggattgcc 720

ggtaagcagt gtcctgacaa tttgaatgtc actttcgcta acaatgtgct gcaaaacaga 780

aaggagtcat gatctcacac cgcaacgtga tcgccaatac ccttcagatc aaggcgtttg 840

agcagaacta ccgggatggt gggggcacaa agcctgcgag tactgaggtt gctcttggtc 900

tccttccgca gagccatatc tatgctcttg tggtcattgg ccatgctggg gcataccgag 960

gcgaccaaac aatcgttctc cccaaattcg aattgaaatc ctacctgaac gccatccaac 1020

agtacaagat cagtgcgctg ttcctggtga gcatgtcttt cccgatgaga aggggccgtc 1080

gaatactaac cgcaaggact taggtacctc cgatcatcat tcacatgctg ggcactcaag 1140

acgtgtgctc caagtatgac ctgagttccg tgacgtctct gttcacggga gcggcacccc 1200

tgggtatgga gacagctgcc gatttcctca aactctaccc gaacattttg atccgccaag 1260

gatacggtac gtggtaatcg atgcttctaa cctgcgcagc actgacagat ttgcgcacag 1320

gtctgacaga gacatgcacg gtcgtaagct cgacccaccc gcacgatatc tggctaggtt 1380

catccggcgc tttgctccct ggagtcgagg cacgaattgt gacgcctgaa aacaaggaaa 1440

tcacaacgta cgactcaccg ggcgaattgg tggtccgaag cccaagcgtc gtcctgggct 1500

atttgaacaa cgaaaaagcc accgcagaga catttgtgga cggatggatg cgtacgggag 1560

acgaggctgt catccgtaga agcccgaagg gcatcgagca cgtgtttatt gtcgatcgga 1620

tcaaggagtt gatcaaggtc aaggttcgtg atcgcatcgg ctgatgggaa tattcgcaat 1680

ctaatacgtc attcctaggg tctgcaagtc gcgcctgccg aactcgaagc ccatatcctc 1740

gcccaccccg atgtctcgga ctgtgctgtc atcgctattc cggatgatcg tgcaggagaa 1800

gtacccaagg ccattgttgt gaagtccgcc agcgcaggat cggacgaatc tgtctcccag 1860

gctctcgtga agtatgttga ggaccacaag gctcgtcaca agtggttgaa gggaggtatc 1920

agatttgtgg atgccattcc caagagcccg agtggtaaga ttcttcgtcg gttgatccgt 1980

gaccaagaga aggaggcacg gagaaaggct ggtagcaaga tctaa 2025

<210> 15
<211> 2249
<212> DNA
<213> Penicillium chrysogenum

<400> 15
atgagctaca gcggaattgt aaccgggctt ggcctaagag gtcgtgtatc ctgtcgaaga 60

tctgcgttcg gaggaaagag aatccttcag caaccatcct tggttaagag ggggctactg 120

acagagagct atgcacgggg gccatcaggt gtgagtatcc gctgtccaag aacaatggcg 180

tgcagtatgt ctctgacagt agtactttca atatagccgc ccttgatcga gtccacagtt 240

ggcgaacact tcgccaagat tgtcgcggaa tgcggagata gaacagcgtg agaaccgtgt 300

cgcaagattg gacaattgag acttgagata ctaactatac tgtcaagggt tgtctcaaga 360

catcagaatg atcgggcaac ctatgcatcc ttggacacaa gaagcaattc tctcgctcgg 420

gggctggagt cacttggtgt tgggaagggt gagcgggttg gagtcatgct tgggaactcg 480

atggaatacg ctgtggtatg ttgggtgacc gcggaaatgg gtaggaagaa aagagcgagg 540

ctaatatccg tgacaaggca acatacgcgt tattcaaact tggtgccgtt ttggtaggtc 600

agctctctca ttgccacggg atcattgggt ctaataatcc aataggttcc gctcaacccg 660

tcattcaata ctgcacaagt gattgcggcc ctgggtcacc tggaagccag tcatctccta 720

atcagcacag agtccaatct gccacgaaag aagccacgca gcaatgtccc tttactcaat 780

gaccttgttg aagacctcca caagtcgaag ttggagtccg cacctgtccc gtctctgaga 840

aatatcatta tggtcgacaa ctccgaaggc cgcgtggata tatcgtccta taagagcttg 900

acgcagtatg catctattat gtcccagctt actgtagacg gcagcccgct tcctcctcga 960

aacctttcgc cggatgaaac tgtgaatatc cagttcacct caggaaccac atcaatgccc 1020

aaggctgcat gcctcaccca tcgctccatt ctgaataatg gctctcagat tggtgaccgc 1080

atgcttctca cgccgaatga tattatctgt tgcccaccgc cgttgtttca ttgcttcggc 1140

agtatattag gctatatggc gacagcgacc cacggctcag ccatcgtctt ccccagtgaa 1200

tccttcaatg ctcgtgctgc gttggaggct gtccaagaag agaaatgtac cgcgctatat 1260

ggtgtaccga caatgtttct cgaggagtta agcttgatcg agacaggaga agtctccagc 1320

gagggattcc aacatcttcg cacgggcatc gctgccggaa gcagtattcc tgccgagata 1380

atgaaaaagc tgcataaggt actgaatttg accgaattga ctatttgtta tggaatgacg 1440

gaaacaagtc ccgtctcggc tatgacaacg actgatgatc ccattgataa gcggatctac 1500

tctgtcggaa agctcatgcc acatgtcgag gcaaagattg tgaatccggt ggacaagaac 1560

gtaattctac ccatagagag acggggtgag ttggcagtca gcgggtacct gctgatgaag 1620

gaatactggg ccgatccaga aaagacagcg gaagtgatga ttccggatga ttctggcaaa 1680

gtgtggatgc atgtgagttc aattttctat ttgttgtctc gagtaaagct gtttcctctg 1740

accccggggt tttagactgg agatgaagct tccatgtcac cagatggata tatcactatc 1800

accggacgga tcaaagatct gataatccgt ggtggcgaga acatacaccc cctggagatt 1860

gagaattgct tgttagccaa caatggcgtt gcagacgtgt cggtcgtcgg cgtccctgac 1920

gtgagatatg gcgaggcagt agctgctttc attgttcctc gggaccacag ctcaaataca 1980

gtcactgctg aggagatcca gcaatgggtg cgtgaaaagc tcagcaacca tcttagtaag 2040

tcgtatccta ggcaactgct ttacgtcgtt tttgaactat cctgacaatt gtcgcggggg 2100

ctgagctgat gttttgtctg tttacagttc cgaagcatgt cttctttttg ggccctttgg 2160

agtctttccc caagacagca agcgggaaaa ttcagaagtt caagctcaga gagacggcca 2220

ttgcactttt gaccaagtgc gctgcatga 2249

<210> 16
<211> 1960
<212> DNA
<213> Penicillium chrysogenum

<400> 16
atgtctagca gtttactctc ccttctttcc cgttcgaatg cgcgcgtctg ctttactccg 60

cggcgaacgc ccctcagcgc aagactgttg tcgactctcc cgaacacacc tatctttcgc 120

gcactccaga accatgaccc tgaaagagtg gctgttgttc atagcgtctc ctctcgatca 180

ttcacctacg gtagtttaat tgccgacatc gtccgcgcta aggatgacct ggaacaaaag 240

gctgcgaaag cgcagggcca attggctggc gagcgggtcg ccttcctcgc ggagaacagc 300

tatgattacg taggtactgt tgtcttggac cccattgaag acgatatata aaatactaat 360

agacttatag tgacactgct ggcgatattt gctagtgatg ccattgcgct cccgctctcg 420

ccatcattcc cgactgggga gctgaagtat atcctggata attcacaggc caagatgctg 480

ctgacgactg agaaatatgc cgataaaggt atggaactat tgcgagaggg attggagcgt 540

gagcctctat ttgctatccg caataagctc acggagggcg catcgagcgg cgagagcgta 600

accctgcacg atctgaagca gccttcgtcc ggtgggatga tgttgtatac atctgggact 660

acgaatcgac cagtaggatt agttccctcc ctgcaccgaa ggaactatta ctaactccaa 720

acagaaaggt gttctcatcc cccaatcggc actggccgcg caagcatctt cactactaga 780

agcatggaaa tactcaccgg atgatcgact tcttcacctg ctccccctcc accacatcca 840

cggcgtggta aacgcaatcg tggcaccaat agtagccggc tcctcagtcg agttcatgta 900

ccccttcaac ccggacaaag tctggaaacg tctagcagca cccttcctct catccaacgc 960

ctcaaaacca gccattacat tcctcactgc ggtcccaaca atctacaacc gcctcatggc 1020

caccttcccc aaactaaccc cagagctcca aaacgccgca aaagaaggaa tctccccgca 1080

aaacctccgc ttgaacatct ccggctccgc cgccctcccg accccaacaa aaaccgcctg 1140

gacaaacctc agcaacggca acgtcctcct cgagcgcttc ggcatgaccg aagtcggcat 1200

ggcgattagc tgcggtctcg acgccgcaga ccgggtggac ggcagcgtcg gctggcctct 1260

cccaggcgtc gaagcccgac tcgccgatac cgagacaggt gctgtcatcc cagttgaaga 1320

aaaggaaccc agtggccgag aacgtgaagg cgagatccag ctccgcggcg aaacaatctt 1380

cgaccattac tggggcaacg agaaagccac gcgcgagagc ttcgtgcaga gcgatgatgg 1440

aggcagaccg tggttctgca caggtgatgt cgctacccgt cgggtagttg atggagccgg 1500

tagtggagcg agcggggcat gggctcaggg accgatgtat ttcatacagg gtcggaagag 1560

cgttgacatc atcaagactg ggggggagaa ggttagcgcg ttggaggtgg agagggagtt 1620

actctctctg tatgttgttt ctttctttcg ggtatccacc ttgaactact ttatactgac 1680

gatggatctt agtccccaaa tcaccgaagc tgcagtcgtt ggtctgccgt ctgaacaatg 1740

gggtcagaag atcgctgctg tggttgttct cgcccctgat gcggctgctt caggtcgtaa 1800

tgggcagtct tggggtccga tggatatgcg gcgcgcgctg aagggttccc ttgcttcttt 1860

caagattccg caggagatga aggttttggc ggctattccc aggaatgcga tggggaaggt 1920

taataagaag gcactggtga aggaagtctt tggagtttga 1960

<210> 17
<211> 1701
<212> DNA
<213> Penicillium chrysogenum

<400> 17
atgccaacaa tttaccgctc accatatcca gatctggata tccaatctgt tgatttggtc 60

tcttatctgt tctccaatcc tttcaacacg cccctggacc ggcctatgta catcaatgcc 120

atctcaggag agcaatatac ttttggagat gtggtgcaac gtacacgctc tctgtccaac 180

gggcttcggc aaagtatagg ccttaagcct aacgatgttg tggcattatt cagtcccaac 240

acaatcgatt accctgtggt atgccatgca attgtcggct cgagagcaat cgttgcaccg 300

accagtgcag ctttgacagc acttgagctt aacgctcagc tcaagaccag cggggccaga 360

ttcatcgtcg tccattcaac tctgctggag actgctcaaa aggcagccaa ggggacatct 420

gtcgagaaag tcctcctgat agatggccaa acaccagtca atggtcaacc tacatgcaat 480

tatctcgcca atacatttgc ccccgatgat ctcctcactg tcgatccggc cgaggcagat 540

cgccaaccga cattcatctg cttttcatct ggaacatcag gtgcagcaaa gggtgtcatc 600

acaactcatc agaacatcac ctcgaacctc cagcaatggc gccaacacat gttagaatct 660

ggtcttccct ctcagcgacc aagaagacaa tccgccattg cattcctgcc attcagccac 720

atctatggcc taaacttgtt catgtgccaa tgcttgatat ggggtacaac agtggtcgta 780

atgcccagat tcgaccttga tttatacctc agttgcatcc agaaataccg accagacgag 840

ttggcactgg tgccgcccat tgccctgatg ttggttaaag acccacgagt ctccaaatac 900

gatctaagca gcgtgcgcaa gatcatgtct gccgccgcac cccttactat tgaattatcc 960

tccgccctcg aagccaagtt cacggagatc tgcaagacag aggtattctg tacccagtca 1020

tggggtctga cagagacctc accaatggca acagccgtcc cgaatgaccg catggacaag 1080

cggaacacgg gcgtgggctg tatcgcgccg aacatgcagc tccgctttgt ggatcccgaa 1140

tctatgaaag acgcagccgt caaacctgac gggtccactg agcctgcgga gatctggtgt 1200

cgtggtccga acgtggtgat ggggtattac aacaatgaga aagcgacgaa ggaagctttc 1260

cacgtggacg aagatggaac gaggtggttc cgaacgggtg atatcggcac catcgatgga 1320

gatgggtacg tcaccatcca ggatcggatc aaggagatga tcaagtacaa gggactgcag 1380

gtcattccta gcgagttgga gggaaagctg gtcgaccacc ctgacgtgga ggatgcggct 1440

gtaactggaa tgtgggtgga tgacatggct actgagctgc cagttgggtt tgtggttttg 1500

agtccgcagg ctaaagacag ggatcagaag gctgtccttg atggaatcca cgcgtggctg 1560

aatgagcgca ttgctaacca taagcgtctg cgtgggggaa tccatgtctt gtcgcagatt 1620

cctaagtcgc cgagtgggaa gattctgcgt agacagctga gggatttgct gaagagtcaa 1680

gcgcctaaag ctcgtctgtg a 1701

<210> 18
<211> 1991

<212>  DNA
<213>  Penicillium chrysogenum

<400>  18
atggcggccc caagaagtgt acagcgcttg cagcagacgt tgtcgcacgt acagcctcct      60

attaatccac agctatctct tgtcgcaggc ccgacggagc cacagctgct ggatattacc     120

ttgggtgagc tgctaacact acaagcactg cagtatggga gaattgagtg cttggtcttc     180

ccctggactg gtgcacgatg gacgtatggc cagctgaaag atgagaccga ccgtctagct     240

aggggaatgt tggcttcggg tattcaaaaa ggcgatcgcg tcggaatcat ggcgggcaat     300

tgtgaacaat acatctcggt gttcttcgcg gccgcacgag ttggggctat tttggtcgtt     360

ttgaacaaca cgtacacccc ctccgagctg tattacgccc taaatcacag tggtgggtga     420

tatccacttc ccgtatttat tgactccata ctaatctcgg cgttttgttc tattttcatt     480

cagaatgccg aatgcttttc atgacaccac gaattggacg tcataatctc gaggaggtcc     540

ttacccaact tggccctaat cctaaaaaag cggcgacctc ggaaacactg gaggaaattg     600

tcattctccg cgaaagctat aacaacttcc ccacctatca cgatgtcatg gaacgcggcc     660

tgtcccaggc tgcacatgtg ctccaggacc gcgaggccga gttacagccg gatgatgtct     720

gcaatctgca attcacaagc ggttcaacag gcaatcctaa agctgcaatg ttgacacatc     780

agtatgtgga ccatgtacaa caaactcaat cttaaagata tagctaactc ctccgcagca     840

acttggttaa caactcgcgc tttattggtg atcgcatgaa tctcacctct tttgatgttc     900

tgtgttgccc gccgccattg ttccactgct tcgggctggt cctgggcatg cttgcggtag     960

tcacccatgg cgccaagatt gtcttcccta gcgaaacctt cgaccccaag gcggttttgc    1020

atgccatctc cgacgagaaa tgcactgctc tacacggtgt cccaaccatg tttgaggcga    1080

tcctttccgt tcccaagccc tcaaactttg atacctcaaa cctccgcaca ggcattattg    1140

ccggggcccc agtcccacgg ccactgatga agcggctctt tgcagaatta aatatgaccg    1200

aatacactag tagctatggt acagtcccct tctcccttgg tctgctgatc aagtgctaac    1260

ctaagcctct aggtctcacg gaggcctcgc ccacttgctt caatgctttc accaccgaca    1320

gcatccacac aagattgacg accgtcggaa aggttctgcc ccacgctcgc gcgaagatta    1380

tcgataccca tgggaacatc gttcccgttg gtcagcgtgg cgagctttgc atggccggct    1440

accagctcac caagggatac tggaataacc cggagaagac ggcagagaca ttcataaccg    1500

atgccgaggg tgtcaaatgg ctgaagactg gtgacgaagc ttccttcaac gccgaagggt    1560

actgcactat caccggtcgc ttcaaagata tcatcattcg tggtaagttc gaacagccgg    1620

acattgatag cgatttctaa ttctgaggtc tgataggcgg cgaaaacatt tatcctctcg 1680

aaatcgaaga acgattggcc gcacatccgt caattgagtt agcatctgtg attggtattg 1740

ccgaccccaa atacggcgag gttgttgggg cttttatcgc gatcgcttcg ggagcagaac 1800

gtccgaccga cgatgaactg cgcacatgga ctcgggatac tttgggtcga cacaaagccc 1860

cacagcatgt gtttgtattc ggagaagagg gggcttcgag cacggttccc gtgacgggaa 1920

gcgggaaagt tcgcaaggtg gagttacggc aaatggcgat ggcggtgctg gccgagcgcc 1980

ggaaggcatg a 1991

<210> 19
<211> 1696
<212> DNA
<213> Penicillium chrysogenum

<400> 19
atgccaatcc cttccctacc cctcttcgcc gcagcggagc agcatgcgcg ccagaatccc 60

gagaagatcg cggtcattga caccacgaaa caacaatctt tcacgttcgt tcaattgctg 120

gccgacgcag cagcgttaag gaagcgcata attgagcaat tgggattaac ggacgacttg 180

gaagagcgac gcattgcgtt cttagtcccg aatggatatg actacgtcgc tacgcaatgg 240

gccgtgtggg cggcaggagg tgtctgcgtt ccactttgta ggttgaaacc atgaagcgca 300

atgcgaaaca cttgtattca atactgacat ctgctaggca tcagccaccc agtgaaagaa 360

ctgctataca ccatcggaga ttcggacccc tcattgatta tcgtgcatcc ggagttcgag 420

aagatcgcac cctcgctgcg cgaaggctac accaccgata taccattcat agggctggag 480

ccattctctc gaaatgagac cccgacttta ccatctttct ccccgccgtt tgcgctcacg 540

cgccgcgccc tgatgatcta tacctccgga accacatcaa accccaaagg ctgcgtgaca 600

acacatgaga acatcacctt ccaagcgagt tgtctggtta aagcctggga gtacaaaccc 660

tcagaccatc ttatccacgt gctgccactc caccacgtcc acggcatcat caatggccta 720

gctgccagtt tcctaagtgg tgcgacagtg gagatgcatc cgaagtttga cccgaaggta 780

atctggggcc ggtggcagga ccacagctcc tcgaccctgt tcatggccgt gccgaccatt 840

tactcccgtc tgaatgacta cttcgatgct catatccgcg gcacggaaca agaagatgca 900

gcgcgtgccg gagctcgtgc tctacgactt gtcgtcagtg gatccgcggc cctcccgacg 960

cccattaaag aaaaattcgc cgaaatcact ggtcaggttc tattggagcg gtatggaatg 1020

accgagatcg gcatggcgct cagttgcggg ctggaagtcc aaaagcgaat cgacggtagt 1080

gtggggtggc cgttgcccgg cgtggaggta cgactgaccg ataaagaaac cggtcggatt 1140

gtggacggag tggatgagga cggcatgatc gaggtcaagg gcgggaatgt cttccgcgag 1200

tactggcgga aaccggaagc cacggcctcg gagttcacgg cggatggatg gttcaagacg 1260

ggtgatgtgg ctaaacgcga tccaagcggt gcatacttca tccaaggccg tgcttctgtt 1320

gacctgatca aatccggtgg atataagatt tcggcgttgg aagttgagcg caagatgctg 1380

gccattcatg cgattcagga agttgcagtg gtcgggttga ccgatcaaga atggggacag 1440

cgagtcgctg cagtcgtcaa gttccgagaa ggggtatgtt tgcgttgtgt ccagggaaac 1500

tgttctattt gcatggctaa cactgataca gactgcaccg atggaacttc ctactctacg 1560

ggctgagctc aagaatgaga tggctcctta taagatccca accgttctga aggttgtgga 1620

tgggatcgag cgcaatgcta tgggcaaggt gaataagaag gtcattgtac aaaagtactg 1680

gccggataag gcatga 1696

<210> 20
<211> 1922
<212> DNA
<213> Penicillium chrysogenum

<400> 20
atgattttgg aaccagcaga gagagtcctg cttccaacaa aggacctatt gtcctatatt 60

tttgatgacc caccatatga ccaagaccag ccagtgagtg acaaacaacg cttcaacctt 120

atcatcaacc gggtgccaaa ttgacacggg cagatctaca tcgacgtcaa caatccctct 180

cggtcgatct cctgtaacca ggcgaaaagg ctagttcgtc agctcatcgc cggtctacgg 240

gaatctggtc tccaaaaagg cgattgcgtt ctcattcatt ctttcaatga tgtacggagg 300

ttccaccatg cagttggatg aggttacagg atgctgactt attctagata aattatagca 360

ttcttgtgtt ggccattatt ggagctggag gatgtttcac gggctcgaac ccatcttaca 420

ctcctcatga gctcgctcat catatcaagg cctccgaaag caggttcctt ttctccgagc 480

ccgagatctt cgattctctc atgcgtgcca ctgaggggaa gaatataccc aaacaaaaca 540

tttggatctt cgacaatctg ggacaatcta tcccggctgg aatgcgatcg tggaaacagc 600

tattggaagt cggggaggag gattgggtgc gattcaacga tctcgaaacc tgccaacaga 660

ctacagcggc gaggttgttt agcagtggca ccactggtct acccaaagct gtgacaatca 720

cacaccacaa cctcattgga cagcatgagc ttgttcacgg tgtcaatccg cgctcttacc 780

ctgttagttc acccgttatc ggcttggaaa gagccttgac aagttaatgc gatggcatca 840

ctgacccatg acactgttga atagatttca cgggtcattg cagtacccat ctttcatgca 900

tccgcagctc cagtgtcgca tatttcaaca ctaaaagctg gctcagttgc atatatgatg 960

cgtcggttcg acctcgagac atacctcact acagtcgaaa agtacaacgt caccgacctt 1020

gctgttgttc caccaatcgt gatagcaatt ctgatgtctc ctctttctcg aaacaaaccc 1080

tatttgcgaa aggctcggat ggcagggtgc ggagcggctc ctttggacaa ggacgttcaa 1140

gccaggttcc gctccctgat gggcaacgat accccattca ctcaggtatg ggggatgact 1200

gaaacatcgt gtgtggctac aatgtttgcc cacccagagc acgatgatac gggctctgtc 1260

ggtcggctca tcccaaacct cgaagccaag tgcgttgtat cgaactcccc tgttggatgt 1320

gtagcgttgt ctcaagtctg caactaatat cgatttcccc cagactcatt gatgaagacg 1380

gaaagaatat ctcagccttc ggcgtccgtg gcgagttgtg tgtccgaggt ccgacagtga 1440

caccggggta cttcaacaat cccgaggcta attctgaggc ctttgacgct gagggttggt 1500

tcaaaacggg cgatatagcc tattgtgacc agcgcactcg caaatggtat attgtggatc 1560

gaaggaagga actgatcaaa gtccgcggct tccaggtcgc tccacccgag ctagaggcgg 1620

tgctcttgtc gcatccgcag attgtggacg cagccgtcat cgggataacc ttcgctgatg 1680

ccgatataga gtatccacga gcctatgtgg ttcgacgatc cggcgatcaa ggagccaagc 1740

taacggaagc tgagattcaa gaatacgtct taagccgact tgccaagtac aaagcactca 1800

caggtggcgt gaaatttgtt ggtgcgattg cacggaaccc aagtggcaag atcttgaaga 1860

gggtattgcg cgaagatgct aggaaagaaa tcgaggctgg actcctgaaa gccaagcttt 1920

aa 1922

<210> 21
<211> 2079
<212> DNA
<213> Penicillium chrysogenum

<400> 21
   atggtgtaca ctgctgaacc ccttgagtac ccagaaggga caactctacc cgagcttttg 60
   ctcgagcgca acgtgaacaa tgttccacct gatatgcctg ctataattga cggggtttct 120
   ggggcaactg tgtacaacta tagcagcttc cgagccactg tgagaagagt cgccaactac 180
   ctctgccagg atatcaacct tcctcgtggt gcagttgtcg gaattcttgc ggcgaacagt 240
   gtgagaggaa ttacccgatc aacacacgga tgaccgctat aattgataca ctaacatgtc 300
   ctgggtacgt gcagaataat tacccagtca ttgtgcatgg gattctggct gcaggaggtg 360
   ttgtctcggc atttaatcct ctacatcagg cgcaggttag ttatgaaagc aagatgacag 420
   ctctgtcgat tcggtgtttg cttattcagt gccgctagga aattctccat tatttgcgca 480
   ttgcaaggcc caaagtcgtc ctagtagatc agcacttgac taaaacactc acagacggac 540
   tttcacgagc aaagctggac tactcaccag atctttatgt cctatcgccc gatagctccc 600
   atcctgctcc ctggatccat tttgatcttg gacatatcat agccaccggc gcagaaaagt 660
   ccgacactgt ggagccacga tctaatacga caagaagcga cctagccttc ttatgcttca 720
   gctctggtac cacagggccg atgaaagggg tctatctcac ccatgaaaac atcatcacaa 780
   atatttttca acaccgacag cgtctaccgg aaatgttcca aaaccaccag acggttgcag 840
   ccctcatcac gccatttttc cacattctcg gcttgggtgt atttgtctgt caatatattt 900
   gccaagtaag ggtcctccgt gttcccgtat ccctgtatca caccaacccc ggtaataatt 960
   ttcgctaggg catcccgatt gtagttttcc ccaactttga actgccattg ctgctggacg 1020
   ccatcccgcg agaccggagt aagtcatgat aaatattttc gctgcaaggg agcgccagct 1080
   gactgagttg ctagttaccc atatcaacat tgtacccccc cattgctctg agactgcttc 1140
   aagcaaccac caccagcacc acggatttct catctctgcg tggcctcatc aatgctgctg 1200
   cacccttgaa ggagattgtt tccagcgaac tgtcgcgacg catgggatgc aatatcactc 1260
   agtggtatgg gatgactgag gctagcccaa gcgtgatttc ccagagggag gacgaagtag 1320
   agataacagg cacagtggga cgacttctcc caggcatgtc gatgaaaatt gtggattcgg 1380
   aaggggaagg tatgacgctt cgattcgttc agtagtttat cctaaccgcg agatagagtg 1440
   tgagccgaat aagcctggtg agcttctgat acagggttcg aatctaatgt ctggctacgt 1500
   tcaaaacacg gagtcaaatg acgcgtttat cgacggttac ttcaaaactg gcgatattgg 1560
   atatgttaac gaggcgggct atgtattcct tgttggtcgt tccaaagagc ttatcaaggt 1620
   gaaagggtac gttgtttcgc gctgtgtgtt cggatctttg ggaacaaggc taacgctatg 1680
   ttctgggaac tctagacacc aggtggctcc agccgacttg gagtctatct tgctcagcca 1740
   tcccaaagtg tgcgatgccg ccgtcaaagg agtctacttc ccagaacagg aaactgaata 1800
   tccagcggcg tacatcacaa tcgactccgc gcaaactgag ccagggcagc tacaagccga 1860
   ggttgaggct ttcgtgaata accaagtagc caaatacaag tggctacgga gcggcgtgca 1920
   catcattccc gcgattcctc gcaagtaagt caccaaactc gtggggccca ccctaaaaca 1980
   cacctgtctg acttgtttcg acacaatagt gcatctggta aaattctccg acatcagctc 2040
   cccgatctga aaacgagcca gcgacaaact aaaatatag 2079

<210> 22
<211> 5001
<212> DNA
<213> Penicillium chrysogenum

<400> 22
atggcggact tatcaagatc ttcgtatgtt ttatcagatt tatcattttc gtctttcttt 60

tccttctttt tggggtgtcc atatgaagtt tcttccgttc ctgactgtca aacagactta 120

tagaaaagct tctatacctc cgcagtgagg cctcccatcc agattccttc caccaactct 180

ccgccttatt gtcctcacac accggtcctt ggcccatccg acacctaatc tcaacattcc 240

aggccatcta caagtccatt cccgaatccc tcaaggccaa taacaatgaa tcaatcaccc 300

gtctctgttc acaagtcaaa gcagccacca ccagcgttgc agaattaata gggacagact 360

cccgcgccgg tcttatcgac gctaacaccg gacggcatct gacacataat gccattcggc 420

agttcttgga gaatttcaag attccagtgg gactctcccg ccatggaaag cctcgtatcg 480

ctgtgatctt gcccaacggc cctctgatgg cagttgctgt ccttgctttc gtcaacagat 540

acaccattgt gcccatgact acgaacacgg tggcagagca actgcagaca gatatagaaa 600

actcacaagc tgatgctgtt gtcgcgctgg acgcggacat tggcaagttg caactcgaca 660

acggtacccg gccagtgttt ggcattgagc agcttgaaga cttgactttc cgcgtggtct 720

ctgcaggaaa tacttctact gcatacgacc atccccccaa ttctggtgat gacattgcca 780

ttatcctgtt cactagtggt accagtggca ccaagaagct ggttcccatc acaacttaca 840

acctcatcgc tggtaccatc gctactatag agtctgtgga actgtctgag acagatacat 900

gccttaacat gatgccactg aaccatgtgt atgtccccct cccgaattaa atataggaag 960

gcaactaaca gcatcagcgg tggcatcatg cgcagtatat tctcccccat cctcgccggc 1020

ggtgctacga tatgttgtcc ctcgtttgac cccagcatgt tctgggatgc tgtacaagcc 1080

cctcacacca aaccaacttg gtactacgcc acacccacga tgcatcaaat gattcttgcc 1140

gaggccgagc accgtcctga tgccgtaaaa caaagcgcca tccagtttat ctgcaacgct 1200

ggtggtggtc tccctccaac ccttgcagtg cagcttcata gcactttcca ctgcgttgtc 1260

ctccccagct acggcatgac tgaatgtatg cccattgctg cgccgccgag ggactataag 1320

cttgatcgcc ctggaacatc gggaaggatt gttggccctg aggttgcaat tctgactgaa 1380

agtggcaacc ctgtcacgca aaatggcatg ctcgggcaca tttgcattcg tggatcaccc 1440

gccttcgagg gctatctgac ccctggcggc aagattgata ccagtgcgtt caatgagtct 1500

ggctggtttg atactggtga tcttggacac ctagatgatg acaattacct gtacatcact 1560

ggccgcagca aggaggttat caaccgcggt ggtgaaatta tctctcctgt cgaagtggaa 1620

aatgcggtcc tgacctcggc caaggacccc gagtccccac tgtatggtcg cgtgactgag 1680

acattggcct tttctgtccc agacgaggtc ctgcaggaag tcgtcggtgt ggttatcgtc 1740

acgcctccag gcttcactag gccggatctt cgccagatac acgaagcgct gcagcctatt 1800

atacatcagc ccaagtggcc tgctctggtg gtgtacatgg acggcgtgcc gaaggcaaac 1860

aacaagattc agcgcattaa gctcgcggac cgcttgtcgt tggatacctt aaccacaact 1920

acgcccctgg cgagccggca ctacgaagct gtctgtccac ccactggtgc tccgctcagt 1980

gcattgattc cgaagaagcc atgtgtcatc gatgaaaaaa tgattcgttc agtcctcagc 2040

aagaaggcaa acacacctga cgtgcacgtg caaataaacc cgcgcgacgg gctcgcgcag 2100

gttgttttgt ttgtcgagaa cctcgacgat gaccatgtga cacctgccga gctccacgac 2160

cagctagatg gctatcttgt acctagccgt atcattcccc tgaaagggcc catgcctgtc 2220

gatttctacg gaaaacctga tcaggccgct atcaatgagg caatccacgc ccgaaactcc 2280

gatggcgacc tgtcacccat tcagcggcgc gtgcgtggaa tcttcgctgt caccttgtct 2340

tgttcaccag aggaagtatc cagcggtacc gacttttttg ctgctggagg tgatagtctt 2400

agtgctggcc gtatggtctc tcaacttcgt cgggaatttg gtattttctt ggccggtgac 2460

attctcttcc accacagcac cgttggagca atagagcaga agattatcga agctgttgac 2520

gttaaggctg ccaagggcga agacggcgga gttgaattgc cgggttgcga gaagacatac 2580

agcagcacca acccaattat cttggttctg catctgttcc cgactgtgtt cttcttccct 2640

atgaagcgtg ccttccaatg gatggtgttc gcatacgtcg tggccgagtg ctccaatcga 2700

tttccgattc gtgagaacct catcgctcgt ttgatgctgg ttgttttcgc cgtgatgtct 2760

gctcgcttat gctcacaaat tgtgtctccc attctcgcta tcatcttcaa gtggcttgtc 2820

atcggtcgct acaaggaagg catgtcccca atgtggggcc catatcatac gcgctggtgg 2880

ttgacccaga aagcgacgca ggtgtgtgga aaggggctct tcaacaacta taattggtct 2940

cgcatcctat tctaccgttt gatgggagca aagattggaa agaatgttac cgtgtctgcg 3000

tccgccaagc ttggtgagta tgatctcatt gagatcgggg acaatgttgt tctggatacc 3060

tgcgtgtgtc gcccgttcgc cgtggagcgc aacacttcta tgctgctgaa gcgcattcgc 3120

attggtaagg actcgtctgt cggcatcaag tctattgtcg ctcctggtgc cgatataccc 3180

gagaggacct gcattggtcc caactcgtcg agctgggaac tccaagacgc cgatgagtcc 3240

aaccgccaac tgctaacgtc tcagattcct aagcctcact ggctctggat tctctttatt 3300

gttgaaccga tcaagttggt cacttggacc gctactcgta ttacctggat ggccggtctt 3360

attcctatgg ttctccagtt tcctacccca gcggccgaca tgttccggtc taccctagac 3420

tggtacacca gtgaccaccg gattgcatat cacattaccg cacggatctg tcgtgcggtt 3480

ggtggcccga ttgttctttt tattgcggta ttggtcatca agttcttcct ggatctcatc 3540

tgtggcaagc ccaagcctgg tcccgcctca aaacagacga ctcgccaaaa gattcggagt 3600

gctgtgctcg cacagattct acccgcaggt gacattcacg agctcactcg cctgacaggt 3660

cgtcactacg aatttgtctc gatggcagtc cgtgcccttg gtggtaaggt tggcaagcat 3720

gtctactggc caagtgttgg tccagtcacg gtcgactttg atctcattga ggttggtaac 3780

gatgttgtat ttggttcccg ctccactctg gtcacatccg atggctacgg ccgggaccgc 3840

atcgtgattg gcgatggcac tatggtgggc gatcgtgtcg tcgctttgcc gggcgctacc 3900

atcggccgtc aagccatgat cggttccggt gctctgcttc gccgtaacgg tgaatatccc 3960

tccaacactg tctggactgg aagcaagggt ggtgaggcca tacagttccc aagttccacc 4020

tcgaccacca cttcgaccgc tccaaccatc attggggatg gtagcagcag ccccaacagc 4080

agcggcagtg aagacgagag gtcacccact gagaagatgc ccactgagaa gcagacctac 4140

tatggcagta aggacaagac tgttacccag atcgctgagc aggatatcga tacctgcaag 4200

ccattcggtc gcgccttcta ccgccatgag gccaactact acgtcctccg catgtggcaa 4260

attgtgatct actctacttt tgcagtcatt gtcactactg tctattggct cctcactgtc 4320

ttgttctccc tttttgctct ccgcactgtc ctccagtaca gtgacgccgc tggtttcaag 4380

caaggcgcct ggcgcccatt cgttctatac ggcactctcg cctcaatcct ctccgtcatc 4440

actgtcgctc aagtcgttct agccttcgcc atcatcctct gcattaaatg ggcagtcgtg 4500

ggccgccgca aggaaggatc ctaccactgg gacaagagca gttacaacca gcgctggcaa 4560

ttcctccttt cctgcgagac cctgatcaaa gactgctatg acggagttgg tctcctgcct 4620

atgatctccg gctctgccta catttcctgg tactaccgcc tcttgggcgc aaaaattgga 4680

aaggattgtg cgatacacgc caatggtagt cccagcatct tctttaccga gcctgatctc 4740

ctcacccttg gtgaccgggt tgctgtcgac gatgccagct tggtctgcca cttgaactca 4800

cgtggtggat tcgagttgca taccttgaag gttggggatc ggagtattct gcgcgctgga 4860

tcccgtttga tgtccggcgc ttcaatgggt caggatgctt gtcttttgga acatactctg 4920

gtcttgtcgg gtgaccatgt tgaggatgga gatacgctcc agggatggcc cgctgaaggg 4980

tttgaatgga aacgggttta a 5001

<210> 23
<211> 2158
<212> DNA
<213> Penicillium chrysogenum

<400> 23
atggacattt tatccgaagt cggtgcgcct gctgtagcta tggcatcggc cttgtcggtg 60

gccgccggcg cttatctgaa cgcgaagctt gccattgcca ctgatttgcg aacaatccac 120

agcgacaagg ctgccgcaaa gcggttaagt gagcgcatcg ctaagctcga tggctcaaca 180

accatctaca agatgctcga gcgggcggtg gaagtggaag gacgagctac cacagatgcg 240

ctctggtttg agcagaagac ctggtcgtat ggccaattga aagattgtta gtatcgctcc 300

tccatcgaag aggaaatatc acctaggtac atctgattgc tgatacgact gtttgcagtg 360

gtcgacagaa tggctgctct tttgaagagt cgtgatatca atcccggtga tacagtgggt 420

gtctttacca ccaactcacc ggagatggtc atgaccgtat acgctctatc aaaactgggc 480

gctgtggcag cgatgatcaa taccaacctg cgaggtttgt ggatcatcca attccaaccc 540

aggggaatag actgttgaca ctagtgacag acgatacttt cacccattgc gtcaacgtct 600

ccggctccaa attgatcatc tcaaccgcag acctgtgtca acacgtctgc gtcgacctac 660

cacacttcac actcagcctg ggttcattcg aaggtgccga agcaggcgca attgagccca 720

tcacttctgg aaccctccag caattctccc cattaggact tgcagcagcc aagcgtagcc 780

caaaggacct ctccctcctg atctacacat caggaacaac cgggaaaccc aaagcctgcg 840

cgatccgcaa catgctcact ctaatcacct ccaacccaca ctcggcagat gtaagcaacc 900

actcaaagta ccacccattc cgcgtgtatt cccctctacc attattccac ggtacagcct 960

tcttcaccgg cctttgtgcc gccatcggaa acggaggcac attatgcctt ggacgtaaat 1020

tctccgcctc gaaattctgg aaggaagtgc acgattcagg tgcaacccgc atcctataca 1080

tcggcgagct atgccggtac cttctcgcga caccgccatc accctacgac caggaccaca 1140

agtgcattgt ggcaagcggt aacgggctgc gcggggagat ctgggagaag ttccgtgagc 1200

gcttcaacgt ccctgagatc cgcgagttct accgctcaac cgagggagtt gccaaattcg 1260

acaaccacgg tgttggagct tggggcgctg gcaaggtcgg gttttctggg cctatccgcc 1320

ggttcttgga agatgacact ttcatcgtca agtacgatac taacaccgag atgccgtacc 1380

gggatcctgc cactgggttc tgtgtgaggg ctgcgcttgg acaggaggga gaggcaattg 1440

gtcgcgtccg ggaccgcggt atgctgattg agtatctggg taatgagggt gcgacggaag 1500

agaagcttct gcgtgatgtg ttccagaagg gagatctgtt ccagcggact ggtgatctgg 1560

ttgttcaaga tgaatctggg tgggtgaggt tccaggaccg ggttggtgac actttccggt 1620

ggaagggtga gaacgttagt gcgggtgaga ttcggaccca tatttgccgg attgagggtg 1680

tgcatgatgc tgtggtatat ggtgtgaagc taagcgggta tgtctcgagg ctgtctttct 1740

ctcttttgtt cggcaagatt ggattgctga cggtctatac taggtatgat ggtcaagctg 1800

gagccgctgg tattacacta gagtcaccag aggttgagac tgagttgatg tcgaccttat 1860

gcaaggcgct caagaagaag ggtgttcctt catacgctct gcctcgactg gttcgactca 1920

ctgagaagta agcattccct tgattgcgat aatttggccg cgctgctaat caagcgtaat 1980

ctgcagggtt gctacaggtg tcactttcaa gcaggctaaa ggtgatttag cgaagaaggg 2040

ctggaatcct cgccaggact cgggcggcga tattttgtac tggttgaacg gcacaaagta 2100

tcagaagctg gaagagcaaa gttgggcgga gattgagagt ggtaaagcca aaatctga 2158

<210> 24
<211> 1959
<212> DNA
<213> Penicillium chrysogenum

<400> 24
atgtcttcaa acaatatgct cccccaggag gcttactctg acttgctgag tttcacattc 60

gatggcccaa agccatacaa ccagaatcaa cctctgttta ttgatgctga ggatccatcc 120

cgatcattta cagctgccca atttcgacaa ctagtccgga ccttgattgc tggtctgaaa 180

gcccataacg tccagccagg ggattgtgta ttgctacact tgggaaatag cgtgagtgaa 240

agagagaaaa aaaaaataat cagaaatcag aaatccaata ttggactgaa gatttccgga 300

aaataaaaga aacaatgctg actcagacct gtcaaatgca tcagatacta tatcccgccc 360

tcttcttcgg tataattggc gccggcggtg tctatatggg atcaaacccc cgaagccacc 420

ctcaagaact cgaccacgtc ctcagcctcg ccgaacccaa actgatcctc acaacacgtg 480

atgccctccc ctccgtcctc gatgcctccg ctggccgagg cattcatcca gcgcaggtct 540

gcctcgtgga cgagcacgcc atcgaccact gcgcacaact attcctgtgg tacgaactgg 600

gatactcatc ggcgggacaa ttctgcgcca tgaatggcga cgcgcgtcac aacaatttcg 660

ccaatttact ctgcttcggt gaaagcgact ggctgaagtt cgcggaccct gtcgtcgcac 720

aagccacgcc tgcggccatg tatccgacaa gcggtacagg cggacttccc aaagcagcaa 780

tactgtccca ctatgcgctt gtatcacagc accgtactat ctactacgaa gtgccgcatc 840

cagttagccg gctcatctcg ctgcccatgt tccatctgtt tggcgcgctt tggacacatc 900

tgttccctgt ccgatacggg cacccgctgt ttgtcatgcc gcgctttgag gtcaatgatt 960

tcttggccgc tgtccacaag taccagatct ccgagactta cctggtcccg gctatcatcc 1020

acgctatcaa ccagtcatcc gtgcccatcg gtgacctatt gaaatcgctg cgctatgttg 1080

gcgttgctgg cgctcctatt gatggtcact ccatgcagca gttccgcagc catatcaacc 1140

ctatgggata tgcgtgccag atctggggga tgacagaggt cggtgtgaca ttccaaactc 1200

gttggggcca gcagggtgac cctggaagta ttggacggtg tattgctggg tatgaggccc 1260

gcttggtgca accagatgga aagaccgtgc agggtgataa ctgctcgggt gaactctacg 1320

tccgtgggcc gggtctgctc actgcataca agggccgcac agacgcattg gagccacatg 1380

gatggttccg gactggagac atagcgtatg tcaagcaggg ccagtattat attgttggac 1440

ggacgaagga gcttatcaaa gtacgagggt aagtctatta gtaccagttt ctttatcaaa 1500

atgaaaatgc taatatcatt caacagatgg caagtagccc cagctgaagt tgaaggcgtc 1560

ctcctccaac acccgggaat ccttgatgcc ggcgtgatag gcgtgaacaa ggatggtgtg 1620

ggcgaagtcc cacgcgcttt cgtcgtgcgc tctcgcgacc catccgtgcg ccgcctaacc 1680

ggcgaacaag tttacaatta ttcacgccag cagctggcga ggtacaaagc cctcgatgga 1740

ggcgttgtat tcgtggaaga gatcccgcgc actgcaagcg gaaagatcca gcgcttcaag 1800

ctgtcccaga tgaactcgta ccgggagatg gtggcgtccc tgctttcccg attcgaaggc 1860

gagggctctg ctgcagccac cgcggcccga cgtggactac ccgctggggc tgagatgtcg 1920

ccagttagtc tcattcctga gggacgggtt gcggtctaa 1959

<210> 25
<211> 1947
<212> DNA
<213> Penicillium chrysogenum

<400> 25
atggaagcca gactcgcgct ccttcaaggc tccaaagagc ctgccttgtg gtttgaaaca 60

ctgggaaact tcatcgacaa gcaagcttcc cagtatgaag accgagtagc tgctattttc 120

ccttggcaat ccgtccgact ctcataccgt caattggcag agcggagcaa gattcttgca 180

aaggcaatgc tagagatggg actacgcaag ggagactgtg ttggagtcat ggcagggaac 240

tgttatcagt atattgaggt tttcctcggt ggcgggcgca ttggatgccc tgttgttgtt 300

ctgaacaata cctacattcc aagggaacta atgagtgctg tgcaaaagag ctgtaagttc 360

tggatagttc aattggtcaa ataccacaag ttctgatcac tcatctcata tatttagcgt 420

gcaaattggt tttcgttgcc tccgatattg gttcgcggag tctctcggct catatcaacg 480

cgctttgtgg tgatcaatcg agaaaccctg ctctgccaga actccgccgg gttgtcaact 540

ttgggaataa ggatccaagt agcaccggag tcgaaatgca gtcttacagc gcctttacct 600

ctggcgccca atctgtcttc atgaaagact caatgctgct ccgtgcagag aagtccgtgg 660

agcccgaaga tgtcctcaac ctacaattca cctccggtat tatcctttaa tcaccttcaa 720

aatgttccgc catctaatat ccaagtagga accactggct ccccaaaagc tgccatgcta 780

agccacatgt gagtaaattt ccccaatatc caaagataat ctcactaaga agcgcagtaa 840

cctcctcaac aacgcccgct ttgtcggcga cgcaatgcac ctcaccccaa cagacataat 900

ctgctgcccc ccacctctat tccactgctt tggtttagtc ttaggcttcc tcgcgtcttt 960

cgtacacggc agctcaatag tatttccatc agacttcttc gacgtccgga aattcgtttc 1020

aacaatcctc tccgaaaacg caaccgtcct cctcggcgtt ccgacaatgt acatttcgga 1080

gctggaggtc ctttcaaaaa gtcgacagcg tccccaccat ctgaggacag gtcttgcgtc 1140

gggctcggct gtttcacaag gtcttatgaa tcagctcaga gaggaaatgg gcgtacagaa 1200

gatgttgatc gcgtatggca tgaccgaaac cagtccagtc acattcatta cctctattga 1260

ggatggggat gagaagggga catcgaccgt gggtagagtc ctgccgcata cgggtgccaa 1320

ggttgttggg aaagggggcg agattgttcg gagaggggag aggggagagt tgtgtactag 1380

tgggtttgcc ctgcagaagg ggtattgggg gaatgaggag aagacccggg aggtcatgag 1440

ggtggatggg gatggagttt tgtggatgca tacaggggat gaggctttta ttgatgaaga 1500

tggatatgct catattacgg ggaggatcaa ggatctcatt atacggggta agttctggta 1560

taacctgatt ttgatctggt ttgctaacca ctaatggtgc taggcgggga aaatatattt 1620

ccgagagaga ttgaagagcg acttacgttg catccgtcta tcagcgaagc cagtgtggtt 1680

ggaatcaaag atgaaagata cggcgaggtt gttggatgct ttttgaaaat ggctgaagga 1740

tatcccaaag tccctgatac agaggtgaaa cagtgggtag gcgagaagct tggtcgacac 1800

aagacgccac agtatacatt ctggattgga gatgaaaaag tgggaaccga tttccccaag 1860

actgggagcg ggaaacatca gaagcacatc atgcgggacc ttggaaattt gctagtgcag 1920

agggatacgg cgagagccaa gctatag 1947

<210> 26
<211> 1650
<212> DNA
<213> Penicillium chrysogenum

<400> 26
atggcaacta gacttgttcc caaaggttgc cctctagtca ggactaccat acaagttttg 60

gaacatcgca aacatcgtat ccatttcgct tcttacttag ggaatcatgc tcaatttcgt 120

cggagtcacg ctttggcacg tgccaatggt cccttgttgg ggattcgaat tcttgatctg 180

acgcgtgttc tcgcagtaag tctctgatat tgatacattg ggagtgttca atgcttgaca 240

catatcaggg accattctgt acacagattc tcgctaatta tggggctgag gttctcaagg 300

ttgagcatcc aaaagggggt gtatgtcctt attttctttt cggttctgtt aagtggacat 360

gatactcacc aagtatatat catgcaggat gacactcgct tgtggcggac ggcagccgag 420

aaacatgttt ggaaatccac tggcaaagat atgtctacat atttttgtgc gatcaatcgg 480

aacaagctat ctatgacttt gaacctcaaa caagagaaag gccgggagat tctctttcga 540

cttgtcaaag aggccgatgt ggtgtcagta atcctccgtc tcgaggtaaa accctccgta 600

ctgaattatc atagagtgga aaattttgtt ccaggaaaga tggatgaaat gggcattgga 660

tatgaaaagc tacgggaaat aaatccatcg atcatatatg caagcgtatc tggtcagtcc 720

tcgatcatcg tgaaggcata tgcaaccttg gaactgtgag tgctgacttc aattcaggat 780

acggcgctag tggtccatat tctcatcgcg caggttacga cgccattgcc gctgccgaag 840

caggtatgct atacatcact ggcgagcgca atggaccccc aacacgacct ggtctcggcc 900

tcactgatat gagcaccggt ctatacctgc acggggcgat acttgccgca ctttatgctc 960

gcagggatac aggtcggggc cagaagattg atacgtcact atttgagagc caggtgtccc 1020

tgctttctaa tgtggctatg tcatggctga atggcgggga acatgcaaag cgttggggga 1080

cagagcatcc gagtattgtc ccctaccaag cttttaagac aaaggacggt cacttggttc 1140

taggcgctac gaacaaccgc cagttccaaa ctctttgtcg gttggtgaag ctatcggagt 1200

tggcgactga cccacgattt gtggacaaca gctctcgtgt ccaaaatcgg gctgagctaa 1260

aagagatact tgagccaatc atcagaagga agccgacgaa agtctggctg tcagacttgg 1320

atggtagtgg actgccctat gggcctgtga acacgattga agaagtgttc tcgcacccac 1380

aaaccgctgc gagggatatg gttcacagcc ttccccacga agcgtcggaa tcagaggaga 1440

tcagactact aggtttgtta ctcataaggg cctgtgtctg tatatactga cgccgagaat 1500

tattcaggga cccctgtcaa gtttagtggg acgaagcccc aaattcgacg atcgccgcca 1560

agtctaggag agcataccaa tttcacactc gagagaatgg gattctcagc gaaggagata 1620

gaggtaatgc gagaacaggg tattgtgtga 1650

<210> 27
<211> 1649
<212> DNA
<213> Penicillium chrysogenum

<400> 27
atgacggtcg cgctcaggtt tcaggttgct gcccgtcgat cgggcttgtc ctggggtagc 60

aatgccctgc ggcgcacaat accaggacga agctactcca gtgcagttgc agctgacggg 120

tccctgcctt tggctggtat tcgggtgctt gatatgacac gcgttttggc tggtgtaagt 180

acatctctcg agacgctact gtgtgtctac aatttgagct gacatgttct agccctattg 240

tactcaaawa ctaggtgatc tagggtatgt gttgagctta agagctatga aagagctgca 300

atgtatgatt tctacgatac taacaacacg aaacatagag cggatgttat caaggtcgaa 360

caccctgtgc gcggcgacga tactcgtgct tggggtccgc cgtatgcgaa gtacgaagat 420

gcttccagaa agggtcctgg cgagagcgcc tactacctcg ctgtacgagc cttatcttaa 480

gtttgtgaaa cacattatca tggcactaat cacggatata ggtcaaccga aataagaaat 540

ctctcggtct atcattccaa cacaagtcag gtgtcgagat tctacacaag cttgcaaagg 600

aatgtgatgt cctagtcgag aactatctcc cagggggact taaaaaatac ggcatggact 660

atgaaacgct gcgggaaatc aaccccaagt tgatctatgc cagtatcacc gggtatggcc 720

agacgggccc atatagcaac cgtgcaggct acgacgtcat ggtggaagcc gagatggggt 780

tgatgcacat caccggcgct cgggatggtg cgccagtcaa ggttggtgtt gccgttaccg 840

atctgaccac cggactatac acatcaaatg ccatcatggc cgctttgatt gctcggggtc 900

ggaccggtag aggccagcat atcgatgcct gtttgagtga ttgccaggtc gctacactat 960

cgaacctggc aagctctgca cttatcagtg ggcaaaagga ttccggccgt tggggcacag 1020

cacaccgtaa gcagcgaaca gtctcttgtg ccgggtgtaa taattatcaa actgacagca 1080

tccagcatcc attgtaccct accggagcta caaaaccagc gacggtgata ttctcttcgg 1140

cggtggcaac gataagctgt ttggcgtgtt gtgtgatcga ctgggcttct ctgagtggaa 1200

gactgacgcc cgcttcgtca ctaacagtga ccgagtgcag catcgtgcgg agatagatgg 1260

tatgatcgaa agcaccacta tgcagaagac cacgcaggag tggttggata tattcgaggg 1320

tagcggtatg ccttatgcgg cggtcaacga cattcaaggg accctgaatc acaagcacgg 1380

tgagtctgtc catttccttc acatgtgaca aaagaacggt gctgacccta ccaacagtct 1440

tggcacgaga tatggtgacg gaagttgagc accctgcatg tggccccatc aagttggtca 1500

acacgcctat caagtactcc gaagcgacac cgggtgtgcg cacgcctcct cctactctag 1560

gccagcatac ggatgagatc ttaggtagtg tcctcgatta tggggaggca gatatcgctc 1620

gcttgaagga tgagggagtc gtgtcctga 1649

<210> 28
<211> 1185
<212> DNA
<213> Penicillium chrysogenum

<400> 28
   atgtcaccat tgaaaggaat caaagtcatt gagctagcag gcctagcccc aggtacgcga 60
   tcaatctaag aaacgtcatt accacaacta acaaccactc caggcccatt cacaggtctc 120
   ctgctagccg acaacggcgc ctcagtcctc cgcatcgacc gacccaaatc cataagtggc 180
   gaccaactaa cccgcaataa aacctccata acactcgatt tacgcgataa aacctcccta 240
   aacatcctcc tccgcctcct cacagcagcg gacatcctaa tcgacccctt ccgcccaggc 300
   gtgctcgaac gcctcggcct ctccccaaca gaagtcctcc taaagcacaa tccccgccta 360
   atcgtcgcgc gtatgactgg cttccgccgc gacgggaaat acaaagatat ggccggccac 420
   gacataaact acatcgccgt ctcaggcgtg ctgtccatgc tcggccgcac aggggaaaag 480
   ccccacgccc cgggaaacat cctcggcgac ttcgccggcg gcggcgcagt ctgtttccag 540
   ggaattctac ttgcgctcat ctcccgctct catactggtc gcggccaggt cgtcgaggca 600
   aatatggtgg atggctcggc gtaccttgct acgttcccgc ggttagggag gaacactccc 660
   gcgtggtcgg agccgcgtgg cgagaacctg cttgatggtg ggtgtccgta ctatgatacg 720
   tacgaaacta aggatgccgg gcggtatttc gccgtcgggg ctttggagcc gcagttttat 780
   gcggcgttgg tgcgcgggct agggtttgat atggggcagc ttccgaaaag ggaggatcga 840
   gagcagtggc cggtgctgag ggagatcttt acgaagaggt ttaaggagaa gacgcgcgcg 900
   gagtgggagg ctgtttttga tggcaccgat gcttgtgcta cgcctgtttt ggagcaggcg 960
   gagcttgagg acgatgggta tgagcagagg cctatggtgc atctggttaa tacgcctgcg 1020
   gcaccgattc ccgcggatca gggtggttgg tcgggtgggg gacttttacc tggggatggg 1080
   ggtgttgata cgcttaggga gtgggtggga tgggagcagg gtaaggactt tgaggtaagg 1140
   aaggatgggg ctttcgtggc gatagatggg aagtcaaaga tttga 1185

<210> 29
<211> 1688
<212> DNA
<213> Penicillium chrysogenum

<400> 29
atgccttcgc aagtggcaac atgcctgcgc ctcgcgcgtc agttctccgc caatcctgtc 60

caggtaagtg atttgccaat agctcatggc ccatgtcgct tttcgagccg cggagactga 120

catgtctttt ttaaactagc accaacgttt catggcccgc gcattctcca gcagcgtccg 180

ccgaagtgag atcaacaagg tgtacccctc cgccgaggta gcagtcaagg acatgaagtc 240

caactcgact ctgctcgccg gtggattcgg gttgtctggt gtacctgact cgctgatcaa 300

cgccgtactg gcgaacccct ctatcactgg cctgaccgtg gccagtaaca acgctggtgt 360

ggacggagct ggtctcggtc tgctcctcaa gtctaagcag atcaaaaaga tgattgcttc 420

ttacgtcggc gagaacaaaa ccttcgagcg catgtacctc actggtgaga tcgagttgga 480

gctcagtccc cagggaactc ttgctgaacg atgccgttct ggcggtgcgg gtatccccgc 540

attctacaca cccgccgcct tcggtaccgt ggtgcagacc ggtgcggtgc ccctacgaca 600

caatgctgat ggcacagtcg caaagtacag cgaggcgcgc gacaccaagg tgttcgacgg 660

caagagctac gtcatggagg aggcgatcaa gggtgactac gctttcgtca aggcgtggaa 720

ggccgacaag cttggtaact gccagttccg ctacgcggcc gccaacttca acggtgccat 780

gggccgtaac gccaagatga ccatcgtcga ggctgagcac atcgtcgagc ccggcgagat 840

cgaaccggct gccatccacc tgcccggtat ctacgtcaag cgtgttatcc agagcaccac 900

ccccaagaat atcgagaagt tcaccttcgc caaggaggag ggtgcagaca cctctgctct 960

cggcaagggt gagaccgcca acaagcgcga gcgtatcgtc cgccgtgctg ctaaggagtt 1020

caagaacggc atgtacgcca accttggtat tggtatgccc atgcttgctc ccagctttgt 1080

cgacccctcc gttgaggtga ccctgcaatc tgagaacggt atcctcggtc tcggccctta 1140

ccccaagcag ggccaggagg accctgatct catcaacgcc ggcaaggaga ctgtcactct 1200

caagcccggt gctagctgct tcggcagcga cgagtccttc ggcatgatcc gctccggccg 1260

tatcgacctg accatcctgg gtgccatgca ggtcagcgcc aagggtgact tggccaactg 1320

tacgttttct cacttttcct ctgtttcatg cacataacta acattctcgc gcagggatgc 1380

ttcccggtaa gatcaagggc ttcggcggtg ccatggactt ggtctccaac ccctctgcca 1440

ccaaggtcgt cgtgaccatg gaacacaccg acaagaaggg taaccccaag attgtcaaga 1500

actgcgaatt ccctctgacc ggccctgctt gcgtcagccg tatcatcacc gacctctgcg 1560

tcttcgacgt tgacttcacc cacggcctga ctcttattga gatcgctgac ggtgtcaccg 1620

tcgaggaggt ccaggctaag accgaggccc ccttcaaggt cgccgatgac ctgaagccca 1680

tgctgtaa 1688

<210> 30
<211> 1941
<212> DNA
<213> Penicillium chrysogenum

<400> 30
atgacagctc ttagaatatc tactactttg tcgcgcaaag cgctcactgc ctgtcgacct 60

cgactcttcc tcggagtccg caccagtgtg cgcccttggc agccattcag taccagcacc 120

agcactagat actccataac cacgccatta aaatccctcg ctcctaaggt cgaacgcagc 180

ggttcgaaat tatacaaaga tgccgatgcc gctgtcgccg atatcaagag cggatcgaca 240

atccttagct ctgggttcgg tctttgcgga gttgcaggta cgtggacgaa caaccaacca 300

gtgcaagaag cgaagctgac acatagcata gaaacgctca tcaatgctat gcaccgccgg 360

ggcgctgatc aattgcactc cctgacagcc gtttcgaaca atgccggcgc cgcgggcaag 420

ggtggccttt cgacgctctc gcagaatggc cagatcaacc gtctgatact ttcatacctc 480

gggaataata aggcattgga gaagaaatat ttgacgggac acattgctat tgaactttgt 540

ccgcaaggta ctttggctga acgtctccgt gctgggggtg ctgggattcc ggcgttcttt 600

accccaacag gagtccgtat gtatagatct cctcgaacca tgtttgttgc tgacaagcga 660

agacaccttt atccaggagg gaaagattcc cgttcgtatg gacgagtccg gtaaagttct 720

ggagtctggc aagccgcgcg agacgcggga attcaacggc aagacatacc tcatggaaga 780

agctttgact ggggatgtag ctatcctcag agcctggaaa gcggatgagg ctggtaactg 840

cgtgttccgg tgagtatatg tactagtacg tcctggaaac tggatactga cggtggttag 900

atatactacc aaggcatttg gccctatcat ggccaaggcc gcaaccctga ccattgtgga 960

agcggagaac attgtcccaa tcggctccat tgatcccaat gatgtagact taccgggtat 1020

cttcgtggat cggatcgtcc ctgccacgga cgacaagcac attgaaatca ggaagctgcg 1080

ctcgggcgag accactgtag ctggttctgg aaaagatgag gctcggattc aaagagaact 1140

cattggtcgc cgggcggcca aggagcttaa gccgggattc tatgtgaacc tgggtgttgg 1200

tattcccacg ttggcaccgt cgttcctgcc aaaagatgtg aaggtttgga ttcagtcgga 1260

gaacggcatc ttgggaatgg taagttttgc aaaatctctt ccacaggctg gactaaccat 1320

aaatttgaat ccagggtgat tatccaactg agcaagaact ggatccgtaa gttgttccta 1380

gacgtgttga tccgaactcc cgctaactca gaccagcgat atcatcaatg ccggaaaaga 1440

aaccgtgacg ttggttcctg gagctgctac attcgatagc agtgagtcct tcggtatgat 1500

ccgcggaggt catgtagatg tgtctattct cggcgctctg caagtaagtg ccaacggtga 1560

tttggccaat tatatgatcc ccggcaaggt cttcaagggt atgggaggag caatggattt 1620

gatctcgaac ccggagaaga ccaagatcgt ggtggctacg agccacgtcg ccaaggatgg 1680

gtctcctaag atcgtacaaa agtgcagtct gccgttgact ggtgccaatg ttgtcagcac 1740

tatcatcacg gacctggtaa gtgcaatgat cccagttgat ttaaaccaag gctgacagag 1800

gtacagtgtg tcttccaggt tgaccgagct accggagagc tcacactgac ggaacttgct 1860

ccgggtgttg aggtagaaga ggtgcagagt aagacggatg ccaagttcac cattgcggat 1920

acccttgaga ttatggaatg a 1941

<210> 31
<211> 1278
<212> DNA
<213> Penicillium chrysogenum

<400> 31
atgagattat tatcctcact tgcctcccgg ccccatgccc tgtcgggcgc agttcaacct 60

gcaagacgat cacatctcac atggcgtctt tactccagca caccagctgc taaaaacagg 120

cctctgcctc tcgctggggt tactgtggtc agtctcgagc aagctattgc tgcgcccttt 180

tgcacgcgac agcttgccga tcttggagct cgagtcatca aggtcgaacg gccaggtgtt 240

ggagactttg cacgacacta tgacacgcga gttggtggtc tagcatcaca tttcgtatgg 300

acgaaccgtt ctaaggagag tttggctctg gacctcaaac acgaaaagga ccatgccgtc 360

ctgatgaagc tccttgaaaa ggccgacgtt ctcgttcaga acttggcccc aggagccagt 420

gcgcgactgg gcctttctca cgaggcattg aaaggggaac accctgatct tatagtgtgc 480

gatatatctg gatatgggca agacggtcca tatcgcgaca agaaggccta cgaccttctt 540

attcaaaatg aggctggaat gctctccgtc acgggcactg gaaaagagcc ggtaaaggtt 600

ggtatctcta tcgctgatat tgctgcgggg atgtacgcct acagcaatat tctctcggca 660

ctactgcagc gtggtaaaga tggacaaggt tgtcatattg acatctctat gctggaaagc 720

atggtggaat ggatgggctt ccccatgtac tatgcattcg acaatgcccc gggtccagtc 780

ccagcaggtg cctcgcatgc agccatctac ccttatggac cgttcgaagc tggtgatggc 840

cagtcggtta tgttgggcat tcaaaatgag cgcgaatggg tcagtttctg caacggcgtc 900

ctctcacaac cagatctggc caccgaccct agatttccca gcaactcaca acgaactcaa 960

aatcgggatg agctcaagtc cattattcac gatgcattct cgaagtttac gacaactgaa 1020

acaattgctc ggttggacga agcatctatc gccaacgcta ctgtcaatga catgaaagat 1080

gtatggggcc atgctcagct tcaggcacga gatcgatgga ccaaggttca aacccctgta 1140

ggggctattc cggctctttt gcctcctgga tcaaccaatt cagcgggaag agatggacat 1200

ggcgctcaga tgggttcagt tccacgggtc ggagagcaca atgaggccat ttttgcggag 1260

ctgggtttat caaattga 1278

<210> 32
<211> 1695
<212> DNA
<213> Penicillium chrysogenum

<400> 32
atgggagacc gcactctatt catacctcca catgtgggag ataacgtgtt gccaaatctt 60

cccttttttc acagactatt gcgttatgcg caacgtaagc cttcgcccat tgtggttcgg 120

gacttggttg ccgacgcgga aaagacgtac caccaccttg tttcggacgt gttggcattt 180

cgcaaggtct tggaaagatc catcagccat gaagctcgac gcgatttgaa tgcggacaag 240

gaggtatata ttggattgct tgcaccggga ggatatgaat acaccgtcgg cttcatcgcc 300

atcctggcaa tcggtgcagc agtcgtacct atggctgctg cattaccagc agaagaagcc 360

tcgtacttcc tattgaaggc gcgatgtgta ggtctggtag ccagcacagc cagcgaaaag 420

accgcccagt ccgtggttcg ctatatggga gaaagcaaag gcatgcacat cccctgcata 480

tctccaatcg catctcattt ccgccacacg cttctcccgt cagatgaaat gaccatctcc 540

tccggtccag ttccggatat gaacgccgca gcactggtaa tattcacttc aggcacgaca 600

ggacccccaa agggcgctgt gcagcgtcgc agttatattt ccggaaatgg agaggccgat 660

gcagcctatt accgaatcac agacaaggac acagtcctcc atgtccttcc agtgcatcat 720

gcctccggtg tgggattgac tttcctcccg ttccttgcag ccggcgcatg tattgagttc 780

cgctgtggta gtttcgatac agcgtggacg tgggagcgct ggcgacgggg aggtcttacc 840

tttttctctg gagttcccac catatacatg cgtatgatgc ggtactacga agagaacatt 900

tcgcaccaag ctcctgagat acgtgaccag tatgtcgccg gtgcgcgtca gattagagcg 960

atgctttgtg gtacttcggc tcttccaggg ccagtacaag agttctggca taacattagg 1020

agcaaaccaa ttctcacccg atatggtgcg acggaatttg gtgcagtcat caagactgaa 1080

cttgattccg atggaacgcc gcaaaacagt gttggttgtg ttgctgaggc ggtctcactc 1140

aagcttaccg aggaaggtca gatcttagtg aagtgtcctt atatgttttc caagtatctc 1200

ttcgacgaga aagcgacagc ggatgcacat gacgccgagg ggtacttcaa gacgggggat 1260

attgctcgcc gtgaaggaaa atattatttc atcctaggcc gtgcctcgat cgacatcatc 1320

aagtcaggtg gctacaagat ctccgcattg gatatcgagc gcgagattct gggtctggac 1380

tatgtttctg aggtcatggt tgtgggcgtc gaagatgagg agttcggtca acgagttgct 1440

gctactgtca gcctcaaaca agatcaaaag acaacacgca aaagcctcac aatcgcggag 1500

ctgcgggaag atctgcggag taagatggct gggtataaga tgccaactgt ccttcgtgtg 1560

gtccaaggtg aattgcccaa gtcaggcacg gggaaggtgc agaagaagat attgggaccg 1620

cagttttttc cgcctaacta tcgacagctg cctgaggtgc aagtctggag cagggagaat 1680

aaggcgaggc tatag 1695

<210> 33
<211> 1683
<212> DNA
<213> Penicillium chrysogenum

<400> 33
atgaggaaga ccttgcgtca tggcttggga ttacctactt acatatacat acctagccat 60

cacatagata tccctccaat tgatacttgg gattgtctct tcgaaagaga agacagaccc 120

ttcctagatg accacatcat cttcaagtcg ccgccgggcg aagtccgaac cttcaacgac 180

atccggtccc aagccctaag cttcggcgcc aatctccaag tccaatggga atggcaaaaa 240

ggcgatatcc tcttactctt tgcgccgaat gacatcgatg tccccacgtt gttttgggga 300

tgccattggg ctgggggagt agtctcacca gccaacccgg cattcactgc tgatgagttg 360

aaataccaat tgagtgacac aggggcaaag gcaatggttg tccacacctc gctcttagac 420

actgcactca ccgctgcaaa gcgcattgac tttcctattg cgcacatgtt ggtttttggc 480

cctccctcct cagaagcaga gctgcagcat gtcgagtcca tgttgggagg tggagctcct 540

ggagcaagac gtccgaggat agacccagtc gtcgatacag ccttcctggt atactcgtcc 600

gggactaccg gacggcccaa ggggacaaga ataactcata ccaatcttgt gacgaacctc 660

gtcttgcagg gacgagttga aggtccgcac atgaactgga gacaggatcg gttcctgtca 720

ttcttaccaa cataccacat atatgtacat ttccctcttt tcctgggaat aaagacaata 780

gtaatggaga agttctccgt caaaggtttc ttacacaatg tgaaagccga atcgatcacc 840

cacatctacg ccgtcccacc tgttgtcctg tacctcgcca aagatcccgc aatgacgcgc 900

gaacaactat catcactgag aatggtaacc tcagctgctg caccactagc atcagatctg 960

atccacgctg tgtacgatcg tctgaaggtc cctgtccgcc aggcatacgg actgaccgag 1020

tcgacggctg ttacgcatct ccagagatgg gacgaatggg ataaagcaat gggatcaagc 1080

ggccctctat atcccgcagt cgaaacgaag ttcatcgacg accaagggaa ccccgtaaca 1140

aaaggcgacg gcgaactatg tctccgagga ccgaccctct tcccaggcta ccataacaac 1200

gctgaagcca cggcacggtc tatcaccagc gatggatggt tcaaaaccgg tgatattgga 1260

ttccaagacg aggagggaaa tctatttatc acagatcgat taaaggacct tattaaattc 1320

aagggtttcc aaattccccc ggcggagata gagagtgccc tgcatgaaca tccgcttgtg 1380

catgacgccg ctgtgattgg acttgctgtt gagaagattg cgaccgaggt accggttgct 1440

tatgtggttc ttgagaagac ggataaaccg gccgagcagg ttgcagagga attggtggct 1500

tatgttagtg ggaaactggc tcctcataag aggcttcgtg gtgggattgt cctgatcgac 1560

gagattccca agggtcccgc tgggaagatt ctgaagcggg tgttgaagac tagggcagaa 1620

ggtgtcgatc aagggaaggc tattggtgct tcaatctatg acgatcgtcc ttccaagctg 1680

tga 1683

<210> 34
<211> 1689
<212> DNA
<213> Penicillium chrysogenum

<400> 34
   atgcccgtat cctctaacta ccccctggtc gacatcccag aagtggatct ttggaccttt 60
   ctatttgagc gcaaggacag agcttaccca gatgacaaga ttatctacca agatgccgat 120
   acccaacgtc attacacata caaatcgcta cgtgatgcct cgctagattt cggcaagggc 180
   ctcaaagccc tgtatgagtg gcgcaagggc gatgtactgg ccctcttcac gccaaacagc 240
   atcgatacac ccgtggtgat gtggggcaca ctttgggccg gcggaaccat ctcgcctgca 300
   aacccgggtt acacggttga cgagctggcc ttccagctca agaactccca tgccaagggc 360
   cttgtaacgc aagcctcggt gctgcccgtt gcgcgggaag cggcgaagaa agttggcatg 420
   cctgaagacc gcattatcct gattggggac cagcgtgatc ccgatgcgcg cgtcaagcat 480
   ttcacctctg tgcgcaatat ctctggtgct acccgctacc gcaagcagaa gatcactcct 540
   gcgaaggatg tggctttttt ggtttattct tctgggacca cgggtgtgcc taagggtgtc 600
   atgatatcgc atcggaacat cgttgccaat atcaggcagc agttcattgc tgagggtgaa 660
   atgttaagct ggaatggtgg tcctgatgga aagggtgatc gggtcctggc atttttgccg 720
   ttctatcata tctatggatt gacttgctta attacccagg ctttatacaa gggttatcac 780
   ttgattgtca tgtccaaatt cgacattgaa aagtggtgcg cccacgtgca gaactatcgc 840
   tgctcgttta gttatatcgt ccctcccgtg gtgctattgt taggcaagca cccggtggtc 900
   gacaaatatg acctttcaag tctgcgcatg atgaactcag gcgctgcacc actcactcaa 960
   gagctggtag aagcagtcta ctcccgcatc aaggttggca ttaagcaggg ctacggactc 1020
   agcgagacca gcccgactac acactcccaa cggtgggagg actggcgcga agccatgggt 1080
   tccgtcggcc gcttgatgcc taatatgcaa gccaagtaca tgactatgcc tgaggatggg 1140
   tccgagccta aggaggttgg cgagggcgag gttggcgaat tgtacctgaa aggacctaac 1200
   gttttcctgg gctatcacga gaacccagag gctaccaagg gctgcctgtc tgaggatggg 1260
   tggttccaga ctggtgacgt tggataccag gacgccaagg gtaatttcta cattaccgat 1320
   cgtgtcaagg aacttatcaa gtataagggc ttccaggtgc ctcctgctga gttggaaggg 1380
   tatctggttg acaacgatgc tattgatgat gttgctgtca ttggtattga gagtgagaca 1440
   cacggttccg aggttcctat ggcttgtgtg gttcgcagcg cgaagagcaa gtcttccgga 1500
   acaagcgaga aggatgaagc agcaaggatt atcaaatggc tggatagcaa ggtcgcaagc 1560
   cacaagcgtc tgcgcggtgg cgtccacttt gtggatgaga tccccaagaa tcccagtggg 1620
   aagatcctac gtcggattct gaagcagaag ttcaaggggg cggccgaggc accaaaagcc 1680
   aagctgtaa 1689

<210> 35
<211> 2097
<212> DNA
<213> Penicillium chrysogenum

<400> 35
atgacatctc ttccgcgtaa actatggcag catccagccc ccgagtcgac ccagatgggt 60

cgcttccagc gagacctaga gaaatccacc ggacacaagt tcgactcctt ccatgatatg 120

tacctgtatt cagtcaagaa ccgctcagcg ttctgggaat tctgctggaa atacttccaa 180

ctgatccacg agggctccta cacaaaggta gttgatgaaa ccgctcgaat ggatagcgtg 240

cccgaatggt tccccggtgt acgtctgaac ttcgccgaga acctgctttt ctcacgcatt 300

gctggcgaca aaacagacaa ggaagacgac aagattgctg ttagcgaagt tcgagaaggc 360

gctgcgcatg aggttatcca tctcacttgg ggtgagctcc gacgacggac tggagcgttg 420

gtccaggcta tgaaggccca tggggtcgtt cagggtgatc gtattgctct ctgcgcggcg 480

aatagcatcg atacactatt tgtgttcttg gcgtcaacgg ctctgggtgc tatcttctca 540

tctagttcca cggatatggg tactaaaggc gttttggata gactgcttca gatcaagcct 600

cgctggctgt ttatggacga cctggcggtt tataatggta agaccatcga cttgcgatcg 660

aagattgggg aaatcgtgaa aggcatggag tctgtccctg aatttgaggg agtcgtctca 720

ctacctcggt tccattcgcg gccggcggat atcaactcca tctctaggac caagactctg 780

gcggagttcc tcgaaaaggc gggcggaaat gagaaacttg aatttgaaag agttggattt 840

cgcgatccct tcttggtggt ttactcgtct ggcacgacag gacagccgaa gtgtatcgtc 900

cattctgtgg gcggcgtgct tctaaactcg agcaaggagg gaagattgca tagtgatctc 960

gggcctgact gtgtgacctt acaatatacg acaaccggat ggattatgta tatgagtgct 1020

gtgcagactt tactctttgg ggcacgggtg gtgctgtatg atggaagccc atttatccca 1080

ggtatcactg ctttggttga ccttgcagca caggagaagg tgacccacct tggaatatca 1140

ccccggtggc tccatgagtt acaacaggct aagatcaaac cccgcgagaa agttgatctc 1200

agctcgttac gggttgtcac gagcactggc atggtccttc gggatgcatt gttcgagtgg 1260

ttctatgacg aagggttccc accccatact cgtctgaata atatttctgg tggcactgat 1320

attgcaggat cctttggaac aggcaacccc cttgtcccac tatatgttgg tgggtgcgcc 1380

ggatgcagcc tgggcattcc cgtggaggtt tacgactcta cgattgaggg cggcgacggg 1440

atcaagggag tacccgtgga ggacggtgtt ccaggcgaat tggtcgccac atctgctttt 1500

cccaacatgc ccacactatt ctggggggat gagagcggca agaagtacca tgatgcgtat 1560

tttgagagat tcgacaacgt gtggacacac ggcgacttcg tttccatccg tccaatcacg 1620

aaacaaattg tgtttcacgg acgcgctgat ggggtgctca acccgtcggg agtgcgattt 1680

ggatcggccg agatctatcg ggtacttgag ggccaatttt cgaaagagat tagcgattcc 1740

atatgcgtag gccagcgacg accaacagat acggacgagc gggtgatact ctttttgctg 1800

atgaggccag ggatggcgtt cacaccggac ctggttgcac gagtgaagag cgccatccgt 1860

tcagagctga gccctcgcca tgtacccatg ttcacgtttg agacccatga aatcccgacc 1920

acggtcaacc tgaaaaaggt ggagctccct gtgaagcaga ttgtgtctgg aaaaatcatt 1980

aagccgtcgg gtacactgct caacccgaag agcttggact tctactatca attcgccaaa 2040

gtggaaactc tgcgcgagaa aatattgaac aacgcatatg aaaggaatag taagtag 2097

<210> 36
<211> 2124
<212> DNA
<213> Penicillium chrysogenum

<400> 36
atgttcttct catcacctcc ccatttggct aaggccgagg agcttaagca agcgcctcca 60

aagggcgtag cttactctgt cgccattcct ggctctgaac agcccggccg aagtcgggta 120

taccgagcat ggaatgcgca gaaggaactt ttgacgacac tggactccca ggtcaccacg 180

gcccatgata tgttcgaatc gaccgccaac cgccagccca agaaccactg cctcggttgg 240

cgtccgtata actccacgac caagtccttc gatccatacc agtggctcac ttacgagact 300

gtgcagaaga gacgcgctgc gttcggtgct ggtctagtag agctgcacca caagcacgac 360

tgccatcgct ccggccagta tggtgttggt ctctggtccc agaaccgacc ggaatggcag 420

attaccgatt tggcttgcat ttcccaatct ctttactccg tctcgatcta cgacgtcctg 480

tccccagacg ctaccgaata tattatcaac cacgctgagc tcagctgtgt tgtcacctct 540

ctaccccata tcccaacgct cctcaagctc aagccttctc tcccgaacct gaagatcatc 600

atcagtttgg acccattaga tggcggtgag caggatggtc actccaagcg tgcccttatc 660

gagtccatgg ccgcgggtca gggcctggcg atctacacta tggatcaggt tgaggagctg 720

ggcttggcct caaagcgcgg gtacaatgcc ccttctgctt cggacattgt cactatcaac 780

tatacctccg gaacgaccgg cccccctaag ggtgttgttc tgacgcatgg caatgccgtg 840

gcggccacgt cctgcggttt gatcactgtc ggccaatccc gaggtgatac gatggtctct 900

tatctgccgc tggcccatat ttatgcccgt ctggccgagc acactgcttt ctggggtggt 960

gcccggattg gttatttcca cgggaacatt gtggagttgg tcgatgattt gaagttgttg 1020

aaacccactg gtttcatgtc tgttcctcgt ctttacagtc ggtttggtag cgcaatccgt 1080

tctgcgacag ttgagcagcc tggtttcaag ggcgctcttt cacgacacat cattgcggcc 1140

aagactgcga acatgaagaa ccccgatccc tccaaggcga cagtccggca cgctgtgtac 1200

gatcgcctgt gggctaagaa ggtggctgcc gctctcggtc tggagcgagc caggtacatg 1260

gtgtctggct ccgcgccctt ggacccgact ctacatgact tcctgcgtgt tgccaccggt 1320

accgatatcc ttcaggttta cggcttgact gagtcctacg cctcagccac cgcccagccg 1380

acctacgatc tgacctccgg caactgcggt agccttgctc cctgtgtcga ggcatgcctg 1440

gcctctctgc cagacatgga gtactccgtg gatgacaagc cgttcccccg tggtgagctc 1500

ttgctgcgcg gcaacaacat gttccgcgag tactacaaga acgaggaaga gaccagcaag 1560

gccgtcactg aggatggctg gttccgcacc ggcgatgtct gcacaatcga cgaaaagggc 1620

cgcttcatca tcattgaccg ccgcaagaac gtactcaagc tcgcccaggg cgagtacatc 1680

tcccccgagc gtctggaggg tgttgtcctt gccgagcttg gctacatcgc ccaggcttat 1740

gtccacggcg acagcatgca gacattccta gttggcattt tcggtgtcgc acctgacctc 1800

ttcgctccgt ttgccagcaa ggttctgggc aagaccattg ccccgaccga cttggaagcg 1860

attaaggaat ctctgcgcga tgacaagatc cgccgtgctg tgctccggga cctcgaacgc 1920

gtggccaaga agcacaagtt tgctggctac gagagaatcc gcaacgtctc tctcaaggtg 1980

gagccattca ccgttgagaa caacttgctg accccgactc tcaagctcaa gcgtccccct 2040

gctgtcaagc tttaccgttc ccttctggat cagctctacg aacaggccac cgaggagcag 2100

tctgccccca aggctaagct ataa 2124

<210> 37
<211> 1671
<212> DNA
<213> Penicillium chrysogenum

<400> 37
atgccgtata aatcccgctg gacgattgac atcccagaca cccatttagc ctctcttctt 60

ctcaaatccc caacggcacc tctctccaaa acacacaagt gctacctgga cgccgcccgc 120

ccagaaaccc actacctcac cacccatgat ctccgcctct ggagccagcg gctagcagcc 180

ggcctacgca aatccggact gcagcgcgga gaccgcgtcc tcctcttctc aggcaacgac 240

ctcttcttcc ccgtagtctt cctaggcgtg ataatggcgg gggggatctt caccggcgcg 300

aatccaacct tcgtagcccg cgaactagcc taccaactcc aagacagcgg cgccacatac 360

ctcctctgcg caagcaacag tctagaaacg ggactggaag ccgccaaaca agccaaacta 420

ccgcagagcc acatcttcgc ctacgacacc tccatctacg acggcgtaac aaacccccaa 480

aagggctgcg catactggag cgacctcctc gcctcagagg aagaaggtgc agccttcacc 540

tgggacgagc tgagtacacc agccctctcg tccacaacac tcgcactcaa ctactccagc 600

ggcacaacgg gccggcccaa gggcgtcgag atctcgcacc ggaactacgt cgccaatatg 660

ctccagtatt gtcatacggc aagtctacat ccggactaca aggctcgtct tgagcgctcc 720

cgctggctat gtttcttgcc tatgtatcac gcgatggcgc agaatatctt tattgcggcg 780

gcgctgtacc gcgccacgcc ggtgtatatc atgtctaagt ttgactttgt caagatgctg 840

gagtatacgc agcgattccg gattacggat tttatactcg tgccgccggt tgttgttgcg 900

ttggcgaaac atccggctgt tgggcagtat gatttgagta gtgtggagtt ggttgggagt 960

ggtgcagcgc cgttggggag ggaggtttgt gaggaggttg agaagttgtg gcctcccggg 1020

aagattaata ttaaacaggg atgggggatg actgaggcga cttgctcggt cacgggatgg 1080

aatccagccg aaattagtac atctgcgtct gtcggtgagc tcaatgccaa ttgcgaggcg 1140

aagattatgt tcgatggggt tgaggtgaag gaacgcaact ctcgtggcga gttgtgggtt 1200

cgtgctccca atgtgatgaa aggctactgg cgcaatgaga aggccactaa agagaccaag 1260

acggaggatg ggtggctcct gacgggggat attgcgtttg tggatgacga tggcaaattc 1320

catgtggtgg atcgaatgaa ggaattaatc aaagtcaaag gcaaccaggt ggcgcccgcc 1380

gagttagagg ctcttttact tgaacatccg gcgatatcag atgttgcggt gatcggggtt 1440

gtcatcaaca acgatgagcg ccctcgcgca tatgtggtgc ttcgacccgg ccaatccgcc 1500

accgccaacg agatcgcgca ttacttggac aacaaggtct ccgcgttcaa gcggattacc 1560

ggcggagttg tattcctcga ggccataccg aagaatcctt cgggcaagat tcttcgcatg 1620

aaattgcgtg aacaggccaa agaggagcta cgtgtcaccg ctaaactcta g 1671

<210> 38
<211> 1977
<212> DNA
<213> Penicillium chrysogenum

<400> 38
atggctaaca agcggtctct ttgcgcagaa attcccttga gtcttgcact ccctgccgcg 60

gcaactacac ttgcatatct caacgcaaga tggtcgctct cctacgattt gaatctcctc 120

aagggcttgc tgaagatgtc tgcgaagtcc cgcatcgccg aacgtggcga ccgcttgaac 180

ctcttctaca cacttgaagc ctatgcactc gatcctaaaa ctgcagacaa cgatttcatt 240

gtgtacaatg gacgaacgac gacattccac gaaacctaca tcacggctct tcgctacggg 300

gcgtggttca agacagtcca cggcattaag cgcaaggaga tcgtcgcgat cgatttcatg 360

aactcatcta ccttcatctt catgctgttg ggactctgga gcattggagc agtccctgca 420

tttatcaact acaacttgtc tggcaagccg ctcacccact ctattcggac gtcctccgca 480

aggctcgtgg tggttgacga agaagtccgc cactgcttcc cggaggagca ggagaagatt 540

ttgacatccc cgaacttccg tgatggcaag ggaccggttg aaattgtgtt ccatacccca 600

gaggtagaag cgcagatctt ggggatggag cctatgcgag aggatgacaa ggctcgcagt 660

ggcttgatcc ctcgcgatat ggccattttg atttacacaa gtggcaccac cgggcttcct 720

aaacccgcta ttgtcagctg gaagaagtgc tggtctggta gtttgttcgt taaagattgg 780

ttgaacatta ctccctctga cagattcttc acgtgcatgc ctctttacca tagttccgct 840

gcgatacttg ggtttataac ttgcttgatg ggcgggtcca ccctgatcat tggccgccgg 900

ttctctgccc gcaacttcat gaaggaggct cgggagaacg gtgccacagt tatccaatac 960

gtgggcgaaa cgctgcgtta tctattgggc gtagcacctg aaattgaccc ggtcaccgga 1020

gacgaccttg acaagaagca caatatccgc ttggcgtttg gaaatggatt gcgtcctgat 1080

atctggaacc gcttcaagga gcgcttcaac attcccacaa tcgccgagtt ctacgccgcc 1140

accgaaggca ctgcgggctc atggaacatt tcgtcaaacg acttttccgc gggtgccatc 1200

ggccgaaatg gtgctttagg aaacattatt cttggtcgtg gctctgccat tgtggatgtt 1260

gaccacgaga cccaggaacc atggcgtgat cccaagactg gtctctgcaa gaaggtgcct 1320

cgcggtgacc ccggtgagtt gctgttcgcc atcgatgcag cagatcccac tgccaatttc 1380

cagggctact tcggaaacaa gaaggcgaca gaaggcaaga ttatccgtga tgtaatcaag 1440

aagggcgatg cctatttccg aaccggtgac atggttcgat gggacaaaga cggccgctgg 1500

ttcttctcgg atcgactggg tgataccttc cgatggaaga gcgagaacgt atcaaccagc 1560

gaggtttctg aggttctagg cgcgcatcct gaagtgcatg aagccaatgt ttacggcgtg 1620

tctcttccta atcatgatgg tcgtgccggt tgtgcagcca tcctcttcaa ccagcagata 1680

acaagcggtg gcctctctga ttccgcactg gagccctcgc gcgaggtgct tgatactctg 1740

gctgcacaca ctctccagaa cctgccccgg tttgcggctc cgctgttctt gcgggttacg 1800

cctgtgacgc agtcaacggg taacaacaag caacagaagc atgttctccg tactgaaggt 1860

gtggatccgg ctcttgtgtc gaagaaggac agactgtact ggcttcaggg caatacatat 1920

gtcccctttg ggcagaggga ctgggaacgg ttgactggtg gtcaagttcg actgtga 1977

<210> 39
<211> 1899

<212> DNA
<213> Penicillium chrysogenum

<400> 39
atggatatag gaaccgccgc cgctgccctc gtcggaggag ctacaatcgc aggatatttg 60

aatgccaagt ttcatatcca gaaggatata tcaggactgt tcacgctgaa gaacgctgaa 120

cgacaatatg ccaatgccgc tagtcaaaac caaggaaatc catggttcgt gctagtgcag 180

accgtgaagc gataccccga catgatctgt ctctggactc gcgagagatc atacacatac 240

cgagaaatcc aagaccaagc ctgccaatat gctcatttct tcctatcaca tggcgtgaag 300

aaaggcgacc tcgttgccct ttacctccag aacagcaacg agtacctcgt tgcatgggtc 360

gccctctgga gcattggctg tgcccccgcc gccataaatt acaacctcac cggtgacgca 420

ttactgcact gcctgaaaat cagcgacgcg accattctcc tcgtcgacga aaatgccgac 480

tgtcgcgccc gcgtcgaaga aagccatgac gctatcacag gcaatctggg aatgaagcca 540

atgacactag atagcgcctt aaaggcgcat atcagaacat tcccaacaac cctcccaccc 600

aaggaactga gcaagcatgt ggccggggac ttccccgcaa ttcttctata cacctctggc 660

accacgggta tgcccaaggg ctgtgcgttc acaatgtcca gactatatac cactctattc 720

gtccgccgtg ccatgatggg agatacgcca ggcccaggag gtgatcgctg gtacagctgt 780

atgcctttat atcacggaac ggcggctatc accataatct cgtgtctggt aatgggtgta 840

agcgtcgcca ttgcgcccaa gttcagcgtc agtagattct ggacagatgt ccgcgactcg 900

gagtcgacca tcttcgtgta cgttggagag actgcacggt atttgctcgc accgcctccg 960

tcgcctctag accgaaacca caaggtacgc tgtatgtacg gcaacggtct ccgtccggat 1020

atctgggaac ggttccgtga acggtttggt gttgcggaag tcggggagtt cttcaatagc 1080

acagaaggtg tctttggtct cttcaactac aaccgcggcc ctttcacggc tggaagtgtt 1140

ggtcatcacg gtttgatcat gcgtggtata ctgcataatg tcttcgtccc tgttgccatt 1200

gatccagaaa ctggagatat tctgcgggat cccaagtctg gctttgcggt tcgctcgcct 1260

tatgagcagg gaggtgaaat tatcgtcaac gttcccagtg aagaagcttt ccagggttac 1320

tggcacaatg atgaggcgac caataagaag tttttacgtg atgtcttcaa gaagggggat 1380

ctctattatc gatctggaga tgctctgcgc cggcagagcg atggccggtg gtacttcctc 1440

gaccgactcg gggatacctt ccgatggaag agtgagaatg tagcaaccgc cgaggtttca 1500

gaggtactag gccagttccc tggcatcacc gaagccaacg tttacggcgt ccgtctcccc 1560

aatcacgagg gacgcgccgg ctgtgcagcg attcaaatta gcccagatgc gcgtcagacg 1620

tttgactaca cggagcttgc gagattcgta cgctccaagc tgccgaaata tgctgttcca 1680

ttgttccttc gcatcgtcga gaatcccact cacatccaca accataagca gaacaaggtg 1740

ccacttcgtg atgagggggt tgatactgcc ttgattggga ctaaagcccc tgagggtaaa 1800

gacgaccact tcctgtggat tgcgcctggt gaggagagtt attcaccgta tgggcagaaa 1860

gagtgggagc aattgtcgag tgggagtgtt cggctgtga 1899

<210> 40
<211> 1719
<212> DNA
<213> Penicillium chrysogenum

<400> 40
atgtctggtt caaagtctcg gttgtctggg ctccttggcc atttcacagg ctcaacaccg 60

ccggttgaac accgtgttaa tactcatacg ctatccccaa ctttctttct tccgcgtgct 120

gctgccgttg agcccaatgc tgaggcgatc tatcatgtaa ccgccaacaa caaaatcctt 180

cgtaggagct atggcgagac tgcagaccga gcgaggggta tggcttacta cctcaaaaaa 240

catgggctca gcagagtggg gatactatgc ccgaataccc cagccttcct tgagtcaatt 300

ttcggaatcg ccgcagctgg cgctgttaat atcgctgtca attatcgtct gaaacaagaa 360

gacattgctt atatatttga ccatggtgat gcggaggtga tcatcgtcga cgaggaatat 420

gttccacttc tggagcacta tcgatcccaa cacccccgca ttcctattat agtagacacc 480

gacactgatg cgacggaggg tgagctaacg ggcccctttg acgaggctgt tctggaaggc 540

ctcagacacg acatcgatac aggttctcga ggctgggaag gactcgaaag ccaagctgcc 600

gatgaagaat ccacaattgc attggcatat actagcggga ctacagcccg tcctaagggt 660

gttgagttca gtcatcgggg ctgctatctg gcaacattgg gaaacgtcat tgaaactggt 720

ctgaactacc accgcggacg tgcccgttat ctatggacgc tacccatgtt ccacgctatg 780

ggctggacat ttccatgggc agtgacagcc gttcgcggaa ctcattattg cctacgcaag 840

atagactacc ccgaaatctg gaggctgctg aaagaggagc atatcaccca cttcaatgct 900

gcaccgactg tgaacacctt gctctgtaac gcgaaagaag cagagaggct gcccgagcct 960

gttcgcgtca cggtggcagc aagcccgccc acaccacttc tcttcgaaca aatgaccgac 1020

ctaaacttgc accctgtaca tgtttatgga atgacggaga catacggccc aatcaccaag 1080

ggctaccatt tgccagaatg ggacgagctc cctcttaagg acaaatacca gaggatggct 1140

cgccaaggcc acggctttat tacaagtctc ccagctcggg tcattaagac cgaagtgcct 1200

gcagggacta tcacagatgt tcgcaaggat ggccaggaga ttggcgagat tgtttttgtt 1260

ggaaatatct gtgcccgggg atactacaag gaccccgagg cgactcggaa attatttgca 1320

ggaggtgtgt tgcactctgg ggacttggct gtctggcatc ccgacggtgc aattcagatt 1380

cttgatcgtg caaaggatat catcatcagt ggcggcgaaa acatctcctc cgttgcactg 1440

gagtctatgc tagttatgca tcctgatatt ttggaggctg gtgtcgttgc tgttcctgac 1500

tcccactggg gcgaacgacc taaagccttt gttacagtga aggaaggcaa gagcctacaa 1560

ggctcagact tgatagactg ggctcgaaat actagcggaa tcagcaagtt catgattcca 1620

cgagaagtgg aagtggttac tgaactacct aagaccagca ctggcaaaat ccggaagaac 1680

atcctacgtg actgggtgaa gggtccgaga gaagcttag 1719

<210> 41
<211> 1734
<212> DNA
<213> Penicillium chrysogenum

<400> 41
atgagcctgc aagactcaca tcacagtttc cagccaaagc cttcctataa acagggtctt 60

ctggagccgg cacttcggaa ctggacaatt ggggatctac tccagcgtca agccgagcaa 120

tttccaggca acattgcgat ttcctgcccc ggcacgtcaa actccatcac ctatcgccaa 180

ttgaacgacc gtaccaagct tcttggaaaa gctctgatag caagtggaat ctccgttgga 240

gatcgcgttg gcatctttgc aggaaatgtt ttggaatatg tggaggttgc gctggcaact 300

gcgcgcattg gagcaatcat cgttctgttg aacacctttt acacaacgga ggaaatcaaa 360

agagcccttc ggtttactgg gtgttcattg ctattcatca ctgagagcct tggaaaacgc 420

agtctgttgc catgcatcga ccaactcaac gagataattg aaaaccagaa aagcgagttc 480

ccagatctcc gaagcatggt tcttctctca ggacagtgtt cagaatcgtc caatctgcag 540

tcctatgcgg actttttcaa tatcccaccc accggtgcga aggccgcttc tgcttgcaag 600

gcagcagaaa cacaagttac tccagagacg atctgtaact tccaattcac tagtggcacc 660

actgggatgc cgaaggcggt gatgctcact cactttaacg tcgtcaacaa tggttttcta 720

ataggtgacc gagtgtgcct cagtccaaat gataccatat gttgtccgtg gcctttgttc 780

cactcgtctg gatttgttgt tggcctgata acatcactgt gtcatggtgc aactctcgtt 840

ctaccgtcac cagtattcga tcccgctgcc acggcacgag cactgatctc cgaaaggtgt 900

actgggcttc aaggcgtccc aaccatgttc gctgctgttc tcgagtggta ccgtcaacgt 960

ggtactcgtc cgccacctct tcgaacagga attatcggtg gatcaccagt ttccccggct 1020

cttctcagag agctccagca tgaatttgct ctggaagacc tgggtattgc atatggtatg 1080

actgaaacct cgccactgag cttcctgtca aaaggctttg agccagaagg gacccactca 1140

tggatggaaa tcttaccaca cacgacggca aagatagtgg atgcgcaagg aaccattgtc 1200

cccattggaa gtcctggaga actgtgcgtc tcaggatatc tcttgcagca aggatactat 1260

caaaaccccg ggaagacgag tgaggccatg cgcgtgcatg aggatggagt tctctggata 1320

cattcaggcg acgaagcaat catggatgag caaggacgtt gtcggatcag cggcaggatc 1380

aaggatacca tcattcgcgg cggagagaac atatatccag cagagatcga ggatcgcctg 1440

aatgagcacc cggcaatctc gatgtcggcg gtagtgggta tccaagatgc aaagtacgga 1500

gaggcagtgg cggcattctt gcaactcaaa catggcgaaa acccctgtgc acaggcacac 1560

atctcggaat gggttcagca aaccctgggg aaacataagg taccaacact cgtatttcac 1620

ctcggtgttg atggcgttcc tggcgatttc cctaagacag caagcgggaa gatcaagaag 1680

gttgacctgg tggccattgg aactcagctt gtccgcggcg cgggtaaact gtag 1734

<210> 42
<211> 2091
<212> DNA
<213> Penicillium chrysogenum

<400> 42
atgtctccgc acccccaaac aacaacccac tcacactccc ttggcgaccc agaaacattc 60

tggtcccatc acgcggccag actccactgg caccacaaac cttcacgtgc cctaacacgg 120

aagacaaagt tcctagcgag cggaaccaag catgagcact ggtcgtggtt cgctgacggc 180

gaaatctcca caacctacaa ttgtgtcgac cggcatgtgc tcgcaggcca tggcgacaat 240

gttgcgatcg tatgggaatc gccagttacc ggtgtaacag agaagtatac ttatgcccgt 300

ctactcgatg aggtcgaggt gcttgccggt gtgctgaggg aggaaggggt acagaaaggc 360

gatgttgtta ttatctacat gccaatgatc cccgcagccc taatagctgc gttggcaata 420

acccgcctag gcgcaatcca cgccgccgtc ttcggtggct tcgccccgca agctctagca 480

cagcgcattg aggccgcgcg tccgagagct ataatgacag cgtcatgtgg aatagagggg 540

tccaaggggc ccattcccta ccgaccgctc gtcgagggcg ccctcaaggc aagcagcttc 600

aagccgagca aggtcattgt ctggcagcgt gaccaactcc gctggaatca gcccgataag 660

cgcggtggcc agcgtaattg gcagagactc gttaagagtg cacggttccg tggggttaag 720

gctggacctg tccctgttaa gagtacggac gcgctttata ttatttatac aagtggaaca 780

actggcctcc ccaaaggcgt cctccgcgaa gcaggcgggc atgccgtggg cctggaactc 840

tccataaagt ccctcttcgg gataacaggt cccggggaca caatgttctg cgcatcagac 900

atcggctggg tcgtcggaca ctcgtacatc ctctatgcac cgctgctagt cggcgccaca 960

accgtcctgt tcgaaggtaa acccgtcggc acgccggatg caggaacgtt ctggcgtatc 1020

atcgagaaac acaatgtgaa tgcgctcttc acggcaccaa ctgctatgcg agccatccgg 1080

aaagacgacc cggacaacca gcttatcggc gaggtcggga agcggggagg attgaaatcg 1140

ctccgtgcgc ttttcctcgc cggtgagcgt agcgagccgg gtattgttag ctcataccaa 1200

ggcctgcttg gtaagtatgc cgcggccggt gctcttgtta ttgataactg gtggtcctcc 1260

gaatccggat ccccgattac gggtgttgcc ttggatggga gggctgccct tgctagtccg 1320

gcatccggac cagatcatcc tcccctcacg ccccgtcccg gctcagcagg cttcccgatg 1380

cctgggtttg atgtccgcat tgttgatgac gagggtaacg aagtcccgcg cggaacaatg 1440

ggcaatattg ttatggctcc gccgctcgcc ccgtcagcgt tcacaaccct tttcgaggac 1500

gaagaacggt tttaccgtgg gtatcttaag cgctttaatg gccgctggat tgataccggt 1560

gatgcgggga tgattgataa ggatggctac gtgcatatta tgtcgcggag tgacgatatc 1620

atcaacgttg ccgcgcatag gttcagcacc ggatccattg aacaagcaat cctctcgcat 1680

ccgtcgatta gcgaagcaag cgtcgtcggc atccccgacc cgctaaaagg ccacctcccc 1740

ttcgcattta tccagcccaa agctggcgca gtccctggag aaaatgggca tcttccggca 1800

acgccgagcc cgcagttatt tgccgaggtt aacgcgctgg tccgcgagca gatcggggcg 1860

attgcgtcgc tgggtgggat tatacagggt cgggggatga ttcccaagac gcggagtggg 1920

aagacgctta ggcgtgttct tagggagctt gttgaaattg gagtgaaggg ggagtatgcg 1980

gcggaggtta gtgtgccgcc tactgttgag gatgcggagg tcgtttctgt tgcgaggaag 2040

agggttagag agtattttga ggcaaagggg gttgggaagt cgaagcttta g 2091

<210> 43
<211> 2082
<212> DNA
<213> Penicillium chrysogenum

<400> 43
atgcctcata tcgacgtaaa cgttgagcca gaggccaagg agctctggcg gccctcctct 60

cctgagacaa ctcagatcta cgatttcatg actaaagtga acaagaagta tggcttgtca 120

ctgaacaact acgatacact ctggaaatgg agtgtctctg aaccagccca gttctgggag 180

gaaatctggc actataccaa gatcaacgcc cacaccccat accaacacgt ccttgattca 240

aaagatgtgc tatttcctag accttcattc ttcgaaggga gcactttgaa cttcgccgag 300

aatctgttgt atcccgccac ctcgccggat gaagattccg tagcagtgat tggcgccact 360

gaagtagccc gcgaattcgt ctcgtggaag gagctacgag aacgagttcg cctgtgcgca 420

aacgctctga aagaagcagg tctgaagacc ggcgatcggg tcgctggctt cgtgggcaat 480

catgctaata ccgtcgtggc aatgttggcc gctgcttcaa tcggggcctt ttggactggc 540

gtgtcacctg acactggtgt gcatgctgtt ttggagcgtt tgaagcagat ccaacctaag 600

atcctctttg cggacaatgg atccttctac aatggcaagg tgcatagctc gcatgccaaa 660

gtacggagaa ttgtctctga attgccggac cttgagcttt tggtgcttct tattaccacc 720

ccggaacttg aagttaacct ggatgacctt cgacctgcaa atggaaagca aaaagtttat 780

ggtgatttcg tgtctgaggt caaggactct caggcgccat tagagtttgc aagcttgaga 840

ccagatcatc ccgtctatat cctgtattcg tctggtacaa cgggggctcc caaacctatc 900

gttcatgggt ctctggcaca ttgttgcagc acaagaaagg aacatcttct tcactgtgac 960

attcgaccag gtgatcgctt gttctacttc acaacgacaa cctggatgat gtggcactgg 1020

ctggtgtcgg gcttggccag cggagcaaca atcgtcctgt acgatggatc acccttccga 1080

ccctttgatg ctgaaggcgg gaagggagaa atggccatgc cgcggctgat cgaagagctt 1140

caaattaccc atttcggtac atcggccaaa tatctctctc tcctcgaaca agccgctttg 1200

aacccgcgca agcatgccca tagaccagtg tcgttgaaga ccctaagggc catcttcagt 1260

actggatccc cgttggcacc atcaacattc gaatacgtct attcctcttt ccaccacgac 1320

atcatgcttg gttccatcac cggtggcaca gatatcctct ctctgttcgc ttccggctgc 1380

cccattctcc cagtttacaa aggagaaatt caatgccgtt ctttggggat ggacatctca 1440

gtgtttgact atgctggcaa agacattagt gcaactggtg aacccggtga tcttgtctgc 1500

gtcacaccct tcccggccca accagtcatg ttctggcccc cgggaccaac aggcttggag 1560

aagtatcgga aaagttactt cgatgtgttt gggtcctcgg tctggcacca tggggactac 1620

gtgcgtttga acccacaaac cggaggtgtg gtcatgctag gccgcagcga cggcgtactg 1680

aagccatccg gagtgcgatt tggaagtgcc gagatataca atattctact taagcatttc 1740

gccgaagatg tggaagactc cctttgcatt ggtcggaggc gagatggcgt tgacaccgat 1800

gagacggtcg ttctgtttgt ccgactttct tctgccaacg agtccggtat gccggcggat 1860

ctcgccgcac gcatccaggc cactattcgg aaggagctta gcccccgcca tgtgcctggc 1920

attatcgatg catgtcccga gattccggtg acttcaaatg gcaagaaggt cgaaaatgct 1980

gtgaagcaga ttttgtgtgg gcttaacatc aagattggtg cgagtgttgc gaatgcttca 2040

tgtcttgatt ggtatagaaa ttgggcctcg gaaacccctt ag 2082

<210> 44
<211> 2010
<212> DNA
<213> Penicillium chrysogenum

<400> 44
atgtcggacg gcccaattca gcctcccaag cccgcagtgg tgcatgaggc acacgaggtc 60

gacactttcc acgtccccaa ggcgttccac gataagcacc cctccggcac tcacatcaag 120

gacattgagg agtacaagaa gctttacgaa gaatcaatca agagccccga caccttctgg 180

gcacgcatgg cccgcgagct cctcacattt gacaaggact ttgaaaccac acatcacggc 240

tcgtttgaga acggcgacaa tgcctggttc gtcgagggtc ggttgaacgc atcgttcaac 300

tgtgtcgatc gccatgccct caagaaccca gataaggtcg ccattattta tgaggccgac 360

gagcccaacg agggccgtaa gatcacctac ggagagctga tgcgcgaggt gtcccgggtt 420

gcctggactc tgaaggagcg tggcgtcaag aagggcgaca cggtcggtat ctacctgccc 480

atgattcccg aggccgtaat cgctttcctg gcttgctcgc gtattggtgc cgtgcactcc 540

gttgtcttcg ctggtttctc ttccgactcc ctccgggacc gtgtcctgga cgcctcctcc 600

aaggtcatca ttacctccga cgagggcaag cgcggtggca agatcattgg cactaagaag 660

attgtggacg aggccatgaa gcagtgcccc gatgtgcaca ccgtgctggt gtacaagcgc 720

accggtgccg aggtgccctg gaccgctggc cgtgacattt ggtggcacga ggaggtcgag 780

aagtacccca actacctcgc ccctgagtcg gtcagctccg aggatcctct cttcctgttg 840

tacacctccg gttccaccgg taagcccaag ggtgttatgc acaccactgc cggttacctg 900

ctcggtgcgg ccatgactgg aaagtacgtg tttgatatcc acgacgatga tcgctacttc 960

tgcggtggtg atgtcggttg gattacaggt cacacctatg tcgtgtacgc ccctctattg 1020

cttggctgcg ccaccgtcgt gttcgagagt acccccgcct accctaactt ctcgcgctac 1080

tgggatgtca ttgacaagca cgacgtcaca caattctacg ttgcacccac cgctctgcgt 1140

ctgctgaagc gcgctggaga tgagcacatt caccacaaga tgcacagtct gcgtattctt 1200

ggctccgtcg gagagcccat tgccgcggaa gtctggaagt ggtacttcga gtgtgttggc 1260

aaggaggaag ctcacatctg cgacacatac tggcaaaccg agaccggctc acatgtcatc 1320

acccctctcg gcggtatcac ccccaccaag cccggcagtg cctccctacc cttcttcggt 1380

atcgagcctg ccattatcga ccccgtctcc ggagaggaga ttgtcggcaa tgatgtcgag 1440

ggtgttttgg ccttcaagca gccgtggccc agcatggccc gcaccgtgtg gggtgcccac 1500

aagcgttaca tggacactta cttgaacgtg tacaagggtt actacttcac cggagatggt 1560

gctggccgtg accacgacgg ctattactgg atccgcggtc gtgttgacga tgtcgtcaac 1620

gtttctggac accgtctgtc caccgctgag atcgaggccg ctcttctcga gcacccttcc 1680

gttgccgagg ctgctgtcgt tggtattgcc gacgagctga ccggtcaggc tgtcaatgcc 1740

tttgtctctc tcaaggaggg caagcccaca gaacagatca gcaaggacct tgcaatgcaa 1800

gttcgcaagt ccattggtcc cttcgccgcc cccaaggctg tcttcgtcgt ggatgacctc 1860

cccaagaccc gcagtggcaa gatcatgcgc cgaatcctcc ggaagattct cagtggcgag 1920

gaggacagcc tcggtgatac atcaacgctc tccgacccca gtgtcgtgga caagatcata 1980

gaaaccgtcc acagtgctcg ccagaagtaa 2010

<210> 45
<211> 1737
<212> DNA
<213> Penicillium chrysogenum

<400> 45
atggtttttt tacctccaaa ggagtccggt caattggacc caattcccga caatattcca 60

atcagcgagt ttatgctcaa tgagagatat ggacgagtgc gacacgccag ctcccgggac 120

ccatacacct gtggtattac cgggaagtca tactcgtcga aagaggtagc caatcgcgtc 180

gactcgctgg ctcgtagtct atcaaaggaa tttggttggg cgccgaatga agggtcagaa 240

tgggataaga cattggccgt gtttgccctc aacactatcg attccttacc cctattctgg 300

gccgttcaca gactgggcgg tgttctcact cccgccaacg catcatactc cgccgccgag 360

ctgacgcatc agctgcttga ttccaaggcc aaggcccttg tgacttgtgt tcctctcctc 420

tccatctcac tggaagctgc agccaaagct ggtctcccga agaacagaat ctacttactc 480

gatgtacctg agcagcttct tggcggagtc aagcctccag caggatacaa gtccgtttcc 540

gaactgaccc aggctgggaa gtctctcccg ccagtggatg aattgcgatg gagcgcgggt 600

gaaggtgccc ggcgaacagc atttgtgtgc tactcaagtg gaacgtctgg attgccgaaa 660

ggagtcatga tctcacaccg caacgtgatc gccaataccc ttcagatcaa ggcgtttgag 720

cagaactacc gggatggtgg gggcacaaag cctgcgagta ctgaggttgc tcttggtctc 780

cttccgcaga gccatatcta tgctcttgtg gtcattggcc atgctggggc ataccgaggc 840

gaccaaacaa tcgttctccc caaattcgaa ttgaaatcct acctgaacgc catccaacag 900

tacaagatca gtgcgctgtt cctggtacct ccgatcatca ttcacatgct gggcactcaa 960

gacgtgtgct ccaagtatga cctgagttcc gtgacgtctc tgttcacggg agcggcaccc 1020

ctgggtatgg agacagctgc cgatttcctc aaactctacc cgaacatttt gatccgccaa 1080

ggatacggtc tgacagagac atgcacggtc gtaagctcga cccacccgca cgatatctgg 1140

ctaggttcat ccggcgcttt gctccctgga gtcgaggcac gaattgtgac gcctgaaaac 1200

aaggaaatca caacgtacga ctcaccgggc gaattggtgg tccgaagccc aagcgtcgtc 1260

ctgggctatt tgaacaacga aaaagccacc gcagagacat ttgtggacgg atggatgcgt 1320

acgggagacg aggctgtcat ccgtagaagc ccgaagggca tcgagcacgt gtttattgtc 1380

gatcggatca aggagttgat caaggtcaag ggtctgcaag tcgcgcctgc cgaactcgaa 1440

gcccatatcc tcgcccaccc cgatgtctcg gactgtgctg tcatcgctat tccggatgat 1500

cgtgcaggag aagtacccaa ggccattgtt gtgaagtccg ccagcgcagg atcggacgaa 1560

tctgtctccc aggctctcgt gaagtatgtt gaggaccaca aggctcgtca caagtggttg 1620

aagggaggta tcagatttgt ggatgccatt cccaagagcc cgagtggtaa gattcttcgt 1680

cggttgatcc gtgaccaaga gaaggaggca cggagaaagg ctggtagcaa gatctaa 1737

<210> 46
<211> 1854
<212> DNA
<213> Penicillium chrysogenum

<400> 46
atgagctaca gcggaattgt aaccgggctt ggcctaagag gtcgtgtatc ctgtcgaaga 60

tctgcgttcg gaggaaagag aatccttcag caaccatcct tggttaagag ggggctactg 120

acagagagct atgcacgggg gccatcaggt ccgcccttga tcgagtccac agttggcgaa 180

cacttcgcca agattgtcgc ggaatgcgga gatagaacag cggttgtctc aagacatcag 240

aatgatcggg caacctatgc atccttggac acaagaagca attctctcgc tcgggggctg 300

gagtcacttg gtgttgggaa gggtgagcgg gttggagtca tgcttgggaa ctcgatggaa 360

tacgctgtgg caacatacgc gttattcaaa cttggtgccg ttttggttcc gctcaacccg 420

tcattcaata ctgcacaagt gattgcggcc ctgggtcacc tggaagccag tcatctccta 480

atcagcacag agtccaatct gccacgaaag aagccacgca gcaatgtccc tttactcaat 540

gaccttgttg aagacctcca caagtcgaag ttggagtccg cacctgtccc gtctctgaga 600

aatatcatta tggtcgacaa ctccgaaggc cgcgtggata tatcgtccta taagagcttg 660

acgcagtatg catctattat gtcccagctt actgtagacg gcagcccgct tcctcctcga 720

aacctttcgc cggatgaaac tgtgaatatc cagttcacct caggaaccac atcaatgccc 780

aaggctgcat gcctcaccca tcgctccatt ctgaataatg gctctcagat tggtgaccgc 840

atgcttctca cgccgaatga tattatctgt tgcccaccgc cgttgtttca ttgcttcggc 900

agtatattag gctatatggc gacagcgacc cacggctcag ccatcgtctt ccccagtgaa 960

tccttcaatg ctcgtgctgc gttggaggct gtccaagaag agaaatgtac cgcgctatat 1020

ggtgtaccga caatgtttct cgaggagtta agcttgatcg agacaggaga agtctccagc 1080

gagggattcc aacatcttcg cacgggcatc gctgccggaa gcagtattcc tgccgagata 1140

atgaaaaagc tgcataaggt actgaatttg accgaattga ctatttgtta tggaatgacg 1200

gaaacaagtc ccgtctcggc tatgacaacg actgatgatc ccattgataa gcggatctac 1260

tctgtcggaa agctcatgcc acatgtcgag gcaaagattg tgaatccggt ggacaagaac 1320

gtaattctac ccatagagag acggggtgag ttggcagtca gcgggtacct gctgatgaag 1380

gaatactggg ccgatccaga aaagacagcg gaagtgatga ttccggatga ttctggcaaa 1440

gtgtggatgc atactggaga tgaagcttcc atgtcaccag atggatatat cactatcacc 1500

ggacggatca aagatctgat aatccgtggt ggcgagaaca tacaccccct ggagattgag 1560

aattgcttgt tagccaacaa tggcgttgca gacgtgtcgg tcgtcggcgt ccctgacgtg 1620

agatatggcg aggcagtagc tgctttcatt gttcctcggg accacagctc aaatacagtc 1680

actgctgagg agatccagca atgggtgcgt gaaaagctca gcaaccatct tattccgaag 1740

catgtcttct ttttgggccc tttggagtct ttccccaaga cagcaagcgg gaaaattcag 1800

aagttcaagc tcagagagac ggccattgca cttttgacca agtgcgctgc atga 1854

<210> 47
<211> 1788
<212> DNA
<213> Penicillium chrysogenum

<400> 47
atgtctagca gtttactctc ccttctttcc cgttcgaatg cgcgcgtctg ctttactccg 60

cggcgaacgc ccctcagcgc aagactgttg tcgactctcc cgaacacacc tatctttcgc 120

gcactccaga accatgaccc tgaaagagtg gctgttgttc atagcgtctc ctctcgatca 180

ttcacctacg gtagtttaat tgccgacatc gtccgcgcta aggatgacct ggaacaaaag 240

gctgcgaaag cgcagggcca attggctggc gagcgggtcg ccttcctcgc ggagaacagc 300

tatgattacg tagtgacact gctggcgata tttgctagtg atgccattgc gctcccgctc 360

tcgccatcat tcccgactgg ggagctgaag tatatcctgg ataattcaca ggccaagatg 420

ctgctgacga ctgagaaata tgccgataaa ggtatggaac tattgcgaga gggattggag 480

cgtgagcctc tatttgctat ccgcaataag ctcacggagg gcgcatcgag cggcgagagc 540

gtaaccctgc acgatctgaa gcagccttcg tccggtggga tgatgttgta tacatctggg 600

actacgaatc gaccaaaagg tgttctcatc ccccaatcgg cactggccgc gcaagcatct 660

tcactactag aagcatggaa atactcaccg gatgatcgac ttcttcacct gctccccctc 720

caccacatcc acggcgtggt aaacgcaatc gtggcaccaa tagtagccgg ctcctcagtc 780

gagttcatgt accccttcaa cccggacaaa gtctggaaac gtctagcagc acccttcctc 840

tcatccaacg cctcaaaacc agccattaca ttcctcactg cggtcccaac aatctacaac 900

cgcctcatgg ccaccttccc caaactaacc ccagagctcc aaaacgccgc aaaagaagga 960

atctccccgc aaaacctccg cttgaacatc tccggctccg ccgccctccc gaccccaaca 1020

aaaaccgcct ggacaaacct cagcaacggc aacgtcctcc tcgagcgctt cggcatgacc 1080

gaagtcggca tggcgattag ctgcggtctc gacgccgcag accgggtgga cggcagcgtc 1140

ggctggcctc tcccaggcgt cgaagcccga ctcgccgata ccgagacagg tgctgtcatc 1200

ccagttgaag aaaaggaacc cagtggccga gaacgtgaag gcgagatcca gctccgcggc 1260

gaaacaatct tcgaccatta ctggggcaac gagaaagcca cgcgcgagag cttcgtgcag 1320

agcgatgatg gaggcagacc gtggttctgc acaggtgatg tcgctacccg tcgggtagtt 1380

gatggagccg gtagtggagc gagcggggca tgggctcagg gaccgatgta tttcatacag 1440

ggtcggaaga gcgttgacat catcaagact gggggggaga aggttagcgc gttggaggtg 1500

gagagggagt tactctctct tccccaaatc accgaagctg cagtcgttgg tctgccgtct 1560

gaacaatggg gtcagaagat cgctgctgtg gttgttctcg cccctgatgc ggctgcttca 1620

ggtcgtaatg ggcagtcttg gggtccgatg gatatgcggc gcgcgctgaa gggttccctt 1680

gcttctttca agattccgca ggagatgaag gttttggcgg ctattcccag gaatgcgatg 1740

gggaaggtta ataagaaggc actggtgaag gaagtctttg gagtttga 1788

<210> 48
<211> 1701
<212> DNA
<213> Penicillium chrysogenum

<400> 48
atgccaacaa tttaccgctc accatatcca gatctggata tccaatctgt tgatttggtc 60

tcttatctgt tctccaatcc tttcaacacg cccctggacc ggcctatgta catcaatgcc 120

atctcaggag agcaatatac ttttggagat gtggtgcaac gtacacgctc tctgtccaac 180

gggcttcggc aaagtatagg ccttaagcct aacgatgttg tggcattatt cagtcccaac 240

acaatcgatt accctgtggt atgccatgca attgtcggct cgagagcaat cgttgcaccg 300

accagtgcag ctttgacagc acttgagctt aacgctcagc tcaagaccag cggggccaga 360

ttcatcgtcg tccattcaac tctgctggag actgctcaaa aggcagccaa ggggacatct 420

gtcgagaaag tcctcctgat agatggccaa acaccagtca atggtcaacc tacatgcaat 480

tatctcgcca atacatttgc ccccgatgat ctcctcactg tcgatccggc cgaggcagat 540

cgccaaccga cattcatctg cttttcatct ggaacatcag gtgcagcaaa gggtgtcatc 600

acaactcatc agaacatcac ctcgaacctc cagcaatggc gccaacacat gttagaatct 660

ggtcttccct ctcagcgacc aagaagacaa tccgccattg cattcctgcc attcagccac 720

atctatggcc taaacttgtt catgtgccaa tgcttgatat ggggtacaac agtggtcgta 780

atgcccagat tcgaccttga tttatacctc agttgcatcc agaaataccg accagacgag 840

ttggcactgg tgccgcccat tgccctgatg ttggttaaag acccacgagt ctccaaatac 900

gatctaagca gcgtgcgcaa gatcatgtct gccgccgcac cccttactat tgaattatcc 960

tccgccctcg aagccaagtt cacggagatc tgcaagacag aggtattctg tacccagtca 1020

tggggtctga cagagacctc accaatggca acagccgtcc cgaatgaccg catggacaag 1080

cggaacacgg gcgtgggctg tatcgcgccg aacatgcagc tccgctttgt ggatcccgaa 1140

tctatgaaag acgcagccgt caaacctgac gggtccactg agcctgcgga gatctggtgt 1200

cgtggtccga acgtggtgat ggggtattac aacaatgaga aagcgacgaa ggaagctttc 1260

cacgtggacg aagatggaac gaggtggttc cgaacgggtg atatcggcac catcgatgga 1320

gatgggtacg tcaccatcca ggatcggatc aaggagatga tcaagtacaa gggactgcag 1380

gtcattccta gcgagttgga gggaaagctg gtcgaccacc ctgacgtgga ggatgcggct 1440

gtaactggaa tgtgggtgga tgacatggct actgagctgc cagttgggtt tgtggttttg 1500

agtccgcagg ctaaagacag ggatcagaag gctgtccttg atggaatcca cgcgtggctg 1560

aatgagcgca ttgctaacca taagcgtctg cgtgggggaa tccatgtctt gtcgcagatt 1620

cctaagtcgc cgagtgggaa gattctgcgt agacagctga gggatttgct gaagagtcaa 1680

gcgcctaaag ctcgtctgtg a 1701

<210> 49
<211> 1755
<212> DNA
<213> Penicillium chrysogenum

<400> 49
atggcggccc caagaagtgt acagcgcttg cagcagacgt tgtcgcacgt acagcctcct 60

attaatccac agctatctct tgtcgcaggc ccgacggagc cacagctgct ggatattacc 120

ttgggtgagc tgctaacact acaagcactg cagtatggga gaattgagtg cttggtcttc 180

ccctggactg gtgcacgatg gacgtatggc cagctgaaag atgagaccga ccgtctagct 240

aggggaatgt tggcttcggg tattcaaaaa ggcgatcgcg tcggaatcat ggcgggcaat 300

tgtgaacaat acatctcggt gttcttcgcg gccgcacgag ttggggctat tttggtcgtt 360

ttgaacaaca cgtacacccc ctccgagctg tattacgccc taaatcacag tgaatgccga 420

atgcttttca tgacaccacg aattggacgt cataatctcg aggaggtcct tacccaactt 480

ggccctaatc ctaaaaaagc ggcgacctcg gaaacactgg aggaaattgt cattctccgc 540

gaaagctata acaacttccc cacctatcac gatgtcatgg aacgcggcct gtcccaggct 600

gcacatgtgc tccaggaccg cgaggccgag ttacagccgg atgatgtctg caatctgcaa 660

ttcacaagcg gttcaacagg caatcctaaa gctgcaatgt tgacacatca caacttggtt 720

aacaactcgc gctttattgg tgatcgcatg aatctcacct cttttgatgt tctgtgttgc 780

ccgccgccat tgttccactg cttcgggctg gtcctgggca tgcttgcggt agtcacccat 840

ggcgccaaga ttgtcttccc tagcgaaacc ttcgacccca aggcggtttt gcatgccatc 900

tccgacgaga aatgcactgc tctacacggt gtcccaacca tgtttgaggc gatcctttcc 960

gttcccaagc cctcaaactt tgatacctca aacctccgca caggcattat tgccggggcc 1020

ccagtcccac ggccactgat gaagcggctc tttgcagaat taaatatgac cgaatacact 1080

agtagctatg gtctcacgga ggcctcgccc acttgcttca atgctttcac caccgacagc 1140

atccacacaa gattgacgac cgtcggaaag gttctgcccc acgctcgcgc gaagattatc 1200

gatacccatg ggaacatcgt tcccgttggt cagcgtggcg agctttgcat ggccggctac 1260

cagctcacca agggatactg gaataacccg gagaagacgg cagagacatt cataaccgat 1320

gccgagggtg tcaaatggct gaagactggt gacgaagctt ccttcaacgc cgaagggtac 1380

tgcactatca ccggtcgctt caaagatatc atcattcgtg gcggcgaaaa catttatcct 1440

ctcgaaatcg aagaacgatt ggccgcacat ccgtcaattg agttagcatc tgtgattggt 1500

attgccgacc ccaaatacgg cgaggttgtt ggggctttta tcgcgatcgc ttcgggagca 1560

gaacgtccga ccgacgatga actgcgcaca tggactcggg atactttggg tcgacacaaa 1620

gccccacagc atgtgtttgt attcggagaa gagggggctt cgagcacggt tcccgtgacg 1680

ggaagcggga aagttcgcaa ggtggagtta cggcaaatgg cgatggcggt gctggccgag 1740

cgccggaagg catga 1755

<210> 50
<211> 1578
<212> DNA
<213> Penicillium chrysogenum

<400> 50
atgccaatcc cttccctacc cctcttcgcc gcagcggagc agcatgcgcg ccagaatccc 60

gagaagatcg cggtcattga caccacgaaa caacaatctt tcacgttcgt tcaattgctg 120

gccgacgcag cagcgttaag gaagcgcata attgagcaat tgggattaac ggacgacttg 180

gaagagcgac gcattgcgtt cttagtcccg aatggatatg actacgtcgc tacgcaatgg 240

gccgtgtggg cggcaggagg tgtctgcgtt ccactttgca tcagccaccc agtgaaagaa 300

ctgctataca ccatcggaga ttcggacccc tcattgatta tcgtgcatcc ggagttcgag 360

aagatcgcac cctcgctgcg cgaaggctac accaccgata taccattcat agggctggag 420

ccattctctc gaaatgagac cccgacttta ccatctttct ccccgccgtt tgcgctcacg 480

cgccgcgccc tgatgatcta tacctccgga accacatcaa accccaaagg ctgcgtgaca 540

acacatgaga acatcacctt ccaagcgagt tgtctggtta aagcctggga gtacaaaccc 600

tcagaccatc ttatccacgt gctgccactc caccacgtcc acggcatcat caatggccta 660

gctgccagtt tcctaagtgg tgcgacagtg gagatgcatc cgaagtttga cccgaaggta 720

atctggggcc ggtggcagga ccacagctcc tcgaccctgt tcatggccgt gccgaccatt 780

tactcccgtc tgaatgacta cttcgatgct catatccgcg gcacggaaca agaagatgca 840

gcgcgtgccg gagctcgtgc tctacgactt gtcgtcagtg gatccgcggc cctcccgacg 900

cccattaaag aaaaattcgc cgaaatcact ggtcaggttc tattggagcg gtatggaatg 960

accgagatcg gcatggcgct cagttgcggg ctggaagtcc aaaagcgaat cgacggtagt 1020

gtggggtggc cgttgcccgg cgtggaggta cgactgaccg ataaagaaac cggtcggatt 1080

gtggacggag tggatgagga cggcatgatc gaggtcaagg gcgggaatgt cttccgcgag 1140

tactggcgga aaccggaagc cacggcctcg gagttcacgg cggatggatg gttcaagacg 1200

ggtgatgtgg ctaaacgcga tccaagcggt gcatacttca tccaaggccg tgcttctgtt 1260

gacctgatca aatccggtgg atataagatt tcggcgttgg aagttgagcg caagatgctg 1320

gccattcatg cgattcagga agttgcagtg gtcgggttga ccgatcaaga atggggacag 1380

cgagtcgctg cagtcgtcaa gttccgagaa gggactgcac cgatggaact tcctactcta 1440

cgggctgagc tcaagaatga gatggctcct tataagatcc caaccgttct gaaggttgtg 1500

gatgggatcg agcgcaatgc tatgggcaag gtgaataaga aggtcattgt acaaaagtac 1560

tggccggata aggcatga 1578

<210> 51
<211> 1650
<212> DNA
<213> Penicillium chrysogenum

<400> 51
atgattttgg aaccagcaga gagagtcctg cttccaacaa aggacctatt gtcctatatt 60

tttgatgacc caccatatga ccaagaccag ccaatctaca tcgacgtcaa caatccctct 120

cggtcgatct cctgtaacca ggcgaaaagg ctagttcgtc agctcatcgc cggtctacgg 180

gaatctggtc tccaaaaagg cgattgcgtt ctcattcatt ctttcaatga tataaattat 240

agcattcttg tgttggccat tattggagct ggaggatgtt tcacgggctc gaacccatct 300

tacactcctc atgagctcgc tcatcatatc aaggcctccg aaagcaggtt ccttttctcc 360

gagcccgaga tcttcgattc tctcatgcgt gccactgagg ggaagaatat acccaaacaa 420

aacatttgga tcttcgacaa tctgggacaa tctatcccgg ctggaatgcg atcgtggaaa 480

cagctattgg aagtcgggga ggaggattgg gtgcgattca acgatctcga aacctgccaa 540

cagactacag cggcgaggtt gtttagcagt ggcaccactg gtctacccaa agctgtgaca 600

atcacacacc acaacctcat tggacagcat gagcttgttc acggtgtcaa tccgcgctct 660

taccctattt cacgggtcat tgcagtaccc atctttcatg catccgcagc tccagtgtcg 720

catatttcaa cactaaaagc tggctcagtt gcatatatga tgcgtcggtt cgacctcgag 780

acatacctca ctacagtcga aaagtacaac gtcaccgacc ttgctgttgt tccaccaatc 840

gtgatagcaa ttctgatgtc tcctctttct cgaaacaaac cctatttgcg aaaggctcgg 900

atggcagggt gcggagcggc tcctttggac aaggacgttc aagccaggtt ccgctccctg 960

atgggcaacg ataccccatt cactcaggta tgggggatga ctgaaacatc gtgtgtggct 1020

acaatgtttg cccacccaga gcacgatgat acgggctctg tcggtcggct catcccaaac 1080

ctcgaagcca aactcattga tgaagacgga aagaatatct cagccttcgg cgtccgtggc 1140

gagttgtgtg tccgaggtcc gacagtgaca ccggggtact tcaacaatcc cgaggctaat 1200

tctgaggcct ttgacgctga gggttggttc aaaacgggcg atatagccta ttgtgaccag 1260

cgcactcgca aatggtatat tgtggatcga aggaaggaac tgatcaaagt ccgcggcttc 1320

caggtcgctc cacccgagct agaggcggtg ctcttgtcgc atccgcagat tgtggacgca 1380

gccgtcatcg ggataacctt cgctgatgcc gatatagagt atccacgagc ctatgtggtt 1440

cgacgatccg gcgatcaagg agccaagcta acggaagctg agattcaaga atacgtctta 1500

agccgacttg ccaagtacaa agcactcaca ggtggcgtga aatttgttgg tgcgattgca 1560

cggaacccaa gtggcaagat cttgaagagg gtattgcgcg aagatgctag gaaagaaatc 1620

gaggctggac tcctgaaagc caagctttaa 1650

<210> 52
<211> 1512
<212> DNA
<213> Penicillium chrysogenum

<400> 52
atggtgtaca ctgctgaacc ccttgagtac ccagaaggga caactctacc cgagcttttg 60

ctcgagcgca acgtgaacaa tgttccacct gatatgcctg ctataattga cggggtttct 120

ggggcaactg tgtacaacta tagcagcttc cgagccactg tgagaagagt cgccaactac 180

ctctgccagg atatcaacct tcctcgtggt gcagttgtcg gaattcttgc ggcgaacaac 240

ggactttcac gagcaaagct ggactactca ccagatcttt atgtcctatc gcccgatagc 300

tcccatcctg ctccctggat ccattttgat cttggacata tcatagccac cggcgcagaa 360

aagtccgaca ctgtggagcc acgatctaat acgacaagaa gcgacctagc cttcttatgc 420

ttcagctctg gtaccacagg gccgatgaaa ggggtctatc tcacccatga aaacatcatc 480

acaaatattt ttcaacaccg acagcgtcta ccggaaatgt tccaaaacca ccagacggtt 540

gcagccctca tcacgccatt tttccacatt ctcggcttgg gtgtatttgt ctgtcaatat 600

atttgccaag taagggtcct ccgtgttccc gtatccctgt atcacaccaa ccccggtaat 660

aattttcgct agggcatccc gattttaccc atatcaacat tgtacccccc cattgctctg 720

agactgcttc aagcaaccac caccagcacc acggatttct catctctgcg tggcctcatc 780

aatgctgctg cacccttgaa ggagattgtt tccagcgaac tgtcgcgacg catgggatgc 840

aatatcactc agtggtatgg gatgactgag gctagcccaa gcgtgatttc ccagagggag 900

gacgaagtag agataacagg cacagtggga cgacttctcc caggcatgtc gatgaaaatt 960

gtggattcgg aaggggaagt agtttatcct aaccgcgaga tagagtgtga gccgaataag 1020

cctggtgagc ttctgataca gggttcgaat ctaatgtctg gctacgttca aaacacggag 1080

tcaaatgacg cgtttatcga cggttacttc aaaactggcg atattggata tgttaacgag 1140

gcgggctatg tattccttgt tggtcgttcc aaagagctta tcaaggtgaa aggacaccag 1200

gtggctccag ccgacttgga gtctatcttg ctcagccatc ccaaagtgtg cgatgccgcc 1260

gtcaaaggag tctacttccc agaacaggaa actgaatatc cagcggcgta catcacaatc 1320

gactccgcgc aaactgagcc agggcagcta caagccgagg ttgaggcttt cgtgaataac 1380

caagtagcca aatacaagtg gctacggagc ggcgtgcaca tcattcccgc gattcctcgc 1440

aatgcatctg gtaaaattct ccgacatcag ctccccgatc tgaaaacgag ccagcgacaa 1500

actaaaatat ag 1512

<210> 53
<211> 4860
<212> DNA
<213> Penicillium chrysogenum

<400> 53
atggcggact tatcaagatc ttcacttata gaaaagcttc tatacctccg cagtgaggcc 60

tcccatccag attccttcca ccaactctcc gccttattgt cctcacacac cggtccttgg 120

cccatccgac acctaatctc aacattccag gccatctaca agtccattcc cgaatccctc 180

aaggccaata acaatgaatc aatcacccgt ctctgttcac aagtcaaagc agccaccacc 240

agcgttgcag aattaatagg gacagactcc cgcgccggtc ttatcgacgc taacaccgga 300

cggcatctga cacataatgc cattcggcag ttcttggaga atttcaagat tccagtggga 360

ctctcccgcc atggaaagcc tcgtatcgct gtgatcttgc ccaacggccc tctgatggca 420

gttgctgtcc ttgctttcgt caacagatac accattgtgc ccatgactac gaacacggtg 480

gcagagcaac tgcagacaga tatagaaaac tcacaagctg atgctgttgt cgcgctggac 540

gcggacattg gcaagttgca actcgacaac ggtacccggc cagtgtttgg cattgagcag 600

cttgaagact tgactttccg cgtggtctct gcaggaaata cttctactgc atacgaccat 660
ccccccaatt ctggtgatga cattgccatt atcctgttca ctagtggtac cagtggcacc 720
aagaagctgg ttcccatcac aacttacaac ctcatcgctg gtaccatcgc tactatagag 780
tctgtggaac tgtctgagac agatacatgc cttaacatga tgccactgaa ccatgtcggt 840
ggcatcatgc gcagtatatt ctcccccatc ctcgccggcg gtgctacgat atgttgtccc 900
tcgtttgacc ccagcatgtt ctgggatgct gtacaagccc ctcacaccaa accaacttgg 960
tactacgcca cacccacgat gcatcaaatg attcttgccg aggccgagca ccgtcctgat 1020
gccgtaaaac aaagcgccat ccagtttatc tgcaacgctg gtggtggtct ccctccaacc 1080
cttgcagtgc agcttcatag cactttccac tgcgttgtcc tccccagcta cggcatgact 1140
gaatgtatgc ccattgctgc gccgccgagg gactataagc ttgatcgccc tggaacatcg 1200
ggaaggattg ttggccctga ggttgcaatt ctgactgaaa gtggcaaccc tgtcacgcaa 1260
aatgggatgc tcgggcacat ttgcattcgt ggatcacccg ccttcgaggg ctatctgacc 1320
cctggcggca agattgatac cagtgcgttc aatgagtctg gctggtttga tactggtgat 1380
cttggacacc tagatgatga caattacctg tacatcactg gccgcagcaa ggaggttatc 1440
aaccgcggtg gtgaaattat ctctcctgtc gaagtggaaa atgcggtcct gacctcggcc 1500
aaggaccccg agtccccact gtatggtcgc gtgactgaga cattggcctt ttctgtccca 1560
gacgaggtcc tgcaggaagt cgtcggtgtg gttatcgtca cgcctccagg cttcactagg 1620
ccggatcttc gccagataca cgaagcgctg cagcctatta tacatcagcc caagtggcct 1680
gctctggtgg tgtacatgga cggcgtgccg aaggcaaaca acaagattca gcgcattaag 1740
ctcgcggacc gcttgtcgtt ggatacctta accacaacta cgcccctggc gagccggcac 1800
tacgaagctg tctgtccacc cactggtgct ccgctcagtg cattgattcc gaagaagcca 1860
tgtgtcatcg atgaaaaaat gattcgttca gtcctcagca agaaggcaaa cacacctgac 1920
gtgcacgtgc aaataaaccc gcgcgacggg ctcgcgcagg ttgttttgtt tgtcgagaac 1980
ctcgacgatg accatgtgac acctgccgag ctccacgacc agctagatgg ctatcttgta 2040
cctagccgta tcattcccct gaaagggccc atgcctgtcg atttctacgg aaaacctgat 2100
caggccgcta tcaatgaggc aatccacgcc cgaaactccg atggcgacct gtcacccatt 2160
cagcggcgcg tgcgtggaat cttcgctgtc accttgtctt gttcaccaga ggaagtatcc 2220
agcggtaccg acttttttgc tgctggaggt gatagtctta gtgctggccg tatggtctct 2280

caacttcgtc gggaatttgg tattttcttg gccggtgaca ttctcttcca ccacagcacc 2340
gttggagcaa tagagcagaa gattatcgaa gctgttgacg ttaaggctgc caagggcgaa 2400
gacggcggag ttgaattgcc gggttgcgag aagacataca gcagcaccaa cccaattatc 2460
ttggttctgc atctgttccc gactgtgttc ttcttcccta tgaagcgtgc cttccaatgg 2520
atggtgttcg catacgtcgt ggccgagtgc tccaatcgat ttccgattcg tgagaacctc 2580
atcgctcgtt tgatgctggt tgttttcgcc gtgatgtctg ctcgcttatg ctcacaaatt 2640
gtgtctccca ttctcgctat catcttcaag tggcttgtca tcggtcgcta caaggaaggc 2700
atgtccccaa tgtggggccc atatcatacg cgctggtggt tgacccagaa agcgacgcag 2760
gtgtgtggaa aggggctctt caacaactat aattggtctc gcatcctatt ctaccgtttg 2820
atgggagcaa agattggaaa gaatgttacc gtgtctgcgt ccgccaagct tggtgagtat 2880
gatctcattg agatcgggga caatgttgtt ctggatacct gcgtgtgtcg cccgttcgcc 2940
gtggagcgca acacttctat gctgctgaag cgcattcgca ttggtaagga ctcgtctgtc 3000
ggcatcaagt ctattgtcgc tcctggtgcc gatatacccg agaggacctg cattggtccc 3060
aactcgtcga gctgggaact ccaagacgcc gatgagtcca accgccaact gctaacgtct 3120
cagattccta agcctcactg gctctggatt ctctttattg ttgaaccgat caagttggtc 3180
acttggaccg ctactcgtat tacctggatg gccggtctta ttcctatggt tctccagttt 3240
cctaccccag cggccgacat gttccggtct accctagact ggtacaccag tgaccaccgg 3300
attgcatatc acattaccgc acggatctgt cgtgcggttg gtggcccgat tgttcttttt 3360
attgcggtat tggtcatcaa gttcttcctg gatctcatct gtggcaagcc caagcctggt 3420
cccgcctcaa aacagacgac tcgccaaaag attcggagtg ctgtgctcgc acagattcta 3480
cccgcaggtg acattcacga gctcactcgc ctgacaggtc gtcactacga atttgtctcg 3540
atggcagtcc gtgcccttgg tggtaaggtt ggcaagcatg tctactggcc aagtgttggt 3600
ccagtcacgg tcgactttga tctcattgag gttggtaacg atgttgtatt tggttcccgc 3660
tccactctgg tcacatccga tggctacggc cgggaccgca tcgtgattgg cgatggcact 3720
atggtgggcg atcgtgtcgt cgctttgccg ggcgctacca tcggccgtca agccatgatc 3780
ggttccggtg ctctgcttcg ccgtaacggt gaatatccct ccaacactgt ctggactgga 3840
agcaagggtg gtgaggccat acagttccca agttccacct cgaccaccac ttcgaccgct 3900
ccaaccatca ttggggatgg tagcagcagc cccaacagca gcggcagtga agacgagagg 3960
tcacccactg agaagatgcc cactgagaag cagacctact atggcagtaa ggacaagact 4020

gttacccaga tcgctgagca ggatatcgat acctgcaagc cattcggtcg cgccttctac 4080

cgccatgagg ccaactacta cgtcctccgc atgtggcaaa ttgtgatcta ctctactttt 4140

gcagtcattg tcactactgt ctattggctc ctcactgtct tgttctccct ttttgctctc 4200

cgcactgtcc tccagtacag tgacgccgct ggtttcaagc aaggcgcctg gcgcccattc 4260

gttctatacg gcactctcgc ctcaatcctc tccgtcatca ctgtcgctca agtcgttcta 4320

gccttcgcca tcatcctctg cattaaatgg gcagtcgtgg gccgccgcaa ggaaggatcc 4380

taccactggg acaagagcag ttacaaccag cgctggcaat tcctcctttc ctgcgagacc 4440

ctgatcaaag actgctatga cggagttggt ctcctgccta tgatctccgg ctctgcctac 4500

atttcctggt actaccgcct cttgggcgca aaaattggaa aggattgtgc gatacacgcc 4560

aatggtagtc ccagcatctt ctttaccgag cctgatctcc tcacccttgg tgaccgggtt 4620

gctgtcgacg atgccagctt ggtctgccac ttgaactcac gtggtggatt cgagttgcat 4680

accttgaagg ttggggatcg gagtattctg cgcgctggat cccgtttgat gtccggcgct 4740

tcaatgggtc aggatgcttg tcttttggaa catactctgg tcttgtcggg tgaccatgtt 4800

gaggatggag atacgctcca gggatggccc gctgaagggt ttgaatggaa acgggtttaa 4860

<210> 54
<211> 1905
<212> DNA
<213> Penicillium chrysogenum

<400> 54
atggacattt tatccgaagt cggtgcgcct gctgtagcta tggcatcggc cttgtcggtg 60

gccgccggcg cttatctgaa cgcgaagctt gccattgcca ctgatttgcg aacaatccac 120

agcgacaagg ctgccgcaaa gcggttaagt gagcgcatcg ctaagctcga tggctcaaca 180

accatctaca agatgctcga gcgggcggtg gaagtggaag gacgagctac cacagatgcg 240

ctctggtttg agcagaagac ctggtcgtat ggccaattga aagatttggt cgacagaatg 300

gctgctcttt tgaagagtcg tgatatcaat cccggtgata cagtgggtgt ctttaccacc 360

aactcaccgg agatggtcat gaccgtatac gctctatcaa aactgggcgc tgtggcagcg 420

atgatcaata ccaacctgcg agacgatact ttcacccatt gcgtcaacgt ctccggctcc 480

aaattgatca tctcaaccgc agacctgtgt caacacgtct gcgtcgacct accacacttc 540

acactcagcc tgggttcatt cgaaggtgcc gaagcaggcg caattgagcc catcacttct 600

ggaaccctcc agcaattctc cccattagga cttgcagcag ccaagcgtag cccaaaggac 660

ctctccctcc tgatctacac atcaggaaca accgggaaac ccaaagcctg cgcgatccgc 720

aacatgctca ctctaatcac ctccaaccca cactcggcag atgtaagcaa ccactcaaag 780

taccacccat tccgcgtgta ttcccctcta ccattattcc acggtacagc cttcttcacc 840

ggcctttgtg ccgccatcgg aaacggaggc acattatgcc ttggacgtaa attctccgcc 900

tcgaaattct ggaaggaagt gcacgattca ggtgcaaccc gcatcctata catcggcgag 960

ctatgccggt accttctcgc gacaccgcca tcaccctacg accaggacca caagtgcatt 1020

gtggcaagcg gtaacgggct gcgcggggag atctgggaga agttccgtga gcgcttcaac 1080

gtccctgaga tccgcgagtt ctaccgctca accgagggag ttgccaaatt cgacaaccac 1140

ggtgttggag cttggggcgc tggcaaggtc gggttttctg ggcctatccg ccggttcttg 1200

gaagatgaca ctttcatcgt caagtacgat actaacaccg agatgccgta ccgggatcct 1260

gccactgggt tctgtgtgag ggctgcgctt ggacaggagg gagaggcaat tggtcgcgtc 1320

cgggaccgcg gtatgctgat tgagtatctg ggtaatgagg gtgcgacgga agagaagctt 1380

ctgcgtgatg tgttccagaa gggagatctg ttccagcgga ctggtgatct ggttgttcaa 1440

gatgaatctg ggtgggtgag gttccaggac cgggttggtg acactttccg gtggaagggt 1500

gagaacgtta gtgcgggtga gattcgggac catatttgcc ggattgaggg tgtgcatgat 1560

gctgtggtat atggtgtgaa gctaagcggg tatgatggtc aagctggagc cgctggtatt 1620

acactagagt caccagaggt tgagactgag ttgatgtcga ccttatgcaa ggcgctcaag 1680

aagaagggtg ttccttcata cgctctgcct cgactggttc gactcactga gaaggttgct 1740

acaggtgtca ctttcaagca ggctaaaggt gatttagcga agaagggctg gaatcctcgc 1800

caggactcgg gcggcgatat tttgtactgg ttgaacggca caaagtatca gaagctggaa 1860

gagcaaagtt gggcggagat tgagagtggt aaagccaaaa tctga 1905

<210> 55
<211> 1788
<212> DNA
<213> Penicillium chrysogenum

<400> 55
atgtcttcaa acaatatgct cccccaggag gcttactctg acttgctgag tttcacattc 60

gatggcccaa agccatacaa ccagaatcaa cctctgttta ttgatgctga ggatccatcc 120

cgatcattta cagctgccca atttcgacaa ctagtccgga ccttgattgc tggtctgaaa 180

gcccataacg tccagccagg ggattgtgta ttgctacact tgggaaatag catactatat 240

cccgccctct tcttcggtat aattggcgcc ggcggtgtct atatgggatc aaacccccga 300

agccaccctc aagaactcga ccacgtcctc agcctcgccg aacccaaact gatcctcaca 360

acacgtgatg ccctcccctc cgtcctcgat gcctccgctg gccgaggcat tcatccagcg 420

caggtctgcc tcgtggacga gcacgccatc gaccactgcg cacaactatt cctgtggtac 480

gaactgggat actcatcggc gggacaattc tgcgccatga atggcgacgc gcgtcacaac 540

aatttcgcca atttactctg cttcggtgaa agcgactggc tgaagttcgc ggaccctgtc 600

gtcgcacaag ccacgcctgc ggccatgtat ccgacaagcg gtacaggcgg acttcccaaa 660

gcagcaatac tgtcccacta tgcgcttgta tcacagcacc gtactatcta ctacgaagtg 720

ccgcatccag ttagccggct catctcgctg cccatgttcc atctgtttgg cgcgctttgg 780

acacatctgt tccctgtccg atacgggcac ccgctgtttg tcatgccgcg ctttgaggtc 840

aatgatttct tggccgctgt ccacaagtac cagatctccg agacttacct ggtcccggct 900

atcatccacg ctatcaacca gtcatccgtg cccatcggtg acctattgaa atcgctgcgc 960

tatgttggcg ttgctggcgc tcctattgat ggtcactcca tgcagcagtt ccgcagccat 1020

atcaacccta tgggatatgc gtgccagatc tgggggatga cagaggtcgg tgtgacattc 1080

caaactcgtt ggggccagca gggtgaccct ggaagtattg gacggtgtat tgctgggtat 1140

gaggcccgct tggtgcaacc agatggaaag accgtgcagg gtgataactg ctcgggtgaa 1200

ctctacgtcc gtgggccggg tctgctcact gcatacaagg gccgcacaga cgcattggag 1260

ccacatggat ggttccggac tggagacata gcgtatgtca agcagggcca gtattatatt 1320

gttggacgga cgaaggagct tatcaaagta cgaggatggc aagtagcccc agctgaagtt 1380

gaaggcgtcc tcctccaaca cccgggaatc cttgatgccg gcgtgatagg cgtgaacaag 1440

gatggtgtgg gcgaagtccc acgcgctttc gtcgtgcgct ctcgcgaccc atccgtgcgc 1500

cgcctaaccg gcgaacaagt ttacaattat tcacgccagc agctggcgag gtacaaagcc 1560

ctcgatggag gcgttgtatt cgtggaagag atcccgcgca ctgcaagcgg aaagatccag 1620

cgcttcaagc tgtcccagat gaactcgtac cgggagatgg tggcgtccct gctttcccga 1680

ttcgaaggcg agggctctgc tgcagccacc gcggcccgac gtggactacc cgctggggct 1740

gagatgtcgc cagttagtct cattcctgag ggacgggttg cggtctaa 1788

<210> 56
<211> 1725
<212> DNA

<213>  Penicillium chrysogenum

<400>  56
atggaagcca gactcgcgct ccttcaaggc tccaaagagc ctgccttgtg gtttgaaaca      60

ctgggaaact tcatcgacaa gcaagcttcc cagtatgaag accgagtagc tgctattttc     120

ccttggcaat ccgtccgact ctcataccgt caattggcag agcggagcaa gattcttgca     180

aaggcaatgc tagagatggg actacgcaag ggagactgtg ttggagtcat ggcagggaac     240

tgttatcagt atattgaggt tttcctcggt ggcgggcgca ttggatgccc tgttgttgtt     300

ctgaacaata cctacattcc aagggaacta atgagtgctg tgcaaaagag ctcgtgcaaa     360

ttggttttcg ttgcctccga tattggttcg cggagtctct cggctcatat caacgcgctt     420

tgtggtgatc aatcgagaaa ccctgctctg ccagaactcc gccgggttgt caactttggg     480

aataaggatc caagtagcac cggagtcgaa atgcagtctt acagcgcctt tacctctggc     540

gcccaatctg tcttcatgaa agactcaatg ctgctccgtg cagagaagtc cgtggagccc     600

gaagatgtcc tcaacctaca attcacctcc ggaaccactg gctccccaaa agctgccatg     660

ctaagccaca ttaacctcct caacaacgcc cgctttgtcg gcgacgcaat gcacctcacc     720

ccaacagaca taatctgctg ccccccacct ctattccact gctttggttt agtcttaggc     780

ttcctcgcgt ctttcgtaca cggcagctca atagtatttc catcagactt cttcgacgtc     840

cggaaattcg tttcaacaat cctctccgaa aacgcaaccg tcctcctcgg cgttccgaca     900

atgtacattt cggagctgga ggtcctttca aaaagtcgac agcgtcccca ccatctgagg     960

acaggtcttg cgtcgggctc ggctgtttca caaggtctta tgaatcagct cagagaggaa    1020

atgggcgtac agaagatgtt gatcgcgtat ggcatgaccg aaaccagtcc agtcacattc    1080

attacctcta ttgaggatgg ggatgagaag gggacatcga ccgtgggtag agtcctgccg    1140

catacgggtg ccaaggttgt tgggaaaggg ggcgagattg ttcggagagg ggagagggga    1200

gagttgtgta ctagtgggtt tgccctgcag aaggggtatt gggggaatga ggagaagacc    1260

cgggaggtca tgagggtgga tggggatgga gttttgtgga tgcatacagg ggatgaggct    1320

tttattgatg aagatggata tgctcatatt acggggagga tcaaggatct cattatacgg    1380

ggcggggaaa atatatttcc gagagagatt gaagagcgac ttacgttgca tccgtctatc    1440

agcgaagcca gtgtggttgg aatcaaagat gaaagatacg gcgaggttgt tggatgcttt    1500

ttgaaaatgg ctgaaggata tcccaaagtc cctgatacag aggtgaaaca gtgggtaggc    1560

gagaagcttg gtcgacacaa gacgccacag tatacattct ggattggaga tgaaaaagtg    1620

ggaaccgatt tccccaagac tgggagcggg aaacatcaga agcacatcat gcgggacctt 1680

ggaaatttgc tagtgcagag ggatacggcg agagccaagc tatag 1725

<210> 57
<211> 1359
<212> DNA
<213> Penicillium chrysogenum

<400> 57
atggcaacta gacttgttcc caaaggttgc cctctagtca ggactaccat acaagttttg 60

gaacatcgca aacatcgtat ccatttcgct tcttacttag ggaatcatgc tcaatttcgt 120

cggagtcacg ctttggcacg tgccaatggt cccttgttgg ggattcgaat tcttgatctg 180

acgcgtgttc tcgcaggacc attctgtaca cagattctcg ctaattatgg ggctgaggtt 240

ctcaaggttg agcatccaaa agggggtgat gacactcgct tgtggcggac ggcagccgag 300

aaacatgttt ggaaatccac tggcaaagat atgtctacat atttttgtgc gatcaatcgg 360

aacaagctat ctatgacttt gaacctcaaa caagagaaag gccgggagat tctctttcga 420

cttgtcaaag aggccgatgt ggtagtggaa aattttgttc caggaaagat ggatgaaatg 480

ggcattggat atgaaaagct acgggaaata aatccatcga tcatatatgc aagcgtatct 540

ggatacggcg ctagtggtcc atattctcat cgcgcaggtt acgacgccat tgccgctgcc 600

gaagcaggta tgctatacat cactggcgag cgcaatggac ccccaacacg acctggtctc 660

ggcctcactg atatgagcac cggtctatac ctgcacgggg cgatacttgc cgcactttat 720

gctcgcaggg atacaggtcg gggccagaag attgatacgt cactatttga gagccaggtg 780

tccctgcttt ctaatgtggc tatgtcatgg ctgaatggcg gggaacatgc aaagcgttgg 840

gggacagagc atccgagtat tgtcccctac caagctttta agacaaagga cggtcacttg 900

gttctaggcg ctacgaacaa ccgccagttc caaactcttt gtcggttggt gaagctatcg 960

gagttggcga ctgacccacg atttgtggac aacagctctc gtgtccaaaa tcgggctgag 1020

ctaaaagaga tacttgagcc aatcatcaga aggaagccga cgaaagtctg gctgtcagac 1080

ttggatggta gtggactgcc ctatgggcct gtgaacacga ttgaagaagt gttctcgcac 1140

ccacaaaccg ctgcgaggga tatggttcac agccttcccc acgaagcgtc ggaatcagag 1200

gagatcagac tactagggac ccctgtcaag tttagtggga cgaagcccca aattcgacga 1260

tcgccgccaa gtctaggaga gcataccaat ttcacactcg agagaatggg attctcagcg 1320

aaggagatag aggtaatgcg agaacagggt attgtgtga 1359

<210> 58
<211> 1341
<212> DNA
<213> Penicillium chrysogenum

<400> 58
atgacggtcg cgctcaggtt tcaggttgct gcccgtcgat cgggcttgtc ctggggtagc 60

aatgccctgc ggcgcacaat accaggacga agctactcca gtgcagttgc agctgacggg 120

tccctgcctt tggctggtat tcgggtgctt gatatgacac gcgttttggc tggtccctat 180

tgtactcaaa wactaggtga tctaggagcg gatgttatca aggtcgaaca ccctgtgcgc 240

ggcgacgata ctcgtgcttg gggtccgccg tatgcgaagt acgaagatgc ttccagaaag 300

ggtcctggcg agagcgccta ctacctcgct gtcaaccgaa ataagaaatc tctcggtcta 360

tcattccaac acaagtcagg tgtcgagatt ctacacaagc ttgcaaagga atgtgatgtc 420

ctagtcgaga actatctccc aggggacttt aaaaaatacg gcatggacta tgaaacgctg 480

cgggaaatca accccaagtt gatctatgcc agtatcaccg ggtatggcca gacgggccca 540

tatagcaacc gtgcaggcta cgacgtcatg gtggaagccg agatggggtt gatgcacatc 600

accggcgctc gggatggtgc gccagtcaag gttggtgttg ccgttaccga tctgaccacc 660

ggactataca catcaaatgc catcatggcc gctttgattg ctcggggtcg gaccggtaga 720

ggccagcata tcgatgcctg tttgagtgat tgccaggtcg ctacactatc gaacctggca 780

agctctgcac ttatcagtgg gcaaaaggat tccggccgtt ggggcacagc acacccatcc 840

attgtaccct accggagcta caaaaccagc gacggtgata ttctcttcgg cggtggcaac 900

gataagctgt ttggcgtgtt gtgtgatcga ctgggcttct ctgagtggaa gactgacgcc 960

cgcttcgtca ctaacagtga ccgagtgcag catcgtgcgg agatagatgg tatgatcgaa 1020

agcaccacta tgcagaagac cacgcaggag tggttggata tattcgaggg tagcggtatg 1080

ccttatgcgg cggtcaacga cattcaaggg accctgaatc acaagcacgt cttggcacga 1140

gatatggtga cggaagttga gcaccctgca tgtggcccca tcaagttggt caacacgcct 1200

atcaagtact ccgaagcgac accgggtgtg cgcacgcctc ctcctactct aggccagcat 1260

acggatgaga tcttaggtag tgtcctcgat tatggggagg cagatatcgc tcgcttgaag 1320

gatgagggag tcgtgtcctg a 1341

<210> 59
<211> 1134
<212> DNA

<213>  Penicillium chrysogenum

<400>  59
atgtcaccat tgaaaggaat caaagtcatt gagctagcag gcctagcccc aggcccattc      60

acaggtctcc tgctagccga caacggcgcc tcagtcctcc gcatcgaccg acccaaatcc     120

ataagtggcg accaactaac ccgcaataaa acctccataa cactcgattt acgcgataaa     180

acctccctaa acatcctcct ccgcctcctc acagcagcgg acatcctaat cgaccccttc     240

cgcccaggcg tgctcgaacg cctcggcctc tccccaacag aagtcctcct aaagcacaat     300

ccccgcctaa tcgtcgcgcg tatgactggc ttccgccgcg acgggaaata caaagatatg     360

gccggccacg acataaacta catcgccgtc tcaggcgtgc tgtccatgct cggccgcaca     420

ggggaaaagc ccacgccccc gggaaacatc ctcggcgact tcgccggcgg cggcgcagtc     480

tgtttccagg gaattctact tgcgctcatc tcccgctctc atactggtcg cggccaggtc     540

gtcgaggcaa atatggtgga tggctcggcg taccttgcta cgttcccgcg gttagggagg     600

aacactcccg cgtggtcgga gccgcgtggc gagaacctgc ttgatggtgg gtgtccgtac     660

tatgatacgt acgaaactaa ggatgccggg cggtatttcg ccgtcggggc tttggagccg     720

cagttttatg cggcgttggt gcgcgggcta gggtttgata tggggcagct tccgaaaagg     780

gaggatcgag agcagtggcc ggtgctgagg gagatcttta cgaagaggtt taaggagaag     840

acgcgcgcgg agtgggaggc tgtttttgat ggcaccgatg cttgtgctac gcctgttttg     900

gagcaggcgg agcttgagga cgatgggtat gagcagaggc ctatggtgca tctggttaat     960

acgcctgcgg caccgattcc cgcggatcag ggtggttggt cgggtggggg acttttacct    1020

ggggatgggg gtgttgatac gcttagggag tgggtgggat gggagcaggg taaggacttt    1080

gaggtaagga aggatggggc tttcgtggcg atagatggga agtcaaagat ttga          1134

<210>  60
<211>  1557
<212>  DNA
<213>  Penicillium chrysogenum

<400>  60
atgccttcgc aagtggcaac atgcctgcgc ctcgcgcgtc agttctccgc caatcctgtc      60

cagcaccaac gtttcatggc ccgcgcattc tccagcagcg tccgccgaag tgagatcaac     120

aaggtgtacc cctccgccga ggtagcagtc aaggacatga agtccaactc gactctgctc     180

gccggtggat tcgggttgtc tggtgtacct gactcgctga tcaacgccgt actggcgaac     240

ccctctatca ctggcctgac cgtggccagt aacaacgctg gtgtggacgg agctggtctc     300

ggtctgctcc tcaagtctaa gcagatcaaa aagatgattg cttcttacgt cggcgagaac 360

aaaaccttcg agcgcatgta cctcactggt gagatcgagt tggagctcag tccccaggga 420

actcttgctg aacgatgccg ttctggcggt gcgggtatcc ccgcattcta cacacccgcc 480

gccttcggta ccgtggtgca gaccggtgcg gtgcccctac gacacaatgc tgatggcaca 540

gtcgcaaagt acagcgaggc gcgcgacacc aaggtgttcg acggcaagag ctacgtcatg 600

gaggaggcga tcaagggtga ctacgctttc gtcaaggcgt ggaaggccga caagcttggt 660

aactgccagt tccgctacgc ggccgccaac ttcaacggtg ccatgggccg taacgccaag 720

atgaccatcg tcgaggctga gcacatcgtc gagcccggcg agatcgaacc ggctgccatc 780

cacctgcccg gtatctacgt caagcgtgtt atccagagca ccacccccaa gaatatcgag 840

aagttcacct tcgccaagga ggagggtgca gacacctctg ctctcggcaa gggtgagacc 900

gccaacaagc gcgagcgtat cgtccgccgt gctgctaagg agttcaagaa cggcatgtac 960

gccaaccttg gtattggtat gcccatgctt gctcccagct ttgtcgaccc ctccgttgag 1020

gtgaccctgc aatctgagaa cggtatcctc ggtctcggcc cttaccccaa gcagggccag 1080

gaggaccctg atctcatcaa cgccggcaag gagactgtca ctctcaagcc cggtgctagc 1140

tgcttcggca gcgacgagtc cttcggcatg atccgctccg gccgtatcga cctgaccatc 1200

ctgggtgcca tgcaggtcag cgccaagggt gacttggcca actggatgct tcccggtaag 1260

atcaagggct tcggcggtgc catggacttg gtctccaacc cctctgccac caaggtcgtc 1320

gtgaccatgg aacacaccga caagaagggt aaccccaaga ttgtcaagaa ctgcgaattc 1380

cctctgaccg gccctgcttg cgtcagccgt atcatcaccg acctctgcgt cttcgacgtt 1440

gacttcaccc acggcctgac tcttattgag atcgctgacg gtgtcaccgt cgaggaggtc 1500

caggctaaga ccgaggcccc cttcaaggtc gccgatgacc tgaagcccat gctgtaa 1557

<210> 61
<211> 1617
<212> DNA
<213> Penicillium chrysogenum

<400> 61
atgacagctc ttagaatatc tactactttg tcgcgcaaag cgctcactgc ctgtcgacct 60

cgactcttcc tcggagtccg caccagtgtg cgcccttggc agccattcag taccagcacc 120

agcactagat actccataac cacgccatta aaatccctcg ctcctaaggt cgaacgcagc 180

ggttcgaaat tatacaaaga tgccgatgcc gctgtcgccg atatcaagag cggatcgaca 240

atccttagct ctgggttcgg tctttgcgga gttgcagaaa cgctcatcaa tgctatgcac 300

cgccggggcg ctgatcaatt gcactccctg acagccgttt cgaacaatgc cggcgccgcg 360

ggcaagggtg gcctttcgac gctctcgcag aatggccaga tcaaccgtct gatactttca 420

tacctcggga ataataaggc attggagaag aaatatttga cgggacacat tgctattgaa 480

ctttgtccgc aaggtacttt ggctgaacgt ctccgtgctg ggggtgctgg gattccggcg 540

ttctttaccc caacaggaga gggaaagatt cccgttcgta tggacgagtc cggtaaagtt 600

ctggagtctg gcaagccgcg cgagacgcgg gaattcaacg gcaagacata cctcatggaa 660

gaagctttga ctggggatgt agctatcctc agagcctgga aagcggatga ggctggtaac 720

tgcgtgttcc gatatactac caaggcattt ggccctatca tggccaaggc cgcaaccctg 780

accattgtgg aagcggagaa cattgtccca atcggctcca ttgatcccaa tgatgtagac 840

ttaccgggta tcttcgtgga tcggatcgtc cctgccacgg acgacaagca cattgaaatc 900

aggaagctgc gctcgggcga gaccactgta gctggttctg gaaaagatga ggctcggatt 960

caaagagaac tcattggtcg ccgggcggcc aaggagctta agccgggatt ctatgtgaac 1020

ctgggtgttg gtattcccac gttggcaccg tcgttcctgc caaaagatgt gaaggtttgg 1080

attcagtcgg agaacggcat cttgggaatg ggtgattatc caactgagca agaactggat 1140

cccgatatca tcaatgccgg aaaagaaacc gtgacgttgg ttcctggagc tgctacattc 1200

gatagcagtg agtccttcgg tatgatccgc ggaggtcatg tagatgtgtc tattctcggc 1260

gctctgcaag taagtgccaa cggtgatttg gccaattata tgatccccgg caaggtcttc 1320

aagggtatgg gaggagcaat ggatttgatc tcgaacccgg agaagaccaa gatcgtggtg 1380

gctacgagcc acgtcgccaa ggatgggtct cctaagatcg tacaaaagtg cagtctgccg 1440

ttgactggtg ccaatgttgt cagcactatc atcacggacc tgtgtgtctt ccaggttgac 1500

cgagctaccg gagagctcac actgacggaa cttgctccgg gtgttgaggt agaagaggtg 1560

cagagtaaga cggatgccaa gttcaccatt gcggataccc ttgagattat ggaatga 1617

<210> 62
<211> 1278
<212> DNA
<213> Penicillium chrysogenum

<400> 62
atgagattat tatcctcact tgcctcccgg ccccatgccc tgtcgggcgc agttcaacct 60

gcaagacgat cacatctcac atggcgtctt tactccagca caccagctgc taaaaacagg 120

cctctgcctc tcgctggggt tactgtggtc agtctcgagc aagctattgc tgcgcccttt 180

tgcacgcgac agcttgccga tcttggagct cgagtcatca aggtcgaacg gccaggtgtt 240

ggagactttg cacgacacta tgacacgcga gttggtggtc tagcatcaca tttcgtatgg 300

acgaaccgtt ctaaggagag tttggctctg gacctcaaac acgaaaagga ccatgccgtc 360

ctgatgaagc tccttgaaaa ggccgacgtt ctcgttcaga acttggcccc aggagccagt 420

gcgcgactgg gcctttctca cgaggcattg aaaggggaac accctgatct tatagtgtgc 480

gatatatctg gatatgggca agacggtcca tatcgcgaca agaaggccta cgaccttctt 540

attcaaaatg aggctggaat gctctccgtc acgggcactg gaaaagagcc ggtaaaggtt 600

ggtatctcta tcgctgatat tgctgcgggg atgtacgcct acagcaatat tctctcggca 660

ctactgcagc gtggtaaaga tggacaaggt tgtcatattg acatctctat gctggaaagc 720

atggtggaat ggatgggctt ccccatgtac tatgcattcg acaatgcccc gggtccagtc 780

ccagcaggtg cctcgcatgc agccatctac ccttatggac cgttcgaagc tggtgatggc 840

cagtcggtta tgttgggcat tcaaaatgag cgcgaatggg tcagtttctg caacggcgtc 900

ctctcacaac cagatctggc caccgaccct agattttcca gcaactcaca acgaactcaa 960

aatcgggatg agctcaagtc cattattcac gatgcattct cgaagtttac gacaactgaa 1020

acaattgctc ggttggacga agcatctatc gccaacgcta ctgtcaatga catgaaagat 1080

gtatggggcc atgctcagct tcaggcacga gatcgatgga ccaaggttca aacccctgta 1140

ggggctattc cggctctttt gcctcctgga tcaaccaatt cagcgggaag agatggacat 1200

ggcgctcaga tgggttcagt tccacgggtc ggagagcaca atgaggccat ttttgcggag 1260

ctgggtttat caaattga 1278

<210> 63
<211> 564
<212> PRT
<213> Penicillium chrysogenum

<400> 63

Met Gly Asp Arg Thr Leu Phe Ile Pro Pro His Val Gly Asp Asn Val
1               5                   10                  15

Leu Pro Asn Leu Pro Phe Phe His Arg Leu Leu Arg Tyr Ala Gln Arg
            20                  25                  30

Lys Pro Ser Pro Ile Val Val Arg Asp Leu Val Ala Asp Ala Glu Lys
        35                  40                  45

Thr Tyr His His Leu Val Ser Asp Val Leu Ala Phe Arg Lys Val Leu
    50                  55                  60

Glu Arg Ser Ile Ser His Glu Ala Arg Arg Asp Leu Asn Ala Asp Lys
65                  70                  75                  80

Glu Val Tyr Ile Gly Leu Leu Ala Pro Gly Gly Tyr Glu Tyr Thr Val
                85                  90                  95

Gly Phe Ile Ala Ile Leu Ala Ile Gly Ala Ala Val Val Pro Met Ala
            100                 105                 110

Ala Ala Leu Pro Ala Glu Glu Ala Ser Tyr Phe Leu Leu Lys Ala Arg
        115                 120                 125

Cys Val Gly Leu Val Ala Ser Thr Ala Ser Glu Lys Thr Ala Gln Ser
    130                 135                 140

Val Val Arg Tyr Met Gly Glu Ser Lys Gly Met His Ile Pro Cys Ile
145                 150                 155                 160

Ser Pro Ile Ala Ser His Phe Arg His Thr Leu Leu Pro Ser Asp Glu
                165                 170                 175

Met Thr Ile Ser Ser Gly Pro Val Pro Asp Met Asn Ala Ala Ala Leu
            180                 185                 190

Val Ile Phe Thr Ser Gly Thr Thr Gly Pro Pro Lys Gly Ala Val Gln
        195                 200                 205

Arg Arg Ser Tyr Ile Ser Gly Asn Gly Glu Ala Asp Ala Ala Tyr Tyr
    210                 215                 220

Arg Ile Thr Asp Lys Asp Thr Val Leu His Val Leu Pro Val His His
225                 230                 235                 240

Ala Ser Gly Val Gly Leu Thr Phe Leu Pro Phe Leu Ala Ala Gly Ala
                245                 250                 255

Cys Ile Glu Phe Arg Cys Gly Ser Phe Asp Thr Ala Trp Thr Trp Glu

260                 265                 270

Arg Trp Arg Arg Gly Gly Leu Thr Phe Phe Ser Gly Val Pro Thr Ile
        275                 280                 285

Tyr Met Arg Met Met Arg Tyr Tyr Glu Glu Asn Ile Ser His Gln Ala
    290                 295                 300

Pro Glu Ile Arg Asp Gln Tyr Val Ala Gly Ala Arg Gln Ile Arg Ala
305                 310                 315                 320

Met Leu Cys Gly Thr Ser Ala Leu Pro Gly Pro Val Gln Glu Phe Trp
                325                 330                 335

His Asn Ile Arg Ser Lys Pro Ile Leu Thr Arg Tyr Gly Ala Thr Glu
            340                 345                 350

Phe Gly Ala Val Ile Lys Thr Glu Leu Asp Ser Asp Gly Thr Pro Gln
        355                 360                 365

Asn Ser Val Gly Cys Val Ala Glu Ala Val Ser Leu Lys Leu Thr Glu
    370                 375                 380

Glu Gly Gln Ile Leu Val Lys Cys Pro Tyr Met Phe Ser Lys Tyr Leu
385                 390                 395                 400

Phe Asp Glu Lys Ala Thr Ala Asp Ala His Asp Ala Glu Gly Tyr Phe
                405                 410                 415

Lys Thr Gly Asp Ile Ala Arg Arg Glu Gly Lys Tyr Tyr Phe Ile Leu
            420                 425                 430

Gly Arg Ala Ser Ile Asp Ile Ile Lys Ser Gly Gly Tyr Lys Ile Ser
        435                 440                 445

Ala Leu Asp Ile Glu Arg Glu Ile Leu Gly Leu Asp Tyr Val Ser Glu
    450                 455                 460

Val Met Val Val Gly Val Glu Asp Glu Glu Phe Gly Gln Arg Val Ala
465                 470                 475                 480

Ala Thr Val Ser Leu Lys Gln Asp Gln Lys Thr Thr Arg Lys Ser Leu
                485                 490                 495

Thr Ile Ala Glu Leu Arg Glu Asp Leu Arg Ser Lys Met Ala Gly Tyr
            500                 505                 510

Lys Met Pro Thr Val Leu Arg Val Val Gln Gly Glu Leu Pro Lys Ser
        515                 520                 525

Gly Thr Gly Lys Val Gln Lys Lys Ile Leu Gly Pro Gln Phe Phe Pro
    530                 535                 540

Pro Asn Tyr Arg Gln Leu Pro Glu Val Gln Val Trp Ser Arg Glu Asn
545                 550                 555                 560

Lys Ala Arg Leu

<210> 64
<211> 560
<212> PRT
<213> Penicillium chrysogenum

<400> 64

Met Arg Lys Thr Leu Arg His Gly Leu Gly Leu Pro Thr Tyr Ile Tyr
1               5                   10                  15

Ile Pro Ser His His Ile Asp Ile Pro Pro Ile Asp Thr Trp Asp Cys
            20                  25                  30

Leu Phe Glu Arg Glu Asp Arg Pro Phe Leu Asp Asp His Ile Ile Phe
        35                  40                  45

Lys Ser Pro Pro Gly Glu Val Arg Thr Phe Asn Asp Ile Arg Ser Gln
    50                  55                  60

Ala Leu Ser Phe Gly Ala Asn Leu Gln Val Gln Trp Glu Trp Gln Lys
65                  70                  75                  80

Gly Asp Ile Leu Leu Leu Phe Ala Pro Asn Asp Ile Asp Val Pro Thr
                85                  90                  95

Leu Phe Trp Gly Cys His Trp Ala Gly Gly Val Val Ser Pro Ala Asn
            100                 105                 110

Pro Ala Phe Thr Ala Asp Glu Leu Lys Tyr Gln Leu Ser Asp Thr Gly
        115                 120                 125

Ala Lys Ala Met Val Val His Thr Ser Leu Leu Asp Thr Ala Leu Thr
    130                 135                 140

Ala Ala Lys Arg Ile Asp Phe Pro Ile Ala His Met Leu Val Phe Gly
145                 150                 155                 160

Pro Pro Ser Ser Glu Ala Glu Leu Gln His Val Glu Ser Met Leu Gly
                165                 170                 175

Gly Gly Ala Pro Gly Ala Arg Arg Pro Arg Ile Asp Pro Val Val Asp
            180                 185                 190

Thr Ala Phe Leu Val Tyr Ser Ser Gly Thr Thr Gly Arg Pro Lys Gly
        195                 200                 205

Thr Arg Ile Thr His Thr Asn Leu Val Thr Asn Leu Val Leu Gln Gly
    210                 215                 220

Arg Val Glu Gly Pro His Met Asn Trp Arg Gln Asp Arg Phe Leu Ser
225                 230                 235                 240

Phe Leu Pro Thr Tyr His Ile Tyr Val His Phe Pro Leu Phe Leu Gly
                245                 250                 255

Ile Lys Thr Ile Val Met Glu Lys Phe Ser Val Lys Gly Phe Leu His
            260                 265                 270

Asn Val Lys Ala Glu Ser Ile Thr His Ile Tyr Ala Val Pro Pro Val
        275                 280                 285

Val Leu Tyr Leu Ala Lys Asp Pro Ala Met Thr Arg Glu Gln Leu Ser
    290                 295                 300

Ser Leu Arg Met Val Thr Ser Ala Ala Ala Pro Leu Ala Ser Asp Leu
305                 310                 315                 320

Ile His Ala Val Tyr Asp Arg Leu Lys Val Pro Val Arg Gln Ala Tyr
                325                 330                 335

Gly Leu Thr Glu Ser Thr Ala Val Thr His Leu Gln Arg Trp Asp Glu

            340                 345                 350

Trp Asp Lys Ala Met Gly Ser Ser Gly Pro Leu Tyr Pro Ala Val Glu
        355                 360                 365

Thr Lys Phe Ile Asp Asp Gln Gly Asn Pro Val Thr Lys Gly Asp Gly
    370                 375                 380

Glu Leu Cys Leu Arg Gly Pro Thr Leu Phe Pro Gly Tyr His Asn Asn
385                 390                 395                 400

Ala Glu Ala Thr Ala Arg Ser Ile Thr Ser Asp Gly Trp Phe Lys Thr
                405                 410                 415

Gly Asp Ile Gly Phe Gln Asp Glu Glu Gly Asn Leu Phe Ile Thr Asp
            420                 425                 430

Arg Leu Lys Asp Leu Ile Lys Phe Lys Gly Phe Gln Ile Pro Pro Ala
        435                 440                 445

Glu Ile Glu Ser Ala Leu His Glu His Pro Leu Val His Asp Ala Ala
    450                 455                 460

Val Ile Gly Leu Ala Val Glu Lys Ile Ala Thr Glu Val Pro Val Ala
465                 470                 475                 480

Tyr Val Val Leu Glu Lys Thr Asp Lys Pro Ala Glu Gln Val Ala Glu
                485                 490                 495

Glu Leu Val Ala Tyr Val Ser Gly Lys Leu Ala Pro His Lys Arg Leu
            500                 505                 510

Arg Gly Gly Ile Val Leu Ile Asp Glu Ile Pro Lys Gly Pro Ala Gly
        515                 520                 525

Lys Ile Leu Lys Arg Val Leu Lys Thr Arg Ala Glu Gly Val Asp Gln
    530                 535                 540

Gly Lys Ala Ile Gly Ala Ser Ile Tyr Asp Asp Arg Pro Ser Lys Leu
545                 550                 555                 560

<210> 65
<211> 562

<212>  PRT
<213>  Penicillium chrysogenum

<400>  65

Met Pro Val Ser Ser Asn Tyr Pro Leu Val Asp Ile Pro Glu Val Asp
1               5                   10                  15

Leu Trp Thr Phe Leu Phe Glu Arg Lys Asp Arg Ala Tyr Pro Asp Asp
            20                  25                  30

Lys Ile Ile Tyr Gln Asp Ala Asp Thr Gln Arg His Tyr Thr Tyr Lys
        35                  40                  45

Ser Leu Arg Asp Ala Ser Leu Asp Phe Gly Lys Gly Leu Lys Ala Leu
    50                  55                  60

Tyr Glu Trp Arg Lys Gly Asp Val Leu Ala Leu Phe Thr Pro Asn Ser
65                  70                  75                  80

Ile Asp Thr Pro Val Val Met Trp Gly Thr Leu Trp Ala Gly Gly Thr
                85                  90                  95

Ile Ser Pro Ala Asn Pro Gly Tyr Thr Val Asp Glu Leu Ala Phe Gln
            100                 105                 110

Leu Lys Asn Ser His Ala Lys Gly Leu Val Thr Gln Ala Ser Val Leu
        115                 120                 125

Pro Val Ala Arg Glu Ala Ala Lys Lys Val Gly Met Pro Glu Asp Arg
    130                 135                 140

Ile Ile Leu Ile Gly Asp Gln Arg Asp Pro Asp Ala Arg Val Lys His
145                 150                 155                 160

Phe Thr Ser Val Arg Asn Ile Ser Gly Ala Thr Arg Tyr Arg Lys Gln
                165                 170                 175

Lys Ile Thr Pro Ala Lys Asp Val Ala Phe Leu Val Tyr Ser Ser Gly
            180                 185                 190

Thr Thr Gly Val Pro Lys Gly Val Met Ile Ser His Arg Asn Ile Val
        195                 200                 205

Ala Asn Ile Arg Gln Gln Phe Ile Ala Glu Gly Glu Met Leu Ser Trp
    210                 215                 220

Asn Gly Gly Pro Asp Gly Lys Gly Asp Arg Val Leu Ala Phe Leu Pro
225                 230                 235                 240

Phe Tyr His Ile Tyr Gly Leu Thr Cys Leu Ile Thr Gln Ala Leu Tyr
                245                 250                 255

Lys Gly Tyr His Leu Ile Val Met Ser Lys Phe Asp Ile Glu Lys Trp
            260                 265                 270

Cys Ala His Val Gln Asn Tyr Arg Cys Ser Phe Ser Tyr Ile Val Pro
        275                 280                 285

Pro Val Val Leu Leu Leu Gly Lys His Pro Val Val Asp Lys Tyr Asp
    290                 295                 300

Leu Ser Ser Leu Arg Met Met Asn Ser Gly Ala Ala Pro Leu Thr Gln
305                 310                 315                 320

Glu Leu Val Glu Ala Val Tyr Ser Arg Ile Lys Val Gly Ile Lys Gln
                325                 330                 335

Gly Tyr Gly Leu Ser Glu Thr Ser Pro Thr Thr His Ser Gln Arg Trp
            340                 345                 350

Glu Asp Trp Arg Glu Ala Met Gly Ser Val Gly Arg Leu Met Pro Asn
        355                 360                 365

Met Gln Ala Lys Tyr Met Thr Met Pro Glu Asp Gly Ser Glu Pro Lys
    370                 375                 380

Glu Val Gly Glu Gly Glu Val Gly Glu Leu Tyr Leu Lys Gly Pro Asn
385                 390                 395                 400

Val Phe Leu Gly Tyr His Glu Asn Pro Glu Ala Thr Lys Gly Cys Leu
                405                 410                 415

Ser Glu Asp Gly Trp Phe Gln Thr Gly Asp Val Gly Tyr Gln Asp Ala
            420                 425                 430

Lys Gly Asn Phe Tyr Ile Thr Asp Arg Val Lys Glu Leu Ile Lys Tyr

        435                 440                 445

Lys Gly Phe Gln Val Pro Pro Ala Glu Leu Glu Gly Tyr Leu Val Asp
    450                 455                 460

Asn Asp Ala Ile Asp Asp Val Ala Val Ile Gly Ile Glu Ser Glu Thr
465                 470                 475                 480

His Gly Ser Glu Val Pro Met Ala Cys Val Val Arg Ser Ala Lys Ser
                485                 490                 495

Lys Ser Ser Gly Thr Ser Glu Lys Asp Glu Ala Ala Arg Ile Ile Lys
            500                 505                 510

Trp Leu Asp Ser Lys Val Ala Ser His Lys Arg Leu Arg Gly Gly Val
        515                 520                 525

His Phe Val Asp Glu Ile Pro Lys Asn Pro Ser Gly Lys Ile Leu Arg
    530                 535                 540

Arg Ile Leu Lys Gln Lys Phe Lys Gly Ala Ala Glu Ala Pro Lys Ala
545                 550                 555                 560

Lys Leu

<210> 66
<211> 698
<212> PRT
<213> Penicillium chrysogenum

<400> 66

Met Thr Ser Leu Pro Arg Lys Leu Trp Gln His Pro Ala Pro Glu Ser
1               5                   10                  15

Thr Gln Met Gly Arg Phe Gln Arg Asp Leu Glu Lys Ser Thr Gly His
            20                  25                  30

Lys Phe Asp Ser Phe His Asp Met Tyr Leu Tyr Ser Val Lys Asn Arg
        35                  40                  45

Ser Ala Phe Trp Glu Phe Cys Trp Lys Tyr Phe Gln Leu Ile His Glu
    50                  55                  60

Gly Ser Tyr Thr Lys Val Val Asp Glu Thr Ala Arg Met Asp Ser Val
65                  70                  75                  80

Pro Glu Trp Phe Pro Gly Val Arg Leu Asn Phe Ala Glu Asn Leu Leu
                85                  90                  95

Phe Ser Arg Ile Ala Gly Asp Lys Thr Asp Lys Glu Asp Asp Lys Ile
            100                 105                 110

Ala Val Ser Glu Val Arg Glu Gly Ala Ala His Glu Val Ile His Leu
        115                 120                 125

Thr Trp Gly Glu Leu Arg Arg Arg Thr Gly Ala Leu Val Gln Ala Met
    130                 135                 140

Lys Ala His Gly Val Val Gln Gly Asp Arg Ile Ala Leu Cys Ala Ala
145                 150                 155                 160

Asn Ser Ile Asp Thr Leu Phe Val Phe Leu Ala Ser Thr Ala Leu Gly
                165                 170                 175

Ala Ile Phe Ser Ser Ser Ser Thr Asp Met Gly Thr Lys Gly Val Leu
            180                 185                 190

Asp Arg Leu Leu Gln Ile Lys Pro Arg Trp Leu Phe Met Asp Asp Leu
        195                 200                 205

Ala Val Tyr Asn Gly Lys Thr Ile Asp Leu Arg Ser Lys Ile Gly Glu
    210                 215                 220

Ile Val Lys Gly Met Glu Ser Val Pro Glu Phe Glu Gly Val Val Ser
225                 230                 235                 240

Leu Pro Arg Phe His Ser Arg Pro Ala Asp Ile Asn Ser Ile Ser Arg
                245                 250                 255

Thr Lys Thr Leu Ala Glu Phe Leu Glu Lys Ala Gly Gly Asn Glu Lys
            260                 265                 270

Leu Glu Phe Glu Arg Val Gly Phe Arg Asp Pro Phe Leu Val Val Tyr
        275                 280                 285

Ser Ser Gly Thr Thr Gly Gln Pro Lys Cys Ile Val His Ser Val Gly
    290                 295                 300

Gly Val Leu Leu Asn Ser Ser Lys Glu Gly Arg Leu His Ser Asp Leu
305                 310                 315                 320

Gly Pro Asp Cys Val Thr Leu Gln Tyr Thr Thr Thr Gly Trp Ile Met
                325                 330                 335

Tyr Met Ser Ala Val Gln Thr Leu Leu Phe Gly Ala Arg Val Val Leu
            340                 345                 350

Tyr Asp Gly Ser Pro Phe Ile Pro Gly Ile Thr Ala Leu Val Asp Leu
        355                 360                 365

Ala Ala Gln Glu Lys Val Thr His Leu Gly Ile Ser Pro Arg Trp Leu
    370                 375                 380

His Glu Leu Gln Gln Ala Lys Ile Lys Pro Arg Glu Lys Val Asp Leu
385                 390                 395                 400

Ser Ser Leu Arg Val Val Thr Ser Thr Gly Met Val Leu Arg Asp Ala
                405                 410                 415

Leu Phe Glu Trp Phe Tyr Asp Glu Gly Phe Pro Pro His Thr Arg Leu
            420                 425                 430

Asn Asn Ile Ser Gly Gly Thr Asp Ile Ala Gly Ser Phe Gly Thr Gly
        435                 440                 445

Asn Pro Leu Val Pro Leu Tyr Val Gly Gly Cys Ala Gly Cys Ser Leu
    450                 455                 460

Gly Ile Pro Val Glu Val Tyr Asp Ser Thr Ile Glu Gly Gly Asp Gly
465                 470                 475                 480

Ile Lys Gly Val Pro Val Glu Asp Gly Val Pro Gly Glu Leu Val Ala
                485                 490                 495

Thr Ser Ala Phe Pro Asn Met Pro Thr Leu Phe Trp Gly Asp Glu Ser
            500                 505                 510

Gly Lys Lys Tyr His Asp Ala Tyr Phe Glu Arg Phe Asp Asn Val Trp

515                 520                 525

Thr His Gly Asp Phe Val Ser Ile Arg Pro Ile Thr Lys Gln Ile Val
    530                 535                 540

Phe His Gly Arg Ala Asp Gly Val Leu Asn Pro Ser Gly Val Arg Phe
545                 550                 555                 560

Gly Ser Ala Glu Ile Tyr Arg Val Leu Glu Gly Gln Phe Ser Lys Glu
                565                 570                 575

Ile Ser Asp Ser Ile Cys Val Gly Gln Arg Arg Pro Thr Asp Thr Asp
            580                 585                 590

Glu Arg Val Ile Leu Phe Leu Leu Met Arg Pro Gly Met Ala Phe Thr
        595                 600                 605

Pro Asp Leu Val Ala Arg Val Lys Ser Ala Ile Arg Ser Glu Leu Ser
    610                 615                 620

Pro Arg His Val Pro Met Phe Thr Phe Glu Thr His Glu Ile Pro Thr
625                 630                 635                 640

Thr Val Asn Leu Lys Lys Val Glu Leu Pro Val Lys Gln Ile Val Ser
                645                 650                 655

Gly Lys Ile Ile Lys Pro Ser Gly Thr Leu Leu Asn Pro Lys Ser Leu
            660                 665                 670

Asp Phe Tyr Tyr Gln Phe Ala Lys Val Glu Thr Leu Arg Glu Lys Ile
        675                 680                 685

Leu Asn Asn Ala Tyr Glu Arg Asn Ser Lys
    690                 695

<210>  67
<211>  707
<212>  PRT
<213>  Penicillium chrysogenum

<400>  67

Met Phe Phe Ser Ser Pro Pro His Leu Ala Lys Ala Glu Glu Leu Lys
1               5                   10                  15

Gln Ala Pro Pro Lys Gly Val Ala Tyr Ser Val Ala Ile Pro Gly Ser
            20                  25                  30

Glu Gln Pro Gly Arg Ser Arg Val Tyr Arg Ala Trp Asn Ala Gln Lys
        35                  40                  45

Glu Leu Leu Thr Thr Leu Asp Ser Gln Val Thr Thr Ala His Asp Met
    50                  55                  60

Phe Glu Ser Thr Ala Asn Arg Gln Pro Lys Asn His Cys Leu Gly Trp
65                  70                  75                  80

Arg Pro Tyr Asn Ser Thr Thr Lys Ser Phe Asp Pro Tyr Gln Trp Leu
                85                  90                  95

Thr Tyr Glu Thr Val Gln Lys Arg Arg Ala Ala Phe Gly Ala Gly Leu
            100                 105                 110

Val Glu Leu His His Lys His Asp Cys His Arg Ser Gly Gln Tyr Gly
        115                 120                 125

Val Gly Leu Trp Ser Gln Asn Arg Pro Glu Trp Gln Ile Thr Asp Leu
    130                 135                 140

Ala Cys Ile Ser Gln Ser Leu Tyr Ser Val Ser Ile Tyr Asp Val Leu
145                 150                 155                 160

Ser Pro Asp Ala Thr Glu Tyr Ile Ile Asn His Ala Glu Leu Ser Cys
                165                 170                 175

Val Val Thr Ser Leu Pro His Ile Pro Thr Leu Leu Lys Leu Lys Pro
            180                 185                 190

Ser Leu Pro Asn Leu Lys Ile Ile Ile Ser Leu Asp Pro Leu Asp Gly
        195                 200                 205

Gly Glu Gln Asp Gly His Ser Lys Arg Ala Leu Ile Glu Ser Met Ala
    210                 215                 220

Ala Gly Gln Gly Leu Ala Ile Tyr Thr Met Asp Gln Val Glu Glu Leu
225                 230                 235                 240

Gly Leu Ala Ser Lys Arg Gly Tyr Asn Ala Pro Ser Ala Ser Asp Ile
                245                 250                 255

Val Thr Ile Asn Tyr Thr Ser Gly Thr Thr Gly Pro Pro Lys Gly Val
            260                 265                 270

Val Leu Thr His Gly Asn Ala Val Ala Ala Thr Ser Cys Gly Leu Ile
        275                 280                 285

Thr Val Gly Gln Ser Arg Gly Asp Thr Met Val Ser Tyr Leu Pro Leu
    290                 295                 300

Ala His Ile Tyr Ala Arg Leu Ala Glu His Thr Ala Phe Trp Gly Gly
305                 310                 315                 320

Ala Arg Ile Gly Tyr Phe His Gly Asn Ile Val Glu Leu Val Asp Asp
                325                 330                 335

Leu Lys Leu Leu Lys Pro Thr Gly Phe Met Ser Val Pro Arg Leu Tyr
            340                 345                 350

Ser Arg Phe Gly Ser Ala Ile Arg Ser Ala Thr Val Glu Gln Pro Gly
        355                 360                 365

Phe Lys Gly Ala Leu Ser Arg His Ile Ile Ala Ala Lys Thr Ala Asn
    370                 375                 380

Met Lys Asn Pro Asp Pro Ser Lys Ala Thr Val Arg His Ala Val Tyr
385                 390                 395                 400

Asp Arg Leu Trp Ala Lys Lys Val Ala Ala Ala Leu Gly Leu Glu Arg
                405                 410                 415

Ala Arg Tyr Met Val Ser Gly Ser Ala Pro Leu Asp Pro Thr Leu His
            420                 425                 430

Asp Phe Leu Arg Val Ala Thr Gly Thr Asp Ile Leu Gln Gly Tyr Gly
        435                 440                 445

Leu Thr Glu Ser Tyr Ala Ser Ala Thr Ala Gln Pro Thr Tyr Asp Leu
    450                 455                 460

Thr Ser Gly Asn Cys Gly Ser Leu Ala Pro Cys Val Glu Ala Cys Leu

465                 470                 475                 480

Ala Ser Leu Pro Asp Met Glu Tyr Ser Val Asp Asp Lys Pro Phe Pro
                485                 490                 495

Arg Gly Glu Leu Leu Leu Arg Gly Asn Asn Met Phe Arg Glu Tyr Tyr
            500                 505                 510

Lys Asn Glu Glu Glu Thr Ser Lys Ala Val Thr Glu Asp Gly Trp Phe
        515                 520                 525

Arg Thr Gly Asp Val Cys Thr Ile Asp Glu Lys Gly Arg Phe Ile Ile
    530                 535                 540

Ile Asp Arg Arg Lys Asn Val Leu Lys Leu Ala Gln Gly Glu Tyr Ile
545                 550                 555                 560

Ser Pro Glu Arg Leu Glu Gly Val Val Leu Ala Glu Leu Gly Tyr Ile
                565                 570                 575

Ala Gln Ala Tyr Val His Gly Asp Ser Met Gln Thr Phe Leu Val Gly
            580                 585                 590

Ile Phe Gly Val Ala Pro Asp Leu Phe Ala Pro Phe Ala Ser Lys Val
        595                 600                 605

Leu Gly Lys Thr Ile Ala Pro Thr Asp Leu Glu Ala Ile Lys Glu Ser
    610                 615                 620

Leu Arg Asp Asp Lys Ile Arg Arg Ala Val Leu Arg Asp Leu Glu Arg
625                 630                 635                 640

Val Ala Lys Lys His Lys Phe Ala Gly Tyr Glu Arg Ile Arg Asn Val
                645                 650                 655

Ser Leu Lys Val Glu Pro Phe Thr Val Glu Asn Asn Leu Leu Thr Pro
            660                 665                 670

Thr Leu Lys Leu Lys Arg Pro Pro Ala Val Lys Leu Tyr Arg Ser Leu
        675                 680                 685

Leu Asp Gln Leu Tyr Glu Gln Ala Thr Glu Glu Gln Ser Ala Pro Lys
    690                 695                 700

Ala Lys Leu
705

<210> 68
<211> 556
<212> PRT
<213> Penicillium chrysogenum

<400> 68

Met Pro Tyr Lys Ser Arg Trp Thr Ile Asp Ile Pro Asp Thr His Leu
1               5                   10                  15

Ala Ser Leu Leu Leu Lys Ser Pro Thr Ala Pro Leu Ser Lys Thr His
            20                  25                  30

Lys Cys Tyr Leu Asp Ala Ala Arg Pro Glu Thr His Tyr Leu Thr Thr
        35                  40                  45

His Asp Leu Arg Leu Trp Ser Gln Arg Leu Ala Ala Gly Leu Arg Lys
    50                  55                  60

Ser Gly Leu Gln Arg Gly Asp Arg Val Leu Leu Phe Ser Gly Asn Asp
65                  70                  75                  80

Leu Phe Phe Pro Val Val Phe Leu Gly Val Ile Met Ala Gly Gly Ile
                85                  90                  95

Phe Thr Gly Ala Asn Pro Thr Phe Val Ala Arg Glu Leu Ala Tyr Gln
            100                 105                 110

Leu Gln Asp Ser Gly Ala Thr Tyr Leu Leu Cys Ala Ser Asn Ser Leu
        115                 120                 125

Glu Thr Gly Leu Glu Ala Ala Lys Gln Ala Lys Leu Pro Gln Ser His
    130                 135                 140

Ile Phe Ala Tyr Asp Thr Ser Ile Tyr Asp Gly Val Thr Asn Pro Gln
145                 150                 155                 160

Lys Gly Cys Ala Tyr Trp Ser Asp Leu Leu Ala Ser Glu Glu Glu Gly
                165                 170                 175

Ala Ala Phe Thr Trp Asp Glu Leu Ser Thr Pro Ala Leu Ser Ser Thr
            180                 185                 190

Thr Leu Ala Leu Asn Tyr Ser Ser Gly Thr Thr Gly Arg Pro Lys Gly
        195                 200                 205

Val Glu Ile Ser His Arg Asn Tyr Val Ala Asn Met Leu Gln Tyr Cys
    210                 215                 220

His Thr Ala Ser Leu His Pro Asp Tyr Lys Ala Arg Leu Glu Arg Ser
225                 230                 235                 240

Arg Trp Leu Cys Phe Leu Pro Met Tyr His Ala Met Ala Gln Asn Ile
                245                 250                 255

Phe Ile Ala Ala Ala Leu Tyr Arg Ala Thr Pro Val Tyr Ile Met Ser
            260                 265                 270

Lys Phe Asp Phe Val Lys Met Leu Glu Tyr Thr Gln Arg Phe Arg Ile
        275                 280                 285

Thr Asp Phe Ile Leu Val Pro Pro Val Val Val Ala Leu Ala Lys His
    290                 295                 300

Pro Ala Val Gly Gln Tyr Asp Leu Ser Ser Val Glu Leu Val Gly Ser
305                 310                 315                 320

Gly Ala Ala Pro Leu Gly Arg Glu Val Cys Glu Glu Val Glu Lys Leu
                325                 330                 335

Trp Pro Pro Gly Lys Ile Asn Ile Lys Gln Gly Trp Gly Met Thr Glu
            340                 345                 350

Ala Thr Cys Ser Val Thr Gly Trp Asn Pro Ala Glu Ile Ser Thr Ser
        355                 360                 365

Ala Ser Val Gly Glu Leu Asn Ala Asn Cys Glu Ala Lys Ile Met Phe
    370                 375                 380

Asp Gly Val Glu Val Lys Glu Arg Asn Ser Arg Gly Glu Leu Trp Val
385                 390                 395                 400

Arg Ala Pro Asn Val Met Lys Gly Tyr Trp Arg Asn Glu Lys Ala Thr

405                 410                 415

Lys Glu Thr Lys Thr Glu Asp Gly Trp Leu Leu Thr Gly Asp Ile Ala
            420                 425                 430

Phe Val Asp Asp Asp Gly Lys Phe His Val Val Asp Arg Met Lys Glu
        435                 440                 445

Leu Ile Lys Val Lys Gly Asn Gln Val Ala Pro Ala Glu Leu Glu Ala
    450                 455                 460

Leu Leu Leu Glu His Pro Ala Ile Ser Asp Val Ala Val Ile Gly Val
465                 470                 475                 480

Val Ile Asn Asn Asp Glu Arg Pro Arg Ala Tyr Val Val Leu Arg Pro
                485                 490                 495

Gly Gln Ser Ala Thr Ala Asn Glu Ile Ala His Tyr Leu Asp Asn Lys
            500                 505                 510

Val Ser Ala Phe Lys Arg Ile Thr Gly Gly Val Val Phe Leu Glu Ala
        515                 520                 525

Ile Pro Lys Asn Pro Ser Gly Lys Ile Leu Arg Met Lys Leu Arg Glu
    530                 535                 540

Gln Ala Lys Glu Glu Leu Arg Val Thr Ala Lys Leu
545                 550                 555

<210>  69
<211>  658
<212>  PRT
<213>  Penicillium chrysogenum

<400>  69

Met Ala Asn Lys Arg Ser Leu Cys Ala Glu Ile Pro Leu Ser Leu Ala
1               5                   10                  15

Leu Pro Ala Ala Ala Thr Thr Leu Ala Tyr Leu Asn Ala Arg Trp Ser
            20                  25                  30

Leu Ser Tyr Asp Leu Asn Leu Leu Lys Gly Leu Leu Lys Met Ser Ala
        35                  40                  45

Lys Ser Arg Ile Ala Glu Arg Gly Asp Arg Leu Asn Leu Phe Tyr Thr
    50                  55                  60

Leu Glu Ala Tyr Ala Leu Asp Pro Lys Thr Ala Asp Asn Asp Phe Ile
65                  70                  75                  80

Val Tyr Asn Gly Arg Thr Thr Thr Phe His Glu Thr Tyr Ile Thr Ala
                85                  90                  95

Leu Arg Tyr Gly Ala Trp Phe Lys Thr Val His Gly Ile Lys Arg Lys
            100                 105                 110

Glu Ile Val Ala Ile Asp Phe Met Asn Ser Ser Thr Phe Ile Phe Met
        115                 120                 125

Leu Leu Gly Leu Trp Ser Ile Gly Ala Val Pro Ala Phe Ile Asn Tyr
    130                 135                 140

Asn Leu Ser Gly Lys Pro Leu Thr His Ser Ile Arg Thr Ser Ser Ala
145                 150                 155                 160

Arg Leu Val Val Val Asp Glu Glu Val Arg His Cys Phe Pro Glu Glu
                165                 170                 175

Gln Glu Lys Ile Leu Thr Ser Pro Asn Phe Arg Asp Gly Lys Gly Pro
            180                 185                 190

Val Glu Ile Val Phe His Thr Pro Glu Val Glu Ala Gln Ile Leu Gly
        195                 200                 205

Met Glu Pro Met Arg Glu Asp Asp Lys Ala Arg Ser Gly Leu Ile Pro
    210                 215                 220

Arg Asp Met Ala Ile Leu Ile Tyr Thr Ser Gly Thr Thr Gly Leu Pro
225                 230                 235                 240

Lys Pro Ala Ile Val Ser Trp Lys Lys Cys Trp Ser Gly Ser Leu Phe
                245                 250                 255

Val Lys Asp Trp Leu Asn Ile Thr Pro Ser Asp Arg Phe Phe Thr Cys
            260                 265                 270

Met Pro Leu Tyr His Ser Ser Ala Ala Ile Leu Gly Phe Ile Thr Cys
        275                 280                 285

Leu Met Gly Gly Ser Thr Leu Ile Ile Gly Arg Arg Phe Ser Ala Arg
    290                 295                 300

Asn Phe Met Lys Glu Ala Arg Glu Asn Gly Ala Thr Val Ile Gln Tyr
305                 310                 315                 320

Val Gly Glu Thr Leu Arg Tyr Leu Leu Gly Val Ala Pro Glu Ile Asp
                325                 330                 335

Pro Val Thr Gly Asp Asp Leu Asp Lys Lys His Asn Ile Arg Leu Ala
            340                 345                 350

Phe Gly Asn Gly Leu Arg Pro Asp Ile Trp Asn Arg Phe Lys Glu Arg
        355                 360                 365

Phe Asn Ile Pro Thr Ile Ala Glu Phe Tyr Ala Ala Thr Glu Gly Thr
    370                 375                 380

Ala Gly Ser Trp Asn Ile Ser Ser Asn Asp Phe Ser Ala Gly Ala Ile
385                 390                 395                 400

Gly Arg Asn Gly Ala Leu Gly Asn Ile Ile Leu Gly Arg Gly Ser Ala
                405                 410                 415

Ile Val Asp Val Asp His Glu Thr Gln Glu Pro Trp Arg Asp Pro Lys
            420                 425                 430

Thr Gly Leu Cys Lys Lys Val Pro Arg Gly Asp Pro Gly Glu Leu Leu
        435                 440                 445

Phe Ala Ile Asp Ala Ala Asp Pro Thr Ala Asn Phe Gln Gly Tyr Phe
    450                 455                 460

Gly Asn Lys Lys Ala Thr Glu Gly Lys Ile Ile Arg Asp Val Ile Lys
465                 470                 475                 480

Lys Gly Asp Ala Tyr Phe Arg Thr Gly Asp Met Val Arg Trp Asp Lys
                485                 490                 495

Asp Gly Arg Trp Phe Phe Ser Asp Arg Leu Gly Asp Thr Phe Arg Trp

            500                 505                 510

Lys Ser Glu Asn Val Ser Thr Ser Glu Val Ser Glu Val Leu Gly Ala
        515                 520                 525

His Pro Glu Val His Glu Ala Asn Val Tyr Gly Val Ser Leu Pro Asn
    530                 535                 540

His Asp Gly Arg Ala Gly Cys Ala Ala Ile Leu Phe Asn Gln Gln Ile
545                 550                 555                 560

Thr Ser Gly Gly Leu Ser Asp Ser Ala Leu Glu Pro Ser Arg Glu Val
                565                 570                 575

Leu Asp Thr Leu Ala Ala His Thr Leu Gln Asn Leu Pro Arg Phe Ala
            580                 585                 590

Ala Pro Leu Phe Leu Arg Val Thr Pro Val Thr Gln Ser Thr Gly Asn
        595                 600                 605

Asn Lys Gln Gln Lys His Val Leu Arg Thr Glu Gly Val Asp Pro Ala
    610                 615                 620

Leu Val Ser Lys Lys Asp Arg Leu Tyr Trp Leu Gln Gly Asn Thr Tyr
625                 630                 635                 640

Val Pro Phe Gly Gln Arg Asp Trp Glu Arg Leu Thr Gly Gly Gln Val
                645                 650                 655

Arg Leu

<210> 70
<211> 632
<212> PRT
<213> Penicillium chrysogenum

<400> 70

Met Asp Ile Gly Thr Ala Ala Ala Ala Leu Val Gly Gly Ala Thr Ile
1               5                   10                  15

Ala Gly Tyr Leu Asn Ala Lys Phe His Ile Gln Lys Asp Ile Ser Gly
            20                  25                  30

Leu Phe Thr Leu Lys Asn Ala Glu Arg Gln Tyr Ala Asn Ala Ala Ser
        35                  40                  45

Gln Asn Gln Gly Asn Pro Trp Phe Val Leu Val Gln Thr Val Lys Arg
    50                  55                  60

Tyr Pro Asp Met Ile Cys Leu Trp Thr Arg Glu Arg Ser Tyr Thr Tyr
65                  70                  75                  80

Arg Glu Ile Gln Asp Gln Ala Cys Gln Tyr Ala His Phe Phe Leu Ser
                85                  90                  95

His Gly Val Lys Lys Gly Asp Leu Val Ala Leu Tyr Leu Gln Asn Ser
            100                 105                 110

Asn Glu Tyr Leu Val Ala Trp Val Ala Leu Trp Ser Ile Gly Cys Ala
        115                 120                 125

Pro Ala Ala Ile Asn Tyr Asn Leu Thr Gly Asp Ala Leu Leu His Cys
    130                 135                 140

Leu Lys Ile Ser Asp Ala Thr Ile Leu Leu Val Asp Glu Asn Ala Asp
145                 150                 155                 160

Cys Arg Ala Arg Val Glu Glu Ser His Asp Ala Ile Thr Gly Asn Leu
                165                 170                 175

Gly Met Lys Pro Met Thr Leu Asp Ser Ala Leu Lys Ala His Ile Arg
            180                 185                 190

Thr Phe Pro Thr Thr Leu Pro Pro Lys Glu Leu Ser Lys His Val Ala
        195                 200                 205

Gly Asp Phe Pro Ala Ile Leu Leu Tyr Thr Ser Gly Thr Thr Gly Met
    210                 215                 220

Pro Lys Gly Cys Ala Phe Thr Met Ser Arg Leu Tyr Thr Thr Leu Phe
225                 230                 235                 240

Val Arg Arg Ala Met Met Gly Asp Thr Pro Gly Pro Gly Gly Asp Arg
                245                 250                 255

Trp Tyr Ser Cys Met Pro Leu Tyr His Gly Thr Ala Ala Ile Thr Ile
            260                 265                 270

Ile Ser Cys Leu Val Met Gly Val Ser Val Ala Ile Ala Pro Lys Phe
        275                 280                 285

Ser Val Ser Arg Phe Trp Thr Asp Val Arg Asp Ser Glu Ser Thr Ile
    290                 295                 300

Phe Val Tyr Val Gly Glu Thr Ala Arg Tyr Leu Leu Ala Pro Pro Pro
305                 310                 315                 320

Ser Pro Leu Asp Arg Asn His Lys Val Arg Cys Met Tyr Gly Asn Gly
                325                 330                 335

Leu Arg Pro Asp Ile Trp Glu Arg Phe Arg Glu Arg Phe Gly Val Ala
            340                 345                 350

Glu Val Gly Glu Phe Phe Asn Ser Thr Glu Gly Val Phe Gly Leu Phe
        355                 360                 365

Asn Tyr Asn Arg Gly Pro Phe Thr Ala Gly Ser Val Gly His His Gly
    370                 375                 380

Leu Ile Met Arg Gly Ile Leu His Asn Val Phe Val Pro Val Ala Ile
385                 390                 395                 400

Asp Pro Glu Thr Gly Asp Ile Leu Arg Asp Pro Lys Ser Gly Phe Ala
                405                 410                 415

Val Arg Ser Pro Tyr Glu Gln Gly Gly Glu Ile Ile Val Asn Val Pro
            420                 425                 430

Ser Glu Glu Ala Phe Gln Gly Tyr Trp His Asn Asp Glu Ala Thr Asn
        435                 440                 445

Lys Lys Phe Leu Arg Asp Val Phe Lys Lys Gly Asp Leu Tyr Tyr Arg
    450                 455                 460

Ser Gly Asp Ala Leu Arg Arg Gln Ser Asp Gly Arg Trp Tyr Phe Leu
465                 470                 475                 480

Asp Arg Leu Gly Asp Thr Phe Arg Trp Lys Ser Glu Asn Val Ala Thr

                485                 490                 495

Ala Glu Val Ser Glu Val Leu Gly Gln Phe Pro Gly Ile Thr Glu Ala
            500                 505                 510

Asn Val Tyr Gly Val Arg Leu Pro Asn His Glu Gly Arg Ala Gly Cys
        515                 520                 525

Ala Ala Ile Gln Ile Ser Pro Asp Ala Arg Gln Thr Phe Asp Tyr Thr
    530                 535                 540

Glu Leu Ala Arg Phe Val Arg Ser Lys Leu Pro Lys Tyr Ala Val Pro
545                 550                 555                 560

Leu Phe Leu Arg Ile Val Glu Asn Pro Thr His Ile His Asn His Lys
                565                 570                 575

Gln Asn Lys Val Pro Leu Arg Asp Glu Gly Val Asp Thr Ala Leu Ile
            580                 585                 590

Gly Thr Lys Ala Pro Glu Gly Lys Asp Asp His Phe Leu Trp Ile Ala
        595                 600                 605

Pro Gly Glu Glu Ser Tyr Ser Pro Tyr Gly Gln Lys Glu Trp Glu Gln
    610                 615                 620

Leu Ser Ser Gly Ser Val Arg Leu
625                 630

<210> 71
<211> 572
<212> PRT
<213> Penicillium chrysogenum

<400> 71

Met Ser Gly Ser Lys Ser Arg Leu Ser Gly Leu Leu Gly His Phe Thr
1               5                   10                  15

Gly Ser Thr Pro Pro Val Glu His Arg Val Asn Thr His Thr Leu Ser
            20                  25                  30

Pro Thr Phe Phe Leu Pro Arg Ala Ala Ala Val Glu Pro Asn Ala Glu
        35                  40                  45

Ala Ile Tyr His Val Thr Ala Asn Asn Lys Ile Leu Arg Arg Ser Tyr
    50                  55                  60

Gly Glu Thr Ala Asp Arg Ala Arg Gly Met Ala Tyr Tyr Leu Lys Lys
65                  70                  75                  80

His Gly Leu Ser Arg Val Gly Ile Leu Cys Pro Asn Thr Pro Ala Phe
                85                  90                  95

Leu Glu Ser Ile Phe Gly Ile Ala Ala Ala Gly Ala Val Asn Ile Ala
            100                 105                 110

Val Asn Tyr Arg Leu Lys Gln Glu Asp Ile Ala Tyr Ile Phe Asp His
        115                 120                 125

Gly Asp Ala Glu Val Ile Ile Val Asp Glu Glu Tyr Val Pro Leu Leu
    130                 135                 140

Glu His Tyr Arg Ser Gln His Pro Arg Ile Pro Ile Ile Val Asp Thr
145                 150                 155                 160

Asp Thr Asp Ala Thr Glu Gly Glu Leu Thr Gly Pro Phe Asp Glu Ala
                165                 170                 175

Val Leu Glu Gly Leu Arg His Asp Ile Asp Thr Gly Ser Arg Gly Trp
            180                 185                 190

Glu Gly Leu Glu Ser Gln Ala Ala Asp Glu Glu Ser Thr Ile Ala Leu
        195                 200                 205

Ala Tyr Thr Ser Gly Thr Thr Ala Arg Pro Lys Gly Val Glu Phe Ser
    210                 215                 220

His Arg Gly Cys Tyr Leu Ala Thr Leu Gly Asn Val Ile Glu Thr Gly
225                 230                 235                 240

Leu Asn Tyr His Arg Gly Arg Ala Arg Tyr Leu Trp Thr Leu Pro Met
                245                 250                 255

Phe His Ala Met Gly Trp Thr Phe Pro Trp Ala Val Thr Ala Val Arg
            260                 265                 270

Gly Thr His Tyr Cys Leu Arg Lys Ile Asp Tyr Pro Glu Ile Trp Arg
        275                 280                 285

Leu Leu Lys Glu Glu His Ile Thr His Phe Asn Ala Ala Pro Thr Val
    290                 295                 300

Asn Thr Leu Leu Cys Asn Ala Lys Glu Ala Glu Arg Leu Pro Glu Pro
305                 310                 315                 320

Val Arg Val Thr Val Ala Ala Ser Pro Pro Thr Pro Leu Leu Phe Glu
                325                 330                 335

Gln Met Thr Asp Leu Asn Leu His Pro Val His Val Tyr Gly Met Thr
            340                 345                 350

Glu Thr Tyr Gly Pro Ile Thr Lys Gly Tyr His Leu Pro Glu Trp Asp
        355                 360                 365

Glu Leu Pro Leu Lys Asp Lys Tyr Gln Arg Met Ala Arg Gln Gly His
    370                 375                 380

Gly Phe Ile Thr Ser Leu Pro Ala Arg Val Ile Lys Thr Glu Val Pro
385                 390                 395                 400

Ala Gly Thr Ile Thr Asp Val Arg Lys Asp Gly Gln Glu Ile Gly Glu
                405                 410                 415

Ile Val Phe Val Gly Asn Ile Cys Ala Arg Gly Tyr Tyr Lys Asp Pro
            420                 425                 430

Glu Ala Thr Arg Lys Leu Phe Ala Gly Gly Val Leu His Ser Gly Asp
        435                 440                 445

Leu Ala Val Trp His Pro Asp Gly Ala Ile Gln Ile Leu Asp Arg Ala
    450                 455                 460

Lys Asp Ile Ile Ile Ser Gly Gly Glu Asn Ile Ser Ser Val Ala Leu
465                 470                 475                 480

Glu Ser Met Leu Val Met His Pro Asp Ile Leu Glu Ala Gly Val Val
                485                 490                 495

Ala Val Pro Asp Ser His Trp Gly Glu Arg Pro Lys Ala Phe Val Thr

            500                 505                 510

Val Lys Glu Gly Lys Ser Leu Gln Gly Ser Asp Leu Ile Asp Trp Ala
        515                 520                 525

Arg Asn Thr Ser Gly Ile Ser Lys Phe Met Ile Pro Arg Glu Val Glu
    530                 535                 540

Val Val Thr Glu Leu Pro Lys Thr Ser Thr Gly Lys Ile Arg Lys Asn
545                 550                 555                 560

Ile Leu Arg Asp Trp Val Lys Gly Pro Arg Glu Ala
                565                 570

<210> 72
<211> 577
<212> PRT
<213> Penicillium chrysogenum

<400> 72

Met Ser Leu Gln Asp Ser His His Ser Phe Gln Pro Lys Pro Ser Tyr
1               5                   10                  15

Lys Gln Gly Leu Leu Glu Pro Ala Leu Arg Asn Trp Thr Ile Gly Asp
            20                  25                  30

Leu Leu Gln Arg Gln Ala Glu Gln Phe Pro Gly Asn Ile Ala Ile Ser
        35                  40                  45

Cys Pro Gly Thr Ser Asn Ser Ile Thr Tyr Arg Gln Leu Asn Asp Arg
    50                  55                  60

Thr Lys Leu Leu Gly Lys Ala Leu Ile Ala Ser Gly Ile Ser Val Gly
65                  70                  75                  80

Asp Arg Val Gly Ile Phe Ala Gly Asn Val Leu Glu Tyr Val Glu Val
                85                  90                  95

Ala Leu Ala Thr Ala Arg Ile Gly Ala Ile Ile Val Leu Leu Asn Thr
            100                 105                 110

Phe Tyr Thr Thr Glu Glu Ile Lys Arg Ala Leu Arg Phe Thr Gly Cys
        115                 120                 125

Ser Leu Leu Phe Ile Thr Glu Ser Leu Gly Lys Arg Ser Leu Leu Pro
    130                 135                 140

Cys Ile Asp Gln Leu Asn Glu Ile Ile Glu Asn Gln Lys Ser Glu Phe
145                 150                 155                 160

Pro Asp Leu Arg Ser Met Val Leu Leu Ser Gly Gln Cys Ser Glu Ser
                165                 170                 175

Ser Asn Leu Gln Ser Tyr Ala Asp Phe Phe Asn Ile Pro Pro Thr Gly
            180                 185                 190

Ala Lys Ala Ala Ser Ala Cys Lys Ala Ala Glu Thr Gln Val Thr Pro
        195                 200                 205

Glu Thr Ile Cys Asn Phe Gln Phe Thr Ser Gly Thr Thr Gly Met Pro
    210                 215                 220

Lys Ala Val Met Leu Thr His Phe Asn Val Val Asn Asn Gly Phe Leu
225                 230                 235                 240

Ile Gly Asp Arg Val Cys Leu Ser Pro Asn Asp Thr Ile Cys Cys Pro
                245                 250                 255

Trp Pro Leu Phe His Ser Ser Gly Phe Val Val Gly Leu Ile Thr Ser
            260                 265                 270

Leu Cys His Gly Ala Thr Leu Val Leu Pro Ser Pro Val Phe Asp Pro
        275                 280                 285

Ala Ala Thr Ala Arg Ala Leu Ile Ser Glu Arg Cys Thr Gly Leu Gln
    290                 295                 300

Gly Val Pro Thr Met Phe Ala Ala Val Leu Glu Trp Tyr Arg Gln Arg
305                 310                 315                 320

Gly Thr Arg Pro Pro Pro Leu Arg Thr Gly Ile Ile Gly Gly Ser Pro
                325                 330                 335

Val Ser Pro Ala Leu Leu Arg Glu Leu Gln His Glu Phe Ala Leu Glu
            340                 345                 350

Asp Leu Gly Ile Ala Tyr Gly Met Thr Glu Thr Ser Pro Leu Ser Phe
        355                 360                 365

Leu Ser Lys Gly Phe Glu Pro Glu Gly Thr His Ser Trp Met Glu Ile
    370                 375                 380

Leu Pro His Thr Thr Ala Lys Ile Val Asp Ala Gln Gly Thr Ile Val
385                 390                 395                 400

Pro Ile Gly Ser Pro Gly Glu Leu Cys Val Ser Gly Tyr Leu Leu Gln
                405                 410                 415

Gln Gly Tyr Tyr Gln Asn Pro Gly Lys Thr Ser Glu Ala Met Arg Val
            420                 425                 430

His Glu Asp Gly Val Leu Trp Ile His Ser Gly Asp Glu Ala Ile Met
        435                 440                 445

Asp Glu Gln Gly Arg Cys Arg Ile Ser Gly Arg Ile Lys Asp Thr Ile
    450                 455                 460

Ile Arg Gly Gly Glu Asn Ile Tyr Pro Ala Glu Ile Glu Asp Arg Leu
465                 470                 475                 480

Asn Glu His Pro Ala Ile Ser Met Ser Ala Val Val Gly Ile Gln Asp
                485                 490                 495

Ala Lys Tyr Gly Glu Ala Val Ala Ala Phe Leu Gln Leu Lys His Gly
            500                 505                 510

Glu Asn Pro Cys Ala Gln Ala His Ile Ser Glu Trp Val Gln Gln Thr
        515                 520                 525

Leu Gly Lys His Lys Val Pro Thr Leu Val Phe His Leu Gly Val Asp
    530                 535                 540

Gly Val Pro Gly Asp Phe Pro Lys Thr Ala Ser Gly Lys Ile Lys Lys
545                 550                 555                 560

Val Asp Leu Val Ala Ile Gly Thr Gln Leu Val Arg Gly Ala Gly Lys
                565                 570                 575

Leu

<210>  73
<211>  696
<212>  PRT
<213>  Penicillium chrysogenum

<400>  73

Met Ser Pro His Pro Gln Thr Thr Thr His Ser His Ser Leu Gly Asp
1               5                   10                  15

Pro Glu Thr Phe Trp Ser His His Ala Ala Arg Leu His Trp His His
            20                  25                  30

Lys Pro Ser Arg Ala Leu Thr Arg Lys Thr Lys Phe Leu Ala Ser Gly
        35                  40                  45

Thr Lys His Glu His Trp Ser Trp Phe Ala Asp Gly Glu Ile Ser Thr
    50                  55                  60

Thr Tyr Asn Cys Val Asp Arg His Val Leu Ala Gly His Gly Asp Asn
65                  70                  75                  80

Val Ala Ile Val Trp Glu Ser Pro Val Thr Gly Val Thr Glu Lys Tyr
                85                  90                  95

Thr Tyr Ala Arg Leu Leu Asp Glu Val Glu Val Leu Ala Gly Val Leu
            100                 105                 110

Arg Glu Glu Gly Val Gln Lys Gly Asp Val Val Ile Ile Tyr Met Pro
        115                 120                 125

Met Ile Pro Ala Ala Leu Ile Ala Ala Leu Ala Ile Thr Arg Leu Gly
    130                 135                 140

Ala Ile His Ala Ala Val Phe Gly Gly Phe Ala Pro Gln Ala Leu Ala
145                 150                 155                 160

Gln Arg Ile Glu Ala Ala Arg Pro Arg Ala Ile Met Thr Ala Ser Cys
                165                 170                 175

Gly Ile Glu Gly Ser Lys Gly Pro Ile Pro Tyr Arg Pro Leu Val Glu
            180                 185                 190

Gly Ala Leu Lys Ala Ser Ser Phe Lys Pro Ser Lys Val Ile Val Trp
        195                 200                 205

Gln Arg Asp Gln Leu Arg Trp Asn Gln Pro Asp Lys Arg Gly Gly Gln
    210                 215                 220

Arg Asn Trp Gln Arg Leu Val Lys Ser Ala Arg Phe Arg Gly Val Lys
225                 230                 235                 240

Ala Gly Pro Val Pro Val Lys Ser Thr Asp Ala Leu Tyr Ile Ile Tyr
                245                 250                 255

Thr Ser Gly Thr Thr Gly Leu Pro Lys Gly Val Leu Arg Glu Ala Gly
            260                 265                 270

Gly His Ala Val Gly Leu Glu Leu Ser Ile Lys Ser Leu Phe Gly Ile
        275                 280                 285

Thr Gly Pro Gly Asp Thr Met Phe Cys Ala Ser Asp Ile Gly Trp Val
    290                 295                 300

Val Gly His Ser Tyr Ile Leu Tyr Ala Pro Leu Leu Val Gly Ala Thr
305                 310                 315                 320

Thr Val Leu Phe Glu Gly Lys Pro Val Gly Thr Pro Asp Ala Gly Thr
                325                 330                 335

Phe Trp Arg Ile Ile Glu Lys His Asn Val Asn Ala Leu Phe Thr Ala
            340                 345                 350

Pro Thr Ala Met Arg Ala Ile Arg Lys Asp Asp Pro Asp Asn Gln Leu
        355                 360                 365

Ile Gly Glu Val Gly Lys Arg Gly Gly Leu Lys Ser Leu Arg Ala Leu
    370                 375                 380

Phe Leu Ala Gly Glu Arg Ser Glu Pro Gly Ile Val Ser Ser Tyr Gln
385                 390                 395                 400

Gly Leu Leu Gly Lys Tyr Ala Ala Ala Gly Ala Leu Val Ile Asp Asn
                405                 410                 415

Trp Trp Ser Ser Glu Ser Gly Ser Pro Ile Thr Gly Val Ala Leu Asp
            420                 425                 430

Gly Arg Ala Ala Leu Ala Ser Pro Ala Ser Gly Pro Asp His Pro Pro
        435                 440                 445

Leu Thr Pro Arg Pro Gly Ser Ala Gly Phe Pro Met Pro Gly Phe Asp
    450                 455                 460

Val Arg Ile Val Asp Asp Glu Gly Asn Glu Val Pro Arg Gly Thr Met
465                 470                 475                 480

Gly Asn Ile Val Met Ala Pro Pro Leu Ala Pro Ser Ala Phe Thr Thr
                485                 490                 495

Leu Phe Glu Asp Glu Glu Arg Phe Tyr Arg Gly Tyr Leu Lys Arg Phe
            500                 505                 510

Asn Gly Arg Trp Ile Asp Thr Gly Asp Ala Gly Met Ile Asp Lys Asp
        515                 520                 525

Gly Tyr Val His Ile Met Ser Arg Ser Asp Asp Ile Ile Asn Val Ala
    530                 535                 540

Ala His Arg Phe Ser Thr Gly Ser Ile Glu Gln Ala Ile Leu Ser His
545                 550                 555                 560

Pro Ser Ile Ser Glu Ala Ser Val Val Gly Ile Pro Asp Pro Leu Lys
                565                 570                 575

Gly His Leu Pro Phe Ala Phe Ile Gln Pro Lys Ala Gly Ala Val Pro
            580                 585                 590

Gly Glu Asn Gly His Leu Pro Ala Thr Pro Ser Pro Gln Leu Phe Ala
        595                 600                 605

Glu Val Asn Ala Leu Val Arg Glu Gln Ile Gly Ala Ile Ala Ser Leu
    610                 615                 620

Gly Gly Ile Ile Gln Gly Arg Gly Met Ile Pro Lys Thr Arg Ser Gly
625                 630                 635                 640

Lys Thr Leu Arg Arg Val Leu Arg Glu Leu Val Glu Ile Gly Val Lys

                645                 650                 655

Gly Glu Tyr Ala Ala Glu Val Ser Val Pro Pro Thr Val Glu Asp Ala
            660                 665                 670

Glu Val Val Ser Val Ala Arg Lys Arg Val Arg Glu Tyr Phe Glu Ala
        675                 680                 685

Lys Gly Val Gly Lys Ser Lys Leu
    690                 695

<210> 74
<211> 693
<212> PRT
<213> Penicillium chrysogenum

<400> 74

Met Pro His Ile Asp Val Asn Val Glu Pro Glu Ala Lys Glu Leu Trp
1               5                   10                  15

Arg Pro Ser Ser Pro Glu Thr Thr Gln Ile Tyr Asp Phe Met Thr Lys
            20                  25                  30

Val Asn Lys Lys Tyr Gly Leu Ser Leu Asn Asn Tyr Asp Thr Leu Trp
        35                  40                  45

Lys Trp Ser Val Ser Glu Pro Ala Gln Phe Trp Glu Glu Ile Trp His
    50                  55                  60

Tyr Thr Lys Ile Asn Ala His Thr Pro Tyr Gln His Val Leu Asp Ser
65                  70                  75                  80

Lys Asp Val Leu Phe Pro Arg Pro Ser Phe Phe Glu Gly Ser Thr Leu
                85                  90                  95

Asn Phe Ala Glu Asn Leu Leu Tyr Pro Ala Thr Ser Pro Asp Glu Asp
            100                 105                 110

Ser Val Ala Val Ile Gly Ala Thr Glu Val Ala Arg Glu Phe Val Ser
        115                 120                 125

Trp Lys Glu Leu Arg Glu Arg Val Arg Leu Cys Ala Asn Ala Leu Lys
    130                 135                 140

Glu Ala Gly Leu Lys Thr Gly Asp Arg Val Ala Gly Phe Val Gly Asn
145                 150                 155                 160

His Ala Asn Thr Val Val Ala Met Leu Ala Ala Ala Ser Ile Gly Ala
                165                 170                 175

Phe Trp Thr Gly Val Ser Pro Asp Thr Gly Val His Ala Val Leu Glu
            180                 185                 190

Arg Leu Lys Gln Ile Gln Pro Lys Ile Leu Phe Ala Asp Asn Gly Ser
        195                 200                 205

Phe Tyr Asn Gly Lys Val His Ser Ser His Ala Lys Val Arg Glu Ile
    210                 215                 220

Val Ser Glu Leu Pro Asp Leu Glu Leu Leu Val Leu Leu Ile Thr Thr
225                 230                 235                 240

Pro Glu Leu Glu Val Asn Leu Asp Asp Leu Arg Pro Ala Asn Gly Lys
                245                 250                 255

Ala Lys Val Tyr Gly Asp Phe Val Ser Glu Val Lys Asp Ser Gln Ala
            260                 265                 270

Pro Leu Glu Phe Ala Ser Leu Arg Pro Asp His Pro Val Tyr Ile Leu
        275                 280                 285

Tyr Ser Ser Gly Thr Thr Gly Ala Pro Lys Pro Ile Val His Gly Ser
    290                 295                 300

Leu Gly Thr Leu Leu Gln His Lys Lys Glu His Leu Leu His Cys Asp
305                 310                 315                 320

Ile Arg Pro Gly Asp Arg Leu Phe Tyr Phe Thr Thr Thr Thr Trp Met
                325                 330                 335

Met Trp His Trp Leu Val Ser Gly Leu Ala Ser Gly Ala Thr Ile Val
            340                 345                 350

Leu Tyr Asp Gly Ser Pro Phe Arg Pro Phe Asp Ala Glu Gly Gly Lys
        355                 360                 365

Gly Glu Met Ala Met Pro Arg Leu Ile Glu Glu Leu Gln Ile Thr His
    370                 375                 380

Phe Gly Thr Ser Ala Lys Tyr Leu Ser Leu Leu Glu Gln Ala Ala Leu
385                 390                 395                 400

Asn Pro Arg Lys His Ala His Arg Pro Val Ser Leu Lys Thr Leu Arg
                405                 410                 415

Ala Ile Phe Ser Thr Gly Ser Pro Leu Ala Pro Ser Thr Phe Glu Tyr
            420                 425                 430

Val Tyr Ser Ser Phe His His Asp Ile Met Leu Gly Ser Ile Thr Gly
        435                 440                 445

Gly Thr Asp Ile Leu Ser Leu Phe Ala Ser Gly Cys Pro Ile Leu Pro
    450                 455                 460

Val Tyr Lys Gly Glu Ile Gln Cys Arg Ser Leu Gly Met Asp Ile Ser
465                 470                 475                 480

Val Phe Asp Tyr Ala Gly Lys Asp Ile Ser Ala Thr Gly Glu Pro Gly
                485                 490                 495

Asp Leu Val Cys Val Thr Pro Phe Pro Ala Gln Pro Val Met Phe Trp
            500                 505                 510

Pro Pro Gly Pro Thr Gly Leu Glu Lys Tyr Arg Lys Ser Tyr Phe Asp
        515                 520                 525

Val Phe Gly Ser Ser Val Trp His His Gly Asp Tyr Val Arg Leu Asn
    530                 535                 540

Pro Gln Thr Gly Gly Val Val Met Leu Gly Arg Ser Asp Gly Val Leu
545                 550                 555                 560

Lys Pro Ser Gly Val Arg Phe Gly Ser Ala Glu Ile Tyr Asn Ile Leu
                565                 570                 575

Leu Lys His Phe Ala Glu Asp Val Glu Asp Ser Leu Cys Ile Gly Arg
            580                 585                 590

Arg Arg Asp Gly Val Asp Thr Asp Glu Thr Val Val Leu Phe Val Arg

595                 600                 605

Leu Ser Ser Ala Asn Glu Ser Gly Met Pro Ala Asp Leu Ala Ala Arg
    610                 615                 620

Ile Gln Ala Thr Ile Arg Lys Glu Leu Ser Pro Arg His Val Pro Gly
625                 630                 635                 640

Ile Ile Asp Ala Cys Pro Glu Ile Pro Val Thr Ser Asn Gly Lys Lys
                645                 650                 655

Val Glu Asn Ala Val Lys Gln Ile Leu Cys Gly Leu Asn Ile Lys Ile
            660                 665                 670

Gly Ala Ser Val Ala Asn Ala Ser Cys Leu Asp Trp Tyr Arg Asn Trp
        675                 680                 685

Ala Ser Glu Asn Pro
    690

<210>  75
<211>  669
<212>  PRT
<213>  Penicillium chrysogenum

<400>  75

Met Ser Asp Gly Pro Ile Gln Pro Pro Lys Pro Ala Val Val His Glu
1               5                   10                  15

Ala His Glu Val Asp Thr Phe His Val Pro Lys Ala Phe His Asp Lys
            20                  25                  30

His Pro Ser Gly Thr His Ile Lys Asp Ile Glu Glu Tyr Lys Lys Leu
        35                  40                  45

Tyr Glu Glu Ser Ile Lys Ser Pro Asp Thr Phe Trp Ala Arg Met Ala
    50                  55                  60

Arg Glu Leu Leu Thr Phe Asp Lys Asp Phe Glu Thr Thr His His Gly
65                  70                  75                  80

Ser Phe Glu Asn Gly Asp Asn Ala Trp Phe Val Glu Gly Arg Leu Asn
                85                  90                  95

Ala Ser Phe Asn Cys Val Asp Arg His Ala Leu Lys Asn Pro Asp Lys
            100                 105                 110

Val Ala Ile Ile Tyr Glu Ala Asp Glu Pro Asn Glu Gly Arg Lys Ile
        115                 120                 125

Thr Tyr Gly Glu Leu Met Arg Glu Val Ser Arg Val Ala Trp Thr Leu
    130                 135                 140

Lys Glu Arg Gly Val Lys Lys Gly Asp Thr Val Gly Ile Tyr Leu Pro
145                 150                 155                 160

Met Ile Pro Glu Ala Val Ile Ala Phe Leu Ala Cys Ser Arg Ile Gly
                165                 170                 175

Ala Val His Ser Val Val Phe Ala Gly Phe Ser Ser Asp Ser Leu Arg
            180                 185                 190

Asp Arg Val Leu Asp Ala Ser Ser Lys Val Ile Ile Thr Ser Asp Glu
        195                 200                 205

Gly Lys Arg Gly Gly Lys Ile Ile Gly Thr Lys Lys Ile Val Asp Glu
    210                 215                 220

Ala Met Lys Gln Cys Pro Asp Val His Thr Val Leu Val Tyr Lys Arg
225                 230                 235                 240

Thr Gly Ala Glu Val Pro Trp Thr Ala Gly Arg Asp Ile Trp Trp His
                245                 250                 255

Glu Glu Val Glu Lys Tyr Pro Asn Tyr Leu Ala Pro Glu Ser Val Ser
            260                 265                 270

Ser Glu Asp Pro Leu Phe Leu Leu Tyr Thr Ser Gly Ser Thr Gly Lys
        275                 280                 285

Pro Lys Gly Val Met His Thr Thr Ala Gly Tyr Leu Leu Gly Ala Ala
    290                 295                 300

Met Thr Gly Lys Tyr Val Phe Asp Ile His Asp Asp Asp Arg Tyr Phe
305                 310                 315                 320

Cys Gly Gly Asp Val Gly Trp Ile Thr Gly His Thr Tyr Val Val Tyr
                325                 330                 335

Ala Pro Leu Leu Leu Gly Cys Ala Thr Val Val Phe Glu Ser Thr Pro
            340                 345                 350

Ala Tyr Pro Asn Phe Ser Arg Tyr Trp Asp Val Ile Asp Lys His Asp
        355                 360                 365

Val Thr Gln Phe Tyr Val Ala Pro Thr Ala Leu Arg Leu Leu Lys Arg
    370                 375                 380

Ala Gly Asp Glu His Ile His His Lys Met His Ser Leu Arg Ile Leu
385                 390                 395                 400

Gly Ser Val Gly Glu Pro Ile Ala Ala Glu Val Trp Lys Trp Tyr Phe
                405                 410                 415

Glu Cys Val Gly Lys Glu Glu Ala His Ile Cys Asp Thr Tyr Trp Gln
            420                 425                 430

Thr Glu Thr Gly Ser His Val Ile Thr Pro Leu Gly Gly Ile Thr Pro
        435                 440                 445

Thr Lys Pro Gly Ser Ala Ser Leu Pro Phe Phe Gly Ile Glu Pro Ala
    450                 455                 460

Ile Ile Asp Pro Val Ser Gly Glu Glu Ile Val Gly Asn Asp Val Glu
465                 470                 475                 480

Gly Val Leu Ala Phe Lys Gln Pro Trp Pro Ser Met Ala Arg Thr Val
                485                 490                 495

Trp Gly Ala His Lys Arg Tyr Met Asp Thr Tyr Leu Asn Val Tyr Lys
            500                 505                 510

Gly Tyr Tyr Phe Thr Gly Asp Gly Ala Gly Arg Asp His Asp Gly Tyr
        515                 520                 525

Tyr Trp Ile Arg Gly Arg Val Asp Asp Val Val Asn Val Ser Gly His
    530                 535                 540

Arg Leu Ser Thr Ala Glu Ile Glu Ala Ala Leu Leu Glu His Pro Ser

545                 550                 555                 560

Val Ala Glu Ala Ala Val Val Gly Ile Ala Asp Glu Leu Thr Gly Gln
                565                 570                 575

Ala Val Asn Ala Phe Val Ser Leu Lys Glu Gly Lys Pro Thr Glu Gln
            580                 585                 590

Ile Ser Lys Asp Leu Ala Met Gln Val Arg Lys Ser Ile Gly Pro Phe
        595                 600                 605

Ala Ala Pro Lys Ala Val Phe Val Val Asp Asp Leu Pro Lys Thr Arg
    610                 615                 620

Ser Gly Lys Ile Met Arg Arg Ile Leu Arg Lys Ile Leu Ser Gly Glu
625                 630                 635                 640

Glu Asp Ser Leu Gly Asp Thr Ser Thr Leu Ser Asp Pro Ser Val Val
                645                 650                 655

Asp Lys Ile Ile Glu Thr Val His Ser Ala Arg Gln Lys
            660                 665

<210>  76
<211>  578
<212>  PRT
<213>  Penicillium chrysogenum

<400>  76

Met Val Phe Leu Pro Pro Lys Glu Ser Gly Gln Leu Asp Pro Ile Pro
1               5                   10                  15

Asp Asn Ile Pro Ile Ser Glu Phe Met Leu Asn Glu Arg Tyr Gly Arg
            20                  25                  30

Val Arg His Ala Ser Ser Arg Asp Pro Tyr Thr Cys Gly Ile Thr Gly
        35                  40                  45

Lys Ser Tyr Ser Ser Lys Glu Val Ala Asn Arg Val Asp Ser Leu Ala
    50                  55                  60

Arg Ser Leu Ser Lys Glu Phe Gly Trp Ala Pro Asn Glu Gly Ser Glu
65                  70                  75                  80

Trp Asp Lys Thr Leu Ala Val Phe Ala Leu Asn Thr Ile Asp Ser Leu
                85                  90                  95

Pro Leu Phe Trp Ala Val His Arg Leu Gly Gly Val Leu Thr Pro Ala
            100                 105                 110

Asn Ala Ser Tyr Ser Ala Ala Glu Leu Thr His Gln Leu Leu Asp Ser
        115                 120                 125

Lys Ala Lys Ala Leu Val Thr Cys Val Pro Leu Leu Ser Ile Ser Leu
    130                 135                 140

Glu Ala Ala Ala Lys Ala Gly Leu Pro Lys Asn Arg Ile Tyr Leu Leu
145                 150                 155                 160

Asp Val Pro Glu Gln Leu Leu Gly Gly Val Lys Pro Pro Ala Gly Tyr
                165                 170                 175

Lys Ser Val Ser Glu Leu Thr Gln Ala Gly Lys Ser Leu Pro Pro Val
            180                 185                 190

Asp Glu Leu Arg Trp Ser Ala Gly Glu Gly Ala Arg Arg Thr Ala Phe
        195                 200                 205

Val Cys Tyr Ser Ser Gly Thr Ser Gly Leu Pro Lys Gly Val Met Ile
    210                 215                 220

Ser His Arg Asn Val Ile Ala Asn Thr Leu Gln Ile Lys Ala Phe Glu
225                 230                 235                 240

Gln Asn Tyr Arg Asp Gly Gly Gly Thr Lys Pro Ala Ser Thr Glu Val
                245                 250                 255

Ala Leu Gly Leu Leu Pro Gln Ser His Ile Tyr Ala Leu Val Val Ile
            260                 265                 270

Gly His Ala Gly Ala Tyr Arg Gly Asp Gln Thr Ile Val Leu Pro Lys
        275                 280                 285

Phe Glu Leu Lys Ser Tyr Leu Asn Ala Ile Gln Gln Tyr Lys Ile Ser
    290                 295                 300

Ala Leu Phe Leu Val Pro Pro Ile Ile Ile His Met Leu Gly Thr Gln
305                 310                 315                 320

Asp Val Cys Ser Lys Tyr Asp Leu Ser Ser Val Thr Ser Leu Phe Thr
                325                 330                 335

Gly Ala Ala Pro Leu Gly Met Glu Thr Ala Ala Asp Phe Leu Lys Leu
            340                 345                 350

Tyr Pro Asn Ile Leu Ile Arg Gln Gly Tyr Gly Leu Thr Glu Thr Cys
        355                 360                 365

Thr Val Val Ser Ser Thr His Pro His Asp Ile Trp Leu Gly Ser Ser
    370                 375                 380

Gly Ala Leu Leu Pro Gly Val Glu Ala Arg Ile Val Thr Pro Glu Asn
385                 390                 395                 400

Lys Glu Ile Thr Thr Tyr Asp Ser Pro Gly Glu Leu Val Val Arg Ser
                405                 410                 415

Pro Ser Val Val Leu Gly Tyr Leu Asn Asn Glu Lys Ala Thr Ala Glu
            420                 425                 430

Thr Phe Val Asp Gly Trp Met Arg Thr Gly Asp Glu Ala Val Ile Arg
        435                 440                 445

Arg Ser Pro Lys Gly Ile Glu His Val Phe Ile Val Asp Arg Ile Lys
    450                 455                 460

Glu Leu Ile Lys Val Lys Gly Leu Gln Val Ala Pro Ala Glu Leu Glu
465                 470                 475                 480

Ala His Ile Leu Ala His Pro Asp Val Ser Asp Cys Ala Val Ile Ala
                485                 490                 495

Ile Pro Asp Asp Arg Ala Gly Glu Val Pro Lys Ala Ile Val Val Lys
            500                 505                 510

Ser Ala Ser Ala Gly Ser Asp Glu Ser Val Ser Gln Ala Leu Val Lys
        515                 520                 525

Tyr Val Glu Asp His Lys Ala Arg His Lys Trp Leu Lys Gly Gly Ile

530                 535                 540

Arg Phe Val Asp Ala Ile Pro Lys Ser Pro Ser Gly Lys Ile Leu Arg
545                 550                 555                 560

Arg Leu Ile Arg Asp Gln Glu Lys Glu Ala Arg Arg Lys Ala Gly Ser
                565                 570                 575

Lys Ile

<210>  77
<211>  617
<212>  PRT
<213>  Penicillium chrysogenum

<400>  77

Met Ser Tyr Ser Gly Ile Val Thr Gly Leu Gly Leu Arg Gly Arg Val
1               5                   10                  15

Ser Cys Arg Arg Ser Ala Phe Gly Gly Lys Arg Ile Leu Gln Gln Pro
            20                  25                  30

Ser Leu Val Lys Arg Gly Leu Leu Thr Glu Ser Tyr Ala Arg Gly Pro
        35                  40                  45

Ser Gly Pro Pro Leu Ile Glu Ser Thr Val Gly Glu His Phe Ala Lys
    50                  55                  60

Ile Val Ala Glu Cys Gly Asp Arg Thr Ala Val Val Ser Arg His Gln
65                  70                  75                  80

Asn Asp Arg Ala Thr Tyr Ala Ser Leu Asp Thr Arg Ser Asn Ser Leu
                85                  90                  95

Ala Arg Gly Leu Glu Ser Leu Gly Val Gly Lys Gly Glu Arg Val Gly
            100                 105                 110

Val Met Leu Gly Asn Ser Met Glu Tyr Ala Val Ala Thr Tyr Ala Leu
        115                 120                 125

Phe Lys Leu Gly Ala Val Leu Val Pro Leu Asn Pro Ser Phe Asn Thr
    130                 135                 140

Ala Gln Val Ile Ala Ala Leu Gly His Leu Glu Ala Ser His Leu Leu
145                 150                 155                 160

Ile Ser Thr Glu Ser Asn Leu Pro Arg Lys Lys Pro Arg Ser Asn Val
                165                 170                 175

Pro Leu Leu Asn Asp Leu Val Glu Asp Leu His Lys Ser Lys Leu Glu
            180                 185                 190

Ser Ala Pro Val Pro Ser Leu Arg Asn Ile Ile Met Val Asp Asn Ser
        195                 200                 205

Glu Gly Arg Val Asp Ile Ser Ser Tyr Lys Ser Leu Thr Gln Tyr Ala
    210                 215                 220

Ser Ile Met Ser Gln Leu Thr Val Asp Gly Ser Pro Leu Pro Pro Arg
225                 230                 235                 240

Asn Leu Ser Pro Asp Glu Thr Val Asn Ile Gln Phe Thr Ser Gly Thr
                245                 250                 255

Thr Ser Met Pro Lys Ala Ala Cys Leu Thr His Arg Ser Ile Leu Asn
            260                 265                 270

Asn Gly Ser Gln Ile Gly Asp Arg Met Leu Leu Thr Pro Asn Asp Ile
        275                 280                 285

Ile Cys Cys Pro Pro Pro Leu Phe His Cys Phe Gly Ser Ile Leu Gly
    290                 295                 300

Tyr Met Ala Thr Ala Thr His Gly Ser Ala Ile Val Phe Pro Ser Glu
305                 310                 315                 320

Ser Phe Asn Ala Arg Ala Ala Leu Glu Ala Val Gln Glu Glu Lys Cys
                325                 330                 335

Thr Ala Leu Tyr Gly Val Pro Thr Met Phe Leu Glu Glu Leu Ser Leu
            340                 345                 350

Ile Glu Thr Gly Glu Val Ser Ser Glu Gly Phe Gln His Leu Arg Thr
        355                 360                 365

Gly Ile Ala Ala Gly Ser Ser Ile Pro Ala Glu Ile Met Lys Lys Leu
    370                 375                 380

His Lys Val Leu Asn Leu Thr Glu Leu Thr Ile Cys Tyr Gly Met Thr
385                 390                 395                 400

Glu Thr Ser Pro Val Ser Ala Met Thr Thr Thr Asp Asp Pro Ile Asp
                405                 410                 415

Lys Arg Ile Tyr Ser Val Gly Lys Leu Met Pro His Val Glu Ala Lys
            420                 425                 430

Ile Val Asn Pro Val Asp Lys Asn Val Ile Leu Pro Ile Glu Arg Arg
        435                 440                 445

Gly Glu Leu Ala Val Ser Gly Tyr Leu Leu Met Lys Glu Tyr Trp Ala
    450                 455                 460

Asp Pro Glu Lys Thr Ala Glu Val Met Ile Pro Asp Asp Ser Gly Lys
465                 470                 475                 480

Val Trp Met His Thr Gly Asp Glu Ala Ser Met Ser Pro Asp Gly Tyr
                485                 490                 495

Ile Thr Ile Thr Gly Arg Ile Lys Asp Leu Ile Ile Arg Gly Gly Glu
            500                 505                 510

Asn Ile His Pro Leu Glu Ile Glu Asn Cys Leu Leu Ala Asn Asn Gly
        515                 520                 525

Val Ala Asp Val Ser Val Val Gly Val Pro Asp Val Arg Tyr Gly Glu
    530                 535                 540

Ala Val Ala Ala Phe Ile Val Pro Arg Asp His Ser Ser Asn Thr Val
545                 550                 555                 560

Thr Ala Glu Glu Ile Gln Gln Trp Val Arg Glu Lys Leu Ser Asn His
                565                 570                 575

Leu Ile Pro Lys His Val Phe Phe Leu Gly Pro Leu Glu Ser Phe Pro
            580                 585                 590

Lys Thr Ala Ser Gly Lys Ile Gln Lys Phe Lys Leu Arg Glu Thr Ala

        595                 600                 605

Ile Ala Leu Leu Thr Lys Cys Ala Ala
    610                 615

<210> 78
<211> 595
<212> PRT
<213> Penicillium chrysogenum

<400> 78

Met Ser Ser Ser Leu Leu Ser Leu Leu Ser Arg Ser Asn Ala Arg Val
1               5                   10                  15

Cys Phe Thr Pro Arg Arg Thr Pro Leu Ser Ala Arg Leu Leu Ser Thr
            20                  25                  30

Leu Pro Asn Thr Pro Ile Phe Arg Ala Leu Gln Asn His Asp Pro Glu
        35                  40                  45

Arg Val Ala Val Val His Ser Val Ser Ser Arg Ser Phe Thr Tyr Gly
    50                  55                  60

Ser Leu Ile Ala Asp Ile Val Arg Ala Lys Asp Asp Leu Glu Gln Lys
65                  70                  75                  80

Ala Ala Lys Ala Gln Gly Gln Leu Ala Gly Glu Arg Val Ala Phe Leu
                85                  90                  95

Ala Glu Asn Ser Tyr Asp Tyr Val Val Thr Leu Leu Ala Ile Phe Ala
            100                 105                 110

Ser Asp Ala Ile Ala Leu Pro Leu Ser Pro Ser Phe Pro Thr Gly Glu
        115                 120                 125

Leu Lys Tyr Ile Leu Asp Asn Ser Gln Ala Lys Met Leu Leu Thr Thr
    130                 135                 140

Glu Lys Tyr Ala Asp Lys Gly Met Glu Leu Leu Arg Glu Gly Leu Glu
145                 150                 155                 160

Arg Glu Pro Leu Phe Ala Ile Arg Asn Lys Leu Thr Glu Gly Ala Ser
                165                 170                 175

Ser Gly Glu Ser Val Thr Leu His Asp Leu Lys Gln Pro Ser Ser Gly
        180                 185                 190

Gly Met Met Leu Tyr Thr Ser Gly Thr Thr Asn Arg Pro Lys Gly Val
    195                 200                 205

Leu Ile Pro Gln Ser Ala Leu Ala Ala Gln Ala Ser Ser Leu Leu Glu
    210                 215                 220

Ala Trp Lys Tyr Ser Pro Asp Asp Arg Leu Leu His Leu Leu Pro Leu
225                 230                 235                 240

His His Ile His Gly Val Val Asn Ala Ile Val Ala Pro Ile Val Ala
                245                 250                 255

Gly Ser Ser Val Glu Phe Met Tyr Pro Phe Asn Pro Asp Lys Val Trp
        260                 265                 270

Lys Arg Leu Ala Ala Pro Phe Leu Ser Ser Asn Ala Ser Lys Pro Ala
        275                 280                 285

Ile Thr Phe Leu Thr Ala Val Pro Thr Ile Tyr Asn Arg Leu Met Ala
    290                 295                 300

Thr Phe Pro Lys Leu Thr Pro Glu Leu Gln Asn Ala Ala Lys Glu Gly
305                 310                 315                 320

Ile Ser Pro Gln Asn Leu Arg Leu Asn Ile Ser Gly Ser Ala Ala Leu
                325                 330                 335

Pro Thr Pro Thr Lys Thr Ala Trp Thr Asn Leu Ser Asn Gly Asn Val
            340                 345                 350

Leu Leu Glu Arg Phe Gly Met Thr Glu Val Gly Met Ala Ile Ser Cys
        355                 360                 365

Gly Leu Asp Ala Ala Asp Arg Val Asp Gly Ser Val Gly Trp Pro Leu
    370                 375                 380

Pro Gly Val Glu Ala Arg Leu Ala Asp Thr Glu Thr Gly Ala Val Ile
385                 390                 395                 400

Pro Val Glu Glu Lys Glu Pro Ser Gly Arg Glu Arg Glu Gly Glu Ile
                405                 410                 415

Gln Leu Arg Gly Glu Thr Ile Phe Asp His Tyr Trp Gly Asn Glu Lys
            420                 425                 430

Ala Thr Arg Glu Ser Phe Val Gln Ser Asp Asp Gly Gly Arg Pro Trp
        435                 440                 445

Phe Cys Thr Gly Asp Val Ala Thr Arg Arg Val Val Asp Gly Ala Gly
    450                 455                 460

Ser Gly Ala Ser Gly Ala Trp Ala Gln Gly Pro Met Tyr Phe Ile Gln
465                 470                 475                 480

Gly Arg Lys Ser Val Asp Ile Ile Lys Thr Gly Gly Glu Lys Val Ser
                485                 490                 495

Ala Leu Glu Val Glu Arg Glu Leu Leu Ser Leu Pro Gln Ile Thr Glu
            500                 505                 510

Ala Ala Val Val Gly Leu Pro Ser Glu Gln Trp Gly Gln Lys Ile Ala
        515                 520                 525

Ala Val Val Val Leu Ala Pro Asp Ala Ala Ala Ser Gly Arg Asn Gly
    530                 535                 540

Gln Ser Trp Gly Pro Met Asp Met Arg Arg Ala Leu Lys Gly Ser Leu
545                 550                 555                 560

Ala Ser Phe Lys Ile Pro Gln Glu Met Lys Val Leu Ala Ala Ile Pro
                565                 570                 575

Arg Asn Ala Met Gly Lys Val Asn Lys Lys Ala Leu Val Lys Glu Val
            580                 585                 590

Phe Gly Val
        595

<210>  79
<211>  566
<212>  PRT
<213>  Penicillium chrysogenum

<400> 79

Met Pro Thr Ile Tyr Arg Ser Pro Tyr Pro Asp Leu Asp Ile Gln Ser
1               5                   10                  15

Val Asp Leu Val Ser Tyr Leu Phe Ser Asn Pro Phe Asn Thr Pro Leu
            20                  25                  30

Asp Arg Pro Met Tyr Ile Asn Ala Ile Ser Gly Glu Gln Tyr Thr Phe
        35                  40                  45

Gly Asp Val Val Gln Arg Thr Arg Ser Leu Ser Asn Gly Leu Arg Gln
    50                  55                  60

Ser Ile Gly Leu Lys Pro Asn Asp Val Val Ala Leu Phe Ser Pro Asn
65                  70                  75                  80

Thr Ile Asp Tyr Pro Val Val Cys His Ala Ile Val Gly Ser Arg Ala
                85                  90                  95

Ile Val Ala Pro Thr Ser Ala Ala Leu Thr Ala Leu Glu Leu Asn Ala
            100                 105                 110

Gln Leu Lys Thr Ser Gly Ala Arg Phe Ile Val Val His Ser Thr Leu
        115                 120                 125

Leu Glu Thr Ala Gln Lys Ala Ala Lys Gly Thr Ser Val Glu Lys Val
    130                 135                 140

Leu Leu Ile Asp Gly Gln Thr Pro Val Asn Gly Gln Pro Thr Cys Asn
145                 150                 155                 160

Tyr Leu Ala Asn Thr Phe Ala Pro Asp Asp Leu Leu Thr Val Asp Pro
                165                 170                 175

Ala Glu Ala Asp Arg Gln Pro Thr Phe Ile Cys Phe Ser Ser Gly Thr
            180                 185                 190

Ser Gly Ala Ala Lys Gly Val Ile Thr Thr His Gln Asn Ile Thr Ser
        195                 200                 205

Asn Leu Gln Gln Trp Arg Gln His Met Leu Glu Ser Gly Leu Pro Ser
    210                 215                 220

Gln Arg Pro Arg Arg Gln Ser Ala Ile Ala Phe Leu Pro Phe Ser His
225                 230                 235                 240

Ile Tyr Gly Leu Asn Leu Phe Met Cys Gln Cys Leu Ile Trp Gly Thr
                245                 250                 255

Thr Val Val Val Met Pro Arg Phe Asp Leu Asp Leu Tyr Leu Ser Cys
            260                 265                 270

Ile Gln Lys Tyr Arg Pro Asp Glu Leu Ala Leu Val Pro Pro Ile Ala
        275                 280                 285

Leu Met Leu Val Lys Asp Pro Arg Val Ser Lys Tyr Asp Leu Ser Ser
    290                 295                 300

Val Arg Lys Ile Met Ser Ala Ala Ala Pro Leu Thr Ile Glu Leu Ser
305                 310                 315                 320

Ser Ala Leu Glu Ala Lys Phe Thr Glu Ile Cys Lys Thr Glu Val Phe
                325                 330                 335

Cys Thr Gln Ser Trp Gly Leu Thr Glu Thr Ser Pro Met Ala Thr Ala
            340                 345                 350

Val Pro Asn Asp Arg Met Asp Lys Arg Asn Thr Gly Val Gly Cys Ile
        355                 360                 365

Ala Pro Asn Met Gln Leu Arg Phe Val Asp Pro Glu Ser Met Lys Asp
    370                 375                 380

Ala Ala Val Lys Pro Asp Gly Ser Thr Glu Pro Ala Glu Ile Trp Cys
385                 390                 395                 400

Arg Gly Pro Asn Val Val Met Gly Tyr Tyr Asn Asn Glu Lys Ala Thr
                405                 410                 415

Lys Glu Ala Phe His Val Asp Glu Asp Gly Thr Arg Trp Phe Arg Thr
            420                 425                 430

Gly Asp Ile Gly Thr Ile Asp Gly Asp Gly Tyr Val Thr Ile Gln Asp
        435                 440                 445

Arg Ile Lys Glu Met Ile Lys Tyr Lys Gly Leu Gln Val Ile Pro Ser
    450                 455                 460

Glu Leu Glu Gly Lys Leu Val Asp His Pro Asp Val Glu Asp Ala Ala
465                 470                 475                 480

Val Thr Gly Met Trp Val Asp Asp Met Ala Thr Glu Leu Pro Val Gly
                485                 490                 495

Phe Val Val Leu Ser Pro Gln Ala Lys Asp Arg Asp Gln Lys Ala Val
            500                 505                 510

Leu Asp Gly Ile His Ala Trp Leu Asn Glu Arg Ile Ala Asn His Lys
        515                 520                 525

Arg Leu Arg Gly Gly Ile His Val Leu Ser Gln Ile Pro Lys Ser Pro
    530                 535                 540

Ser Gly Lys Ile Leu Arg Arg Gln Leu Arg Asp Leu Leu Lys Ser Gln
545                 550                 555                 560

Ala Pro Lys Ala Arg Leu
                565

<210>  80
<211>  584
<212>  PRT
<213>  Penicillium chrysogenum

<400>  80

Met Ala Ala Pro Arg Ser Val Gln Arg Leu Gln Gln Thr Leu Ser His
1               5                   10                  15

Val Gln Pro Pro Ile Asn Pro Gln Leu Ser Leu Val Ala Gly Pro Thr
            20                  25                  30

Glu Pro Gln Leu Leu Asp Ile Thr Leu Gly Glu Leu Leu Thr Leu Gln
        35                  40                  45

Ala Leu Gln Tyr Gly Arg Ile Glu Cys Leu Val Phe Pro Trp Thr Gly
    50                  55                  60

Ala Arg Trp Thr Tyr Gly Gln Leu Lys Asp Glu Thr Asp Arg Leu Ala
65                  70                  75                  80

Arg Gly Met Leu Ala Ser Gly Ile Gln Lys Gly Asp Arg Val Gly Ile
                85                  90                  95

Met Ala Gly Asn Cys Glu Gln Tyr Ile Ser Val Phe Phe Ala Ala Ala
            100                 105                 110

Arg Val Gly Ala Ile Leu Val Val Leu Asn Asn Thr Tyr Thr Pro Ser
        115                 120                 125

Glu Leu Tyr Tyr Ala Leu Asn His Ser Glu Cys Arg Met Leu Phe Met
    130                 135                 140

Thr Pro Arg Ile Gly Arg His Asn Leu Glu Glu Val Leu Thr Gln Leu
145                 150                 155                 160

Gly Pro Asn Pro Lys Lys Ala Ala Thr Ser Glu Thr Leu Glu Glu Ile
                165                 170                 175

Val Ile Leu Arg Glu Ser Tyr Asn Asn Phe Pro Thr Tyr His Asp Val
            180                 185                 190

Met Glu Arg Gly Leu Ser Gln Ala Ala His Val Leu Gln Asp Arg Glu
        195                 200                 205

Ala Glu Leu Gln Pro Asp Asp Val Cys Asn Leu Gln Phe Thr Ser Gly
    210                 215                 220

Ser Thr Gly Asn Pro Lys Ala Ala Met Leu Thr His His Asn Leu Val
225                 230                 235                 240

Asn Asn Ser Arg Phe Ile Gly Asp Arg Met Asn Leu Thr Ser Phe Asp
                245                 250                 255

Val Leu Cys Cys Pro Pro Pro Leu Phe His Cys Phe Gly Leu Val Leu
            260                 265                 270

Gly Met Leu Ala Val Val Thr His Gly Ala Lys Ile Val Phe Pro Ser
        275                 280                 285

Glu Thr Phe Asp Pro Lys Ala Val Leu His Ala Ile Ser Asp Glu Lys
    290                 295                 300

Cys Thr Ala Leu His Gly Val Pro Thr Met Phe Glu Ala Ile Leu Ser
305                 310                 315                 320

Val Pro Lys Pro Ser Asn Phe Asp Thr Ser Asn Leu Arg Thr Gly Ile
                325                 330                 335

Ile Ala Gly Ala Pro Val Pro Arg Pro Leu Met Lys Arg Leu Phe Ala
            340                 345                 350

Glu Leu Asn Met Thr Glu Tyr Thr Ser Ser Tyr Gly Leu Thr Glu Ala
        355                 360                 365

Ser Pro Thr Cys Phe Asn Ala Phe Thr Thr Asp Ser Ile His Thr Arg
    370                 375                 380

Leu Thr Thr Val Gly Lys Val Leu Pro His Ala Arg Ala Lys Ile Ile
385                 390                 395                 400

Asp Thr His Gly Asn Ile Val Pro Val Gly Gln Arg Gly Glu Leu Cys
                405                 410                 415

Met Ala Gly Tyr Gln Leu Thr Lys Gly Tyr Trp Asn Asn Pro Glu Lys
            420                 425                 430

Thr Ala Glu Thr Phe Ile Thr Asp Ala Glu Gly Val Lys Trp Leu Lys
        435                 440                 445

Thr Gly Asp Glu Ala Ser Phe Asn Ala Glu Gly Tyr Cys Thr Ile Thr
    450                 455                 460

Gly Arg Phe Lys Asp Ile Ile Ile Arg Gly Gly Glu Asn Ile Tyr Pro
465                 470                 475                 480

Leu Glu Ile Glu Glu Arg Leu Ala Ala His Pro Ser Ile Glu Leu Ala
                485                 490                 495

Ser Val Ile Gly Ile Ala Asp Pro Lys Tyr Gly Glu Val Val Gly Ala
            500                 505                 510

Phe Ile Ala Ile Ala Ser Gly Ala Glu Arg Pro Thr Asp Asp Glu Leu
        515                 520                 525

Arg Thr Trp Thr Arg Asp Thr Leu Gly Arg His Lys Ala Pro Gln His
    530                 535                 540

Val Phe Val Phe Gly Glu Glu Gly Ala Ser Ser Thr Val Pro Val Thr
545                 550                 555                 560

Gly Ser Gly Lys Val Arg Lys Val Glu Leu Arg Gln Met Ala Met Ala
                565                 570                 575

Val Leu Ala Glu Arg Arg Lys Ala
            580

<210>  81
<211>  525
<212>  PRT
<213>  Penicillium chrysogenum

<400>  81

Met Pro Ile Pro Ser Leu Pro Leu Phe Ala Ala Ala Glu Gln His Ala
1               5                   10                  15

Arg Gln Asn Pro Glu Lys Ile Ala Val Ile Asp Thr Thr Lys Gln Gln
            20                  25                  30

Ser Phe Thr Phe Val Gln Leu Leu Ala Asp Ala Ala Ala Leu Arg Lys
        35                  40                  45

Arg Ile Ile Glu Gln Leu Gly Leu Thr Asp Asp Leu Glu Glu Arg Arg
    50                  55                  60

Ile Ala Phe Leu Val Pro Asn Gly Tyr Asp Tyr Val Ala Thr Gln Trp
65                  70                  75                  80

Ala Val Trp Ala Ala Gly Gly Val Cys Val Pro Leu Cys Ile Ser His
                85                  90                  95

Pro Val Lys Glu Leu Leu Tyr Thr Ile Gly Asp Ser Asp Pro Ser Leu
            100                 105                 110

Ile Ile Val His Pro Glu Phe Glu Lys Ile Ala Pro Ser Leu Arg Glu
        115                 120                 125

Gly Tyr Thr Thr Asp Ile Pro Phe Ile Gly Leu Glu Pro Phe Ser Arg
    130                 135                 140

Asn Glu Thr Pro Thr Leu Pro Ser Phe Ser Pro Pro Phe Ala Leu Thr
145                 150                 155                 160

Arg Arg Ala Leu Met Ile Tyr Thr Ser Gly Thr Thr Ser Asn Pro Lys
                165                 170                 175

Gly Cys Val Thr Thr His Glu Asn Ile Thr Phe Gln Ala Ser Cys Leu
            180                 185                 190

Val Lys Ala Trp Glu Tyr Lys Pro Ser Asp His Leu Ile His Val Leu
        195                 200                 205

Pro Leu His His Val His Gly Ile Ile Asn Gly Leu Ala Ala Ser Phe
    210                 215                 220

Leu Ser Gly Ala Thr Val Glu Met His Pro Lys Phe Asp Pro Lys Val
225                 230                 235                 240

Ile Trp Gly Arg Trp Gln Asp His Ser Ser Ser Thr Leu Phe Met Ala
                245                 250                 255

Val Pro Thr Ile Tyr Ser Arg Leu Asn Asp Tyr Phe Asp Ala His Ile
            260                 265                 270

Arg Gly Thr Glu Gln Glu Asp Ala Ala Arg Ala Gly Ala Arg Ala Leu
        275                 280                 285

Arg Leu Val Val Ser Gly Ser Ala Ala Leu Pro Thr Pro Ile Lys Glu
    290                 295                 300

Lys Phe Ala Glu Ile Thr Gly Gln Val Leu Leu Glu Arg Tyr Gly Met
305                 310                 315                 320

Thr Glu Ile Gly Met Ala Leu Ser Cys Gly Leu Glu Val Gln Lys Arg
                325                 330                 335

Ile Asp Gly Ser Val Gly Trp Pro Leu Pro Gly Val Glu Val Arg Leu
            340                 345                 350

Thr Asp Lys Glu Thr Gly Arg Ile Val Asp Gly Val Asp Glu Asp Gly
        355                 360                 365

Met Ile Glu Val Lys Gly Gly Asn Val Phe Arg Glu Tyr Trp Arg Lys
    370                 375                 380

Pro Glu Ala Thr Ala Ser Glu Phe Thr Ala Asp Gly Trp Phe Lys Thr
385                 390                 395                 400

Gly Asp Val Ala Lys Arg Asp Pro Ser Gly Ala Tyr Phe Ile Gln Gly
                405                 410                 415

Arg Ala Ser Val Asp Leu Ile Lys Ser Gly Gly Tyr Lys Ile Ser Ala
            420                 425                 430

Leu Glu Val Glu Arg Lys Met Leu Ala Ile His Ala Ile Gln Glu Val
        435                 440                 445

Ala Val Val Gly Leu Thr Asp Gln Glu Trp Gly Gln Arg Val Ala Ala
    450                 455                 460

Val Val Lys Phe Arg Glu Gly Thr Ala Pro Met Glu Leu Pro Thr Leu
465                 470                 475                 480

Arg Ala Glu Leu Lys Asn Glu Met Ala Pro Tyr Lys Ile Pro Thr Val
                485                 490                 495

Leu Lys Val Val Asp Gly Ile Glu Arg Asn Ala Met Gly Lys Val Asn
            500                 505                 510

Lys Lys Val Ile Val Gln Lys Tyr Trp Pro Asp Lys Ala
        515                 520                 525

<210>  82
<211>  300
<212>  PRT
<213>  Penicillium chrysogenum

<400>  82

Met Ile Phe Glu Pro Ala Glu Arg Val Leu Leu Pro Thr Lys Asp Leu
1               5                   10                  15

Leu Ser Tyr Ile Phe Asp Asp Pro Pro Tyr Asp Gln Asp Gln Pro Ile
            20                  25                  30

Tyr Ile Asp Val Asn Asn Pro Ser Arg Ser Ile Ser Cys Asn Gln Ala

        35                  40                  45

Lys Arg Leu Val Arg Gln Leu Ile Ala Gly Leu Arg Glu Ser Gly Leu
    50                  55                  60

Gln Lys Gly Asp Cys Val Leu Ile His Ser Phe Asn Asp Ile Asn Tyr
65                  70                  75                  80

Ser Ile Leu Val Leu Ala Ile Ile Gly Ala Gly Gly Cys Phe Thr Gly
                85                  90                  95

Ser Asn Pro Ser Tyr Thr Pro His Glu Leu Ala His His Ile Lys Ala
            100                 105                 110

Ser Glu Ser Arg Phe Leu Phe Ser Glu Pro Glu Ile Phe Asp Ser Leu
        115                 120                 125

Met Arg Ala Thr Glu Gly Lys Asn Ile Pro Lys Gln Asn Ile Trp Ile
    130                 135                 140

Phe Asp Asn Leu Gly Gln Ser Ile Pro Ala Gly Met Arg Ser Trp Lys
145                 150                 155                 160

Gln Leu Leu Glu Val Gly Glu Glu Asp Trp Val Arg Phe Asn Asp Leu
                165                 170                 175

Glu Thr Cys Gln Gln Thr Thr Ala Ala Arg Leu Phe Ser Ser Gly Thr
            180                 185                 190

Thr Gly Leu Pro Lys Ala Val Thr Ile Thr His His Asn Leu Ile Gly
        195                 200                 205

Gln His Glu Leu Val His Gly Val Asn Pro Arg Ser Tyr Pro Ile Ser
    210                 215                 220

Arg Val Ile Ala Val Pro Ile Phe His Ala Ser Ala Ala Pro Val Ser
225                 230                 235                 240

His Ile Ser Thr Leu Lys Ala Gly Ser Val Ala Tyr Met Met Arg Arg
                245                 250                 255

Phe Asp Leu Glu Thr Tyr Leu Thr Thr Val Glu Lys Tyr Asn Val Thr
            260                 265                 270

Asp Leu Ala Val Val Pro Pro Ile Val Ile Ala Ile Leu Met Ser Pro
        275                 280                 285

Leu Ser Arg Asn Lys Pro Tyr Leu Arg Lys Ala Arg
    290                 295                 300

<210>  83
<211>  502
<212>  PRT
<213>  Penicillium chrysogenum

<400>  83

Met Val Tyr Thr Ala Glu Pro Leu Glu Tyr Pro Glu Gly Thr Thr Leu
1               5                   10                  15

Pro Glu Leu Leu Leu Glu Arg Asn Val Asn Asn Val Pro Pro Asp Met
            20                  25                  30

Pro Ala Ile Ile Asp Gly Val Ser Gly Ala Thr Val Tyr Asn Tyr Ser
        35                  40                  45

Ser Phe Arg Ala Thr Val Arg Arg Val Ala Asn Tyr Leu Cys Gln Asp
    50                  55                  60

Ile Asn Leu Pro Arg Gly Ala Val Val Gly Ile Leu Ala Ala Asn Asn
65                  70                  75                  80

Gly Leu Ser Arg Ala Lys Leu Asp Tyr Ser Pro Asp Leu Tyr Val Leu
                85                  90                  95

Ser Pro Asp Ser Ser His Pro Ala Pro Trp Ile His Phe Asp Leu Gly
            100                 105                 110

His Ile Ile Ala Thr Gly Ala Glu Lys Ser Asp Thr Val Glu Pro Arg
        115                 120                 125

Ser Asn Thr Thr Arg Ser Asp Leu Ala Phe Leu Cys Phe Ser Ser Gly
    130                 135                 140

Thr Thr Gly Pro Met Lys Gly Val Tyr Leu Thr His Glu Asn Ile Ile
145                 150                 155                 160

Thr Asn Ile Phe Gln His Arg Gln Arg Leu Pro Glu Met Phe Gln Asn
                165                 170                 175

His Gln Thr Val Ala Ala Leu Ile Thr Pro Phe Phe His Ile Leu Gly
180 185 . 190

Leu Gly Val Phe Val Cys Gln Tyr Ile Cys Gln Val Arg Val Leu Arg
195 200 205

Val Pro Val Ser Leu Tyr His Thr Asn Pro Gly Asn Asn Phe Arg Gly
210 215 220

Ile Pro Ile Leu Pro Ile Ser Thr Leu Tyr Pro Pro Ile Ala Leu Arg
225 230 235 240

Leu Leu Gln Ala Thr Thr Thr Ser Thr Thr Asp Phe Ser Ser Leu Arg
245 250 255

Gly Leu Ile Asn Ala Ala Ala Pro Leu Lys Glu Ile Val Ser Ser Glu
260 265 270

Leu Ser Arg Arg Met Gly Cys Asn Ile Thr Gln Trp Tyr Gly Met Thr
275 280 285

Glu Ala Ser Pro Ser Val Ile Ser Gln Arg Glu Asp Glu Val Glu Ile
290 295 300

Thr Gly Thr Val Gly Arg Leu Leu Pro Gly Met Ser Met Lys Ile Val
305 310 315 320

Asp Ser Glu Gly Glu Val Val Tyr Pro Asn Arg Glu Ile Glu Cys Glu
325 330 335

Pro Asn Lys Pro Gly Glu Leu Leu Ile Gln Gly Ser Asn Leu Met Ser
340 345 350

Gly Tyr Val Gln Asn Thr Glu Ser Asn Asp Ala Phe Ile Asp Gly Tyr
355 360 365

Phe Lys Thr Gly Asp Ile Gly Tyr Val Asn Glu Ala Gly Tyr Val Phe
370 375 380

Leu Val Gly Arg Ser Lys Glu Leu Ile Lys Val Lys Gly His Gln Val

385                 390                 395                 400

Ala Pro Ala Asp Leu Glu Ser Ile Leu Leu Ser His Pro Lys Val Cys
                405                 410                 415

Asp Ala Ala Val Lys Gly Val Tyr Phe Pro Glu Gln Glu Thr Glu Tyr
            420                 425                 430

Pro Ala Ala Tyr Ile Thr Ile Asp Ser Ala Gln Thr Glu Pro Gly Gln
        435                 440                 445

Leu Gln Ala Glu Val Glu Ala Phe Val Asn Asn Gln Val Ala Lys Tyr
    450                 455                 460

Lys Trp Leu Arg Ser Gly Val His Ile Ile Pro Ala Ile Pro Arg Asn
465                 470                 475                 480

Ala Ser Gly Lys Ile Leu Arg His Gln Leu Pro Asp Leu Lys Thr Ser
                485                 490                 495

Gln Arg Gln Thr Lys Ile
            500

<210>  84
<211>  1619
<212>  PRT
<213>  Penicillium chrysogenum

<400>  84

Met Ala Asp Leu Ser Arg Ser Ser Leu Ile Glu Lys Leu Leu Tyr Leu
1               5                   10                  15

Arg Ser Glu Ala Ser His Pro Asp Ser Phe His Gln Leu Ser Ala Leu
            20                  25                  30

Leu Ser Ser His Thr Gly Pro Trp Pro Ile Arg His Leu Ile Ser Thr
        35                  40                  45

Phe Gln Ala Ile Tyr Lys Ser Ile Pro Glu Ser Leu Lys Ala Asn Asn
    50                  55                  60

Asn Glu Ser Ile Thr Arg Leu Cys Ser Gln Val Lys Ala Ala Thr Thr
65                  70                  75                  80

Ser Val Ala Glu Leu Ile Gly Thr Asp Ser Arg Ala Gly Leu Ile Asp
                85                  90                  95

Ala Asn Thr Gly Arg His Leu Thr His Asn Ala Ile Arg Gln Phe Leu
            100                 105                 110

Glu Asn Phe Lys Ile Pro Val Gly Leu Ser Arg His Gly Lys Pro Arg
        115                 120                 125

Ile Ala Val Ile Leu Pro Asn Gly Pro Leu Met Ala Val Ala Val Leu
    130                 135                 140

Ala Phe Val Asn Arg Tyr Thr Ile Val Pro Met Thr Thr Asn Thr Val
145                 150                 155                 160

Ala Glu Gln Leu Gln Thr Asp Ile Glu Asn Ser Gln Ala Asp Ala Val
                165                 170                 175

Val Ala Leu Asp Ala Asp Ile Gly Lys Leu Gln Leu Asp Asn Gly Thr
            180                 185                 190

Arg Pro Val Phe Gly Ile Glu Gln Leu Glu Asp Leu Thr Phe Arg Val
        195                 200                 205

Val Ser Ala Gly Asn Thr Ser Thr Ala Tyr Asp His Pro Pro Asn Ser
    210                 215                 220

Gly Asp Asp Ile Ala Ile Ile Leu Phe Thr Ser Gly Thr Ser Gly Thr
225                 230                 235                 240

Lys Lys Leu Val Pro Ile Thr Thr Tyr Asn Leu Ile Ala Gly Thr Ile
                245                 250                 255

Ala Thr Ile Glu Ser Val Glu Leu Ser Glu Thr Asp Thr Cys Leu Asn
            260                 265                 270

Met Met Pro Leu Asn His Val Gly Gly Ile Met Arg Ser Ile Phe Ser
        275                 280                 285

Pro Ile Leu Ala Gly Gly Ala Thr Ile Cys Cys Pro Ser Phe Asp Pro
    290                 295                 300

Ser Met Phe Trp Asp Ala Val Gln Ala Pro His Thr Lys Pro Thr Trp
305                 310                 315                 320

Tyr Tyr Ala Thr Pro Thr Met His Gln Met Ile Leu Ala Glu Ala Glu
                325                 330                 335

His Arg Pro Asp Ala Val Lys Gln Ser Ala Ile Gln Phe Ile Cys Asn
            340                 345                 350

Ala Gly Gly Gly Leu Pro Pro Thr Leu Ala Val Gln Leu His Ser Thr
        355                 360                 365

Phe His Cys Val Val Leu Pro Ser Tyr Gly Met Thr Glu Cys Met Pro
    370                 375                 380

Ile Ala Ala Pro Pro Arg Asp Tyr Lys Leu Asp Arg Pro Gly Thr Ser
385                 390                 395                 400

Gly Arg Ile Val Gly Pro Glu Val Ala Ile Leu Thr Glu Ser Gly Asn
                405                 410                 415

Pro Val Thr Gln Asn Gly Met Leu Gly His Ile Cys Ile Arg Gly Ser
            420                 425                 430

Pro Ala Phe Glu Gly Tyr Leu Thr Pro Gly Gly Lys Ile Asp Thr Ser
        435                 440                 445

Ala Phe Asn Glu Ser Gly Trp Phe Asp Thr Gly Asp Leu Gly His Leu
    450                 455                 460

Asp Asp Asp Asn Tyr Leu Tyr Ile Thr Gly Arg Ser Lys Glu Val Ile
465                 470                 475                 480

Asn Arg Gly Gly Glu Ile Ile Ser Pro Val Glu Val Glu Asn Ala Val
                485                 490                 495

Leu Thr Ser Ala Lys Asp Pro Glu Ser Pro Leu Tyr Gly Arg Val Thr
            500                 505                 510

Glu Thr Leu Ala Phe Ser Val Pro Asp Glu Val Leu Gln Glu Val Val
        515                 520                 525

Gly Val Val Ile Val Thr Pro Pro Gly Phe Thr Arg Pro Asp Leu Arg

530                 535                 540

Gln Ile His Glu Ala Leu Gln Pro Ile Ile His Gln Pro Lys Trp Pro
545                 550                 555                 560

Ala Leu Val Val Tyr Met Asp Gly Val Pro Lys Ala Asn Asn Lys Ile
                565                 570                 575

Gln Arg Ile Lys Leu Ala Asp Arg Leu Ser Leu Asp Thr Leu Thr Thr
            580                 585                 590

Thr Thr Pro Leu Ala Ser Arg His Tyr Glu Ala Val Cys Pro Pro Thr
        595                 600                 605

Gly Ala Pro Leu Ser Ala Leu Ile Pro Lys Lys Pro Cys Val Ile Asp
    610                 615                 620

Glu Lys Met Ile Arg Ser Val Leu Ser Lys Lys Ala Asn Thr Pro Asp
625                 630                 635                 640

Val His Val Gln Ile Asn Pro Arg Asp Gly Leu Ala Gln Val Val Leu
                645                 650                 655

Phe Val Glu Asn Leu Asp Asp Asp His Val Thr Pro Ala Glu Leu His
            660                 665                 670

Asp Gln Leu Asp Gly Tyr Leu Val Pro Ser Arg Ile Ile Pro Leu Lys
        675                 680                 685

Gly Pro Met Pro Val Asp Phe Tyr Gly Lys Pro Asp Gln Ala Ala Ile
    690                 695                 700

Asn Glu Ala Ile His Ala Arg Asn Ser Asp Gly Asp Leu Ser Pro Ile
705                 710                 715                 720

Gln Arg Arg Val Arg Gly Ile Phe Ala Val Thr Leu Ser Cys Ser Pro
                725                 730                 735

Glu Glu Val Ser Ser Gly Thr Asp Phe Phe Ala Ala Gly Gly Asp Ser
            740                 745                 750

Leu Ser Ala Gly Arg Met Val Ser Gln Leu Arg Arg Glu Phe Gly Ile
        755                 760                 765

Phe Leu Ala Gly Asp Ile Leu Phe His His Ser Thr Val Gly Ala Ile
    770                 775                 780

Glu Gln Lys Ile Ile Glu Ala Val Asp Val Lys Ala Ala Lys Gly Glu
785                 790                 795                 800

Asp Gly Gly Val Glu Leu Pro Gly Cys Glu Lys Thr Tyr Ser Ser Thr
                805                 810                 815

Asn Pro Ile Ile Leu Val Leu His Leu Phe Pro Thr Val Phe Phe Phe
            820                 825                 830

Pro Met Lys Arg Ala Phe Gln Trp Met Val Phe Ala Tyr Val Val Ala
        835                 840                 845

Glu Cys Ser Asn Arg Phe Pro Ile Arg Glu Asn Leu Ile Ala Arg Leu
    850                 855                 860

Met Leu Val Val Phe Ala Val Met Ser Ala Arg Leu Cys Ser Gln Ile
865                 870                 875                 880

Val Ser Pro Ile Leu Ala Ile Ile Phe Lys Trp Leu Val Ile Gly Arg
                885                 890                 895

Tyr Lys Glu Gly Met Ser Pro Met Trp Gly Pro Tyr His Thr Arg Trp
            900                 905                 910

Trp Leu Thr Gln Lys Ala Thr Gln Val Cys Gly Lys Gly Leu Phe Asn
        915                 920                 925

Asn Tyr Asn Trp Ser Arg Ile Leu Phe Tyr Arg Leu Met Gly Ala Lys
    930                 935                 940

Ile Gly Lys Asn Val Thr Val Ser Ala Ser Ala Lys Leu Gly Glu Tyr
945                 950                 955                 960

Asp Leu Ile Glu Ile Gly Asp Asn Val Val Leu Asp Thr Cys Val Cys
                965                 970                 975

Arg Pro Phe Ala Val Glu Arg Asn Thr Ser Met Leu Leu Lys Arg Ile
            980                 985                 990

Arg Ile Gly Lys Asp Ser Ser Val Gly Ile Lys Ser Ile Val Ala Pro
        995                 1000                1005

Gly Ala Asp Ile Pro Glu Arg Thr Cys Ile Gly Pro Asn Ser Ser
    1010                1015                1020

Ser Trp Glu Leu Gln Asp Ala Asp Glu Ser Asn Arg Gln Leu Leu
    1025                1030                1035

Thr Ser Gln Ile Pro Lys Pro His Trp Leu Trp Ile Leu Phe Ile
    1040                1045                1050

Val Glu Pro Ile Lys Leu Val Thr Trp Thr Ala Thr Arg Ile Thr
    1055                1060                1065

Trp Met Ala Gly Leu Ile Pro Met Val Leu Gln Phe Pro Thr Pro
    1070                1075                1080

Ala Ala Asp Met Phe Arg Ser Thr Leu Asp Trp Tyr Thr Ser Asp
    1085                1090                1095

His Arg Ile Ala Tyr His Ile Thr Ala Arg Ile Cys Arg Ala Val
    1100                1105                1110

Gly Gly Pro Ile Val Leu Phe Ile Ala Val Leu Val Ile Lys Phe
    1115                1120                1125

Phe Leu Asp Leu Ile Cys Gly Lys Pro Lys Pro Gly Pro Ala Ser
    1130                1135                1140

Lys Gln Thr Thr Arg Gln Lys Ile Arg Ser Ala Val Leu Ala Gln
    1145                1150                1155

Ile Leu Pro Ala Gly Asp Ile His Glu Leu Thr Arg Leu Thr Gly
    1160                1165                1170

Arg His Tyr Glu Phe Val Ser Met Ala Val Arg Ala Leu Gly Gly
    1175                1180                1185

Lys Val Gly Lys His Val Tyr Trp Pro Ser Val Gly Pro Val Thr
    1190                1195                1200

Val Asp Phe Asp Leu Ile Glu Val Gly Asn Asp Val Val Phe Gly
    1205                1210                1215

Ser Arg Ser Thr Leu Val Thr Ser Asp Gly Tyr Gly Arg Asp Arg
    1220                1225                1230

Ile Val Ile Gly Asp Gly Thr Met Val Gly Asp Arg Val Val Ala
    1235                1240                1245

Leu Pro Gly Ala Thr Ile Gly Arg Gln Ala Met Ile Gly Ser Gly
    1250                1255                1260

Ala Leu Leu Arg Arg Asn Gly Glu Tyr Pro Ser Asn Thr Val Trp
    1265                1270                1275

Thr Gly Ser Lys Gly Gly Glu Ala Ile Gln Phe Pro Ser Ser Thr
    1280                1285                1290

Ser Thr Thr Thr Ser Thr Ala Pro Thr Ile Ile Gly Asp Gly Ser
    1295                1300                1305

Ser Ser Pro Asn Ser Ser Gly Ser Glu Asp Glu Arg Ser Pro Thr
    1310                1315                1320

Glu Lys Met Pro Thr Glu Lys Gln Thr Tyr Tyr Gly Ser Lys Asp
    1325                1330                1335

Lys Thr Val Thr Gln Ile Ala Glu Gln Asp Ile Asp Thr Cys Lys
    1340                1345                1350

Pro Phe Gly Arg Ala Phe Tyr Arg His Glu Ala Asn Tyr Tyr Val
    1355                1360                1365

Leu Arg Met Trp Gln Ile Val Ile Tyr Ser Thr Phe Ala Val Ile
    1370                1375                1380

Val Thr Thr Val Tyr Trp Leu Leu Thr Val Leu Phe Ser Leu Phe
    1385                1390                1395

Ala Leu Arg Thr Val Leu Gln Tyr Ser Asp Ala Ala Gly Phe Lys
    1400                1405                1410

Gln Gly Ala Trp Arg Pro Phe Val Leu Tyr Gly Thr Leu Ala Ser

1415                    1420                    1425

Ile Leu Ser Val Ile Thr Val Ala Gln Val Val Leu Ala Phe Ala
    1430                    1435                    1440

Ile Ile Leu Cys Ile Lys Trp Ala Val Val Gly Arg Arg Lys Glu
    1445                    1450                    1455

Gly Ser Tyr His Trp Asp Lys Ser Ser Tyr Asn Gln Arg Trp Gln
    1460                    1465                    1470

Phe Leu Leu Ser Cys Glu Thr Leu Ile Lys Asp Cys Tyr Asp Gly
    1475                    1480                    1485

Val Gly Leu Leu Pro Met Ile Ser Gly Ser Ala Tyr Ile Ser Trp
    1490                    1495                    1500

Tyr Tyr Arg Leu Leu Gly Ala Lys Ile Gly Lys Asp Cys Ala Ile
    1505                    1510                    1515

His Ala Asn Gly Ser Pro Ser Ile Phe Phe Thr Glu Pro Asp Leu
    1520                    1525                    1530

Leu Thr Leu Gly Asp Arg Val Ala Val Asp Asp Ala Ser Leu Val
    1535                    1540                    1545

Cys His Leu Asn Ser Arg Gly Gly Phe Glu Leu His Thr Leu Lys
    1550                    1555                    1560

Val Gly Asp Arg Ser Ile Leu Arg Ala Gly Ser Arg Leu Met Ser
    1565                    1570                    1575

Gly Ala Ser Met Gly Gln Asp Ala Cys Leu Leu Glu His Thr Leu
    1580                    1585                    1590

Val Leu Ser Gly Asp His Val Glu Asp Gly Asp Thr Leu Gln Gly
    1595                    1600                    1605

Trp Pro Ala Glu Gly Phe Glu Trp Lys Arg Val
    1610                    1615

<210>  85
<211>  634

<212>  PRT
<213>  Penicillium chrysogenum

<400>  85

Met Asp Ile Leu Ser Glu Val Gly Ala Pro Ala Val Ala Met Ala Ser
1               5                   10                  15

Ala Leu Ser Val Ala Ala Gly Ala Tyr Leu Asn Ala Lys Leu Ala Ile
            20                  25                  30

Ala Thr Asp Leu Arg Thr Ile His Ser Asp Lys Ala Ala Ala Lys Arg
        35                  40                  45

Leu Ser Glu Arg Ile Ala Lys Leu Asp Gly Ser Thr Thr Ile Tyr Lys
    50                  55                  60

Met Leu Glu Arg Ala Val Glu Val Glu Gly Arg Ala Thr Thr Asp Ala
65                  70                  75                  80

Leu Trp Phe Glu Gln Lys Thr Trp Ser Tyr Gly Gln Leu Lys Asp Leu
                85                  90                  95

Val Asp Arg Met Ala Ala Leu Leu Lys Ser Arg Asp Ile Asn Pro Gly
            100                 105                 110

Asp Thr Val Gly Val Phe Thr Thr Asn Ser Pro Glu Met Val Met Thr
        115                 120                 125

Val Tyr Ala Leu Ser Lys Leu Gly Ala Val Ala Ala Met Ile Asn Thr
    130                 135                 140

Asn Leu Arg Asp Asp Thr Phe Thr His Cys Val Asn Val Ser Gly Ser
145                 150                 155                 160

Lys Leu Ile Ile Ser Thr Ala Asp Leu Cys Gln His Val Cys Val Asp
                165                 170                 175

Leu Pro His Phe Thr Leu Ser Leu Gly Ser Phe Glu Gly Ala Glu Ala
            180                 185                 190

Gly Ala Ile Glu Pro Ile Thr Ser Gly Thr Leu Gln Gln Phe Ser Pro
        195                 200                 205

Leu Gly Leu Ala Ala Ala Lys Arg Ser Pro Lys Asp Leu Ser Leu Leu
    210                 215                 220

Ile Tyr Thr Ser Gly Thr Thr Gly Lys Pro Lys Ala Cys Ala Ile Arg
225                 230                 235                 240

Asn Met Leu Thr Leu Ile Thr Ser Asn Pro His Ser Ala Asp Val Ser
                245                 250                 255

Asn His Ser Lys Tyr His Pro Phe Arg Val Tyr Ser Pro Leu Pro Leu
            260                 265                 270

Phe His Gly Thr Ala Phe Phe Thr Gly Leu Cys Ala Ala Ile Gly Asn
        275                 280                 285

Gly Gly Thr Leu Cys Leu Gly Arg Lys Phe Ser Ala Ser Lys Phe Trp
    290                 295                 300

Lys Glu Val His Asp Ser Gly Ala Thr Arg Ile Leu Tyr Ile Gly Glu
305                 310                 315                 320

Leu Cys Arg Tyr Leu Leu Ala Thr Pro Pro Ser Pro Tyr Asp Gln Asp
                325                 330                 335

His Lys Cys Ile Val Ala Ser Gly Asn Gly Leu Arg Gly Glu Ile Trp
            340                 345                 350

Glu Lys Phe Arg Glu Arg Phe Asn Val Pro Glu Ile Arg Glu Phe Tyr
        355                 360                 365

Arg Ser Thr Glu Gly Val Ala Lys Phe Asp Asn His Gly Val Gly Ala
    370                 375                 380

Trp Gly Ala Gly Lys Val Gly Phe Ser Gly Pro Ile Arg Arg Phe Leu
385                 390                 395                 400

Glu Asp Asp Thr Phe Ile Val Lys Tyr Asp Thr Asn Thr Glu Met Pro
                405                 410                 415

Tyr Arg Asp Pro Ala Thr Gly Phe Cys Val Arg Ala Ala Leu Gly Gln
            420                 425                 430

Glu Gly Glu Ala Ile Gly Arg Val Arg Asp Arg Gly Met Leu Ile Glu

435                 440                 445

Tyr Leu Gly Asn Glu Gly Ala Thr Glu Glu Lys Leu Leu Arg Asp Val
    450                 455                 460

Phe Gln Lys Gly Asp Leu Phe Gln Arg Thr Gly Asp Leu Val Val Gln
465                 470                 475                 480

Asp Glu Ser Gly Trp Val Arg Phe Gln Asp Arg Val Gly Asp Thr Phe
                485                 490                 495

Arg Trp Lys Gly Glu Asn Val Ser Ala Gly Glu Ile Arg Asp His Ile
            500                 505                 510

Cys Arg Ile Glu Gly Val His Asp Ala Val Val Tyr Gly Val Lys Leu
        515                 520                 525

Ser Gly Tyr Asp Gly Gln Ala Gly Ala Ala Gly Ile Thr Leu Glu Ser
    530                 535                 540

Pro Glu Val Glu Thr Glu Leu Met Ser Thr Leu Cys Lys Ala Leu Lys
545                 550                 555                 560

Lys Lys Gly Val Pro Ser Tyr Ala Leu Pro Arg Leu Val Arg Leu Thr
                565                 570                 575

Glu Lys Val Ala Thr Gly Val Thr Phe Lys Gln Ala Lys Gly Asp Leu
            580                 585                 590

Ala Lys Lys Gly Trp Asn Pro Arg Gln Asp Ser Gly Gly Asp Ile Leu
        595                 600                 605

Tyr Trp Leu Asn Gly Thr Lys Tyr Gln Lys Leu Glu Glu Gln Ser Trp
    610                 615                 620

Ala Glu Ile Glu Ser Gly Lys Ala Lys Ile
625                 630

<210>  86
<211>  595
<212>  PRT
<213>  Penicillium chrysogenum

<400>  86

Met Ser Ser Asn Asn Met Leu Pro Gln Glu Ala Tyr Ser Asp Leu Leu
1               5                   10                  15

Ser Phe Thr Phe Asp Gly Pro Lys Pro Tyr Asn Gln Asn Gln Pro Leu
            20                  25                  30

Phe Ile Asp Ala Glu Asp Pro Ser Arg Ser Phe Thr Ala Ala Gln Phe
        35                  40                  45

Arg Gln Leu Val Arg Thr Leu Ile Ala Gly Leu Lys Ala His Asn Val
    50                  55                  60

Gln Pro Gly Asp Cys Val Leu Leu His Leu Gly Asn Ser Ile Leu Tyr
65                  70                  75                  80

Pro Ala Leu Phe Phe Gly Ile Ile Gly Ala Gly Gly Val Tyr Met Gly
                85                  90                  95

Ser Asn Pro Arg Ser His Pro Gln Glu Leu Asp His Val Leu Ser Leu
            100                 105                 110

Ala Glu Pro Lys Leu Ile Leu Thr Thr Arg Asp Ala Leu Pro Ser Val
        115                 120                 125

Leu Asp Ala Ser Ala Gly Arg Gly Ile His Pro Ala Gln Val Cys Leu
    130                 135                 140

Val Asp Glu His Ala Ile Asp His Cys Ala Gln Leu Phe Leu Trp Tyr
145                 150                 155                 160

Glu Leu Gly Tyr Ser Ser Ala Gly Gln Phe Cys Ala Met Asn Gly Asp
                165                 170                 175

Ala Arg His Asn Asn Phe Ala Asn Leu Leu Cys Phe Gly Glu Ser Asp
            180                 185                 190

Trp Leu Lys Phe Ala Asp Pro Val Val Ala Gln Ala Thr Pro Ala Ala
        195                 200                 205

Met Tyr Pro Thr Ser Gly Thr Gly Gly Leu Pro Lys Ala Ala Ile Leu
    210                 215                 220

Ser His Tyr Ala Leu Val Ser Gln His Arg Thr Ile Tyr Tyr Glu Val
225                 230                 235                 240

Pro His Pro Val Ser Arg Leu Ile Ser Leu Pro Met Phe His Leu Phe
                245                 250                 255

Gly Ala Leu Trp Thr His Leu Phe Pro Val Arg Tyr Gly His Pro Leu
            260                 265                 270

Phe Val Met Pro Arg Phe Glu Val Asn Asp Phe Leu Ala Ala Val His
        275                 280                 285

Lys Tyr Gln Ile Ser Glu Thr Tyr Leu Val Pro Ala Ile Ile His Ala
    290                 295                 300

Ile Asn Gln Ser Ser Val Pro Ile Gly Asp Leu Leu Lys Ser Leu Arg
305                 310                 315                 320

Tyr Val Gly Val Ala Gly Ala Pro Ile Asp Gly His Ser Met Gln Gln
                325                 330                 335

Phe Arg Ser His Ile Asn Pro Met Gly Tyr Ala Cys Gln Ile Trp Gly
            340                 345                 350

Met Thr Glu Val Gly Val Thr Phe Gln Thr Arg Trp Gly Gln Gln Gly
        355                 360                 365

Asp Pro Gly Ser Ile Gly Arg Cys Ile Ala Gly Tyr Glu Ala Arg Leu
    370                 375                 380

Val Gln Pro Asp Gly Lys Thr Val Gln Gly Asp Asn Cys Ser Gly Glu
385                 390                 395                 400

Leu Tyr Val Arg Gly Pro Gly Leu Leu Thr Ala Tyr Lys Gly Arg Thr
                405                 410                 415

Asp Ala Leu Glu Pro His Gly Trp Phe Arg Thr Gly Asp Ile Ala Tyr
            420                 425                 430

Val Lys Gln Gly Gln Tyr Tyr Ile Val Gly Arg Thr Lys Glu Leu Ile
        435                 440                 445

Lys Val Arg Gly Trp Gln Val Ala Pro Ala Glu Val Glu Gly Val Leu

450                 455                 460

Leu Gln His Pro Gly Ile Leu Asp Ala Gly Val Ile Gly Val Asn Lys
465                 470                 475                 480

Asp Gly Val Gly Glu Val Pro Arg Ala Phe Val Val Arg Ser Arg Asp
                485                 490                 495

Pro Ser Val Arg Arg Leu Thr Gly Glu Gln Val Tyr Asn Tyr Ser Arg
            500                 505                 510

Gln Gln Leu Ala Arg Tyr Lys Ala Leu Asp Gly Gly Val Val Phe Val
        515                 520                 525

Glu Glu Ile Pro Arg Thr Ala Ser Gly Lys Ile Gln Arg Phe Lys Leu
    530                 535                 540

Ser Gln Met Asn Ser Tyr Arg Glu Met Val Ala Ser Leu Leu Ser Arg
545                 550                 555                 560

Phe Glu Gly Glu Gly Ser Ala Ala Ala Thr Ala Ala Arg Arg Gly Leu
                565                 570                 575

Pro Ala Gly Ala Glu Met Ser Pro Val Ser Leu Ile Pro Glu Gly Arg
            580                 585                 590

Val Ala Val
        595

<210>  87
<211>  300
<212>  PRT
<213>  Penicillium chrysogenum

<400>  87

Met Glu Ala Arg Leu Ala Leu Leu Gln Gly Ser Lys Glu Pro Ala Leu
1               5                   10                  15

Trp Phe Glu Thr Leu Gly Asn Phe Ile Asp Lys Gln Ala Ser Gln Tyr
            20                  25                  30

Glu Asp Arg Val Ala Ala Ile Phe Pro Trp Gln Ser Val Arg Leu Ser
        35                  40                  45

Tyr Arg Gln Leu Ala Glu Arg Ser Lys Ile Leu Ala Lys Ala Met Leu
    50                  55                  60

Glu Met Gly Leu Arg Lys Gly Asp Cys Val Gly Val Met Ala Gly Asn
65                  70                  75                  80

Cys Tyr Gln Tyr Ile Glu Val Phe Leu Gly Gly Gly Arg Ile Gly Cys
                85                  90                  95

Pro Val Val Val Leu Asn Asn Thr Tyr Ile Pro Arg Glu Leu Met Ser
            100                 105                 110

Ala Val Gln Lys Ser Ser Cys Lys Leu Val Phe Val Ala Ser Asp Ile
        115                 120                 125

Gly Ser Arg Ser Leu Ser Ala His Ile Asn Ala Leu Cys Gly Asp Gln
    130                 135                 140

Ser Arg Asn Pro Ala Leu Pro Glu Leu Arg Arg Val Val Asn Phe Gly
145                 150                 155                 160

Asn Lys Asp Pro Ser Ser Thr Gly Val Glu Met Gln Ser Tyr Ser Ala
                165                 170                 175

Phe Thr Ser Gly Ala Gln Ser Val Phe Met Lys Asp Ser Met Leu Leu
            180                 185                 190

Arg Ala Glu Lys Ser Val Glu Pro Glu Asp Val Leu Asn Leu Gln Phe
        195                 200                 205

Thr Ser Gly Thr Thr Gly Ser Pro Lys Ala Ala Met Leu Ser His Ile
    210                 215                 220

Asn Leu Leu Asn Asn Ala Arg Phe Val Gly Asp Ala Met His Leu Thr
225                 230                 235                 240

Pro Thr Asp Ile Ile Cys Cys Pro Pro Pro Leu Phe His Cys Phe Gly
                245                 250                 255

Leu Val Leu Gly Phe Leu Ala Ser Phe Val His Gly Ser Ser Ile Val
            260                 265                 270

Phe Pro Ser Asp Phe Phe Asp Val Arg Lys Phe Val Ser Thr Ile Leu
        275                 280                 285

Ser Glu Asn Ala Thr Val Leu Leu Gly Val Pro Thr
    290                 295                 300

<210>  88
<211>  452
<212>  PRT
<213>  Penicillium chrysogenum

<400>  88

Met Ala Thr Arg Leu Val Pro Lys Gly Cys Pro Leu Val Arg Thr Thr
1               5                   10                  15

Ile Gln Val Leu Glu His Arg Lys His Arg Ile His Phe Ala Ser Tyr
            20                  25                  30

Leu Gly Asn His Ala Gln Phe Arg Arg Ser His Ala Leu Ala Arg Ala
        35                  40                  45

Asn Gly Pro Leu Leu Gly Ile Arg Ile Leu Asp Leu Thr Arg Val Leu
    50                  55                  60

Ala Gly Pro Phe Cys Thr Gln Ile Leu Ala Asn Tyr Gly Ala Glu Val
65                  70                  75                  80

Leu Lys Val Glu His Pro Lys Gly Gly Asp Asp Thr Arg Leu Trp Arg
                85                  90                  95

Thr Ala Ala Glu Lys His Val Trp Lys Ser Thr Gly Lys Asp Met Ser
            100                 105                 110

Thr Tyr Phe Cys Ala Ile Asn Arg Asn Lys Leu Ser Met Thr Leu Asn
        115                 120                 125

Leu Lys Gln Glu Lys Gly Arg Glu Ile Leu Phe Arg Leu Val Lys Glu
    130                 135                 140

Ala Asp Val Val Val Glu Asn Phe Val Pro Gly Lys Met Asp Glu Met
145                 150                 155                 160

Gly Ile Gly Tyr Glu Lys Leu Arg Glu Ile Asn Pro Ser Ile Ile Tyr
                165                 170                 175

Ala Ser Val Ser Gly Tyr Gly Ala Ser Gly Pro Tyr Ser His Arg Ala
            180                 185                 190

Gly Tyr Asp Ala Ile Ala Ala Ala Glu Ala Gly Met Leu Tyr Ile Thr
        195                 200                 205

Gly Glu Arg Asn Gly Pro Pro Thr Arg Pro Gly Leu Gly Leu Thr Asp
    210                 215                 220

Met Ser Thr Gly Leu Tyr Leu His Gly Ala Ile Leu Ala Ala Leu Tyr
225                 230                 235                 240

Ala Arg Arg Asp Thr Gly Arg Gly Gln Lys Ile Asp Thr Ser Leu Phe
                245                 250                 255

Glu Ser Gln Val Ser Leu Leu Ser Asn Val Ala Met Ser Trp Leu Asn
            260                 265                 270

Gly Gly Glu His Ala Lys Arg Trp Gly Thr Glu His Pro Ser Ile Val
        275                 280                 285

Pro Tyr Gln Ala Phe Lys Thr Lys Asp Gly His Leu Val Leu Gly Ala
    290                 295                 300

Thr Asn Asn Arg Gln Phe Gln Thr Leu Cys Arg Leu Val Lys Leu Ser
305                 310                 315                 320

Glu Leu Ala Thr Asp Pro Arg Phe Val Asp Asn Ser Ser Arg Val Gln
                325                 330                 335

Asn Arg Ala Glu Leu Lys Glu Ile Leu Glu Pro Ile Ile Arg Arg Lys
            340                 345                 350

Pro Thr Lys Val Trp Leu Ser Asp Leu Asp Gly Ser Gly Leu Pro Tyr
        355                 360                 365

Gly Pro Val Asn Thr Ile Glu Glu Val Phe Ser His Pro Gln Thr Ala
    370                 375                 380

Ala Arg Asp Met Val His Ser Leu Pro His Glu Ala Ser Glu Ser Glu
385                 390                 395                 400

Glu Ile Arg Leu Leu Gly Thr Pro Val Lys Phe Ser Gly Thr Lys Pro
                405                 410                 415

Gln Ile Arg Arg Ser Pro Pro Ser Leu Gly Glu His Thr Asn Phe Thr
            420                 425                 430

Leu Glu Arg Met Gly Phe Ser Ala Lys Glu Ile Glu Val Met Arg Glu
        435                 440                 445

Gln Gly Ile Val
    450

<210>  89
<211>  446
<212>  PRT
<213>  Penicillium chrysogenum

<220>
<221>  misc_feature
<222>  (64)..(64)
<223>  Xaa can be any naturally occurring amino acid

<400>  89

Met Thr Val Ala Leu Arg Phe Gln Val Ala Ala Arg Arg Ser Gly Leu
1               5                   10                  15

Ser Trp Gly Ser Asn Ala Leu Arg Arg Thr Ile Pro Gly Arg Ser Tyr
            20                  25                  30

Ser Ser Ala Val Ala Ala Asp Gly Ser Leu Pro Leu Ala Gly Ile Arg
        35                  40                  45

Val Leu Asp Met Thr Arg Val Leu Ala Gly Pro Tyr Cys Thr Gln Xaa
    50                  55                  60

Leu Gly Asp Leu Gly Ala Asp Val Ile Lys Val Glu His Pro Val Arg
65                  70                  75                  80

Gly Asp Asp Thr Arg Ala Trp Gly Pro Pro Tyr Ala Lys Tyr Glu Asp
                85                  90                  95

Ala Ser Arg Lys Gly Pro Gly Glu Ser Ala Tyr Tyr Leu Ala Val Asn
            100                 105                 110

Arg Asn Lys Lys Ser Leu Gly Leu Ser Phe Gln His Lys Ser Gly Val
        115                 120                 125

Glu Ile Leu His Lys Leu Ala Lys Glu Cys Asp Val Leu Val Glu Asn
    130                 135                 140

Tyr Leu Pro Gly Gly Leu Lys Lys Tyr Gly Met Asp Tyr Glu Thr Leu
145                 150                 155                 160

Arg Glu Ile Asn Pro Lys Leu Ile Tyr Ala Ser Ile Thr Gly Tyr Gly
                165                 170                 175

Gln Thr Gly Pro Tyr Ser Asn Arg Ala Gly Tyr Asp Val Met Val Glu
            180                 185                 190

Ala Glu Met Gly Leu Met His Ile Thr Gly Ala Arg Asp Gly Ala Pro
        195                 200                 205

Val Lys Val Gly Val Ala Val Thr Asp Leu Thr Thr Gly Leu Tyr Thr
    210                 215                 220

Ser Asn Ala Ile Met Ala Ala Leu Ile Ala Arg Gly Arg Thr Gly Arg
225                 230                 235                 240

Gly Gln His Ile Asp Ala Cys Leu Ser Asp Cys Gln Val Ala Thr Leu
                245                 250                 255

Ser Asn Leu Ala Ser Ser Ala Leu Ile Ser Gly Gln Lys Asp Ser Gly
            260                 265                 270

Arg Trp Gly Thr Ala His Pro Ser Ile Val Pro Tyr Arg Ser Tyr Lys
        275                 280                 285

Thr Ser Asp Gly Asp Ile Leu Phe Gly Gly Gly Asn Asp Lys Leu Phe
    290                 295                 300

Gly Val Leu Cys Asp Arg Leu Gly Phe Ser Glu Trp Lys Thr Asp Ala
305                 310                 315                 320

Arg Phe Val Thr Asn Ser Asp Arg Val Gln His Arg Ala Glu Ile Asp
                325                 330                 335

Gly Met Ile Glu Ser Thr Thr Met Gln Lys Thr Thr Gln Glu Trp Leu
            340                 345                 350

Asp Ile Phe Glu Gly Ser Gly Met Pro Tyr Ala Ala Val Asn Asp Ile
        355                 360                 365

Gln Gly Thr Leu Asn His Lys His Val Leu Ala Arg Asp Met Val Thr
    370                 375                 380

Glu Val Glu His Pro Ala Cys Gly Pro Ile Lys Leu Val Asn Thr Pro
385                 390                 395                 400

Ile Lys Tyr Ser Glu Ala Thr Pro Gly Val Arg Thr Pro Pro Pro Thr
                405                 410                 415

Leu Gly Gln His Thr Asp Glu Ile Leu Gly Ser Val Leu Asp Tyr Gly
            420                 425                 430

Glu Ala Asp Ile Ala Arg Leu Lys Asp Glu Gly Val Val Ser
        435                 440                 445

<210>  90
<211>  377
<212>  PRT
<213>  Penicillium chrysogenum

<400>  90

Met Ser Pro Leu Lys Gly Ile Lys Val Ile Glu Leu Ala Gly Leu Ala
1               5                   10                  15

Pro Gly Pro Phe Thr Gly Leu Leu Leu Ala Asp Asn Gly Ala Ser Val
            20                  25                  30

Leu Arg Ile Asp Arg Pro Lys Ser Ile Ser Gly Asp Gln Leu Thr Arg
        35                  40                  45

Asn Lys Thr Ser Ile Thr Leu Asp Leu Arg Asp Lys Thr Ser Leu Asn
    50                  55                  60

Ile Leu Leu Arg Leu Leu Thr Ala Ala Asp Ile Leu Ile Asp Pro Phe
65                  70                  75                  80

Arg Pro Gly Val Leu Glu Arg Leu Gly Leu Ser Pro Thr Glu Val Leu
                85                  90                  95

Leu Lys His Asn Pro Arg Leu Ile Val Ala Arg Met Thr Gly Phe Arg
            100                 105                 110

Arg Asp Gly Lys Tyr Lys Asp Met Ala Gly His Asp Ile Asn Tyr Ile
        115                 120                 125

Ala Val Ser Gly Val Leu Ser Met Leu Gly Arg Thr Gly Glu Lys Pro
    130                 135                 140

His Ala Pro Gly Asn Ile Leu Gly Asp Phe Ala Gly Gly Gly Ala Val
145                 150                 155                 160

Cys Phe Gln Gly Ile Leu Leu Ala Leu Ile Ser Arg Ser His Thr Gly
                165                 170                 175

Arg Gly Gln Val Val Glu Ala Asn Met Val Asp Gly Ser Ala Tyr Leu
            180                 185                 190

Ala Thr Phe Pro Arg Leu Gly Arg Asn Thr Pro Ala Trp Ser Glu Pro
        195                 200                 205

Arg Gly Glu Asn Leu Leu Asp Gly Gly Cys Pro Tyr Tyr Asp Thr Tyr
    210                 215                 220

Glu Thr Lys Asp Ala Gly Arg Tyr Phe Ala Val Gly Ala Leu Glu Pro
225                 230                 235                 240

Gln Phe Tyr Ala Ala Leu Val Arg Gly Leu Gly Phe Asp Met Gly Gln
                245                 250                 255

Leu Pro Lys Arg Glu Asp Arg Glu Gln Trp Pro Val Leu Arg Glu Ile
            260                 265                 270

Phe Thr Lys Arg Phe Lys Glu Lys Thr Arg Ala Glu Trp Glu Ala Val
        275                 280                 285

Phe Asp Gly Thr Asp Ala Cys Ala Thr Pro Val Leu Glu Gln Ala Glu
    290                 295                 300

Leu Glu Asp Asp Gly Tyr Glu Gln Arg Pro Met Val His Leu Val Asn
305                 310                 315                 320

Thr Pro Ala Ala Pro Ile Pro Ala Asp Gln Gly Gly Trp Ser Gly Gly
                325                 330                 335

Gly Leu Leu Pro Gly Asp Gly Gly Val Asp Thr Leu Arg Glu Trp Val
            340                 345                 350

Gly Trp Glu Gln Gly Lys Asp Phe Glu Val Arg Lys Asp Gly Ala Phe
        355                 360                 365

Val Ala Ile Asp Gly Lys Ser Lys Ile
    370                 375

<210>  91
<211>  518
<212>  PRT
<213>  Penicillium chrysogenum

<400>  91

Met Pro Ser Gln Val Ala Thr Cys Leu Arg Leu Ala Arg Gln Phe Ser
1               5                   10                  15

Ala Asn Pro Val Gln His Gln Arg Phe Met Ala Arg Ala Phe Ser Ser
            20                  25                  30

Ser Val Arg Arg Ser Glu Ile Asn Lys Val Tyr Pro Ser Ala Glu Val
        35                  40                  45

Ala Val Lys Asp Met Lys Ser Asn Ser Thr Leu Leu Ala Gly Gly Phe
    50                  55                  60

Gly Leu Ser Gly Val Pro Asp Ser Leu Ile Asn Ala Val Leu Ala Asn
65                  70                  75                  80

Pro Ser Ile Thr Gly Leu Thr Val Ala Ser Asn Asn Ala Gly Val Asp
                85                  90                  95

Gly Ala Gly Leu Gly Leu Leu Leu Lys Ser Lys Gln Ile Lys Lys Met
            100                 105                 110

Ile Ala Ser Tyr Val Gly Glu Asn Lys Thr Phe Glu Arg Met Tyr Leu
        115                 120                 125

Thr Gly Glu Ile Glu Leu Glu Leu Ser Pro Gln Gly Thr Leu Ala Glu

    130                 135                 140

Arg Cys Arg Ser Gly Gly Ala Gly Ile Pro Ala Phe Tyr Thr Pro Ala
145                 150                 155                 160

Ala Phe Gly Thr Val Val Gln Thr Gly Ala Val Pro Leu Arg His Asn
                165                 170                 175

Ala Asp Gly Thr Val Ala Lys Tyr Ser Glu Ala Arg Asp Thr Lys Val
            180                 185                 190

Phe Asp Gly Lys Ser Tyr Val Met Glu Glu Ala Ile Lys Gly Asp Tyr
        195                 200                 205

Ala Phe Val Lys Ala Trp Lys Ala Asp Lys Leu Gly Asn Cys Gln Phe
    210                 215                 220

Arg Tyr Ala Ala Ala Asn Phe Asn Gly Ala Met Gly Arg Asn Ala Lys
225                 230                 235                 240

Met Thr Ile Val Glu Ala Glu His Ile Val Glu Pro Gly Glu Ile Glu
                245                 250                 255

Pro Ala Ala Ile His Leu Pro Gly Ile Tyr Val Lys Arg Val Ile Gln
            260                 265                 270

Ser Thr Thr Pro Lys Asn Ile Glu Lys Phe Thr Phe Ala Lys Glu Glu
        275                 280                 285

Gly Ala Asp Thr Ser Ala Leu Gly Lys Gly Glu Thr Ala Asn Lys Arg
    290                 295                 300

Glu Arg Ile Val Arg Arg Ala Ala Lys Glu Phe Lys Asn Gly Met Tyr
305                 310                 315                 320

Ala Asn Leu Gly Ile Gly Met Pro Met Leu Ala Pro Ser Phe Val Asp
                325                 330                 335

Pro Ser Val Glu Val Thr Leu Gln Ser Glu Asn Gly Ile Leu Gly Leu
            340                 345                 350

Gly Pro Tyr Pro Lys Gln Gly Gln Glu Asp Pro Asp Leu Ile Asn Ala
        355                 360                 365

Gly Lys Glu Thr Val Thr Leu Lys Pro Gly Ala Ser Cys Phe Gly Ser
    370                 375                 380

Asp Glu Ser Phe Gly Met Ile Arg Ser Gly Arg Ile Asp Leu Thr Ile
385                 390                 395                 400

Leu Gly Ala Met Gln Val Ser Ala Lys Gly Asp Leu Ala Asn Trp Met
                405                 410                 415

Leu Pro Gly Lys Ile Lys Gly Phe Gly Gly Ala Met Asp Leu Val Ser
            420                 425                 430

Asn Pro Ser Ala Thr Lys Val Val Val Thr Met Glu His Thr Asp Lys
        435                 440                 445

Lys Gly Asn Pro Lys Ile Val Lys Asn Cys Glu Phe Pro Leu Thr Gly
    450                 455                 460

Pro Ala Cys Val Ser Arg Ile Ile Thr Asp Leu Cys Val Phe Asp Val
465                 470                 475                 480

Asp Phe Thr His Gly Leu Thr Leu Ile Glu Ile Ala Asp Gly Val Thr
                485                 490                 495

Val Glu Glu Val Gln Ala Lys Thr Glu Ala Pro Phe Lys Val Ala Asp
            500                 505                 510

Asp Leu Lys Pro Met Leu
        515

<210>  92
<211>  538
<212>  PRT
<213>  Penicillium chrysogenum

<400>  92

Met Thr Ala Leu Arg Ile Ser Thr Thr Leu Ser Arg Lys Ala Leu Thr
1               5                   10                  15

Ala Cys Arg Pro Arg Leu Phe Leu Gly Val Arg Thr Ser Val Arg Pro
            20                  25                  30

Trp Gln Pro Phe Ser Thr Ser Thr Ser Thr Arg Tyr Ser Ile Thr Thr
        35                  40                  45

Pro Leu Lys Ser Leu Ala Pro Lys Val Glu Arg Ser Gly Ser Lys Leu
    50                  55                  60

Tyr Lys Asp Ala Asp Ala Ala Val Ala Asp Ile Lys Ser Gly Ser Thr
65                  70                  75                  80

Ile Leu Ser Ser Gly Phe Gly Leu Cys Gly Val Ala Glu Thr Leu Ile
                85                  90                  95

Asn Ala Met His Arg Arg Gly Ala Asp Gln Leu His Ser Leu Thr Ala
            100                 105                 110

Val Ser Asn Asn Ala Gly Ala Ala Gly Lys Gly Gly Leu Ser Thr Leu
        115                 120                 125

Ser Gln Asn Gly Gln Ile Asn Arg Leu Ile Leu Ser Tyr Leu Gly Asn
    130                 135                 140

Asn Lys Ala Leu Glu Lys Lys Tyr Leu Thr Gly His Ile Ala Ile Glu
145                 150                 155                 160

Leu Cys Pro Gln Gly Thr Leu Ala Glu Arg Leu Arg Ala Gly Gly Ala
                165                 170                 175

Gly Ile Pro Ala Phe Phe Thr Pro Thr Gly Glu Gly Lys Ile Pro Val
            180                 185                 190

Arg Met Asp Glu Ser Gly Lys Val Leu Glu Ser Gly Lys Pro Arg Glu
        195                 200                 205

Thr Arg Glu Phe Asn Gly Lys Thr Tyr Leu Met Glu Glu Ala Leu Thr
    210                 215                 220

Gly Asp Val Ala Ile Leu Arg Ala Trp Lys Ala Asp Glu Ala Gly Asn
225                 230                 235                 240

Cys Val Phe Arg Tyr Thr Thr Lys Ala Phe Gly Pro Ile Met Ala Lys
                245                 250                 255

Ala Ala Thr Leu Thr Ile Val Glu Ala Glu Asn Ile Val Pro Ile Gly

            260                 265                 270

Ser Ile Asp Pro Asn Asp Val Asp Leu Pro Gly Ile Phe Val Asp Arg
        275                 280                 285

Ile Val Pro Ala Thr Asp Asp Lys His Ile Glu Ile Arg Lys Leu Arg
    290                 295                 300

Ser Gly Glu Thr Thr Val Ala Gly Ser Gly Lys Asp Glu Ala Arg Ile
305                 310                 315                 320

Gln Arg Glu Leu Ile Gly Arg Arg Ala Ala Lys Glu Leu Lys Pro Gly
                325                 330                 335

Phe Tyr Val Asn Leu Gly Val Gly Ile Pro Thr Leu Ala Pro Ser Phe
            340                 345                 350

Leu Pro Lys Asp Val Lys Val Trp Ile Gln Ser Glu Asn Gly Ile Leu
        355                 360                 365

Gly Met Gly Asp Tyr Pro Thr Glu Gln Glu Leu Asp Pro Asp Ile Ile
    370                 375                 380

Asn Ala Gly Lys Glu Thr Val Thr Leu Val Pro Gly Ala Ala Thr Phe
385                 390                 395                 400

Asp Ser Ser Glu Ser Phe Gly Met Ile Arg Gly Gly His Val Asp Val
                405                 410                 415

Ser Ile Leu Gly Ala Leu Gln Val Ser Ala Asn Gly Asp Leu Ala Asn
            420                 425                 430

Tyr Met Ile Pro Gly Lys Val Phe Lys Gly Met Gly Gly Ala Met Asp
        435                 440                 445

Leu Ile Ser Asn Pro Glu Lys Thr Lys Ile Val Val Ala Thr Ser His
    450                 455                 460

Val Ala Lys Asp Gly Ser Pro Lys Ile Val Gln Lys Cys Ser Leu Pro
465                 470                 475                 480

Leu Thr Gly Ala Asn Val Val Ser Thr Ile Ile Thr Asp Leu Cys Val
                485                 490                 495

Phe Gln Val Asp Arg Ala Thr Gly Glu Leu Thr Leu Thr Glu Leu Ala
            500                 505                 510

Pro Gly Val Glu Val Glu Glu Val Gln Ser Lys Thr Asp Ala Lys Phe
        515                 520                 525

Thr Ile Ala Asp Thr Leu Glu Ile Met Glu
    530                 535

<210>  93
<211>  425
<212>  PRT
<213>  Penicillium chrysogenum

<400>  93

Met Arg Leu Leu Ser Ser Leu Ala Ser Arg Pro His Ala Leu Ser Gly
1               5                   10                  15

Ala Val Gln Pro Ala Arg Arg Ser His Leu Thr Trp Arg Leu Tyr Ser
            20                  25                  30

Ser Thr Pro Ala Ala Lys Asn Arg Pro Leu Pro Leu Ala Gly Val Thr
        35                  40                  45

Val Val Ser Leu Glu Gln Ala Ile Ala Ala Pro Phe Cys Thr Arg Gln
    50                  55                  60

Leu Ala Asp Leu Gly Ala Arg Val Ile Lys Val Glu Arg Pro Gly Val
65                  70                  75                  80

Gly Asp Phe Ala Arg His Tyr Asp Thr Arg Val Gly Gly Leu Ala Ser
                85                  90                  95

His Phe Val Trp Thr Asn Arg Ser Lys Glu Ser Leu Ala Leu Asp Leu
            100                 105                 110

Lys His Glu Lys Asp His Ala Val Leu Met Lys Leu Leu Glu Lys Ala
        115                 120                 125

Asp Val Leu Val Gln Asn Leu Ala Pro Gly Ala Ser Ala Arg Leu Gly
    130                 135                 140

Leu Ser His Glu Ala Leu Lys Gly Glu His Pro Asp Leu Ile Val Cys
145                 150                 155                 160

Asp Ile Ser Gly Tyr Gly Gln Asp Gly Pro Tyr Arg Asp Lys Lys Ala
                165                 170                 175

Tyr Asp Leu Leu Ile Gln Asn Glu Ala Gly Met Leu Ser Val Thr Gly
            180                 185                 190

Thr Gly Lys Glu Pro Val Lys Val Gly Ile Ser Ile Ala Asp Ile Ala
        195                 200                 205

Ala Gly Met Tyr Ala Tyr Ser Asn Ile Leu Ser Ala Leu Leu Gln Arg
    210                 215                 220

Gly Lys Asp Gly Gln Gly Cys His Ile Asp Ile Ser Met Leu Glu Ser
225                 230                 235                 240

Met Val Glu Trp Met Gly Phe Pro Met Tyr Tyr Ala Phe Asp Asn Ala
                245                 250                 255

Pro Gly Pro Val Pro Ala Gly Ala Ser His Ala Ala Ile Tyr Pro Tyr
            260                 265                 270

Gly Pro Phe Glu Ala Gly Asp Gly Gln Ser Val Met Leu Gly Ile Gln
        275                 280                 285

Asn Glu Arg Glu Trp Val Ser Phe Cys Asn Gly Val Leu Ser Gln Pro
    290                 295                 300

Asp Leu Ala Thr Asp Pro Arg Phe Ser Ser Asn Ser Gln Arg Thr Gln
305                 310                 315                 320

Asn Arg Asp Glu Leu Lys Ser Ile Ile His Asp Ala Phe Ser Lys Phe
                325                 330                 335

Thr Thr Thr Glu Thr Ile Ala Arg Leu Asp Glu Ala Ser Ile Ala Asn
            340                 345                 350

Ala Thr Val Asn Asp Met Lys Asp Val Trp Gly His Ala Gln Leu Gln
        355                 360                 365

Ala Arg Asp Arg Trp Thr Lys Val Gln Thr Pro Val Gly Ala Ile Pro

370                 375                 380

Ala Leu Leu Pro Pro Gly Ser Thr Asn Ser Ala Gly Arg Asp Gly His
385                 390                 395                 400

Gly Ala Gln Met Gly Ser Val Pro Arg Val Gly Glu His Asn Glu Ala
                405                 410                 415

Ile Phe Ala Glu Leu Gly Leu Ser Asn
            420                 425

<210>  94
<211>  27
<212>  DNA
<213>  Artificial Sequence

<220>
<223>  Synthetic oligonucleotide

<400>  94
catatggttt ttttacctcc aaaggag                                          27

<210>  95
<211>  66
<212>  DNA
<213>  Artificial Sequence

<220>
<223>  Synhetic oligonucleotide

<400>  95
atgcattggc tcgtcatgaa gagcctacta gatcttgcta ccagcctttc tccgtgcctc      60

cttctc                                                                 66

<210>  96
<211>  25
<212>  DNA
<213>  Artificial Sequence

<220>
<223>  Synhetic oligonucleotide

<400>  96
ccctctcttc gtcgttgtcc acgcc                                            25

<210>  97
<211>  19
<212>  DNA
<213>  Artificial Sequence

<220>
<223>  Synhetic oligonucleotide

<400>  97
cgtgtcgcac tcgtccata                                                    19


<210>  98
<211>  48
<212>  DNA
<213>  Artificial Sequence

<220>
<223>  Synhetic oligonucleotide

<400>  98
ccttcgccga ctgagatcgg ttccgtcgtc ggtatctact ccggtaag                    48


<210>  99
<211>  57
<212>  DNA
<213>  Artificial Sequence

<220>
<223>  Synhetic oligonucleotide

<400>  99
tcttcggcct ccataagtac ggatatgcgg cgaaacaaag ctatggagcc ggtaatg          57


<210>  100
<211>  33
<212>  DNA
<213>  Artificial Sequence

<220>
<223>  Synhetic oligonucleotide

<400>  100
atgcccgtat cctctaacta ccccctggtc gac                                    33


<210>  101
<211>  33
<212>  DNA
<213>  Artificial Sequence

<220>
<223>  Synhetic oligonucleotide

<400>  101
ccttcgccga ctgacagctt ggcttttggt gcctcggccg cccccttg                    48


<210>  102

<211>  4314
<212>  DNA
<213>  plasmid DNA

<400>  102
atccgttcat ttcaataaac cgggcgacct cagccatccc ttcctgattt tccgctttcc      60

agcgttcggc acgcagacga cgggcttcat tctgcatggt tgtgcttacc agaccggaga     120

tattgacatc gtatgccttg agcaactgat agctgtcgct gtcaactgtc actgtaatac     180

gctgcttcat gcctgcccct cccttttggt gaccaaccgg ctcgacgggg gcagcgcaag     240

gcggtgcctc cggcgggcca ctcaatgctt gagtatactc actagacttt gcttcgcaaa     300

gtcgtgaccg cctacggcgg ctgcggcaag catgctgcag aattctagag ggcccgggtt     360

aattaagcgg cctgatgcgg tattttctcc ttacgcatct gtgcggtatt tcacaccgca     420

tatggtgcac tctcagtaca atctgctctg atgccgcata gtttccgctt cctcgctcac     480

tgactcgctg cgctcggtcg ttcggctgcg gcgagcggta tcagctcact caaaggcggt     540

aatacggtta tccacagaat caggggataa cgcaggaaag aacatgtgag caaaaggcca     600

gcaaaaggcc aggaaccgta aaaaggccgc gttgctggcg tttttccata ggctccgccc     660

ccctgacgag catcacaaaa atcgacgctc aagtcagagg tggcgaaacc cgacaggact     720

ataaagatac caggcgtttc cccctggaag ctccctcgtg cgctctcctg ttccgaccct     780

gccgcttacc ggatacctgt ccgcctttct cccttcggga agcgtggcgc tttctcatag     840

ctcacgctgt aggtatctca gttcggtgta ggtcgttcgc tccaagctgg gctgtgtgca     900

cgaacccccc gttcagcccg accgctgcgc cttatccggt aactatcgtc ttgagtccaa     960

cccggtaaga cacgacttat cgccactggc agcagccact ggtaacagga ttagcagagc    1020

gaggtatgta ggcggtgcta cagagttctt gaagtggtgg cctaactacg gctacactag    1080

aagaacagta tttggtatct gcgctctgct gaagccagtt accttcggaa aaagagttgg    1140

tagctcttga tccggcaaac aaaccaccgc tggtagcggt ggtttttttg tttgcaagca    1200

gcagattacg cgcagaaaaa aaggatctca agaagatcct ttgatctttt ctacggggtc    1260

tgacgctcag tggaacgaaa actcacgtta agggattttg gtcatgagat tatcaaaaag    1320

gatcttcacc tagatccttt taaattaaaa atgaagtttt aaatcaatct aaagtatata    1380

tgagtaaact tggtctgaca gttaccaatg cttaatcagt gaggcaccta tctcagcgat    1440

ctgtctattt cgttcatcca tagttgcctg actccccgtc gtgtagataa ctacgatacg    1500

ggagggctta ccatctggcc ccagtgctgc aatgataccg cgagacccac gctcaccggc    1560

tccagattta tcagcaataa accagccagc cggaagggcc gagcgcagaa gtggtcctgc 1620

aactttatcc gcctccatcc agtctattaa ttgttgccgg gaagctagag taagtagttc 1680

gccagttaat agtttgcgca acgttgttgc cattgctaca ggcatcgtgg tgtcacgctc 1740

gtcgtttggt atggcttcat tcagctccgg ttcccaacga tcaaggcgag ttacatgatc 1800

ccccatgttg tgcaaaaaag cggttagctc cttcggtcct ccgatcgttg tcagaagtaa 1860

gttggccgca gtgttatcac tcatggttat ggcagcactg cataattctc ttactgtcat 1920

gccatccgta agatgctttt ctgtgactgg tgagtactca accaagtcat tctgagaata 1980

gtgtatgcgg cgaccgagtt gctcttgccc ggcgtcaata cgggataata ccgcgccaca 2040

tagcagaact ttaaaagtgc tcatcattgg aaaacgttct tcggggcgaa aactctcaag 2100

gatcttaccg ctgttgagat ccagttcgat gtaacccact cgtgcaccca actgatcttc 2160

agcatctttt actttcacca gcgtttctgg gtgagcaaaa acaggaaggc aaaatgccgc 2220

aaaaaaggga ataagggcga cacggaaatg ttgaatactc atactcttcc tttttcaata 2280

ttattgaagc atttatcagg gttattgtct catgagcgga tacatatttg aatgtattta 2340

gaaaaataaa caaatagggg ttccgcgcac atttccccga aaagtgccac ctgacgtcta 2400

agaaaccatt attatcatga cattaaccta taaaaatagg cgtatcacga ggccctttcg 2460

tgctcaatac gcaaaccgcc tctccccgcg cgttggccga ttcattaatg cagcgcgtta 2520

gaatactcaa gctatgcatc aagcttacat atgccaccag ggctacgaaa catcaatggg 2580

ctacatctca tggctaaaca agtacgacga aggggactcg gttctgacaa ccatgctccg 2640

caaagccggt gccgtcttct acgtcaagac ctctgtcccg cagaccctga tggtctgcga 2700

gacagtcaac aacatcatcg ggcgcaccgt caacccacgc aacaagaact ggtcgtgcgg 2760

cggcagttct ggtggtgagg gtgcgatcgt tgggattcgt ggtggcgtca tcggtgtagg 2820

aacggacatc ggtggctcga ttcgagtgcc ggccgcgttc aacttcctgt acggtctaag 2880

gccgagtcat gggcggctgc cgtatgcaaa gatggcgaac agcatggagg gtcaggagac 2940

ggtgcacagc gttgtcgggc cgattacgca ctctgttgag ggtgagtcct tcgcctcttc 3000

cttcttttcc tgctctatac caggcctcca ctgtcctcct ttcttgcttt ttatactata 3060

tacgagaccg gcagtcactg atgaagtatg ttagacctcc gcctcttcac caaatccgtc 3120

ctcggtcagg agccatggaa atacgactcc aaggtcatcc ccatgccctg gcgccagtcc 3180

gagtcggaca ttattgcctc caagatcaag aacggcgggc tcaatatcgg ctactacaac 3240

ttcgacggca atgtccttcc acaccctcct atcctgcgcg gcgtggaaac caccgtcgcc 3300

gcactcgcca aagccggtca caccgtgacc ccgtggacgc catacaagca cgatttcggc 3360

cacgatctca tctcccatat ctacgcggct gacggcagcg ccgacgtaat gcgcgacatc 3420

agtgcatccg gcgagccggc gattccaaat atcaaagacc tactgaaccc gaacatcaaa 3480

gctgttaaca tgaacgagct ctgggacacg catctccaga agtggaatta ccagatggag 3540

taccttgaga aatggcggga ggctgaagaa aaggccggga aggaactgga cgccatcatc 3600

gcgccgatta cgcctaccgc tgcggtacgg catgaccagt tccggtacta tgggtatgcc 3660

tctgtgatca acctgctgga tttcacgagc gtggttgttc cggttacctt tgcggataag 3720

aacatcgata agaagaatga gagtttcaag gcggttagtg agcttgatgc cctcgtgcag 3780

gaagagtatg atccggaggc gtaccatggg gcaccggttg cagtgcaggt tatcggacgg 3840

agactcagtg aagagaggac gttggcgatt gcagaggaag tggggaagtt gctgggaaat 3900

gtggtgactc catagctaat aagtgtcaga tagcaatttg cacaagaaat caataccagc 3960

aactgtaaat aagcgctgaa gtgaccatgc catgctacga aagagcagaa aaaaacctgc 4020

cgtagaaccg aagagatatg acacgcttcc atctctcaaa ggaagaatcc cttcagggtt 4080

gcgtttccag tctagacacg tataacggca caagtgtctc tcaccaaatg ggttatatct 4140

caaatgtgat ctaaggatgg aaagcccaga atattggctg ggttgatggc tgcttcgagt 4200

gcagtctcat gctgccacag gtgactctgg atggccccat accactcaac ataacttcgt 4260

atagcataca ttatacgaag ttatggatcc actagtgaat tcgtttaaac gat 4313

<210> 103
<211> 5664
<212> DNA
<213> plasmid DNA

<400> 103
atccgttcat ttcaataaac cgggcgacct cagccatccc ttcctgattt tccgctttcc 60

agcgttcggc acgcagacga cgggcttcat tctgcatggt tgtgcttacc agaccggaga 120

tattgacatc gtatgccttg agcaactgat agctgtcgct gtcaactgtc actgtaatac 180

gctgcttcat gcctgcccct cccttttggt gaccaaccgg ctcgacgggg gcagcgcaag 240

gcggtgcctc cggcgggcca ctcaatgctt gagtatactc actagacttt gcttcgcaaa 300

gtcgtgaccg cctacggcgg ctgcggcaag catgctgcag aattctagag ggcccgggtt 360

aattaagcgg cctgatgcgg tattttctcc ttacgcatct gtgcggtatt tcacaccgca 420

tatggtgcac tctcagtaca atctgctctg atgccgcata gtttccgctt cctcgctcac 480

tgactcgctg cgctcggtcg ttcggctgcg gcgagcggta tcagctcact caaaggcggt 540

aatacggtta tccacagaat caggggataa cgcaggaaag aacatgtgag caaaaggcca 600

gcaaaaggcc aggaaccgta aaaaggccgc gttgctggcg tttttccata ggctccgccc 660

ccctgacgag catcacaaaa atcgacgctc aagtcagagg tggcgaaacc cgacaggact 720

ataaagatac caggcgtttc cccctggaag ctccctcgtg cgctctcctg ttccgaccct 780

gccgcttacc ggatacctgt ccgcctttct cccttcggga agcgtggcgc tttctcatag 840

ctcacgctgt aggtatctca gttcggtgta ggtcgttcgc tccaagctgg gctgtgtgca 900

cgaacccccc gttcagcccg accgctgcgc cttatccggt aactatcgtc ttgagtccaa 960

cccggtaaga cacgacttat cgccactggc agcagccact ggtaacagga ttagcagagc 1020

gaggtatgta ggcggtgcta cagagttctt gaagtggtgg cctaactacg gctacactag 1080

aagaacagta tttggtatct gcgctctgct gaagccagtt accttcggaa aaagagttgg 1140

tagctcttga tccggcaaac aaaccaccgc tggtagcggt ggtttttttg tttgcaagca 1200

gcagattacg cgcagaaaaa aaggatctca agaagatcct ttgatctttt ctacggggtc 1260

tgacgctcag tggaacgaaa actcacgtta agggattttg gtcatgagat tatcaaaaag 1320

gatcttcacc tagatccttt taaattaaaa atgaagtttt aaatcaatct aaagtatata 1380

tgagtaaact tggtctgaca gttaccaatg cttaatcagt gaggcaccta tctcagcgat 1440

ctgtctattt cgttcatcca tagttgcctg actccccgtc gtgtagataa ctacgatacg 1500

ggagggctta ccatctggcc ccagtgctgc aatgataccg cgagacccac gctcaccggc 1560

tccagattta tcagcaataa accagccagc cggaagggcc gagcgcagaa gtggtcctgc 1620

aactttatcc gcctccatcc agtctattaa ttgttgccgg gaagctagag taagtagttc 1680

gccagttaat agtttgcgca acgttgttgc cattgctaca ggcatcgtgg tgtcacgctc 1740

gtcgtttggt atggcttcat tcagctccgg ttcccaacga tcaaggcgag ttacatgatc 1800

ccccatgttg tgcaaaaaag cggttagctc cttcggtcct ccgatcgttg tcagaagtaa 1860

gttggccgca gtgttatcac tcatggttat ggcagcactg cataattctc ttactgtcat 1920

gccatccgta agatgctttt ctgtgactgg tgagtactca accaagtcat tctgagaata 1980

gtgtatgcgg cgaccgagtt gctcttgccc ggcgtcaata cgggataata ccgcgccaca 2040

tagcagaact ttaaaagtgc tcatcattgg aaaacgttct tcggggcgaa aactctcaag 2100

gatcttaccg ctgttgagat ccagttcgat gtaacccact cgtgcaccca actgatcttc 2160

agcatctttt actttcacca gcgtttctgg gtgagcaaaa acaggaaggc aaaatgccgc 2220

aaaaaaggga ataagggcga cacggaaatg ttgaatactc atactcttcc tttttcaata 2280

ttattgaagc atttatcagg gttattgtct catgagcgga tacatatttg aatgtattta 2340

gaaaaataaa caaatagggg ttccgcgcac atttccccga aaagtgccac ctgacgtcta 2400

agaaaccatt attatcatga cattaaccta taaaaatagg cgtatcacga ggccctttcg 2460

tgctcaatac gcaaaccgcc tctccccgcg cgttggccga ttcattaatg cagcgcgtta 2520

gaatactcaa gctatgcatc aagcttgtcc cagagctcgt tcatgttaac agctttgatg 2580

ttcgggttca gtaggtcttt gatatttgga atcgccggct cgccggatgc actgatgtcg 2640

cgcattacgt cggcgctgcc gtcagccgcg tagatatggg agatgagatc gtggccgaaa 2700

tcgtgcttgt atggcgtcca cggggtcacg gtgtgaccgg ctttggcgag tgcggcgacg 2760

gtggtttcca cgccgcgcag gataggaggg tgtggaagga cattgccgtc gaagttgtag 2820

tagccgatat tgagcccgcc gttcttgatc ttggaggcaa taatgtccga ctcggactgg 2880

cgccagggca tggggatgac cttggagtcg tatttccatg gctcctgacc gaggacggat 2940

ttggtgaaga ggcggaggtc taacatactt catcagtgac tgccggtctc gtatatagta 3000

taaaaagcaa gaaaggagga cagtggaggc ctggtataga gcaggaaaag aaggaagagg 3060

cgaaggactc accctcaaca gagtgcgtaa tcggcccgac aacgctgtgc accgtctcct 3120

gaccctccat gctgttcgcc atctttgcat acggcagccg cccatgactc ggccttagac 3180

cgtacaggaa gttgaacgcg gccggcactc gaatcgagcc accgatgtcc gttcctacac 3240

cgatgacgcc accacgaatc ccaacgatcg caccctcacc accagaactg ccgccgcacg 3300

accagttctt gttgcgtggg ttgacggtgc gcccgatgat gttgttgact gtctcgcaga 3360

ccatcagggt ctgcggacag aggtcttgac gtagaagacg gcaccggctt tgcggagcat 3420

tggttgtcag aaccgagtcc ccttcgtcgt acttgtttag ccatgagatg tagcccattg 3480

atgtttcgta gccctggtgg catatgttag ctgacaaaaa gggacatcta acgacttagg 3540

ggcaacggtg taccttgact cgaagctggt ctttgagaga gatggggagg ccatggagtg 3600

gaccaacggg tctcttgtgc tttgcgtagt attcatcgag ttcccttgcc tgcgcgagag 3660

cggcgtcagg gaagaactcg tgggcgcagt ttgtctgcac agaagccagc gtcagcttga 3720

tagtcccata aggtggcgtt gttacatctc cctgagaggt agaggggacc ctactaactg 3780

ctgggcgatt gctgcccgtt tacagaatgc tagcgtaact tccaccgagg tcaactctcc 3840

ggccgccagc ttggacacaa gatctgcagc ggaggcctct gtgatcttca gttcggcctc 3900

tgaaaggatc cccgatttct ttgggaaatc aataacgctg tcttccgcag gcagcgtctg 3960

gactttccat tcatcaggga tggtttttgc gaggcgggcg cgcttatcag cggccagttc 4020

ttcccaggat tgaggcatgg tgatgtctgc tcaagcgggg tagctgttag tcaagctgcg 4080

atgaagtggg aaagctcgaa ctgaaaggtt caaaggaata agggatggga aggatggagt 4140

atggatgtag caaagtactt acttagggga aataaaggtt cttggatggg aagatgaata 4200

tactgaagat gggaaaagaa agagaaaaga aaagagcagc tggtggggag agcaggaaaa 4260

tatggcaaca aatgttggac tgacgcaacg accttgtcaa ccccgccgac acaccgggcg 4320

gacagacggg gcaaagctgc ctaccaggga ctgagggacc tcagcaggtc gagtgcagag 4380

caccggatgg gtcgactgcc agcttgtgtt cccggtctgc gccgctggcc agctcctgag 4440

cggcctttcc ggtttcatac accgggcaaa gcaggagagg cacgatattt ggacgcccta 4500

cagatgccgg atgggccaat tagggagctt acgcgccggg tactcgctct acctacttcg 4560

gagaaggtac tatctcgtga atcttttacc agatcggaag caattggact tctgtaccta 4620

ggttaatggc atgctatttc gccgacggct atacacccct ggcttcacat tctccttcgc 4680

ttactgccgg tgattcgatg aagctccata ttctccgatg atgcaataga ttcttggtca 4740

acgaggggca caccagcctt tccacttcgg ggcggagggg cggccggtcc cggattaata 4800

atcatccact gcacctcaga gccgccagag ctgtctggcc agtggcttat tactcagccc 4860

ttctctctgc gtccgtccgt ctctccgcat gccagaaaga gtcataactt cgtataatgt 4920

atgctatacg aagttatcct gcaggccgac gccgaccaac accgccggtc cgacggcggc 4980

ccacgggtcc caggagcttg agatccactt aacgttactg aaatcatcaa acagcttgac 5040

gaatctggat ataagatcgt tggtgtcgat gtcagctccg gagttgagac aaatggtgtt 5100

caggatctcg ataagatacg ttcatttgtc caagcagcaa agagtgcctt ctagtgattt 5160

aatagctcca tgtcaacaag aataaaacgc gttttcgggt ttacctcttc cagatacagc 5220

tcatctgcaa tgcattaatg cattgactgc aacctagtaa cgccttcagg ctccggcgaa 5280

gagaagaata gcttagcaga gctattttca ttttcgggag acgagatcaa gcagatcaac 5340

ggtcgtcaag agacctacga gactgaggaa tccgctcttg gctccacgcg actatatatt 5400

tgtctctaat tgtactttga catgctcctc ttctttactc tgatagcttg actatgaaaa 5460

ttccgtcacc agccctgggt tcgcaaagat aattgcatgt ttcttccttg aactctcaag 5520

cctacaggac acacattcat cgtaggtata aacctcgaaa tcattcctac taagatcgta 5580

tacaatagta accatgcatg gttgcctagt gaatgctccg taacacccaa tacggcgcgc 5640

catgcggccg ctagtttaaa cgat 5664

<210> 104
<211> 18
<212> DNA
<213> Artificial sequence

<220>
<223> Synthetic oligonucleotide

<400> 104
tgcagcgcgt tagaatac 18

<210> 105
<211> 19
<212> DNA
<213> Artificial

<220>
<223> Synthetic oligonucleotide

<400> 105
cggatccttc gccgactga 19

<210> 106
<211> 25
<212> DNA
<213> Artificial

<220>
<223> Synthetic oligonucleotide

<400> 106
tcgcagccag cgccttgtga gatgg 25

<210> 107
<211> 21
<212> DNA
<213> Artificial sequence

<220>
<223> Synthetic oligonucleotide

<400> 107
gccacaggtg actctggatg g 21

<210> 108
<211> 21
<212> DNA
<213> Artificial sequence

<220>
<223>  Synthetic oligonucleotide

<400>  108
gtctctccgc atgccagaaa g                                                21


<210>  109
<211>  25
<212>  DNA
<213>  Artificial

<220>
<223>  Synthetic oligonucleotide

<400>  109
actaacacgc aaacacaggc gcggg                                            25